# EUROPEAN PATENT SPECIFICATION

(11) **EP 2 619 211 B1**
(45) Date of publication and mention of the grant of the patent: **09.09.2015**
(21) Application number: 11790674.3
(22) Date of filing: 22.09.2011
(51) Int. Cl.: C07D 487/14

(54) **FUSED TRICYCLIC COMPOUNDS AS ADENOSINE RECEPTOR ANTAGONIST**
KONDENSIERTE TRIZYKLISCHE VERBINDUNGEN ALS ADENOSIN REZEPTOR ANTAGONISTEN
COMPOSÉS TRICYCLIQUES FUSIONNÉS UTILISÉS COMME ANTAGONISTES DU RÉCEPTEUR DE L'ADÉNOSINE

(30) Priority: 24.09.2010 IN MU28082010
(43) Date of publication of application: 31.07.2013
(73) Proprietor: Advinus Therapeutics Limited, Bangalore 560058 (IN)
(72) Inventor: BARAWKAR, Dinesh, Pune 411057 (IN); BASU, Sujay, Pune 411057 (IN); RAMDAS, Vidya, Pune 411057 (IN); NAYKODI, Minakshi, Pune 411057 (IN); PATEL, Meena, Pune 411057 (IN); SHEJUL, Yogesh, Pune 411057 (IN); THORAT, Sachin, Pune 411057 (IN); PANMAND, Anil, Pune 411057 (IN)
(74) Representative: Jones, Nicholas Andrew
(86) International application number: PCT/IN2011/000657
(87) International publication number: WO 2012/038980

(56) References cited:
- WO-A2-2008/121748

## Description

### Technical Field

The present disclosure relates to a series of substituted fused tricyclic compounds, their tautomers, polymorphs, stereoisomers, solvates, pharmaceutically acceptable salts, pharmaceutical compositions containing them and methods of treating conditions and diseases that are mediated by adenosine receptor (AR) activity. These compounds are useful in the treatment, prevention or suppression of diseases and disorders that may be susceptible to improvement by antagonism of the adenosine receptor. The disclosure also relates to methods for the preparation of such compounds, and to pharmaceutical compositions containing them.

### Background

The effects of adenosine are mediated through at least four specific cell membrane receptors so far identified and classified as A₁, A_{2A}, A_{2B} and A₃ belonging to G protein-coupled receptor family. The A₁ and A₃ receptors down-regulate cellular cAMP levels through their coupling to G protein, which inhibit adenylate cyclase. In contrast, A_{2A} and A_{2B} receptors couple to G protein that activate adenylate cyclase and increase intracellular levels of cAMP. Through these receptors, adenosine regulates the wide range of physiological functions. Advances in understanding the role of adenosine and its receptors in physiology and pathophysiology, as well as new developments in medicinal chemistry of these receptors have identified potential therapeutic areas for drug development. With the combination of pharmacological data, using selective ligands and genetically modified mice, important progress has been made toward an understanding of the role of ARs in a variety of diseases, such as inflammatory conditions, sepsis, heart attack, ischemia-reperfusion injury, vascular injury, spinal cord injury, chronic obstructive pulmonary disease (COPD), asthma, diabetes, obesity, inflammatory bowel disease, retinopathy, and Parkinson's Disease (PD).

Movement disorder constitutes a serious health problem, especially among the elderly. These movement disorders can often be the result of brain lesions. Disorders involving the basal ganglia which result in movement disorders include Parkinson's disease, Huntington's chorea and Wilson's disease. Tremor, rigidity, akinesia and postural changes are four classic symptoms of Parkinson's disease, it is also associated with depression, dementia and overall cognitive decline. Parkinson's disease has a prevalence of 1 per 1000 of the total population and increases to 1 per 100 for those aged over 60 years. Degeneration of dopaminergic neurons in the substantia nigra and the subsequent reductions in the interstitial concentrations of dopamine in the striatum are critical to the development of Parkinson's disease. About 80% of cells from the substantia nigra can be destroyed before the clinical symptoms of Parkinson's disease become apparent

PD is a progressive, incurable disorder with no definite preventive treatment, although drugs are available to alleviate the symptoms and/or slow down the progress of the disease. Current therapy is based on dopamine replacement therapy, the most common drug treatments being dopaminomimetic agents, including L-DOPA, a dopamine precursor, as well as direct or indirect dopamine receptor agonists. L-DOPA is the mainstay in the treatment of PD, but because of tolerance problems and a wide range of adverse reactions, including involuntary movements and vomiting, a strong demand for new therapies exists. Among the various strategies, A2A AR blockers are considered a potential approach to treatment of the disease. Within the brain A2A ARs are richly expressed in the striatum, nucleus accumbens, and olfactory tubercle. A coexpression of A2A with D2 dopamine receptors has been reported in the GABAergic striatopallidal neurons where adenosine and dopamine agonists exert antagonistic effects in the regulation of locomotor activity. Activation of A2A ARs in striatopallidal neurons decreases the affinity of D2 receptors for dopamine, antagonizing the effects of D2 receptors. The negative interaction between A2A and D2 receptors is at the basis of the use of A2A antagonists as a novel therapeutic approach in the treatment of PD. (Pharmacol. Ther. 2005, 105, 267). The recent discovery that the A2A can form functional heteromeric receptor complexes with other Gprotein-coupled receptors such as D2 and the mGlu5 receptors has also suggested new opportunities for the potential of A2A antagonists in PD. (J. Mol. Neurosci. 2005, 26, 209).

A2A knockout (KO) mice transient focal ischemia caused less neuronal damage in comparison to their wild-type (WT) littermates (J. Neurosci. 1999, 19, 9192.). Therefore, it seems that tonic activation of A2A ARs may be responsible for dangerous signal during injury, in contrast to the neuroprotective effects induced by endogenous A1 activation. Recently, selective inactivation or reconstitution of A2A ARs in bone-marrow cells revealed their contribution to the development of ischemic brain injury (J.F. Nat. Med. 2004, 10, 1081) Blockade of A2A ARs has recently been implicated in the treatment of movement disorders such as Parkinson's disease (Trends Pharmacol. Sci. 1997, 18, 338-344) and in the treatment of cerebral ischaemia (Life Sci. 1994, 55, 61-65). The potential utility of A2A AR antagonists in the treatment of Parkinson's disease has been reviewed (CNS drugs, 1998, 10, 311-320). One advantage of A2A AR antagonist therapy is that the underlying neurodegenerative disorder may also be treated ((Ann. N. Y. Acad. Sci. 1997, 825 (Neuroprotective Agents), 3048). In particular, blockade of A2A AR function confers neuroprotection against MPTP-induced neurotoxicity in mice (Neurosci. 2001, 21, RC143).

Alzheimer's disease (AD) is a neurodegenerative disorder of the central nervous system manifested by cognitive and memory deterioration, a variety of neuropsychiatric symptoms, behavioral disturbances, and progressive impairment of daily life activities. Recent research suggests that adenosine receptors play important roles in the modulation of cognitive function. Epidemiological studies have found an association between coffee (a nonselective adenosine receptor antagonist) consumption and improved cognitive function in AD patients and in the elderly. Long-term administration of caffeine in transgenic animal models showed a reduced amyloid burden in brain with better cognitive performance. Antagonists of adenosine A2A receptors mimic these beneficial effects of caffeine on cognitive function. Neuronal cell cultures with amyloid beta in the presence of an A2A receptor antagonist completely prevented amyloid beta-induced neurotoxicity. These findings suggest that the adenosinergic system constitutes a new therapeutic target for AD, and caffeine and A2A receptor antagonists may have promise to manage cognitive dysfunction in AD (Curr Neuropharmacol. 2009 September; 7(3): 207-216).

High expression of A2A ARs has been found in platelets, leukocytes, vascular smooth muscle, and endothelial cells with important implications in the regulation of inflammatory responses. It is now well established that stimulation of the A2A AR in immune cells induces anti-inflammatory effects, mostly due to its ability to increase cAMP levels, which has strong immunosuppressive effects (Trends Immunol. 2005, 26, 299). Stimulation of A2A ARs inhibits neutrophil adherence to the endothelium, degranulation of activated neutrophils and monocytes, plus superoxide anion generation. A2A ARs have been recently defined as sensors and terminators of proinflammatory activities. The strongest evidence for the key role of A2A in inflammation is derived by the elegant study using mice deficient in A2A ARs (Nature 2001, 414, 916). In this model the lack of A2A subtype leads to increased tissue inflammation and damage, thus suggesting a negative and nonredundant regulatory role for the A2A AR. This model permits one to appreciate that adenosinergic regulation of immune cells is fundamental in normal physiological control of inflammation in vivo in spite of the fact that other Gs-protein-coupled receptors and cAMP elevating ligands are present, such as cathecolamines, prostaglandins, dopamine, and histamine (Trends Immunol. 2005, 26, 299). Interestingly, the A2A AR has been demonstrated to be involved in promotion of wound healing and angiogenesis in healing wounds (Am. J. Physiol. Regul. Integr. Comp. Physiol. 2005, 289, R283). Moreover, it plays an active role in the pathogenesis of dermal fibrosis, suggesting a role for antagonists as novel therapeutic approach in the treatment and prevention of dermal fibrosis in diseases such as scleroderma (Arthritis Rheum. 2006, 54, 2632) as well as hepatic fibrosis (Br. J. Pharmacol. 2006 Aug;148(8):1144-55). Studies also suggest that A2A receptor antagonists may be beneficial for social memory impairment and hypertension (Behav Brain Res. 2005 Apr 30;159(2):197-205), sepsis (J Immunol. 2006 May 1;176(9):5616-26), spinal cord injury and neuroprotection (J Neuroinflammation. 2011 Apr 12;8:31), retinopathy (IVOS, Jan. 2000, vol. 41 (1), 230-243, depression (Neurology. 2003 Dec 9;61(11 Suppl 6):S82-7), narcolepsy and other sleep related disorders (Prog Neurobiol. 2007 Dec;83(5):332-47), attention-deficit hyperactivity disorder (ADHD) (Behav Pharmacol. 2009 Mar;20(2): 134-45; Clinical Genetics (2000), 58(1), 31-40 and references therein),

Antagonists of the A₂A receptor are potentially useful therapies for the treatment of addiction. Major drugs of abuse (opiates, cocaine, ethanol, and the like) either directly or indirectly modulate dopamine signaling in neurons particularly those found in the nucleus accumbens, which contain high levels OfA_{2A} adenosine receptors. Dependence has been shown to be augmented by the adenosine signaling pathway, and it has been shown that administration of an A₂A receptor antagonist redues the craving for addictive substances ("The Critical Role of Adenosine A2A Receptors and Gi βγ Subunits in Alcoholism and Addiction: From Cell Biology to Behavior", by Ivan Diamond and Lina Yao, (The Cell Biology of Addiction, 2006, pp 291-316) and "Adaptations in Adenosine Signaling in Drug Dependence: Therapeutic Implications", by Stephen P. Hack and Macdonald J. Christie, Critical Review in Neurobiology, Vol. 15, 235-274 (2003)). See also Alcoholism: Clinical and Experimental Research (2007), 31(8), 1302-1307.

A2A receptors may be beneficial for the treatment or prevention of disorders such as a movement disorder, for example, Parkinson's disease or progressive supernuclear palsy, Restless leg syndrome, nocturnal myoclonus, cerebral ischaemia, Huntington's disease, multiple system atrophy, corticobasal degeneration, Wilson's disease or other disorders of basal ganglia which results in dyskinesias, post traumatic stress disorder. See for example WO200013682, WO200012409, WO2009156737, WO200911442, WO2008121748, WO2001092264, WO2007038284, WO2008002596, WO2009111449, WO2009111442, WO2008121748, WO2009156737, WO2003022283, WO2005044245, WO2008077557, WO2009111449, WO2009705138, WO2009111442, WO2007035542, WO20080870661, WO2008070529, WO2005116026, WO2009055548, WO2007133983, WO2010045006, WO2010045015, WO2010045008 WO2009015236.

We have now found out that compounds of the present invention are potent antagonists of the A2A adenosine receptor and can therefore be used in the treatment of the diseases mentioned above.

**Summary** The present disclosure provides compounds of formula (I), their tautomers, polymorphs, stereoisomers, solvates, pharmaceutically acceptable salts, pharmaceutical compositions containing them and methods of treating conditions and diseases that are mediated by adenosine receptor activity, 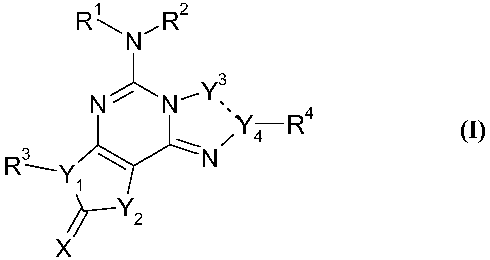 wherein
--- represents a single bond or a double bond;
X is selected from O, S or NR^{a};
Y₁ is selected from N or CH;
Y₂ is selected from NR⁵, O or CR⁵R⁶;
Y₃ is selected from N, CH, CH₂, C(=O) or C(=S);
Y₄ is selected from N, C or CH;
R¹ and R² are independently selected from hydrogen or alkyl;
R³ is -A-Z-B-Q;
   wherein, A is absent or is a group selected from alkylene, alkenylene or alkynylene; wherein one or more methylene groups is optionally replaced by hetero atoms or groups such as -O-, -S(O)p-, -N(R^{a})-, or -C(O); alkylene, alkenylene and alkynylene is optionally substituted with -(CR^{d}R^{e})ₙOR⁷, (CR^{d}R^{e})ₙCOOR⁷, -(CR^{d}R^{e})ₙNR⁸R⁹, cyano, halogen, haloalkyl, perhaloalkyl, alkoxyalkoxy, alkyl or cycloalkyl;
   Z is absent or is selected from a cycloalkyl or a heterocyclyl;
   wherein cycloalkyl and heterocyclyl are unsubstituted or substituted independently with 1, 2, or 3 substituents independently selected from alkyl, alkenyl, alkynyl, acyl, - (CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, halogen, haloalkyl, perhaloalkyl, azido, cyano, keto, thiocarbonyl, -SO₃H, aminocarbonylamino, nitro, -S(O)₂NR^{a}R^{a}, -NR^{b}S(O)₂R^{b} or - S(O)ₚR^{c};
   B is absent or is a group selected from alkylene, alkenylene or alkynylene; wherein one or more methylene groups is optionally replaced by hetero atoms or groups such as -O-, -S(O)p-, -N(R^{a})-, or -C(O); alkylene, alkenylene and alkynylene is optionally substituted with hydroxy, amino, aminoalkyl, cyano, halogen, haloalkyl, perhaloalkyl, carboxy, carboxyalkyl, alkoxy, hydroxyalkyl, alkoxyalkyl, alkoxyalkoxy or alkyl;
   Q is selected from hydrogen, alkyl, cycloalkyl, cycloalkylalkyl, aryl, arylalkyl, heterocyclyl, heterocyclylalkyl, heteroaryl or heteroarylalkyl;
   wherein alkyl, cycloalkyl, cycloalkylalkyl, aryl, arylalkyl, heterocyclyl, heterocyclylalkyl, heteroaryl and heteroarylalkyl are unsubstituted or substituted independently with 1, 2, or 3 substituents independently selected from alkyl, alkenyl, alkynyl, halogen, haloalkyl, perhaloalkyl, azido, cyano, nitro, keto, thiocarbonyl, cyanoalkyl, cyanoalkylcarbonyl, -(CR^{d}R^{e})ₙOR⁷, -(CR^{d}R^{e})ₙC(O)R⁷, - (CR^{d}R^{e})ₙSR⁷, -(CR^{d}R^{e})ₙCOOR⁷, -(CR^{d}R^{e})ₙNR⁸R⁹, -(CR^{d}R^{e})ₙC(O)NR⁸R⁹, - (CR^{d}R^{e})ₙNR⁸C(O)OR⁷, -(CR^{d}R^{e})ₙNR⁸C(O)NR⁸R⁹, -NR^{b}S(O)₂R^{b}, -S(O)ₚR^{c}, - SO₃H, -S(O)₂NR^{a}R^{a}, cycloalkyl, cycloalkenyl, cycloalkylalkyl aryl, arylalkyl, heterocyclyl, heterocyclylalkyl, heteroaryl or heteroarylalkyl;
   wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, alkenyl, alkynyl, haloalkyl, hydroxyalkyl, carboxyalkyl, cycloalkyl, cycloalkylalkyl, aryl, arylalkyl, heterocyclyl, heterocyclylalkyl, heteroaryl and heteroarylalkyl;
   wherein alkyl, alkenyl, alkynyl, cycloalkyl, cycloalkylalkyl, arylalkyl, aryl, heteroaryl, heteroarylalkyl, heterocyclyl, and heterocyclylalkyl are unsubstituted or substituted independently with up to four substituents independently selected from alkyl, alkenyl, alkynyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, aminocarbonylamino, azido, cyano, halogen, haloalkyl, perhaloalkyl, keto, nitro, -S(O)₂NR^{b}R^{b}, -NR^{b}S(O)₂R^{b} or -S(O)ₚR^{c}, thiocarbonyl, -SO₃H, cycloalkyl, cycloalkenyl, aryl, heteroaryl or heterocyclyl ;
R⁵ and R⁶ are independently selected from the group consisting of hydrogen, hydroxy, - (CR^{d}R^{e})ₙOR⁷, (CR^{d}R^{e})ₙCOOR⁷, -(CR^{d}R^{e})ₙNR⁸R⁹, cyanoalkyl, haloalkyl, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, carbonylamino, cycloalkyl, cycloalkylalkyl, aryl, arylalkyl, heteroaryl, heteroarylalkyl, heterocyclyl or heterocyclylalkyl; R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or S, said ring system is further optionally substituted with 1 to 4 substituents independently selected from halo, alkyl, alkenyl, alkynyl, nitro, cyano, -(CR^{d}R^{e})ₙOR⁷, -(CR^{d}R^{e})ₙSR⁷, - (CR^{d}R^{e})ₙNR⁸R⁹, oxo, alkylsulfonyl, -(CR^{d}R^{e})ₙCOOR⁷, -(CR^{d}R^{e})ₙC(O)NR⁸R⁹, cycloalkyl, cycloalkylalkyl, aryl, arylalkyl, heterocyclyl, heterocyclylalkyl, heteroaryl, or heteroarylalkyl;
R^{a} is selected from hydrogen or alkyl;
R^{b} each is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl and heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4, and
p is 0, 1 or 2.

### Detailed description of the invention

### Definitions

In the structural formulae given herein and throughout the present disclosure, the following terms have the indicated meaning, unless specifically stated otherwise.

The term "optionally substituted" as used herein means that the group in question is either unsubstituted or substituted with one or more of the substituents specified. When the group in question is substituted with more than one substituent, the substituent may be same or different.

The term "alkyl" refers to a monoradical branched or unbranched saturated hydrocarbon chain having 1, 2, 3, 4, 5, 6, 7, 8, 9, 10, 11, 12, 13, 14, 15, 16, 17, 18, 19 or 20 carbon atoms, preferably 1, 2, 3, 4, 5, 6, 7, 8, 9 or 10 carbon atoms, more preferably 1, 2, 3, 4, 5 or 6 carbon atoms. This term is exemplified by groups such as methyl, ethyl, n-propyl, iso-propyl, n-butyl, iso-butyl, t-butyl, n-hexyl, n-decyl, tetradecyl, and the like.

The term "alkylene" refers to a diradical of a branched or unbranched saturated hydrocarbon chain, having 1, 2, 3, 4, 5, 6, 7, 8, 9,10, 11,12, 13, 14, 15, 16, 17, 18, 19 or 20 carbon atoms, preferably 1, 2, 3, 4, 5, 6, 7, 8, 9 or 10 carbon atoms, more preferably 1, 2, 3, 4, 5 or 6 carbon atoms. This term is exemplified by groups such as methylene (-CH₂-), ethylene (-CH₂CH₂-), the propylene isomers (e.g., -CH₂CH₂CH₂- and -CH(CH₃)CH₂-) and the like.

The term "substituted alkyl" or "substituted alkylene" refers to: 1) an alkyl group or alkylene group as defined above, having 1, 2, 3, 4 or 5 substituents, preferably 1, 2 or 3 substituents, selected from the group consisting of alkenyl, alkynyl, alkoxy, cycloalkyl, cycloalkenyl, acyl, acylamino, acyloxy, amino, monoalkylamino, dialkylamino, arylamino, heteroarylamino, aminocarbonyl, alkoxycarbonylamino, azido, cyano, halogen, hydroxy, hydroxyalkyl, keto, thiocarbonyl, carboxy, carboxyalkyl, -SO₃H, aryl, aryloxy, heteroaryl, aminocarbonylamino, heteroaryloxy, heterocyclyl, heterocyclyloxy, hydroxyamino, alkoxyamino, nitro, - S(O)₂NR^{a}R^{a}, -NR^{a}S(O)₂R^{a} and -S(O)ₚR^{b}, where each R^{a} is independently selected from the group consisting of hydrogen, alkyl, cycloalkyl, cycloalkylalkyl, aryl, arylalkyl, heteroaryl heteroarylalkyl, heterocyclyl and heterocyclylalkyl; heterocyclyloxy where R^{b} is hydrogen, alkyl, aryl, heteroaryl or heterocyclyl. Unless otherwise constrained by the definition, all substituents may optionally be further substituted by 1, 2, or 3 substituents selected from alkyl, carboxy, carboxyalkyl, aminocarbonyl, hydroxy, alkoxy, halogen, CF₃, amino, substituted amino, cyano, and -S(O)ₚR^{c}, where R^{c} is alkyl, aryl, or heteroaryl and p is 0,1 or 2;
or 2) an alkyl group or alkylene group as defined above that is interrupted by 1, 2, 3, 4, 5, 6, 7, 8, 9 or 10 atoms independently selected from oxygen, sulphur and NR^{d}, where R^{d} is selected from hydrogen, alkyl, cycloalkyl, cycloalkenyl, aryl, heteroaryl and heterocyclyl, carbonylalkyl, carboxyester, carboxyamide and sulfonyl. All substituents may be optionally further substituted by alkyl, alkoxy, halogen, CF₃, amino, substituted amino, cyano, or - S(O)ₚR^{c}, in which R^{c} is alkyl, aryl, or heteroaryl and p is 0, 1, or 2;
or 3) an alkyl or alkylene as defined above that has 1, 2, 3, 4 or 5 substituents as defined above, as well as interrupted by 1, 2, 3, 4, 5, 6, 7, 8, 9 or 10 atoms as defined above.

The term "alkenyl" refers to a monoradical of a branched or unbranched unsaturated hydrocarbon group preferably having from 2, 3, 4, 5, 6, 7, 8, 9, 10, 11, 12, 13, 14, 15, 16, 17, 18, 19 or 20 carbon atoms, more preferably 2, 3, 4, 5, 6, 7, 8, 9 or 10 carbon atoms and even more preferably 2, 3, 4, 5 or 6 carbon atoms and having 1, 2, 3, 4, 5 or 6 double bond (vinyl), preferably 1 double bond. Preferred alkenyl groups include ethenyl or vinyl(-CH=CH₂), 1-propylene or allyl (-CH₂CH=CH₂), isopropylene (-C(CH₃)=CH₂), bicyclo [2.2. 1] heptene, and the like.

The term "alkenylene" refers to a diradical of a branched or unbranched unsaturated hydrocarbon group preferably having from 2, 3, 4, 5, 6, 7, 8, 9, 10, 11, 12, 13, 14, 15, 16, 17, 18, 19 or 20 carbon atoms, more preferably 2, 3, 4, 5, 6, 7, 8, 9 or 10 carbon atoms and even more preferably 2, 3, 4, 5 or 6 carbon atoms and having 1, 3, 4, 5 or 6 double bond (vinyl), preferably 1 double bond.

The term "substituted alkenyl" refers to an alkenyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, thiocarbonyl, carboxy, carboxyalkyl, SO₃H, aryl, aryloxy, heteroaryl, aminocarbonylamino, heteroaryloxy, heterocyclyl, heterocyclyloxy, hydroxyamino, alkoxyamino, nitro, -S(O)₂NR^{a}R^{a}, - NR^{a}S(O)₂R^{a} and -S(O)ₚR^{b} where each R^{a} is independently selected from the group consisting of hydrogen, alkyl, cycloalkyl, cycloalkylalkyl, aryl, arylalkyl, heteroaryl heteroarylalkyl, heterocyclyl, heterocyclylalkyl and heterocyclyloxy, where R^{b} is alkyl, aryl, heteroaryl or heterocyclyl and p is 0, 1 or 2. Unless otherwise constrained by the definition, all substituents may optionally be further substituted by 1, 2, or 3 substituents selected from alkyl, carboxy, carboxyalkyl, aminocarbonyl, hydroxy, alkoxy, halogen, CF₃, amino, substituted amino, cyano, and -S(O)ₚR^{c}, where R^{c} is alkyl, aryl, or heteroaryl and p is 0, 1 or 2.

The term "alkynyl" refers to a monoradical of an unsaturated hydrocarbon, preferably having from 2, 3, 4, 5, 6, 7, 8, 9, 10, 11, 12, 13, 14, 15, 16, 17, 18, 19 or 20 carbon atoms, more preferably 2, 3, 4, 5, 6, 7, 8, 9 or 10 carbon atoms and even more preferably 2, 3, 4, 5 or 6 carbon atoms and having 1, 2, 3, 4, 5 or 6 sites of acetylene (triple bond) unsaturation, preferably 1 triple bond. Preferred alkynyl groups include ethynyl, (-C=CH), propargyl (or prop-1-yn-3-yl,-CH₂C≡CH), homopropargyl (or but-1-yn-4-yl, -CH₂CH₂C≡CH) and the like. The term "alkynylene" refers to a diradical of a branched or an unbranched unsaturated hydrocarbon group preferably having from 2, 3, 4, 5, 6, 7, 8, 9, 10, 11, 12, 13, 14, 15, 16, 17, 18, 19 or 20 carbon atoms, more preferably 2, 3, 4, 5, 6, 7, 8, 9 or 10 carbon atoms and even more preferably 2, 3, 4, 5 or 6 carbon atoms and having 1, 3, 4, 5 or 6 sites of acetylene (triple bond) unsaturation, preferably 1 triple bond.

The term "substituted alkynyl" refers to an alkynyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, -SO₃H, aryl, aryloxy, heteroaryl, aminocarbonylamino, heteroaryloxy, heterocyclyl, heterocyclyloxy, hydroxyamino, alkoxyamino, nitro, -S(O)₂NR^{a}R^{a}, - NR^{a}S(O)₂R^{a} and -S(O)ₚR^{b}, where each R^{a} is independently selected from the group consisting of hydrogen, alkyl, cycloalkyl, cycloalkylalkyl, aryl, arylalkyl, heteroaryl heteroarylalkyl, heterocyclyl, heterocyclylalkyl and heterocyclyloxy, where R^{b} is alkyl, aryl, heteroaryl or heterocyclyl and p is 0, 1 or 2. Unless otherwise constrained by the definition, all substituents may optionally be further substituted by 1, 2, or 3 substituents selected from alkyl, carboxy, carboxyalkyl, aminocarbonyl, hydroxy, alkoxy, halogen, CF₃, amino, substituted amino, cyano, and-S(O)ₚR^{c} where R^{c} is alkyl, aryl, or heteroaryl and p is 0, 1 or 2.

The term "cycloalkyl" refers to unless otherwise mentioned, carbocyclic groups of from 3 to 20 carbon atoms having a single cyclic ring or multiple condensed rings which may be saturated or partially unsaturated. Such cycloalkyl groups include, by way of example, single ring structures such as cyclopropyl, cyclobutyl, cyclopentyl, cyclopentenyl, cyclohexyl, cyclohexenyl, cyclooctyl, and the like, or multiple ring structures such as adamantanyl, bicyclo[2.2.1]heptane, bicyclo[2.2.2]octane, 1,3,3-trimethylbicyclo[2.2.1]hept-2-yl, (2,3,3-trimethylbicyclo[2.2.1]hept-2-yl), or carbocyclic groups to which is fused an aryl group, for example indane, and the like.

The term "substituted cycloalkyl" refers to cycloalkyl groups having 1, 2, 3, 4 or 5 substituents, and preferably 1, 2, or 3 substituents, selected from the group consisting of alkyl, alkoxy, cycloalkyl, cycloalkenyl, acyl, acylamino, acyloxy, amino, aminocarbonyl, alkoxycarbonylamino, azido, cyano, halogen, hydroxy, oxo, thiocarbonyl, aryl, aryloxy, heteroaryl, aminosulfonyl, aminocarbonylamino, heteroaryloxy, heterocyclyl, heterocyclyloxy, hydroxyamino, alkoxyamino, nitro, -C(O)R and -S(O)ₚR^{b}, where R is hydrogen, hydroxyl, alkoxy, alkyl and cyclocalkyl, heterocyclyloxy where R^{b} is alkyl, aryl, heteroaryl or heterocyclyl and p is 0, 1 or 2. Unless otherwise constrained by the definition, all substituents may optionally be further substituted by 1, 2, or 3 substituents selected from alkyl, carboxy, carboxyalkyl, aminocarbonyl, hydroxy, alkoxy, halogen, CF₃, amino, substituted amino, cyano, and-S(O)ₚR^{c}, where R^{c} is alkyl, aryl, or heteroaryl and p is 0, 1 or 2. "Halo" or "Halogen", alone or in combination with any other term means halogens such as chloro (Cl), fluoro (F), bromo (Br) and iodo (I).

"Haloalkyl" refers to a straight chain or branched chain haloalkyl group with 1 to 6 carbon atoms. The alkyl group may be partly or totally halogenated. Representative examples of haloalkyl groups include but are not limited to fluoromethyl, chloromethyl, bromomethyl, difluoromethyl, dichloromethyl, dibromomethyl, trifluoromethyl, trichloromethyl, 2-fluoroethyl, 2-chloroethyl, 2-bromoethyl, 2,2,2-trifluoroethyl, 3-fluoropropyl, 3-chloropropyl, 3-bromopropyl and the like.

The term "alkoxy" refers to the group R"'-O-, where R"' is optionally substituted alkyl or optionally substituted cycloalkyl, or optionally substituted alkenyl or optionally substituted alkynyl; or optionally substituted cycloalkenyl, where alkyl, alkenyl, alkynyl, cycloalkyl and cycloalkenyl are as defined herein. Representative examples of alkoxy groups include but are not limited to methoxy, ethoxy, n-propoxy, iso-propoxy, n-butoxy, tert-butoxy, sec-butoxy, n-pentoxy, n-hexoxy, 1,2-dimethylbutoxy, trifluoromethoxy, and the like.

The term "aminocarbonyl" refers to the group -C(O)NR'R' where each R' is independently hydrogen, alkyl, aryl, heteroaryl, heterocyclyl or both R' groups are joined to form a heterocyclic group (e. g. morpholino). Unless otherwise constrained by the definition, all substituents may optionally be further substituted by 1-3 substituents selected from alkyl, carboxy, carboxyalkyl, aminocarbonyl, hydroxy, alkoxy, halogen, CF₃, amino, substituted amino, cyano, and -S(O)ₚR^{c}, where R^{c} is alkyl, aryl, or heteroaryl and p is 0, 1 or 2.

The term "acylamino" refers to the group -NR"C(O)R" where each R" is independently hydrogen, alkyl, aryl, heteroaryl, or heterocyclyl. Unless otherwise constrained by the definition, all substituents may optionally be further substituted by 1-3 substituents selected from alkyl, carboxy, carboxyalkyl, aminocarbonyl, hydroxy, alkoxy, halogen, CF₃, amino, substituted amino, cyano, and-S(O)ₚR^{c}, where R^{c} is alkyl, aryl, or heteroaryl and p is 0, 1 or 2.

The term "acyloxy" refers to the groups -OC(O)-alkyl, -OC(O)-cycloalkyl, -OC(O)-aryl, - OC(O)-heteroaryl, and -OC(O)-heterocyclyl. Unless otherwise constrained by the definition, all substituents may be optionally further substituted by alkyl, carboxy, carboxyalkyl, aminocarbonyl, hydroxy, alkoxy, halogen, CF₃, amino, substituted amino, cyano, or - S(O)ₚR^{c}, where R^{c} is alkyl, aryl, or heteroaryl and p is 0, 1 or 2.

"Alkoxyalkyl" refers to alkyl groups as defined above wherein at least one of the hydrogen atoms of the alkyl group is replaced by an alkoxy group as defined above. Representative examples of alkoxyalkyl groups include but are not limited to methoxymethyl, methoxyethyl, ethoxymethyl and the like.

"Aryloxyalkyl" refers to the group -alkyl-O-aryl. Representative examples of aryloxyalkyl include but are not limited to phenoxymethyl, naphthyloxymethyl, phenoxyethyl, naphthyloxyethyl and the like.

"Di alkylamino" refers to an amino group, to which two same or different straight chain or branched chain alkyl groups with 1 to 6 carbon atoms are bound. Representative examples of di alkylamino include but are not limited to dimethylamino, diethylamino, methylethylamino, dipropylamino, dibutylamino and the like.

"Cycloalkylalkyl" refers to an alkyl radical as defined above which is substituted by a cycloalkyl radical as defined above. Representative examples of cycloalkylalkyl include but are not limited to cyclopropylmethyl, cyclobutylmethyl, cyclopentylmethyl, cyclohexylmethyl, 1-cyclopentylethyl, 1-cyclohexylethyl, 2-cyclopentylethyl, 2-cyclohexylethyl, cyclobutylpropyl, cyclopentylpropyl, cyclohexylbutyl and the like.

"Aminoalkyl" refers to an amino group that is attached to (C₁₋₆)alkylene as defined herein. Representative examples of aminoalkyl include but are not limited to aminomethyl, aminoethyl, 1-aminopropyl, 2-aminopropyl, and the like. The amino moiety of aminoalkyl may be substituted once or twice with alkyl to provide alkylaminoalkyl and dialkylaminoalkyl respectively. Representative examples of alkylaminoalkyl include but are not limited to methylaminomethyl, methylaminoethyl, methylaminopropyl, ethylaminoethyl and the like. Representative examples of dialkylaminoalkyl include but are not limited to dimethylaminomethyl, dimethylaminoethyl, dimethylaminopropyl, N-methyl-N-ethylaminoethyl and the like.

The term "aryl" refers to an aromatic carbocyclic group of 6 to 20 carbon atoms having a single ring (e.g. phenyl) or multiple rings (e.g. biphenyl), or multiple condensed (fused) rings (e.g. naphthyl or anthranyl). Preferred aryls include phenyl, naphthyl and the like.

The term "arylene" refers to a diradical of an aryl group as defined above. This term is exemplified by groups such as 1,4-phenylene, 1,3-phenylene, 1,2-phenylene, 1,4'-biphenylene, and the like.

Unless otherwise constrained the aryl or arylene groups may optionally be substituted with 1, 2, 3 4 or 5 substituents, preferably 1, 2 or 3 substituents, selected from the group consisting of alkyl, alkoxy, cycloalkyl, cycloalkenyl, acyl, acylamino, acyloxy, amino, aminocarbonyl, alkoxycarbonylamino, azido, cyano, halogen, hydroxy, carboxy, carboxyalkyl, -SO₃H, aryl, aryloxy, heteroaryl, aminosulfonyl, aminocarbonylamino, heteroaryloxy, heterocyclyl, heterocyclyloxy, hydroxyamino, alkoxyamino, nitro, -S(O)₂NR^{a}R^{a}, -NR^{a}S(O)₂R^{a} and - S(O)ₚR^{b} where each R^{a} is independently selected from the group consisting of hydrogen, alkyl, cycloalkyl, cycloalkylalkyl, aryl, arylalkyl, heteroaryl, heteroarylalkyl, heterocyclyl and heterocyclylalkyl; where R^{b} is hydrogen, alkyl, aryl, heterocyclyl or heteroaryl and p is 0, 1 or 2. Unless otherwise constrained by the definition, all substituents may optionally be further substituted by 1, 2 or 3 substituents selected from alkyl, carboxy, carboxyalkyl, aminocarbonyl, hydroxy, alkoxy, halogen, CF₃, amino, substituted amino, cyano, and - S(O)ₚR^{c} where R^{c} is hydrogen, alkyl, aryl, or heteroaryl and p is 0, 1 or 2.

The term "arylalkyl" refers to an aryl group covalently linked to an alkylene group, where aryl and alkylene are defined herein.

"Optionally substituted arylalkyl" refers to an optionally substituted aryl group covalently linked to an optionally substituted alkylene group. Such arylalkyl groups are exemplified by benzyl, phenethyl, naphthylmethyl, and the like.

The term "aryloxy" refers to the group aryl-O- wherein the aryl group is as defined above, and includes optionally substituted aryl groups as also defined above.

The term "arylthio" refers to the group -S-aryl, where aryl is as defined herein including optionally substituted aryl groups as also defined above.

The term "substituted amino" refers to the group -NR'R' where each R' is independently selected from the group consisting of hydrogen, alkyl, cycloalkyl, carboxyalkyl, alkoxycarbonyl, aryl, heteroaryl and heterocyclyl. Unless otherwise constrained by the definition, all substituents may optionally be further substituted by 1, 2 or 3 substituents selected from alkyl, carboxy, carboxyalkyl, aminocarbonyl, hydroxy, alkoxy, halogen, CF₃, amino, substituted amino, cyano, and -S(O)ₚR^{c}, where R^{c} is alkyl, aryl, or heteroaryl and p is 0, 1 or 2.

The term "carboxyalkyl" refers to the groups -alkylene-C(O)OH.

The term "alkylcarboxyalkyl" refers to the groups -alkylene-C(O)OR^{d} where R^{d} is alkyl, cycloalkyl, where alkyl, cycloalkyl are as defined herein, and may be optionally further substituted by alkyl, halogen, CF₃, amino, substituted amino, cyano, or -S(O)ₚR^{c}, in which R^{c} is alkyl, aryl, or heteroaryl and p is 0, 1 or 2.

The term "heteroaryl" refers to an aromatic cyclic group having 1, 2, 3, 4, 5, 6, 7, 8, 9, 10, 11, 12, 13, 14, or 15 carbon atoms and 1, 2, 3 or 4 heteroatoms selected from oxygen, nitrogen and sulphur within at least one ring. Such heteroaryl groups can have a single ring (e.g. pyridinyl or furanyl) or multiple condensed rings (e.g. indolizinyl, benzooxazolyl, benzothiazolyl, or benzothienyl). Examples of heteroaryls include, but are not limited to, [1,2,4] oxadiazole, [1,3,4] oxadiazole, [1,2,4] thiadiazole, [1,3,4] thiadiazole, pyrrole, imidazole, pyrazole, pyridine, pyrazine, pyrimidine, pyridazine, indolizine, isoindole, indole, indazole, purine, quinolizine, isoquinoline, quinoline, phthalazine, quinoxaline, quinazoline, cinnoline, pteridine, carbazole, carboline, phenanthridine, acridine, phenanthroline, isothiazole, phenazine, isoxazole, phenoxazine, phenothiazine, furan, thiophene, oxazole, thiazole, triazole, triazine and the like.

The term "heteroarylene" refers to a diradical of a heteroaryl group as defined above.

Unless otherwise constrained the heteroaryl or heterarylene groups can be optionally substituted with 1, 2, 3, 4 or 5 substituents, preferably 1, 2 or 3 substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, thiocarbonyl, carboxy, carboxyalkyl, -SO₃H, aryl, aryloxy, heteroaryl, aminocarbonylamino, heteroaryloxy, heterocyclyl, heterocyclyloxy, hydroxyamino, alkoxyamino, nitro, - S(O)₂NR^{a}R^{a}, -NR^{a}S(O)₂R^{a} and -S(O)ₚR^{b}, where each R^{a} is independently selected from the group consisting of hydrogen, alkyl, cycloalkyl, cycloalkylalkyl, aryl, arylalkyl, heteroaryl heteroarylalkyl, heterocyclyl and heterocyclylalkyl; where R^{b} is hydrogen, alkyl, aryl, heterocyclyl or heteroaryl, and p is 0, 1 or 2. Unless otherwise constrained by the definition, all substituents may optionally be further substituted by 1-3 substituents selected from alkyl, carboxy, carboxyalkyl, aminocarbonyl, hydroxy, alkoxy, halogen, CF₃, amino, substituted amino, cyano, and-S(O)ₙR^{c}, where R^{c} is alkyl, aryl, or heteroaryl and n is 0,1 or 2.

The term "heteroarylalkyl" refers to a heteroaryl group covalently linked to an alkylene group, where heteroaryl and alkylene are defined herein.

"Optionally substituted heteroarylalkyl" refers to an optionally substituted heteroaryl group covalently linked to an optionally substituted alkylene group. Such heteroarylalkyl groups are exemplified by 3-pyridylmethyl, quinolin-8-ylethyl, 4-methoxythiazol-2-ylpropyl, and the like.

The term "heterocyclyl" refers to a saturated or partially unsaturated group having a single ring or multiple condensed rings, unless otherwise mentioned, having from 1 to 40 carbon atoms and from 1 to 10 hetero atoms, preferably 1, 2, 3 or 4 heteroatoms, selected from nitrogen, sulphur, phosphorus, and/or oxygen within the ring. Heterocyclic groups can have a single ring or multiple condensed rings, and include dihydrofuranyl, tetrahydrofuranyl, morpholinyl, pyrrolidinyl, dihydropyrrole, dihydropyranyl, tetrahydropyranyl, pyrazolidinyl, imidazolidinyl, dihydropyridinyl, tetrahydropyridinyl, piperidinyl, dihydropyrazinyl, tetrahydropyrazinyl, piperazinyl, dihydropyridinyl, benzodioxolyl, tetrahydroquinolinyl, tetrahydroisoquinolinyl, tetrahydronaphthyridinyl, tetrahydrothienopyridinyl and the like. Unless otherwise constrained by the definition for the heterocyclic substituent, such heterocyclic groups can be optionally substituted with 1, 2, 3, 4 or 5, and preferably 1, 2 or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, oxo, -C(O)R where R is hydrogen, hydroxyl, alkoxy, alkyl and cyclocalkyl, thiocarbonyl, carboxy, carboxyalkyl, aryl, aryloxy, heteroaryl, aminosulfonyl, aminocarbonylamino, heteroaryloxy, heterocyclyl, heterocyclyloxy, hydroxyamino, alkoxyamino, nitro, and -S(O)ₚR^{b}, where R^{b} is hydrogen, alkyl, aryl, heterocyclyl or heteroaryl and p is 0, 1 or 2. Unless otherwise constrained by the definition, all substituents may optionally be further substituted by 1-3 substituents selected from alkyl, carboxy, carboxyalkyl, aminocarbonyl, hydroxy, alkoxy, halogen, CF₃, amino, substituted amino, cyano, and -S(O)R^{c}, where R^{c} is alkyl, aryl, or heteroaryl and n is 0, 1 or 2.

The term "heterocyclylalkyl" refers to a heterocyclyl group covalently linked to an alkylene group, where heterocyclyl and alkylene are defined herein.

"Optionally substituted heterocyclylalkyl" refers to an optionally substituted heterocyclyl group covalently linked to an optionally substituted alkylene group.

The term "heteroaryloxy" refers to the group heteroaryl-O-.

The term "thiol" refers to the group -SH.

The term "substituted alkylthio" refers to the group -S-substituted alkyl.

The term "heteroarylthio" refers to the group -S-heteroaryl wherein the heteroaryl group is as defined above including optionally substituted heteroaryl groups as also defined above. The term "sulfoxide" refers to a group -S(O).

"Substituted sulfoxide" refers to a group -S(O)R, in which R is substituted alkyl, substituted aryl, or substituted heteroaryl, as defined herein.

The term "sulfone" refers to a group -S(O)₂R.

The term "substituted sulfone" refers to a group -S(O)₂R, in which R is alkyl, aryl, or heteroaryl.

The compounds of the present disclosure may have the ability to crystallize in more than one form, a characteristic known as polymorphism, and all such polymorphic forms ("polymorphs") are encompassed within the scope of the present disclosure. Polymorphism generally can occur as a response to changes in temperature or pressure or both, and can also result from variations in the crystallization process. Polymorphs can be distinguished by various physical characteristics, and typically the x-ray diffraction patterns, solubility behavior, and melting point of the compound are used to distinguish polymorphs.

The compounds described herein may contain one or more chiral centers and/or double bonds and therefore, may exist as stereoisomers, such as double-bond isomers (i.e., geometric isomers), regioisomers, enantiomers or diastereomers. Accordingly, the chemical structures depicted herein encompass all possible enantiomers and stereoisomers of the illustrated or identified compounds including the stereoisomerically pure form (e.g., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the person skilled in the art. The compounds may also exist in several tautomeric forms including the enol form, the keto form and mixtures thereof. Accordingly, the chemical structures depicted herein encompass all possible tautomeric forms of the illustrated or identified compounds.

Compounds may exist in unsolvated forms as well as solvated forms, including hydrated forms and as N-oxides. In general, compounds may be hydrated, solvated or N-oxides. Certain compounds may exist in multiple crystalline or amorphous forms. In general, unless otherwise indicated, all physical forms are equivalent for the uses contemplated herein and are intended to be within the scope of the present invention.

"Pharmaceutically acceptable salt" embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the present disclosure are quaternary ammonium compounds wherein an equivalent of an anion (M-) is associated with the positive charge of the N atom. M- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. M- is preferably an anion selected from chloride, bromide, iodide, sulphate, nitrate, acetate, maleate, oxalate, succinate or trifluoroacetate. More preferably M- is chloride, bromide, trifluoroacetate or methanesulphonate.

The terms "solvent", "inert organic solvent" or "inert solvent" mean a solvent inert under the conditions of the reaction being described in conjunction therewith [including, for example, benzene, toluene, acetonitrile, tetrahydrofuran ("THF"), dimethylformamide ("DMF"), chloroform, methylene chloride (or dichloromethane), diethyl ether, methanol, pyridine and the like]. Unless specified to the contrary, the solvents used in the reactions of the present disclosure are inert organic solvents. The term "q.s." means adding a quantity sufficient to achieve a stated function, e.g., to bring a solution to the desired volume (i.e., 100%).

The present disclosure provides compounds of formula I, or its tautomers, polymorphs, stereoisomers, solvate or a pharmaceutically acceptable salts thereof, pharmaceutical compositions containing them and methods of treating conditions and diseases that are mediated by adenosine receptor activity, 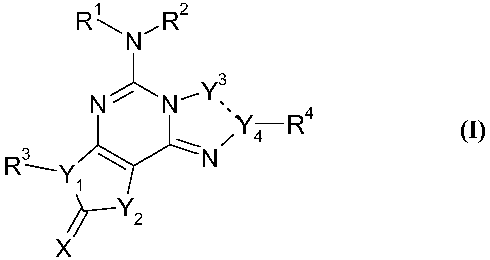 wherein
--- represents a single bond or a double bond;
X is selected from O, S or NR^{a};
Y₁ is selected from N or CH;
Y₂ is selected from NR⁵, O or CR⁵R⁶;
Y₃ is selected from N, CH, CH₂, C(=O) or C(=S);
Y₄ is selected from N, C or CH;
R¹ and R² are independently selected from hydrogen or alkyl;
R³ is -A-Z-B-Q;
   wherein, A is absent or is a group selected from alkylene, alkenylene or alkynylene; wherein one or more methylene groups is optionally replaced by hetero atoms or groups such as -O-, -S(O)p-, -N(R^{a})-, or -C(O); alkylene, alkenylene and alkynylene is optionally substituted with -(CR^{d}R^{e})ₙOR⁷, (CR^{d}R^{e})ₙCOOR⁷, -(CR^{d}R^{e})ₙNR⁸R⁹, cyano, halogen, haloalkyl, perhaloalkyl, alkoxyalkoxy, alkyl or cycloalkyl;
   Z is absent or is selected from a cycloalkyl or a heterocyclyl;
   wherein cycloalkyl and heterocyclyl are unsubstituted or substituted independently with 1, 2, or 3 substituents independently selected from alkyl, alkenyl, alkynyl, acyl, - (CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, halogen, haloalkyl, perhaloalkyl, azido, cyano, keto, thiocarbonyl, -SO₃H, aminocarbonylamino, nitro, -S(O)₂NR^{a}R^{a}, -NR^{b}S(O)₂R^{b} or - S(O)ₚR^{c};
   B is absent or is a group selected from alkylene, alkenylene or alkynylene; wherein one or more methylene groups is optionally replaced by hetero atoms or groups such as -O-, -S(O)p-, -N(R^{a})-, or -C(O); alkylene, alkenylene and alkynylene is optionally substituted with hydroxy, amino, aminoalkyl, cyano, halogen, haloalkyl, perhaloalkyl, carboxy, carboxyalkyl, alkoxy, hydroxyalkyl, alkoxyalkyl, alkoxyalkoxy or alkyl;
   Q is selected from hydrogen, alkyl, cycloalkyl, cycloalkylalkyl, aryl, arylalkyl, heterocyclyl, heterocyclylalkyl, heteroaryl or heteroarylalkyl;
   wherein alkyl, cycloalkyl, cycloalkylalkyl, aryl, arylalkyl, heterocyclyl, heterocyclylalkyl, heteroaryl and heteroarylalkyl are unsubstituted or substituted independently with 1, 2, or 3 substituents independently selected from alkyl, alkenyl, alkynyl, halogen, haloalkyl, perhaloalkyl, azido, cyano, nitro, keto, thiocarbonyl, cyanoalkyl, cyanoalkylcarbonyl, -(CR^{d}R^{e})ₙOR⁷, -(CR^{d}R^{e})ₙC(O)R⁷, - (CR^{d}R^{e})ₙSR⁷, -(CR^{d}R^{e})ₙCOOR⁷, -(CR^{d}R^{e})ₙNR⁸R⁹, -(CR^{d}R^{e})ₙC(O)NR⁸R⁹, - (CR^{d}R^{e})ₙNR⁸C(O)OR⁷, -(CR^{d}R^{e})ₙNR⁸C(O)NR⁸R⁹, -NR^{b}S(O)₂R^{b}, -S(O)ₚR^{c}, - SO₃H, -S(O)₂NR^{a}R^{a}, cycloalkyl, cycloalkenyl, cycloalkylalkyl aryl, arylalkyl, heterocyclyl, heterocyclylalkyl, heteroaryl or heteroarylalkyl;
   wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, alkenyl, alkynyl, haloalkyl, hydroxyalkyl, carboxyalkyl, cycloalkyl, cycloalkylalkyl, aryl, arylalkyl, heterocyclyl, heterocyclylalkyl, heteroaryl and heteroarylalkyl;
   wherein alkyl, alkenyl, alkynyl, cycloalkyl, cycloalkylalkyl, arylalkyl, aryl, heteroaryl, heteroarylalkyl, heterocyclyl, and heterocyclylalkyl are unsubstituted or substituted independently with up to four substituents independently selected from alkyl, alkenyl, alkynyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, aminocarbonylamino, azido, cyano, halogen, haloalkyl, perhaloalkyl, keto, nitro, -S(O)₂NR^{b}R^{b}, -NR^{b}S(O)₂R^{b} or -S(O)ₚR^{c}, thiocarbonyl, -SO₃H, cycloalkyl, cycloalkenyl, aryl, heteroaryl or heterocyclyl ;
R⁵ and R⁶ are independently selected from the group consisting of hydrogen, hydroxy, - (CR^{d}R^{e})ₙOR⁷, (CR^{d}R^{e})ₙCOOR⁷, -(CR^{d}R^{e})ₙNR⁸R⁹, cyanoalkyl, haloalkyl, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, carbonylamino, cycloalkyl, cycloalkylalkyl, aryl, arylalkyl, heteroaryl, heteroarylalkyl, heterocyclyl or heterocyclylalkyl; R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or S, said ring system is further optionally substituted with 1 to 4 substituents independently selected from halo, alkyl, alkenyl, alkynyl, nitro, cyano, -(CR^{d}R^{e})ₙOR⁷, -(CR^{d}R^{e})ₙSR⁷, - (CR^{d}R^{e})ₙNR⁸R⁹, oxo, alkylsulfonyl, -(CR^{d}R^{e})ₙCOOR⁷, -(CR^{d}R^{e})ₙC(O)NR⁸R⁹, cycloalkyl, cycloalkylalkyl, aryl, arylalkyl, heterocyclyl, heterocyclylalkyl, heteroaryl, or heteroarylalkyl;
R^{a} is selected from hydrogen or alkyl;
R^{b} each is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl and heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4 and
p is 0, 1 or 2.

According to another embodiment, the present disclosure relates to compounds of formula (I) or its tautomers, polymorphs, stereoisomers, solvate or a pharmaceutically acceptable salts thereof, wherein,
--- represents a double bond;
X is selected from O, S or NR^{a};
Y₁ is selected from N or CH;
Y₂ is selected from NR⁵ or CR⁵R⁶;
Y₃ is selected from N, CH or CH₂,;
Y₄ is selected from N or C;
R¹ and R² are independently selected from hydrogen or alkyl;
R³ is -A-Z-B-Q;
   wherein, A is absent or is alkylene wherein one or more methylene groups is optionally replaced by hetero atoms or groups such as -O-, -S(O)p-, -N(R^{a})-, or -C(O); alkylene is optionally substituted with -(CR^{d}R^{e})ₙOR⁷, cyano, halogen, haloalkyl, perhaloalkyl, alkyl or cycloalkyl;
   Z is absent or is selected from a cycloalkyl or a heterocyclyl;
   wherein cycloalkyl and heterocyclyl are unsubstituted or substituted independently with 1, 2, or 3 substituents independently selected from alkyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, halogen, haloalkyl, perhaloalkyl, azido, cyano, halogen, keto, thiocarbonyl, -SO₃H, aminocarbonylamino, nitro, -S(O)₂NR^{a}R^{a}, -NR^{b}S(O)₂R^{b} or -S(O)ₚR^{c};
   B is absent or is alkylene wherein one or more methylene groups is optionally replaced by hetero atoms or groups such as -O-, -S(O)p-, -N(R^{a})-, or -C(O); alkylene is optionally substituted with hydroxy, amino, aminoalkyl, cyano, halogen, haloalkyl, perhaloalkyl, carboxy, carboxyalkyl, alkoxy, hydroxyalkyl, alkoxyalkyl, alkoxyalkoxy or alkyl;
   Q is selected from hydrogen, alkyl, cycloalkyl, cycloalkylalkyl, aryl, arylalkyl, heterocyclyl, heterocyclylalkyl, heteroaryl or heteroarylalkyl;
   wherein alkyl, cycloalkyl, cycloalkylalkyl, aryl, arylalkyl, heterocyclyl, heterocyclylalkyl, heteroaryl and heteroarylalkyl are unsubstituted or substituted independently with 1, 2, or 3 substituents independently selected from alkyl, alkenyl, alkynyl, alkoxy, alkoxyalkyl, haloalkyl, perhaloalkyl, azido, cyano, nitro, halogen, keto, thiocarbonyl, cyanoalkyl, cyanoalkylcarbonyl, -(CR^{d}R^{e})ₙOR⁷, - (CR^{d}R^{e})ₙC(O)R⁷, -(CR^{d}R^{e})ₙSR⁷, -(CR^{d}R^{e})ₙCOOR⁷, -(CR^{d}R^{e})ₙNR⁸R⁹, - (CR^{d}R^{e})ₙC(O)NR⁸R⁹, -(CR^{d}R^{e})ₙNR⁸C(O)OR⁷, -(CR^{d}R^{e})ₙNR⁸C(O)NR⁸R⁹, - NR^{b}S(O)₂R^{b}, -S(O)ₚR^{c}, -SO₃H, -S(O)₂NR^{a}R^{a}, cycloalkyl, cycloalkenyl, cycloalkylalkyl aryl, arylalkyl, heterocyclyl, heterocyclylalkyl, heteroaryl or heteroarylalkyl;
   wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, haloalkyl, hydroxyalkyl, carboxyalkyl, cycloalkyl, cycloalkylalkyl, aryl, arylalkyl, heterocyclyl, heterocyclylalkyl, heteroaryl and heteroarylalkyl;
   wherein alkyl, cycloalkyl, cycloalkylalkyl, arylalkyl, aryl, heteroaryl, heteroarylalkyl, heterocyclyl, and heterocyclylalkyl are unsubstituted or independently substituted with up to four substituents independently selected from alkyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, aminocarbonylamino, azido, cyano, halogen, haloalkyl, perhaloalkyl, keto, nitro, - S(O)₂NR^{b}R^{b}, -NR^{b}S(O)₂R^{b} or -S(O)ₚR^{c}, thiocarbonyl, -SO₃H, cycloalkyl, cycloalkenyl, aryl, heteroaryl or heterocyclyl ;
R⁵ and R⁶ are independently selected from the group consisting of hydrogen, hydroxy, - (CR^{d}R^{e})ₙOR⁷, (CR^{d}R^{e})ₙCOOR⁷, -(CR^{d}R^{e})ₙNR⁸R⁹, cyanoalkyl, haloalkyl, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, carbonylamino, cycloalkyl, cycloalkylalkyl, aryl, arylalkyl, heteroaryl, heteroarylalkyl, heterocyclyl or heterocyclylalkyl; R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or S, said ring system is further optionally substituted with 1 to 4 substituents independently selected from halo, alkyl, alkenyl, alkynyl, nitro, cyano, -(CR^{d}R^{e})ₙOR⁷, -(CR^{d}R^{e})ₙSR⁷, - (CR^{d}R^{e})ₙNR⁸R⁹, oxo, alkylsulfonyl, -(CR^{d}R^{e})ₙCOOR⁷, -(CR^{d}R^{e})ₙC(O)NR⁸R⁹, cycloalkyl, cycloalkylalkyl, aryl, arylalkyl, heterocyclyl, heterocyclylalkyl, heteroaryl, or heteroarylalkyl;
R^{a} is selected from hydrogen or alkyl;
R^{b} at each occurrence is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl or heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4 and
p is 0, 1 or 2.

According to another embodiment, the present disclosure relates to compounds of formula (I) or its tautomers, polymorphs, stereoisomers, solvate or a pharmaceutically acceptable salts thereof, wherein,
--- represents a double bond;
X is selected from O or S;
Y₁ represents N;
Y₂ represents NR⁵;
Y₃ represents N;
Y₄ represents C;
R¹ and R² are independently selected from hydrogen or alkyl;
R³ is -A-Z-B-Q;
   wherein, A is absent or is alkylene wherein one or more methylene groups is optionally replaced by hetero atoms or groups such as -O-, -S(O)p-, -N(R^{a})-, or -C(O);
   Z is absent or is a heterocyclyl;
   wherein the heterocyclyl is unsubstituted or substituted independently with 1, 2, or 3 substituents independently selected from alkyl, acyl, -(CR^{d}R^{e})ₙOR⁷, (CR^{d}R^{e})ₙCOOR⁷, -(CR^{d}R^{e})ₙNR⁸R⁹, haloalkyl, perhaloalkyl, cyano, halogen, keto, thiocarbonyl, -SO₃H, nitro, -S(O)₂NR^{a}R^{a}, -NR^{b}S(O)₂R^{b} or -S(O)ₚR^{c};
   B is absent or is alkylene wherein one or more methylene groups is optionally replaced by hetero atoms or groups such as -O-, -S(O)p-, -N(R^{a})-, or -C(O);
   Q is selected from hydrogen, alkyl, cycloalkyl, aryl, heterocyclyl, or heteroaryl;
   wherein alkyl, cycloalkyl, aryl, heterocyclyl and heteroaryl are unsubstituted or independently substituted with 1, 2, or 3 substituents independently selected from alkyl, alkoxy, alkoxyalkyl, haloalkyl, perhaloalkyl, azido, cyano, nitro, halogen, keto, thiocarbonyl, cyanoalkyl, cyanoalkylcarbonyl, -(CR^{d}R^{e})ₙOR⁷, - (CR^{d}R^{e})ₙC(O)R⁷, -(CR^{d}R^{e})ₙSR⁷, -(CR^{d}R^{e})ₙCOOR⁷, -(CR^{d}R^{e})ₙNR⁸R⁹, - (CR^{d}R^{e})ₙC(O)NR⁸R⁹, -(CR^{d}R^{e})ₙNR⁸C(O)OR⁷, -(CR^{d}R^{e})ₙNR⁸C(O)NR⁸R⁹, - NR^{b}S(O)₂R^{b}, -S(O)ₚR^{c}, -SO₃H, -S(O)₂NR^{a}R^{a}, cycloalkyl, cycloalkenyl, cycloalkylalkyl aryl, arylalkyl, heterocyclyl, heterocyclylalkyl, heteroaryl or heteroarylalkyl;
   wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, haloalkyl, hydroxyalkyl, carboxyalkyl, cycloalkyl, cycloalkylalkyl, aryl, arylalkyl, heterocyclyl, heterocyclylalkyl, heteroaryl and heteroarylalkyl;
   wherein alkyl, cycloalkyl, cycloalkylalkyl, arylalkyl, aryl, heteroaryl, heteroarylalkyl, heterocyclyl, and heterocyclylalkyl are unsubstituted or independently substituted with up to four substituents independently selected from alkyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, aminocarbonylamino, azido, cyano, halogen, haloalkyl, perhaloalkyl, keto, nitro, - S(O)₂NR^{b}R^{b}, -NR^{b}S(O)₂R^{b} or -S(O)ₚR^{c}, thiocarbonyl, -SO₃H, cycloalkyl, cycloalkenyl, aryl, heteroaryl or heterocyclyl ;
R⁵ and R⁶ are independently selected from the group consisting of hydrogen, hydroxy, - (CR^{d}R^{e})ₙOR⁷, (CR^{d}R^{e})ₙCOOR⁷, -(CR^{d}R^{e})ₙNR⁸R⁹, cyanoalkyl, haloalkyl, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, carbonylamino, cycloalkyl, cycloalkylalkyl, aryl, arylalkyl, heteroaryl, heteroarylalkyl, heterocyclyl or heterocyclylalkyl; R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or S, said ring system is further optionally substituted with 1 to 4 substituents independently selected from halo, alkyl, alkenyl, alkynyl, nitro, cyano, -(CR^{d}R^{e})ₙOR⁷, -(CR^{d}R^{e})ₙSR⁷, - (CR^{d}R^{e})ₙNR⁸R⁹, oxo, alkylsulfonyl, -(CR^{d}R^{e})ₙCOOR⁷, -(CR^{d}R^{e})ₙC(O)NR⁸R⁹, cycloalkyl, cycloalkylalkyl, aryl, arylalkyl, heterocyclyl, heterocyclylalkyl, heteroaryl, or heteroarylalkyl;
R^{a} is selected from hydrogen or alkyl;
R^{b} at each occurrence is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl or heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4 and
p is 0, 1 or 2.

According to another embodiment, the present disclosure relates to compounds of formula (I) or its tautomers, polymorphs, stereoisomers, solvate or a pharmaceutically acceptable salts thereof, wherein,
--- represents a double bond;
X selected from O or S;
Y₁ represents N;
Y₂ represents NR⁵;
Y₃ represents N;
Y₄ represents C;
R¹ and R² are independently selected from hydrogen or alkyl;
R³ is -A-Z-B-Q;
   wherein, A is absent or is alkylene wherein one or more methylene groups is optionally replaced by hetero atoms or groups such as -O- or -N(R^{a})-;
   Z is absent or is a heterocyclyl;
   wherein the heterocyclyl is unsubstituted or substituted independently with 1, 2, or 3 substituents independently selected from alkyl, -(CR^{d}R^{e})ₙOR⁷, (CR^{d}R^{e})ₙCOOR⁷, haloalkyl, perhaloalkyl, cyano, halogen, keto or thiocarbonyl;
   B is absent or is alkylene wherein one or more methylene groups is optionally replaced by hetero atoms or groups such as -O-, -N(R^{a})-, or -C(O);
   Q is selected from hydrogen, alkyl, cycloalkyl, aryl, heterocyclyl, or heteroaryl;
   wherein alkyl, cycloalkyl, aryl, heterocyclyl and heteroaryl are unsubstituted or independently substituted with 1, 2, or 3 substituents independently selected from alkyl, alkoxy, alkoxyalkyl, haloalkyl, perhaloalkyl, cyano, halogen, keto, thiocarbonyl, cyanoalkyl, -(CR^{d}R^{e})ₙOR⁷, -(CR^{d}R^{e})ₙC(O)R⁷, -(CR^{d}R^{e})ₙCOOR⁷, - (CR^{d}R^{e})ₙNR⁸R⁹, -(CR^{d}R^{e})ₙC(O)NR⁸R⁹, -(CR^{d}R^{e})ₙNR⁸C(O)OR⁷, -S(O)ₚR^{c}, - SO₃H, -S(O)₂NR^{a}R^{a}, cycloalkyl, cycloalkenyl, aryl, heterocyclyl or heteroaryl;
   wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, haloalkyl, hydroxyalkyl, cycloalkyl, aryl, heterocyclyl and heteroaryl;
   wherein alkyl, cycloalkyl, aryl, heteroaryl and heterocyclyl are unsubstituted or independently substituted with up to four substituents independently selected from alkyl, - (CR^{d}R^{e})ₙOR⁷, (CR^{d}R^{e})ₙCOOR⁷, -(CR^{d}R^{e})ₙNR⁸R⁹, cyano, halogen, haloalkyl, perhaloalkyl, nitro, -S(O)₂NR^{b}R^{b}, -NR^{b}S(O)₂R^{b}, -S(O)ₚR^{c}, thiocarbonyl, -SO₃H, cycloalkyl, aryl, heteroaryl or heterocyclyl ;
R⁵ is selected from the group consisting of hydrogen, hydroxy, haloalkyl, -(CR^{d}R^{e})ₙOR⁷, - (CR^{d}R^{e})ₙCOOR⁷, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, cycloalkyl, aryl, heteroaryl or heterocyclyl;
R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or S, said ring system is further optionally substituted with 1 to 4 substituents independently selected from halo, alkyl, nitro, cyano, -(CR^{d}R^{e})ₙOR⁷, -(CR^{d}R^{e})ₙNR⁸R⁹, oxo, alkylsulfonyl, - (CR^{d}R^{e})ₙCOOR⁷ or -(CR^{d}R^{e})ₙC(O)NR⁸R⁹;
R^{a} is selected from hydrogen or alkyl;
R^{b} at each occurrence is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl and heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl or alkyl;
n is 0, 1, 2, 3 or 4 and
p is 0, 1 or 2.

According to another embodiment, the present disclosure relates to compounds of formula (I) or its tautomers, polymorphs, stereoisomers, solvate or a pharmaceutically acceptable salts thereof, wherein,
--- represents a double bond;
X is selected from O or S;
Y₁ represents N;
Y₂ represents NR⁵;
Y₃ represents N;
Y₄ represents C;
R¹ and R² are independently selected from hydrogen or alkyl;
R³ is -A-Z-B-Q;
   wherein, A is absent or is alkylene wherein one or more methylene groups is optionally replaced by hetero atoms or groups such as -O- or -N(R^{a})-;
   Z is absent or is a heterocyclyl selected from dihydrofuranyl, tetrahydrofuranyl, morpholinyl, pyrrolidinyl, dihydropyrrole, dihydropyranyl, tetrahydropyranyl, pyrazolidinyl, imidazolidinyl, dihydropyridinyl, tetrahydropyridinyl, piperidinyl, dihydropyrazinyl, tetrahydropyrazinyl, piperazinyl or dihydropyridinyl;
   wherein the heterocyclyl is unsubstituted or substituted independently with 1, 2, or 3 substituents independently selected from alkyl, -(CR^{d}R^{e})ₙOR⁷, (CR^{d}R^{e})ₙCOOR⁷, haloalkyl, perhaloalkyl, cyano or halogen;
   B is absent or is alkylene wherein one or more methylene groups is optionally replaced by hetero atoms or groups such as -O-, -N(R^{a})-, or -C(O);
   Q is selected from hydrogen, alkyl, cyclopropyl, cyclopentyl, cyclohexyl phenyl, tetrahydrofuranyl, pyrrolidinyl, tetrahydropyridinyl, tetrahydropyranyl, piperazinyl, benzodiaxolyl, tetrahydroquinolinyl, morpholinyl, tetrahydronaphthyridinyl, tetrahydrothienopyridinyl, furanyl, pyridinyl, pyrimidinyl, oxazolyl, thiazolyl, oxadiazolyl, thiadiazolyl, indolyl, quinolinyl, isoquinolinyl or benzooxazolyl;
   wherein Q is unsubstituted or substituted with 1, 2, or 3 substituents independently selected from alkyl, alkoxy, alkoxyalkyl, haloalkyl, perhaloalkyl, cyano, halogen, keto, thiocarbonyl, cyanoalkyl, -(CR^{d}R^{e})ₙOR⁷, -(CR^{d}R^{e})ₙC(O)R⁷,-(CR^{d}R^{e})ₙCOOR⁷, -(CR^{d}R^{e})ₙNR⁸R⁹, -(CR^{d}R^{e})ₙC(O)NR⁸R⁹, - (CR^{d}R^{e})ₙNR⁸C(O)OR⁷, -S(O)ₚR^{c}, -SO₃H, -S(O)₂NR^{a}R^{a}, cycloalkyl, cycloalkenyl, aryl, heterocyclyl or heteroaryl;
   wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, phenyl, naphthyl, furanyl, thiazolyl, oxazolyl, thiadiazolyl, oxadiazolyl, pyrazinyl, pyridinyl and pyrimidinyl;
   wherein R⁴ is unsubstituted or substituted with up to four substituents independently selected from alkyl, -(CR^{d}R^{e})ₙOR⁷, (CR^{d}R^{e})ₙCOOR⁷, -(CR^{d}R^{e})ₙNR⁸R⁹, cyano, halogen, haloalkyl, perhaloalkyl or cycloalkyl;
R⁵ is selected from the group consisting of hydrogen, hydroxy, haloalkyl, -(CR^{d}R^{e})ₙOR⁷, - (CR^{d}R^{e})ₙCOOR⁷, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, cycloalkyl, aryl, heteroaryl or heterocyclyl;
R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or S, said ring system is further optionally substituted with 1 to 4 substituents independently selected from halo, alkyl, nitro, cyano, -(CR^{d}R^{e})ₙOR⁷, -(CR^{d}R^{e})ₙNR⁸R⁹, oxo, alkylsulfonyl, - (CR^{d}R^{e})nCOOR⁷ or -(CR^{d}R^{e})ₙC(O)NR⁸R⁹;
R^{a} is selected from hydrogen or alkyl;
R^{b} each is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl and heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4 and
p is 0, 1 or 2.

Particular embodiments of the present disclosure are compounds of formula I or its tautomers, polymorphs, stereoisomers, solvate or a pharmaceutically acceptable salts thereof, selected from the group consisting of,
5-Amino-8-(2-furyl)-3-[2-[4-[4-(2-methoxyethoxy)phenyl]piperazin-1-yl]ethyl]-1-methyl-[1,2,4]triazolo[5,1-f]purin-2-one,
5-Amino-8-(2-furyl)-3-(2-hydroxyethyl)-1-methyl-[1,2,4]triazolo[5,1-f]purin-2-one,
5-Amino-3-[2-[4-(2,4-difluorophenyl)piperazin-1-yl]ethyl]-8-(2- furyl)-1-methyl-[1,2,4]triazolo[5,1-f]purin-2-one,
5-Amino-8-(2-furyl)-3-[2-[4-(4-methoxyphenyl)piperazin-1-yl]ethyl]-1-methyl-[1,2,4]triazolo[5,1-f]purin-2-one,
5-Amino-8-(2-furyl)-1-methyl-3-(2-morpholinoethyl)-[1,2,4]triazolo[5,1-f]purin-2-one,
5-Amino-3-[2-[4-(2,4-difluorophenyl)-1-piperidyl]ethyl]-8-(2-furyl)-1-methyl- [1,2,4]triazolo[5,1-f]purin-2-one,
5-Amino-8-(2-furyl)-1-methyl-3-[2-[4-(5-methyl-2-pyridyl)piperazin-1-yl]ethyl]-[1,2,4]triazolo[5,1-f]purin-2-one,
5-Amino-8-(2-furyl)-1-methyl-3-[2-[4-(p-tolyl)piperazin-1-yl]ethyl]-[1,2,4]triazolo[5,1-f]purin-2-one,
5-Amino-8-(2-furyl)-1-methyl-3-[2-[4-(3-methyl-2-oxo-butyl)piperazin-1-yl]ethyl]-[1,2,4]triazolo[5,1-f]purin-2-one.
5-Amino-3-[2-[4-(2-fluoro-4-methoxy-phenyl)piperazin-1-yl]ethyl]-8-(2-furyl)-1-methyl-[1,2,4]triazolo[5,1-f]purin-2-one,
5-Amino-8-(2-furyl)-3-[2-[4-[4-(2-methoxy-1,1-dimethyl-ethoxy)phenyl]piperazin-1-yl]ethyl]-1-methyl-[1,2,4]triazolo[5,1-f]purin-2-one,
5-Amino-8-(2-furyl)-3-[2-[4-(6-methoxy-3-pyridyl)piperazin-1-yl]ethyl]-1-methyl-[1,2,4]triazolo[5,1-f]purin-2-one,
5-Amino-3-[2-[4-[3-fluoro-4-(2-methoxyethoxy)phenyl]piperazin-1-yl]ethyl]-8-(2-furyl)-1-methyl-[1,2,4]triazolo[5,1-f]purin-2-one,
5-Amino-8-(2-furyl)-3-[2-[4-[4-(1-hydroxy-1-methyl-ethyl)phenyl]piperazin-1-yl]ethyl]-1-methyl-[1,2,4]triazolo[5,1-f]purin-2-one,
5-Amino-3-[2-[4-(4-fluorophenyl)-4-hydroxy-1-piperidyl]ethyl]-8-(2-furyl)-1-methyl-[1,2,4]triazolo[5,1-f]purin-2-one,
5-Amino-8-(2-furyl)-3-[2-[4-[4-(2-methoxy-2-methyl-propoxy)phenyl]piperazin-1-yl]ethyl]-1-methyl-[1,2,4]triazolo[5,1-f]purin-2-one,
5-Amino-3-[2-[4-[4-(cyclopropoxy)phenyl]piperazin-1-yl]ethyl]-8-(2-furyl)-1-methyl-[1,2,4]triazolo[5,1-f]purin-2-one,
5-Amino-3-[2-[4-(4-fluorophenyl)-3,6-dihydro-2H-pyridin-1-yl]ethyl]-8-(2- furyl)-1-methyl-[1,2,4]triazolo[5,1-f]purin-2-one,
5-Amino-8-(2-furyl)-3-[2-[4-hydroxy-4-(4-methoxyphenyl)-1-piperidyl]ethyl]-1-methyl-[1,2,4]triazolo[5,1-f]purin-2-one,
5-Amino-3-[2-[4-[3,5-difluoro-4-(2-methoxyethoxy)phenyl]piperazin-1-yl]ethyl]-8-(2-furyl)-1-methyl-[1,2,4]triazolo[5,1-f]purin-2-one,
5-Amino-3-[2-[4-[2,5-difluoro-4-(2-methoxyethoxy)phenyl]piperazin-1-yl]ethyl]-8-(2-furyl)-1-methyl-[1,2,4]triazolo[5,1-f]purin-2-one,
5-Amino-3-[2-[4-(2,2-difluoro-1,3-benzodioxol-5-yl)piperazin-1-yl]ethyl]-8-(2-furyl)-1-methyl-[1,2,4]triazolo[5,1-f]purin-2-one,
5-Amino-8-(2-furyl)-3-[2-[4-[4-(2-methoxyethoxy)phenyl]-3,3-dimethyl-piperazin-1-yl]ethyl]-1-methyl-[1,2,4]triazolo[5,1-f]purin-2-one,
5-Amino-3-[2-(4-butylpiperazin-1-yl)ethyl]-8-(2-furyl)-1-methyl-[1,2,4]triazolo[5,1-f]purin-2-one,
5-Amino-8-(2-furyl)-3-[2-(4-hydroxy-4-methyl-1-piperidyl)ethyl]-1-methyl-[1,2,4]triazolo[5,1-f]purin-2-one,
5-Amino-3-[2-[4-[4-[2-(cyclopropoxy)ethoxy]phenyl]piperazin-1-yl]ethyl]-8-(2-furyl)-1-methyl-[1,2,4]triazolo[5,1-f]purin-2-one,
5-Amino-8-(2-furyl)-3-[2-[4-[(4-methoxyphenyl)methyl]piperazin-1-yl]ethyl]-1-methyl-[1,2,4]triazolo[5,1-f]purin-2-one,
5-Amino-8-(2-furyl)-3-[2-[4-[[4-(2-methoxyethoxy)phenyl]methyl]piperazin-1-yl]ethyl]-1-methyl-[1,2,4]triazolo[5,1-f]purin-2-one,
5-Amino-8-(2-furyl)-3-[(4-methoxyphenyl)methyl]-1-methyl-[1,2,4]triazolo[5,1-f]purin-2-one,
5-Amino-8-(2-furyl)-3-[2-[4-(4-methoxyphenyl)piperazin-1-yl]ethyl]-1-methyl-[1,2,4]triazolo[5,1-f]purin-2-one,
5-Amino-8-(2-furyl)-3-[2-[4-[3-(2-methoxyethoxy)phenyl]piperazin-1-yl]ethyl]-1-methyl-[1,2,4]triazolo[5,1-f]purin-2-one,
5-Amino-3-[2-[4-[2-fluoro-4-(2-methoxyethoxy)phenyl]piperazin-1-yl]ethyl]-8-(2-furyl)-1-methyl-[1,2,4]triazolo[5,1-f]purin-2-one,
4-[4-[2-[5-Amino-8-(2-furyl)-1-methyl-2-oxo-[1,2,4]triazolo[5,1-f]purin-3yl]ethyl]piperazin-1-yl]benzonitrile,
4-[4-[2-[5-Amino-8-(2-furyl)-1-methyl-2-oxo-[1,2,4]triazolo[5,1-f]purin-3-yl]ethyl]piperazin-1-yl]-2-fluoro-benzonitrile,
5-Amino-8-(2-furyl)-1-methyl-3-[2-[4-[4-(trifluoromethyl)phenyl]piperazin-1-yl]ethyl]-[1,2,4]triazolo[5,1-f]purin-2-one,
5-Amino-8-(2-furyl)-1-methyl-3-[2-[4-[4-(trifluoromethyl)thiazol-2-yl]piperazin-1-yl]ethyl]-[1,2,4]triazolo[5,1-f]purin-2-one,
5-Amino-3-[2-[4-(cyclopropylmethyl)piperazin-1-yl]ethyl]-8-(2-furyl)-1-methyl-[1,2,4]triazolo[5,1-f]purin-2-one,
5-Amino-3-[2-(4-ethylpiperazin-1-yl)ethyl]-8-(2-furyl)-1-methyl-[1,2,4]triazolo[5,1-f]purin-2-one,
4-[2-[5-Amino-8-(2-furyl)-1-methyl-2-oxo-[1,2,4]triazolo[5,1-f]purin-3-yl]ethyl]-N,N-dimethyl-piperazine-1-sulfonamide,
5-Amino-8-(2-furyl)-1-methyl-3-[2-[4-(4-tetrahydrofuran-3-yloxyphenyl)piperazin-1-yl]ethyl]-[1,2,4]triazolo[5,1-f]purin-2-one,
5-Amino-8-(2-furyl)-1-methyl-3-[2-[4-(4-tetrahydropyran-4-yloxyphenyl)piperazin-1-yl]ethyl]-[1,2,4]triazolo[5,1-f]purin-2-one,
5-Amino-8-(2-furyl)-1-methyl-3-[2-[4-[4-(tetrahydrofuran-2-ylmethoxy)phenyl]piperazin-1-yl]ethyl]-[1,2,4]triazolo[5,1-f]purin-2-one,
5-Amino-8-(2-furyl)-1-methyl-3-[2-(3-methyl-7,8-dihydro-8H-1,6-naphthyridin-6-yl)ethyl]-[1,2,4]triazolo[5,1-f]purin-2-one,
5-Amino-3-[2-(6,7-dihydro-4H-thieno[3,2-c]pyridin-5-yl)ethyl]-8-(2-furyl)-1-methyl-[1,2,4]triazolo[5,1-f]purin-2-one,
5-Amino-8-(2-furyl)-3-[2-[4-[4-(2-methoxyethoxy)phenyl]piperazin-1-yl]propyl]-1-methyl-[1,2,4]triazolo[5,1-f]purin-2-one,
5-Amino-3-[2-[3-(4-fluorophenyl)-2,5-dihydropyrrol-1-yl]ethyl]-8-(2-furyl)-1-methyl-[1,2,4]triazolo[5,1-f]purin-2-one,
5-Amino-3-[2-[4-[4-(2-methoxyethoxy)phenyl]piperazin-1-yl]ethyl]-1-methyl-8-(5-methyl-2-furyl)-[1,2,4]triazolo[5,1-f]purin-2-one,
5-Amino-8-(5-cyclopropyl-2-furyl)-3-[2-[4-[4-(2-methoxyethoxy)phenyl]piperazin-1-yl]ethyl]-1-methyl-[1,2,4]triazolo[5,1-f]purin-2-one,
5-Amino-3-[2-(2,4-difluoroamlino)ethyl]-8-(2-furyl)-1-methyl-[1,2,4]triazolo[5,1-f]purin-2-one,
5-Amino-3-[3-[4-(4-fluorophenyl)piperazin-1-yl]propyl]-8-(2-furyl)-1-methyl-[1,2,4]triazolo[5,1-f]purin-2-one,
5-Amino-8-(2-furyl)-3-[3-[4-[4-(2-methoxyethoxy)phenyl]piperazin-1-yl]propyl]-1-methyl-[1,2,4]triazolo[5,1-f]purin-2-one,
5-Amino-8-(2-furyl)-3-[2-[4-(4-methoxyphenyl)-3,6-dihydro-2H-pyridin-1-yl]ethyl]-1-methyl-[1,2,4]triazolo[5,1-f]purin-2-one,
5-Amino-8-(2-furyl)-3-[2-[4-[4-(2-methoxy-1,1-dimethyl-ethyl)phenyl]piperazin-1-yl]ethyl]-1-methyl-[1,2,4]triazolo[5,1-f]purin-2-one,
5-Amino-8-(2-furyl)-1-methyl-3-(2-piperazin-1-ylethyl)-[1,2,4]triazolo[5,1-f]purin-2-one,
5-Amino-8-(2-furyl)-3-[2-[4-(1H-indole-2-carbonyl)piperazin-1-yl]ethyl]-1-methyl-[1,2,4]triazolo[5,1-f]purin-2-one,
5-Amino-8-(2-furyl)-3-[2-(4-isopropoxyphenyl)ethyl]-1-methyl-[1,2,4]triazolo[5,1-f]purin-2-one,
5-Amino-8-(2-furyl)-1-methyl-3-[2-[4-[(2S)-pyrrolidine-2-carbonyl]piperazin-1-yl]ethyl]-[1,2,4]triazolo[5,1-f]purin-2-one,
5-Amino-8-(2-furyl)-3-[2-(4-methoxyphenyl)ethyl]-1-methyl-[1,2,4]triazolo[5,1-f]purin-2-one,
5-amino-3-[2-[4-[4-(difluoromethoxy)phenyl]piperazin-1-yl]ethyl]-8-(2-furyl)-1-methyl-[1,2,4]triazolo[5,1-f]purin-2-one,
5-Amino-8-(2-furyl)-1-methyl-3-[2-[4-[3-(5-methyl-1,3,4-oxadiazol-2-yl)phenyl]piperazin-1-yl]ethyl]-[1,2,4]triazolo[5,1-f]purin-2-one,
5-Amino-3-[2-[4-[2-fluoro-4-(5-methyl-1,2,4-oxadiazol-3-yl)phenyl]piperazin-1-yl]ethyl]-8-(2-furyl)-1-methyl-[1,2,4]triazolo[5,1-f]purin-2-one,
5-Amino-3-[2-[4-(6-fluoro-2-methyl-1,3-benzoxazol-5-yl)piperazin-1-yl]ethyl]-8-(2-furyl)-1-methyl-[1,2,4]triazolo[5,1-f]purin-2-one,
5-Amino-3-[2-[4-(cyclopropanecarbonyl)piperazin-1-yl]ethyl]-8-(2-furyl)-1-methyl-[1,2,4]triazolo[5,1-f]purin-2-one,
5-Amino-3-[2-[4-(2-cyclopropylacetyl)piperazin-1-yl] ethyl]-8-(2-furyl)-1-methyl-[1,2,4]triazolo[5,1-f]purin-2-one,
5-Amino-8-(2-furyl)-3-[2-[4-[4-(2-hydroxyethoxy)phenyl]piperazin-1-yl]ethyl]-1-methyl-[1,2,4]triazolo[5,1-f]purin-2-one,
5-Amino-8-(2-furyl)-3-[2-[4-(4-hydroxyphenyl)piperazin-1-yl]ethyl]-1-methyl-[1,2,4]triazolo[5,1-f]purin-2-one,
5-Amino-1-(cyclopropylmethyl)-3-[2-[4-(4-ethoxyphenyl)piperazin-1-yl]ethyl]-8-(2-furyl)-[1,2,4]triazolo[5,1-f]purin-2-one,
5-Amino-1-(cyclopropylmethyl)-3-[2-[4-(4-fluorophenyl)piperazin-1-yl]ethyl]-8-(2-furyl)-[1,2,4]triazolo[5,1-f]purin-2-one,
5-Amino-1-(cyclopropylmethyl)-3-[2-[4-(2,4-difluorophenyl)piperazin-1-yl]ethyl]-8-(2-furyl)-[1,2,4]triazolo[5,1-f]purin-2-one,
5-Amino-1-(cyclopropylmethyl)-8-(2-furyl)-3-[2-[4-[4-(2-methoxyethoxy)phenyl]piperazin-1-yl]ethyl]-[1,2,4]triazolo[5,1-f]purin-2-one,
5-Amino-1-(cyclopropylmethyl)-3-[2-(4-fluorophenoxy)ethyl]-8-(2-furyl)-[1,2,4]triazolo[5,1-f]purin-2-one,
5-Amino-8-(2-furyl)-1-methyl-3-[2-[2-oxo-5-(trifluoromethyl)-1-pyridyl]ethyl]-[1,2,4]triazolo[5,1-f]purin-2-one,
5-Amino-3-[2-[4-(2,4-difluorophenyl)pyrazol-1-yl]ethyl]-1-ethyl-8-(2-furyl)-[1,2,4]triazolo[5,1-f]purin-2-one,
1-[2-[5-Amino-1-(cyclopropylmethyl)-8-(2-furyl)-2-oxo-[1,2,4]triazolo[5,1-f]purin-3-yl]ethyl]pyrazole-4-carboxylic acid,
1-[2-[5-Amino-8-(2-furyl)-1-methyl-2-oxo-[1,2,4]triazolo[5,1-f]purin-3-yl]ethyl]pyrazole-4-carboxylic acid,
1-[2-[5-amino-1-(cyclopropylmethyl)-8-(2-furyl)-2-oxo-[1,2,4]triazolo[5,1-f]purin-3-yl]ethyl]-N-cyclopropyl-pyrazole-4-carboxamide,
1-[2-[5-amino-1-(cyclopropylmethyl)-8-(2-furyl)-2-oxo-[1,2,4]triazolo[5,1-f]purin-3-yl]ethyl]-N,N-diethyl-pyrazole-4-carboxamide,
1-[2-[5-Amino-1-(cyclopropylmethyl)-8-(2-furyl)-2-oxo-[1,2,4]triazolo[5,1-f]purin-3-yl]ethyl]-N-cyclopropyl-5-methyl-pyrazole-3-carboxamide,
2-[2-[5-Amino-1-(cyclopropylmethyl)-8-(2-furyl)-2-oxo-[1,2,4]triazolo[5,1-f]purin-3-yl]ethyl]-N-cyclopropyl-5-methyl-pyrazole-3-carboxamide,
1-[2-[5-Amino-8-(2-furyl)-1-methyl-2-oxo-[1,2,4]triazolo[5,1-f]purin-3-yl]ethyl]-N-methylpyrazole-3-carboxamide,
1-[2-[5-Amino-8-(2-furyl)-1-methyl-2-oxo-[1,2,4]triazolo[5,1-f]purin-3-yl]ethyl]-N,N-diethyl-pyrazole-4-carboxamide,
1-[2-[5-amino-8-(2-furyl)-1-methyl-2-oxo-[1,2,4]triazolo[5,1-f]purin-3-yl]ethyl]pyrazole-4-carboxamide,
5-Amino-8-(2-furyl)-3-[2-[4-[(3R)-3-hydroxypyrrolidine-1-carbonyl]pyrazol-1-yl]ethyl]-1-methyl-[1,2,4]triazolo[5,1-f]purin-2-one,
1-[2-[5-Amino-8-(2-furyl)-1-methyl-2-oxo-[1,2,4]triazolo[5,1-f]purin-3-yl]ethyl]-N-methylpyrazole-4-carboxamide,
1-[2-[5-Amino-8-(2-furyl)-1-methyl-2-oxo-[1,2,4]triazolo[5,1-f]purin-3-yl]ethyl]-N-cyclopropyl-pyrazole-3-carboxamide,
1-[2-[5-Amino-8-(2-furyl)-1-methyl-2-oxo-[1,2,4]triazolo[5,1-f]purin-3-yl]ethyl]-N-cyclopropyl-pyrazole-4-carboxamide,
5-Amino-8-(2-furyl)-3-[2-[4-(3-hydroxyazetidine-1-carbonyl)pyrazol-1-yl]ethyl]-1-methyl-[1,2,4]triazolo[5,1-f]purin-2-one,
5-Amino-1-ethyl-8-(2-furyl)-3-[2-[4-[4-(2-methoxyethoxy)phenyl]piperazin-1-yl]ethyl]-[1,2,4]triazolo[5,1-f]purin-2-one,
5-Amino-3-[2-[4-(2,4-difluorophenyl)piperazin-1-yl]ethyl]-1-ethyl-8-(2-furyl)-[1,2,4]triazolo[5,1-f]purin-2-one,
5-Amino-1-ethyl-3-{2-[4-(4-fluoro-phenyl)-piperidin-1-yl]-ethyl}-8-furan-2-yl-1,3-dihydro-[1,2,4]triazolo[5,1-f]purin-2-one,
5-Amino-1-ethyl-8-(2-furyl)-3-[2-(3-methyl-7,8-dihydro-5H-1,6-naphthyridin-6-yl)ethyl]-[1,2,4]triazolo[5,1-f]purin-2-one,
5-Amino-8-(2-furyl)-3-[2-[4-[4-(2-methoxyethoxy)phenyl]piperazin-1-yl]ethyl]-1-(2,2,2-trifluoroethyl)-[1,2,4]triazolo[5,1-f]purin-2-one,
5-Amino-3-{2-[4-(2,4-difluoro-phenyl)-piperazin-1-yl]-ethyl}-8-furan-2-yl-1-(2,2,2-trifluoro-ethyl)-1,3-dihydro-[1,2,4]triazolo[5,1-f]purin-2-one,
5-Amino-8-(2-furyl)-3-[2-[4-[4-(2-methoxyethoxy)phenyl]piperazin-1-yl]ethyl]-1-(2-methoxyethyl)-[1,2,4]triazolo[5,1-f]purin-2-one,
5-amino-3-[2-[4-(4-fluorophenyl)piperazin-1-yl] ethyl]-8-(2-furyl)-1-(2-methoxyethyl)-[1,2,4]triazolo[5,1-f]purin-2-one,
5-Amino-3-[2-[4-(4-fluorophenyl)piperazin-1-yl] ethyl]-8-(2-furyl)-1-(2-hydroxyethyl)-[1,2,4]triazolo[5,1-f]purin-2-one one,
5-Amino-1-cyclopropyl-8-(2-furyl)-3-[2-[4-[4-(2-methoxyethoxy)phenyl]piperazin-1-yl]ethyl]-[1,2,4]triazolo[5,1-f]purin-2-one,
5-Amino-8-(2-furyl)-3-[2-[4-[4-(2-methoxyethoxy)phenyl]piperazin-1-yl]ethyl]-1-(2,2,2-trifluoroethyl)-[1,2,4]triazolo[5,1-f]purin-2-one,
5-Amino-3-[2-[4-[4-(2-methoxyethoxy)phenyl]piperazin-1-yl]ethyl]-1-methyl-8-thiazol-2-yl-[1,2,4]triazolo[5,1-f]purin-2-one,
5-Amino-3-[2-[4-[3-fluoro-4-(2-methoxyethoxy)phenyl]piperazin-1-yl]ethyl]-1-methyl-8-thiazol-2-yl-[1,2,4]triazolo[5,1-f]purin-2-one,
5-Amino-3-[2-[4-(2-cyclopropylacetyl)piperazin-1-yl]ethyl]-1-methyl-8-thiazol-2-yl-[1,2,4]triazolo[5,1-f]purin-2-one,
5-Amino-3-[2-[4-(4-methoxyphenyl)piperazin-1-yl]ethyl]-1-methyl-8-thiazol-2-yl-[1,2,4]triazolo[5,1-f]purin-2-one,
5-Amino-1-methyl-3-[2-[4-(p-tolyl)piperazin-1-yl]ethyl]-8-thiazol-2-yl-[1,2,4]triazolo[5,1-f]purin-2-one,
5-Amino-1-methyl-3-[2-(3-methyl-7,8-dihydro-5H-1,6-naphthyridin-6-yl)ethyl]-8-thiazol-2-yl-[1,2,4]triazolo[5,1-f]purin-2-one,
4-[4-[2-(5-Amino-1-methyl-2-oxo-8-thiazol-2-yl-[1,2,4]triazolo[5,1-f]purin-3-yl)ethyl]piperazin-1-yl]benzonitrile,
5-Amino-1-methyl-3-[2-[4-[3-(5-methyl-1,3,4-oxadiazol-2-yl)phenyl]piperazin-1-yl]ethyl]-8-thiazol-2-yl-[1,2,4]triazolo[5,1-f]purin-2-one,
5-amino-3-[2-[4-[4-(1-hydroxy-1-methyl-ethyl)phenyl]piperazin-1-yl]ethyl]-1-methyl-8-thiazol-2-yl-[1,2,4]triazolo[5,1-f]purin-2-one,
5-Amino-3-[2-[4-[4-(2-methoxyethoxy)phenyl]piperazin-1-yl]ethyl]-1-methyl-8-(2-pyridyl)-[1,2,4]triazolo[5,1-f]purin-2-one,
5-Amino-3-[2-[4-(2,4-difluorophenyl)piperazin-1-yl]ethyl]-1-methyl-8-(2-pyridyl)-[1,2,4]triazolo[5,1-f]purin-2-one,
4-[4-[2-[5-Amino-1-methyl-2-oxo-8-(2-pyridyl)-[1,2,4]triazolo[5,1-f]purin-3-yl]ethyl]piperazin-1-yl]benzonitrile,
5-Amino-3-[2-[4-[4-(1-hydroxy-1-methyl-ethyl)phenyl]piperazin-1-yl]ethyl]-1-methyl-8-(2-pyridyl)-[1,2,4]triazolo[5,1-f]purin-2-one,
5-Amino-3-[2-[4-[4-(2-methoxyethoxy)phenyl]piperazin-1-yl]ethyl]-1-methyl-8-pyrazin-2-yl-[1,2,4]triazolo[5,1-f]purin-2-one,
5-Amino-3-[2-[4-(2,4-difluorophenyl)piperazin-1-yl]ethyl]-1-methyl-8-pyrazin-2-yl-[1,2,4]triazolo[5,1-f]purin-2-one,
5-Amino-3-[2-[4-[2-fluoro-4-(2-methoxyethoxy)phenyl]piperazin-1-yl]ethyl]-1-methyl-8-pyrazin-2-yl-[1,2,4]triazolo[5,1-f]purin-2-one,
5-Amino-8-(2-furyl)-3-[[1-(4-methoxyphenyl)pyrrolidin-3-yl]methyl]-1-methyl-[1,2,4]triazolo[5,1-f]purin-2-one,
5-Amino-8-(2-furyl)-3-[[1-[4-(2-methoxyethoxy)phenyl]pyrrolidin-3-yl]methyl]-1-methyl-[1,2,4]triazolo[5,1-f]purin-2-onehyl}-1-methyl-1,3-dihydro-[1,2,4]triazolo[5,1-i]purin-2-one,
5-amino-8-(2-furyl)-3-[2-[4-[4-(2-methoxyethoxy)phenyl]piperazin-1-yl]ethyl]-1-methyl-[1,2,4]triazolo[5,1-f]purine-2-thione, and
8-(2-furyl)-3-[2-[4-[4-(2-methoxyethoxy)phenyl]piperazin-1-yl]ethyl]-1-methyl-5-(methylamino)-[1,2,4]triazolo[5,1-f]purin-2-one.

Another embodiment of the present disclosure relates to a compound of formula (I) or its tautomers, polymorphs, stereoisomers, solvate or a pharmaceutically acceptable salts thereof, for treating disease or disorder susceptible to improvement by antagonism of A_{2A} receptor.

Yet another embodiment of the present disclosure relates to a compound of formula (I) or its tautomers, polymorphs, stereoisomers, solvate or a pharmaceutically acceptable salts thereof, for treating. Parkinsons disease, restless leg syndrome, Alzheimers disease, neurodegenerative disorder, inflammation, wound healing, dermal fibrosis, nocturnal myoclonus, cerebral ischaemia, myocardial ischemia, Huntington's disease, multiple system atrophy, corticobasal degeneration, Wilson's disease or other disorders of basal ganglia which results in dyskinesias, post traumatic stress disorder, hepatic cirrhosis, sepsis, spinal cord injury, retinopathy, hypertension, social memory impairment, depression, neuroprotection, narcolepsy or other sleep related disorders, attention deficit hyperactivity disorder, drug addiction, post traumatic stress disorder and vascular injury.

The present disclosure also relates to a method of treating a disease in a mammal that is alleviable by treatment with an A_{2A} adenosine receptor antagonist comprising administering a therapeutically effective amount of the compounds of Formula I or its tautomers, polymorphs, stereoisomers, solvate or pharmaceutically acceptable salts thereof.

The present disclosure also relates to a method of treating a disease state in a mammal that is alleviable by treatment with an A_{2A} adenosine receptor antagonist comprising administering a therapeutically effective amount of the compounds of Formula I or its tautomers, polymorphs, stereoisomers, prodrugs, solvate or a pharmaceutically acceptable salts thereof, in particular treatment, prevention or suppression of diseases and disorders that may be susceptible to improvement by antagonism of the adenosine receptor, such as Parkinsons disease, restless leg syndrome, Alzheimers disease, neurodegenerative disorder, inflammation, wound healing, dermal fibrosis, nocturnal myoclonus, cerebral ischaemia, myocardial ischemia, Huntington's disease, multiple system atrophy, corticobasal degeneration, Wilson's disease or other disorders of basal ganglia which results in dyskinesias, post traumatic stress disorder, hepatic cirrhosis, sepsis, spinal cord injury, retinopathy, hypertension, social memory impairment, depression, neuroprotection, narcolepsy or other sleep related disorders, attention deficit hyperactivity disorder, drug addiction, post traumatic stress disorder and vascular injury.

The present disclosure further relates to the process of preparation of compounds of formula I or its tautomers, polymorphs, stereoisomers, solvate or pharmaceutically acceptable salts thereof.

The compounds of formula (I) is prepared as outlined in the Scheme 1 to 5 below:

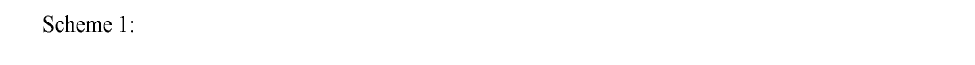

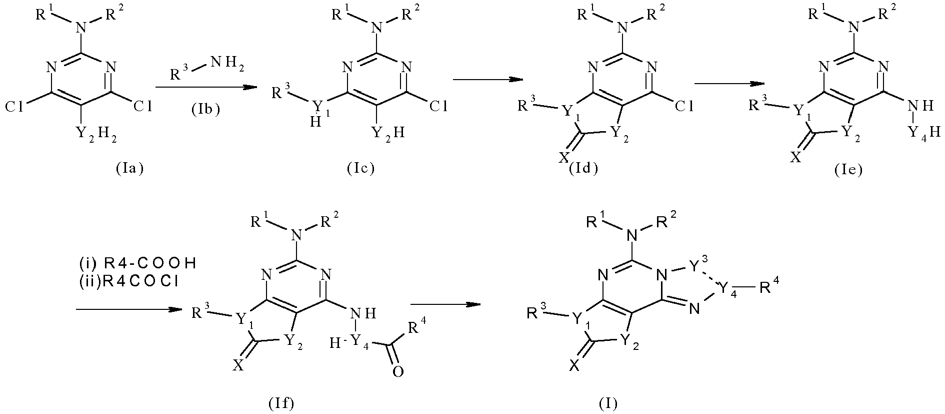

The compound of formula (Ia) wherein all symbols are defined herein above, is either available commercially or may be prepared by methods well known in the art (WO91/01310A1). The compound of formula (1a) may be reacted with amine of formula (Ib) to obtain compound of formula (Ic) wherein all symbols are defined herein above. The reaction temperature may range from 50-100 °C and the reaction time may range from 4 to 24 hours. After completion of reaction the desired product (Ic) may be isolated by conventional methods.

The compound of formula (Ic) may be cyclised to obtain compound of formula (Id) by a known method in the literature. The reaction temperature may range from 10-50°C and the reaction time may range from 4 to 48 hours. After completion of reaction the desired product (Id) may be isolated by conventional methods.

The compound of formula (Id) may be reacted with NH₂Y₄H to obtain compound of formula (Ie). The reaction temperature may range from 50-100 °C and the reaction time may range from 4 to 24 hours. After completion of reaction the desired product (Ie) may be isolated by conventional method.

The compound of formula (Ie) may be reacted with a carboxylic acid R⁴COOH wherein R⁴ is defined earlier, to yield a formula (If). The temperature may range from 20-30 °C and the reaction time may range from 4 to 24 hours. After completion of reaction the product of formula (If) may be isolated by conventional method.

The compound (If) may also be prepared from reaction of (Ie) and with an acid halide R⁴COCl to yield (If). The reaction temperature may range from 0-30 °C and the reaction time may range from 1 to 24 hours. After completion of reaction the product (If) was isolated by conventional method.

Compounds of formula (If) may be dehydrated using dehydrating reagent. The reaction temperature may range from 50-100°C and the reaction time may range from 8 to 24 hours. After completion of reaction, the desired compound of formula (I) wherein all symbols are defined herein above may be isolated by conventional methods.

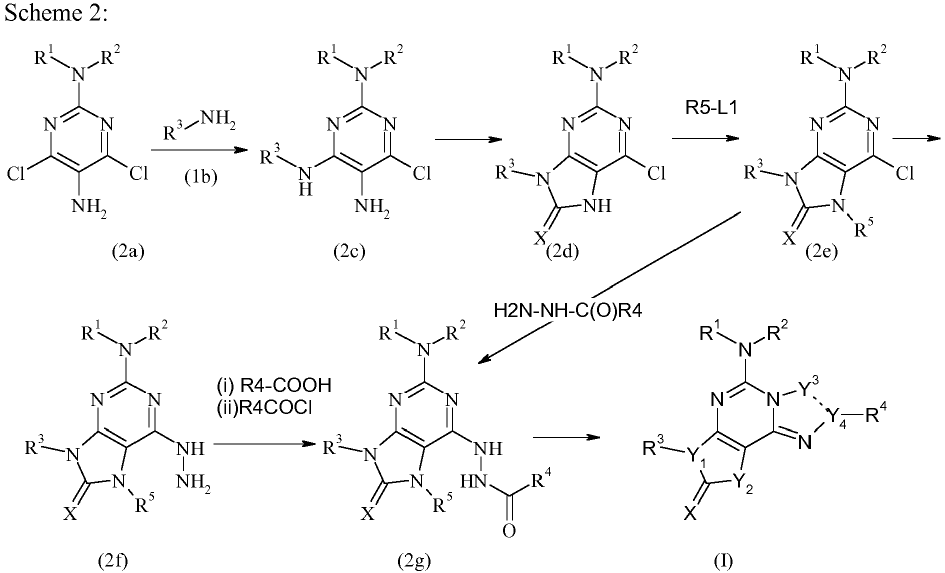

The compound of formula (2a) wherein all symbols are defined herein above, is either available commercially or may be prepared by methods well known in the art (WO91/01310A1). The compound of formula (2a) may be reacted with amine of formula (2b) without or with solvent such as methanol, ethanol, isopropanol or polar solvent such as THF, acetone, DMF and non polar solvent such as toluene, xylene in the presence of a base such as K₂CO₃, NaHCO₃, Et₃N, pyridine and the like, to obtain compound of formula (2c) wherein all symbols are defined herein above. The reaction temperature may range from 50-100 °C and the reaction time may range from 4 to 24 hours. After completion of reaction the desired product (2c) may be isolated by conventional methods.

The compound of formula (2c) may be cyclised to obtain compound of formula (2d) by a known method in the literature. In general the formation of compound (2c) is achieved by using CDI, phosgene, triphosgene, 4-nitriphenyl chloroformate, thiophosgene and the like in the presence of a solvent such as DCM, THF, DMF, ACN, and the like and a base such as K₂CO₃, NaHCO₃, Et3N, pyridine and the like. The reaction temperature may range from 10-50 °C and the reaction time may range from 4 to 48 hours. After completion of reaction the desired product (2d) may be isolated by conventional methods. The compound of formula (2d) may be reacted with formula R⁵-L1 where in R⁵ is as defined above and L1 is a leaving group such as mesylate, tosylate or halo such as bromine, chlorine or iodine and base such as K₂CO₃, Cs₂CO₃ or NaH in an inert solvent such as DMF, DMA to obtain compound of formula (2e) .The reaction temperature may range from 25-60 °C and the reaction time may range from 4 to 24 hours. After completion of reaction the desired product (2e) may be isolated by conventional method.

The compound of formula (2e) may be reacted with hydrazine hydrate without solvent or in a solvent such as methanol, ethanol, isopropanol or polar solvent such as THF, acetone, DMF and non polar solvent such as toluene, xylene and the like, in the absence or presence of a base such as DIPEA, Na₂CO₃ and the like to obtain compound of formula (2f). The reaction temperature may range from 50-100 °C and the reaction time may range from 4 to 24 hours. After completion of reaction the desired product (2f) may be isolated by conventional method.

The compound of formula (2f) may be reacted with a carboxylic acid R⁴COOH wherein R⁴ is defined earlier, to yield a formula (2g). The reaction may be carried out with a suitable coupling agent such as EDCI, DCC, HBTU without base or in the presence of a base such as Et₃N, N-methyl morfoline, N-methyl pyrrolidnone and the like and a solvent such as methanol, ethanol, propanol etc or aprotic solvent such as DMF, CH₂Cl₂. The temperature may range from 20-30 °C and the reaction time may range from 4 to 24 hours. After completion of reaction the product of formula (2g) may be isolated by conventional method. The compound (2g) may also be prepared from reaction of (2f) and with an acid halide R⁴COCl to yield (2g). The acylation reaction may be carried out in the presence of base such as Et₃N, DIPEA, Pyridine and the like in solvent like DCM, DCE and the like. The reaction temperature may range from 0-30 °C and the reaction time may range from 1 to 24 hours. After completion of reaction the product (2g) was isolated by conventional method. Alternatively, compound of formula (2g) may be obtained directly from (2e) by reaction with an appropriate hydrazide of formula H2N-NH-C(O)-R⁴, wherein R⁴ is defined above. The reaction may be carried out using a suitable solvent such as acetonitile, DMF and the like. The reaction temperature may range from 25-100 °C and the reaction time may range from 4 to 24 hours. After completion of reaction the desired product (2g) may be isolated by conventional methods.

Compounds of formula (2g) may be dehydrated using dehydrating reagent such as BSA or mixture of BSA and HMDS. The reaction temperature may range from 50-100°C and the reaction time may range from 8 to 24 hours. After completion of reaction, the desired compound of formula (I) wherein Y1 is N and Y2 is NR⁵ and all other symbols are defined herein above may be isolated by conventional methods.

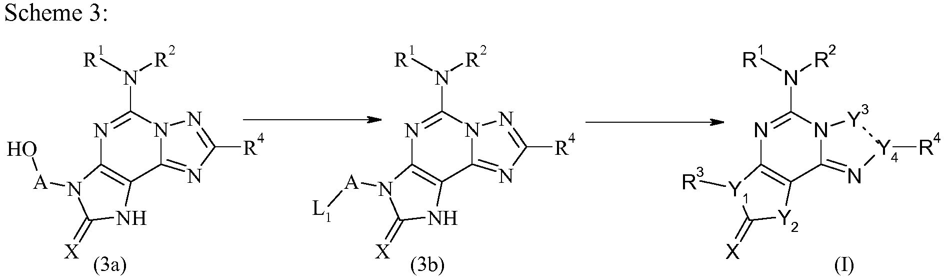

The compound of formula (3a) wherein all symbols are defined herein above may be converted to compound of formula (3b) wherein L1 is leaving group such as tosylate, mesylate, chloride, bromide or iodide and the like and all other symbols are defined herein above, by a suitable method or method known in the literature. The compound of formula (3b) is then reacted with Q-B-ZH, wherein Q, B and Z are defined herein above, in a solvent such as DMF, ACN and the like in presence of a base such as diisopropyl ethyl amine, Et₃N, K₂CO₃,Cs₂CO₃, NaH and the like or without base. The reaction temperature may range from 50-100 °C and the reaction time may range from 2 to 24 hours. After completion of reaction, the desired product (I) wherein all symbols are defined herein above may be isolated by conventional method.

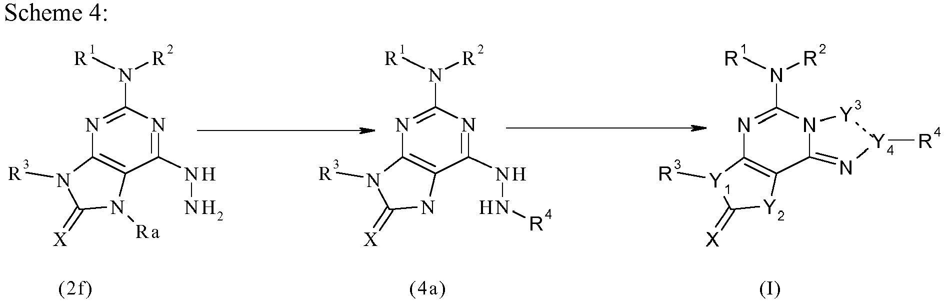

The compound of formula (2f) may be reacted with R⁴CHO in presence of catalytic amount of acid such as acetic acid or without acid followed by reduction with suitable reducing agent such as sodium cyanoborohydride, sodium triacetoxy borohydride and the like to obtain compound of formula (4a).The organic solvent to be used in the present method may be alcohol such as methanol, ethanol or DCM, dichloroethane. The reaction temperature may range from 0-40 °C and the reaction time may range from 4 to 24 hours. After completion of reaction the desired product (4a) may be isolated by conventional method. Compounds of formula (4a) may be treated with phosgene to provide compounds of formula (I) wherein Y³ is C(=O), Y¹ is N and all other symbols are defined herein above.

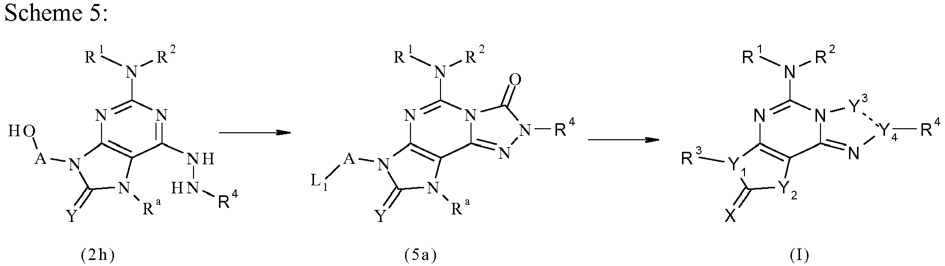

The compound of formula (2h) wherein all symbols are defined herein above may be converted to compounds of formula (5a) wherein L1 is a leaving group such as tosylate, mesylate, chloride, bromide or iodide and the like, or is a suitable hydroxyl protecting group such as a trialkylsilyl group (e.g., t-butyldimethylsilyl group) and all other symbols are defined herein above, by a suitable method or method known in the literature. The compound of formula (5a) may be reacted with Q-B-ZH, wherein Q, B and Z are defined herein above, in a solvent such as DMF, ACN and the like in presence of a base such as diisopropyl ethyl amine, Et₃N, K₂CO₃,Cs₂CO₃, NaH and the like or without base. The reaction temperature may range from 50-100°C and the reaction time may range from 2 to 24 hours. After completion of reaction, the desired product (I) wherein all symbols are defined herein above may be isolated by conventional methods.

Wherever desired or necessary, in any of the above mentioned processes, functional groups is transformed to different functional groups such as an ester function being converted to an acid, amide, hydroxymethyl, keto, aldehyde as well as an ester. The said conversions are carried out using reagents and conditions well documented in the literature.

Wherever desired or necessary, in any of the above mentioned processes, any of the compounds of formula (I) is converted into a pharmaceutically acceptable salt or vice versa or converting one salt form into another pharmaceutically acceptable salt form.

According to an embodiment, the compounds of the present disclosure are adenosine A_{2A} receptor antagonists. Thus, the present disclosure provides a method for the modulation of adenosine A_{2A} receptor activity in mammals which method comprises administering to a mammal in need thereof a therapeutically effective amount of compound of formula (I) or its tautomers, polymorphs, stereoisomers or a pharmaceutically acceptable salts thereof.

As used throughout the specification and in the claims, the term "treatment" embraces all the different forms or modes of treatment as known to those of the pertinent art and in particular includes preventive, curative, delay of progression and palliative treatment.

The term "therapeutically effective amount" as used herein refers to an amount of a drug or a therapeutic agent that will elicit the desired biological or medical response of a tissue, system or an animal (including man) that is being sought by a researcher or clinician.

The term "mammal" or "patient" are used interchangeably herein and include, but are not limited to, humans, dogs, cats, horses, pigs, cows, sheep, monkeys, rabbits, mice and laboratory animals The preferred mammals are humans.

An embodment of the present disclosure relates to a pharmaceutical composition comprising , as an active ingredient, at least one compound of formula (I) or its tautomers, polymorphs, stereoisomers or a pharmaceutically acceptable salts thereof, together with one or more pharmaceutically acceptable carriers or excipients.

The present disclosure further provides pharmaceutical compositions comprising a therapeutically effective amount of a compound of the present disclosure, alone or in combination with one or more pharmaceutically acceptable carriers.

The pharmaceutical compositions according to the present disclosure are those suitable for enteral, such as oral or rectal, transdermal and parenteral administration to mammals, including man, for the treatment of conditions mediated by the adenosine A_{2A} receptor. Such conditions include, but are not limited to, Parkinsons disease, restless leg syndrome, Alzheimers disease, neurodegenerative disorder, inflammation, wound healing, dermal fibrosis, nocturnal myoclonus, cerebral ischaemia, myocardial ischemia, Huntington's disease, multiple system atrophy, corticobasal degeneration, Wilson's disease or other disorders of basal ganglia which results in dyskinesias, post traumatic stress disorder, hepatic cirrhosis, sepsis, spinal cord injury, retinopathy, hypertension, social memory impairment, depression, neuroprotection, narcolepsy or other sleep related disorders, attention deficit hyperactivity disorder, drug addiction, post traumatic stress disorder and vascular injury.

Generally, the concentration of the compound(s) of the present disclosure in a liquid composition, such as a lotion, will be from about 0.01- about 25 wt %, preferably from about 0.1- about 10 wt %. The concentration in a semi-solid or a solid composition such as a gel or a powder will be about 0.1- about 5 wt %, preferably about 0.5- about 25 wt %.

The amount of a compound of the present disclosure required for use in treatment will vary not only with the particular salt selected but also with the route of administration, the nature of the condition being treated and the age and condition of the patient and will be ultimately at the discretion of the administering physician or clinician. In general, a suitable dose will be in the range of from about 0.001 mg/kg/day to about 20 mg/kg/day For example, a dosage may be from about 0.002 mg/kg to about 10 mg/kg of body weight per day, from about 0.01 mg/kg/day to about 1 mg/kg/day, and from about 0.1 mg/kg/day to about 5 mg/kg/day.

The compound is conveniently administered in unit dosage form, e.g, containing 5 to 1000 µg, about 10 to about 750 µg, about 50 to about 500 µg of active ingredient per unit dosage form.

The desired dose may conveniently be presented in a single dose or as divided doses administered at appropriate intervals, for example, as two, three, four or more sub-doses per day. The sub-dose itself may be further divided, e g, into a number of discrete loosely spaced administrations; such as multiple inhalations from an insufflator or by application of a plurality of drops into the eye Dosages above or beiow the range cited herein above are within the scope of the present disclosure and may be administered to the individual patient if desired and necessary.

Accordingly, in various embodiments, the present disclosure provides pharmaceutical compositions as described above for the treatment of conditions mediated by adenosine receptor, such as Parkinsons disease, restless leg syndrome, Alzheimers disease, neurodegenerative disorder, inflammation, wound healing, dermal fibrosis, nocturnal myoclonus, cerebral ischaemia, myocardial ischemia, Huntington's disease, multiple system atrophy, corticobasal degeneration, Wilson's disease or other disorders of basal ganglia which results in dyskinesias, post traumatic stress disorder, hepatic cirrhosis, sepsis, spinal cord injury, retinopathy, hypertension, social memory impairment, depression, neuroprotection, narcolepsy or other sleep related disorders, attention deficit hyperactivity disorder, drug addiction, post traumatic stress disorder and vascular injury.

An embodiment of the present disclosure also relates to a pharmaceutical composition comprising a compound of formula (I) or its tautomers, polymorphs, stereoisomers or a pharmaceutically acceptable salts thereof, in combination with one or more therapeutically active agents.

In various embodimemts, the present disclosure provides pharmaceutical compositions comprising a therapeutically effective amount of a compound of the disclosure in combination with a therapeutically effective amount of another therapeutic agent, preferably selected from anti-inflammatory agents, anti-diabetic agents, anti-hypertensive agents and anti-dyslipidemic agents.

According to an embodiment, the pharmaceutical compositions may contain a therapeutically effective amount of a compound of the disclosure as defined above, either alone or in a combination with another therapeutic agent, e.g., each at an effective therapeutic dose as reported in the art. Such therapeutic agents include: a) anti-inflammatory agents, such as anticholinergic or antimuscarinic agents; steroids; LTB₄ (leukotriene B₄) antagonists; dopamine receptor agonists; PDE₄ (phosphodiesterase 4) inhibitors; and beta-2 adrenergic receptor agonists; b) anti-diabetic agents, such as insulin, insulin derivatives and mimetics; insulin secretagogues; insulinotropic sulfonylurea receptor ligands; thiazolidone derivatives; GSK3 (glycogen synthase kinase-3) inhibitors; sodium-dependent glucose co-transporter inhibitors; glycogen phosphorylase A inhibitors; biguanides; alpha-glucosidase inhibitors; GLP-1 (glucagon like peptide-1), GLP-1 analogs and GLP-1 mimetics; modulators of PPARs (peroxisome proliferator-activated receptors); DPPIV (dipeptidyl peptidase IV) inhibitors; SCD-1 (stearoyl-CoA desaturase-1) inhibitors; DGAT1 and DGAT2 (diacylglycerol acyltransferase 1 and 2) inhibitors; ACC2 (acetyl CoA carboxylase 2) inhibitors; and breakers of AGE (advanced glycation end products); c) anti-hypertensive agents, such as loop diuretics; angiotensin converting enzyme (ACE) inhibitors; inhibitors of the Na-K-ATPase membrane pump such as digoxin; neutralendopeptidase (NEP) inhibitors; ACE/NEP inhibitors; angiotensin II antagonists; renin inhibitors; β-adrenergic receptor blockers; inotropic agents; calcium channel blockers; aldosterone receptor antagonists; and aldosterone synthase inhibitors; and d) anti-dyslipidemic agents such as 3-hydroxy-3-methyl-glutaryl coenzyme A (HMG-CoA) reductase inhibitors; HDL increasing compounds such as cholesterol ester transfer protein (CETP) inhibitors; squalene synthase inhibitors; FXR (farnesoid X receptor) and LXR (liver X receptor) ligands; cholestyramine; fibrates; nicotinic acid; and aspirin.

According to another embodiment, the present disclosure provides pharmaceutical compositions comprising a therapeutically effective amount of a compound of the disclosure in combination with a therapeutically effective amount of one to three other agents useful in treating Parkinson's disease in a pharmaceutically acceptable carrier.

According to yet another embodimemt, the pharmaceutical compositions may contain a therapeutically effective amount of a compound of the disclosure as defined above, either alone or in a combination with a therapeutically effective amount of one to three other agents useful in treating Parkinson's disease. Such agents include L-DOPA, dopaminergic agonists, MAO-B inhibitors, DOPA decarboxylase inhibitors, COMT inhibitors and NMDA receptor.

**Pharmaceutical Compositions** The compounds of Formula I are usually administered in the form of pharmaceutical compositions. This disclosure therefore provides pharmaceutical compositions that contain, as the active ingredient, one or more of the compounds of Formula I or its tautomers, polymorphs, stereoisomers or a pharmaceutically acceptable salts thereof, and one or more pharmaceutically acceptable excipients, carriers, including inert solid diluents and fillers, diluents, including sterile aqueous solution and various organic solvents, permeation enhancers, solubilizers and adjuvants. The compounds of Formula I may be administered alone or in combination with other therapeutic agents. Such compositions are prepared in a manner well known in the pharmaceutical art (see, e.g., Remington's Pharmaceutical Sciences, Mace Publishing Co., Philadelphia, Pa. 17.sup.th Ed. (1985) and "Modern Pharmaceutics", Marcel Dekker, Inc. 3.sup.rd Ed. (G. S. Banker & C. T. Rhodes, Eds.).

### Administration

The compounds of Formula I may be administered in either single or multiple doses by any of the accepted modes of administration of agents having similar utilities, for example as described in those patents and patent applications incorporated by reference, including rectal, buccal, intranasal and transdermal routes, by intra-arterial injection, intravenously, intraperitoneally, parenterally, intramuscularly, subcutaneously, orally, topically, as an inhalant, or via an impregnated or coated device such as a stent, for example, or an artery-inserted cylindrical polymer.

The following examples are included to demonstrate preferred embodiments of the present disclosure. It should be appreciated by those of skill in the art that the techniques disclosed in the examples which follow represent techniques discovered by the inventor to function well in the practice of the present disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the present disclosure.

### Examples

The invention is further illustrated by the following examples which in no way should be construed as being further limiting. One skilled in the art will readily appreciate that the specific methods and results described are merely illustrative. Structures of the intermediates as well as the final compounds were confirmed by nuclear magnetic resonance spectra for proton (¹H NMR) and LCMS.

### Example A1: 5-Amino-8-(2-furyl)-3-[2-[4-[4-(2-methoxyethoxy)phenyl]piperazin-1-yl]ethyl]-1-ethyl-[1,2,4]triazolo[5,1-f]purin-2-one

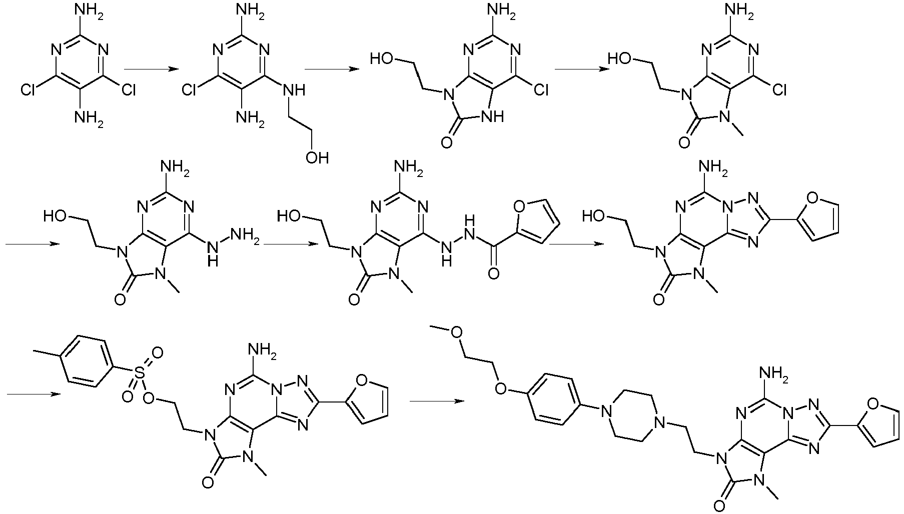

### Step-1: 2-[(2,5-Diamino-6-chloro-pyrimidin-4-yl)amino]ethanol

A mixture of 4,6-dichloropyrimidine-2,5-diamine (28g, 156mmol), ethanolamine (18ml, 312mmol) and ethanol (250ml) were heated at 100-110 °C for 16 hours. The mixture was cooled and solvent was removed. To the residue methanol (100ml) was added and stirred for 20 minutes. The solid was filtered off to obtain 2-[(2,5-diamino-6-chloro-pyrimidin-4-yl)amino]ethanol (22.0g, 70%).
¹HNMR(400MHz, DMSO d6): δ 3.36-3.40 (m, 2H); 3.50-3.54 (m, 2H); 3.88 (bs, 2H); 4.74 (t, J=5.6Hz, 1H); 5.63 (bs, 2H); 6.51 (t, J=5.6Hz, 1H)

### Step-2: 2-Amino-6-chloro-9-(2-hydroxyethyl)-7H-purin-8-one

A mixture of 2-[(2,5-diamino-6-chloro-pyrimidin-4-yl)amino]ethanol obtained in step 1 (10.0g, 49.26mmol) in acetonitrile (400ml) were cooled to 0 °C. To this reaction mixture K₂CO₃ (20.39gm, 147.7mmol) and 4-nitrophenyl chloroformate (19.8g, 98.52mmol)was added and stirred at 25-27 °C for 24 hours. This reaction mixture was filtered and washed with acetonitrile (300ml) and diethyl ether (300ml) respectively. Solid obtained was dried to obtain crude 2-amino-6-chloro-9-(2-hydroxyethyl)-7H-purin-8-one as a yellow solid. Small amount of crude material was purified by column chromatography to obtain pure product.
¹HNMR(400MHz, DMSO d6): δ 3.61-3.66 (m, 2H); 3.72-3.75 (m, 2H); 4.85 (t, J=6Hz, 1H); 6.60 (s, 2H); 11.21 (s, 1H)

### Step-3: 2-Amino-6-chloro-9-(2-hydroxyethyl)-7-methyl-purin-8-one

A mixture of 2-amino-6-chloro-9-(2-hydroxyethyl)-7H-purin-8-one obtained in step 2 (13g, 56.7mmol), K₂CO₃ (11.5g, 84mmol), methyl iodide (12g, 85.15mmol) and DMF (130ml) were stirred at 25-30 °C for 16 hours. The reaction mixture was concentrated and purified by column chromatography using 60-120 silica gel and 4% methanol in DCM as an eluent to obtain 2-amino-6-chloro-9-(2-hydroxyethyl)-7-methyl-purin-8-one (8g, 58%) as an off white solid.
¹HNMR(400MHz, DMSO d6): δ 3.42 (s, 3H); 3.65 (t, J=5.6Hz, 2H); 3.78 (t, J=5.6Hz, 2H); 4.85 (t, J=5.6Hz, 1H); 6.69 (bs, 2H).

### Step-4: 2-Amino-6-hydrazino-9-(2-hydroxyethyl)-7-methyl-purin-8-one

A mixture of 2-amino-6-chloro-9-(2-hydroxyethyl)-7-methyl-purin-8-one obtained in step 3 (8g, 32.9mmol), Hydrazine hydrate (16ml ,32.9mmol) and ethanol (300ml) were heated at 100-110 °C for 16 hours. The reaction mixture was concentrated and solid obtained was filtered off and dried to obtain 2-amino-6-hydrazino-9-(2-hydroxyethyl)-7-methyl-purin-8-one (7g, 89 %) as an off white solid.
¹HNMR(400MHz, DMSO d6): δ 3.37 (s, 3H); 3.58-3.61 (m, 2H); 3.71 (t, J=6Hz, 2H); 4.29 (bs, 2H); 4.87 (t, J=5.6Hz, 1H), 6.00 (bs, 2H); 7.63 (s, 1H).

### Step-5: N'-[2-Amino-9-(2-hydroxyethyl)-7-methyl-8-oxo-purin-6-yl]furan-2-carbohydrazide

2-amino-6-hydrazino-9-(2-hydroxyethyl)-7-methyl-purin-8-one (4.5g, 18.18mmol) obtained in step 4, 2-furoic acid (2.53g, 22.5mmol), HOBT (2.53g, 18.8 mmol) and N-methylmorpholine were taken in dimethylformamide (40ml). 1-Ethyl-3(3'-dimethylaminopropryl)carbodiimide hydrochloride (EDCI.HCl) (5.4g, 28.2mmol) was added to the reaction mixture and stirred at 25-27 °C for 14 hours. The reaction mixture was evaporated and residue was purified by column chromatography to obtain N'-[2-amino-9-(2-hydroxyethyl)-7-methyl-8-oxo-purin-6-yl]furan-2-carbohydrazide (5.3g, 84%) as an off white solid.
¹HNMR (400MHz, DMSO d6): δ 3.43 (s, 3H); 3.59-3.63 (m, 2H); 3.74 (t, J=6Hz, 2H); 4.88 (t, J=5.6Hz, 1H); 5.98 (bs, 2H); 6.67 (bs, 1H); 7.25 (d, J=3.2Hz, 1H); 7.90 (s, 1H); 8.35 (s, 1H); 10.28 (s,1H).

### Step-6: 5-Amino-8-(2-furyl)-3-(2-hydroxyethyl)-1-methyl-[1,2,4]triazolo[5,1-f]purin-2-one

A mixture of N'-[2-amino-9-(2-hydroxyethyl)-7-methyl-8-oxo-purin-6-yl]furan-2-carbohydrazide obtained in step 5 (5.3g, 15.9mmol), N,O-bistrimethylsilylacetamide (27ml, 111.4mmol) and hexamethyldisilazane (83ml, 397mmol) were heated at 110-120 °C for 16 hours. The reaction mixture was quenched with methanol (100ml) and water (100ml) and organic volatiles were evaporated. The solid obtained was filtered off and washed with water (30ml) followed by diethyl ether (100ml) to obtain 5-amino-8-(2-furyl)-3-(2-hydroxyethyl)-1-methyl-[1,2,4]triazolo[5,1-f]purin-2-one (3.50g, 71%) as an off white solid.
¹HNMR (400MHz, DMSO d6): δ 3.56 (s, 3H); 3.67-3.70 (m, 2H); 3.84-3.87 (m, 2H); 4.88 (t, J=5.6Hz, 1H); 6.73 (bs, 1H); 7.20 (bs, 1H); 7.79 (bs, 2H); 7.94 (bs, 1H).

### Step-7: 2-[5-Amino-8-(2-furyl)-1-methyl-2-oxo-[1,2,4]triazolo[5,1-f]purin-3-yl]ethyl 4-methylbenzenesulfonate

A mixture of 5-amino-8-(2-furyl)-3-(2-hydroxyethyl)-1-methyl-[1,2,4]triazolo[5,1-f]purin-2-one obtained in step 6 (3.5g, 11mmol p-toluene sulphonylchloride (5.2 g, 27mmol) were taken in pyridine (30ml)and stirred at 25-27 °C for 16 hours. To the reaction mixture hexane (100ml) was added and solid obtained was filtered off and washed with water (100ml) followed by hexane (100ml) to obtain 2-[5-amino-8-(2-furyl)-1-methyl-2-oxo-[1,2,4]triazolo[5,1-f]purin-3-yl]ethyl 4-methylbenzenesulfonate (4.1g, 78%) as a brown solid. ¹HNMR (400MHz, DMSO d6): δ 2.02 (s, 3H); 3.49 (s, 3H); 3.99 (t, J=4.8Hz, 2H); 4.71 (t, J=4.8Hz, 2H); 6.73-6.75 (m, 1H); 7.01 (d, J=8Hz, 2H); 7.23 (d, J=3.2Hz, 1H); 7.41 (d, J=8.4Hz, 2H); 7.78 (bs, 2H); 7.96 (d, J=1.2Hz, 1H).

### Step-8: : 5-Amino-8-(2-furyl)-3-[2-[4-[4-(2-methoxyethoxy)phenyl]piperazin-1-yl]ethyl]-1-methyl-[1,2,4]triazolo[5,1-f]purin-2-one

A mixture of 2-[5-amino-8-(2-furyl)-1-methyl-2-oxo-[1,2,4]triazolo[5,1-f]purin-3-yl]ethyl 4-methylbenzenesulfonate obtained in step 7 (0.25g, 0.533mmol), 1-[4-(2-Methoxy-ethoxy)-phenyl]-piperazine (0.188g, 0.799mmol) and DIPEA (0.27ml, 1.599mmol) were taken in DMF (5ml) and stirred at 80 °C for 16 hours. To the reaction mixture water (100ml) was added and solid obtained was filtered off. The crude product was purified by column chromatography to obtain 5-amino-8-(2-furyl)-3-[2-[4-[4-(2-methoxyethoxy)phenyl]piperazin-1-yl]ethyl]-1-methyl-[1,2,4]triazolo[5,1-f]purin-2-one (0.135g, 47%) as an off white solid
¹HNMR (400MHz, DMSO d6): δ 2.60 (bs, 4H); 2.68 (t, J=6.4Hz, 2H); 2.96 (bs, 4H); 3.29 (s, 3H); 3.56 (s, 3H); 3.59-3.62 (m, 2H); 3.94-4.00 (m, 4H); 6.71-6.73 (m, 1H); 6.79-6.86 (m, 4H); 7.19 (dd, J=3.2Hz, 1.2Hz, 1H); 7.80 (bs, 2H); 7.94 (bs, 1H).

Examples A2-A63 were prepared by following similar experimental procedure of Example A1 using the appropriate intermediates.

| **Ex. No** | **Structure / IUPAC name** | **NMR** |
|---|---|---|
| A2 | 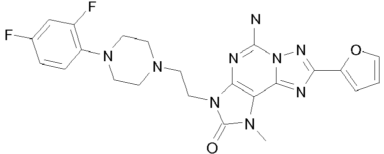 | ¹HNMR (400MHz, DMSO d6): δ 2.62 (bs, 4H); 2.67-2.70 (m, 2H); 2.91 (bs, 4H); 3.57 (s, 3H); 3.95 (bs, 2H); 6.73 (bs, 1H); 6.95-7.04 (m, 2H); 7.15-7.19 (m, 2H); 7.79 (bs, 2H); 7.94 (bs, 1H). |
| | 5-Amino-3-[2-[4-(2,4-difluorophenyl)piperazin-1-yl]ethyl]-8-(2-furyl)-1-methyl-[1,2,4]triazolo[5,1-f]purin-2-one | |
| A3 | 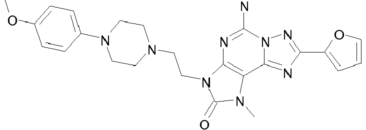 | ¹HNMR (400MHz, DMSO d6): δ 2.61 (bs, 4H); 2.68 (bs, 2H); 2.95 (bs, 4H); 3.57 (s, 3H); 3.67 (s, 3H); 3.96 (bs, 2H); 6.72 (bs, 1H); 6.78-6.87 (m,4H); 7.19 (bs, 1H); 7.81 (bs, 2H); 7.94 (s, 1H). |
| | 5-Amino-8-(2-furyl)-3-[2-[4-(4-methoxyphenyl)piperazin-1-yl]ethyl]-1-methyl-[1,2,4]triazolo[5,1-f]purin-2-one | |
| A4 | 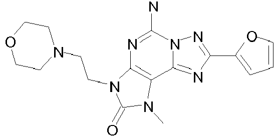 | ¹HNMR (400MHz, CDCl3): δ 2.57 (bs, 4H); 2.76 (t, J=6.4Hz, 2H); 3.67 (t, J=4.4Hz, 4H); 3.76 (s, 3H); 4.06 (t, J=6.4 Hz, 2H); 5.75 (bs, 2H); 6.60 (bs, 1H); 7.23-7.26 (m, 1H); 7.64 (s, 1H). |
| | 5-Amino-8-(2-furyl)-1-methyl-3-(2-morpholmoethyl)-[1,2,4]triazolo[5,1-f]purin-2-one | |
| A5 | 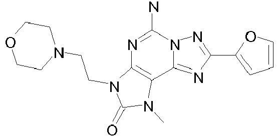 | ¹H NMR(400 MHz, DMSO d6): δ 1.50-1.58 (m, 3H); 1.71 (d, J=10.8Hz, 2H); 2.08 (t, J=10.4Hz, 2H); 2.66 (t, J=6.4Hz, 2H); 3.05 (d, J=11.2Hz, 2H); 3.57 (s, 3H); 3.94 (t, J=6.8Hz, 2H); 6.72-6.73 (m, 1H); 7.08 (t, J=8.8Hz, 2H); 7.20 (d, J=3.2Hz, 1H); 7.23-7.27 (m, 1H); 7.80 (bs, 2H); 7.94 (s, 1H). |
| | 5-Amino-3-[2-[4-(2,4-difluorophenyl)-1-piperidyl]ethyl]-8-(2-furyl)-1-methyl-[1,2,4]triazolo[5,1-f]purin-2-one | |
| A6 | 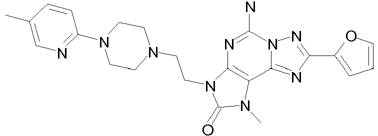 | ¹H NMR(400 MHz, DMSO d6): δ 2.13(s, 3H); 2.55(bs, 4H); 2.67(t, J=6.4Hz, 2H); 3.32-3.34 (m, 4H); 3.56 (s, 3H); 3.96 (t, J=6.4Hz, 2H); 6.71-6.73 (m, 2H); 7.19 (d, J=3.2Hz, 1H); 7.35 (d, J=8.8Hz, 1H); 7.81 (bs, 2H); 7.94 (bs, 2H). |
| | 5-Amino-8-(2-furyl)-1-methyl-3-[2-[4-(5-methyl-2-pyridyl)piperazin-1-yl]ethyl]-[1,2,4]triazolo[5,1-f]purin-2-one | |
| A7 | 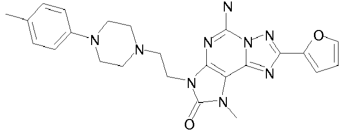 | ¹H NMR(400 MHz, DMSO d6): δ 2.18 (s, 3H); 2.60 (bs, 4H); 2.65-2.70(m, 2H); 3.00 (bs, 4H); 3.56 (s, 3H); 3.96 (t, J= 6.8Hz, 2H); 6.72 (bs, 1H);6.79 (d, J=8.4Hz, 2H); 7.0 (d, J=8.0Hz, 2H); 7.19 (d, J=2.8Hz, 1H); 7.80 (bs, 2H); 7.94 (s, 1H). |
| | 5-Amino-8-(2-furyl)-1-methyl-3-[2-[4-(p-tolyl)piperazin-1-yl]ethyl]-[1,2,4]triazolo[5,1-f]purin-2-one | |
| A8 | 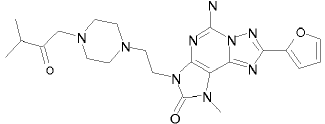 | ¹H NMR(400MHz,DMSO): δ 0.97 (d, J=6.8Hz, 6H); 2.34 (bs, 4H); 2.40-2.42 (m, 4H); 2.62 (t, J=6.4Hz, 2H); 2.67-2.74 (m, 1H); 3.21 (s, 2H); 3.56 (s, 3H); 3.90 (t, J=6.4Hz, 2H); 6.72 (dd, J= 2Hz, 3.6Hz, 1H); 7.19 (d, J=3.6Hz, 1H); 7.79 (bs, 2H); 7.94 (d, J=1.2Hz, 1H). |
| | 5-Amino-8-(2-furyl)-1-methyl-3-[2-[4-(3-methyl-2-oxo-butyl)piperazin-1-yl]ethyl]-[1,2,4]triazolo[5,1-f]purin-2-one | |
| A9 | 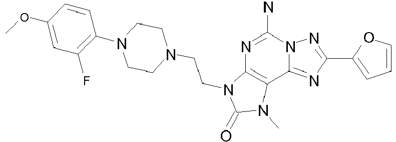 | ¹H NMR(400MHz,DMSO): δ 2.6 (bs, 4H); 2.68 (t, J=6.4Hz, 2H); 2.86 (bs, 4 H); 3.57 (s , 3H); 3.70 (s, 3H); 3.95 (t, J=6.4Hz, 2H); 6.66 (dd, J=8.8Hz, 3.2Hz, 1H); 6.72 (dd, J=2Hz, 3.6Hz, 1H); 6.67-6.81 (m, 1H); 6.94 (t, J=9.2Hz, 1H); 7.19 (d, J=3.6Hz, 1H); 7.80 (bs, 2H); 7.94 (d, J=1.6Hz, 1H). |
| | 5-Amino-3-[2-[4-(2-fluoro-4-methoxyphenyl)piperazin-1-yl]ethyl]-8-(2-furyl)-1-methyl-[1,2,4]triazolo[5,1-f]purin-2-one | |
| A10 | 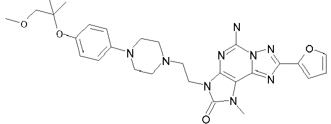 | ¹HNMR(400MHz,DMSO): δ 1.15 (s, 6H); 2.60 (bs, 4H); 2.68 (t, J=6.8Hz, 2H); 3.01 (bs, 4H); 3.23 (s, 2H); 3.29 (s, 3H); 3.57 (s, 3H); 3.96 (t, J=6.4Hz, 2H); 6.72 (dd, J=1.6Hz, 3.6 Hz, 1H); 6.81 (s, 4H); 7.19 (d,J=3.6Hz, 1H); 7.81 (bs, 2H); 7.94 (bs, 1H). |
| | 5-Amino-8-(2-furyl)-3-[2-[4-[4-(2-methoxy-1,1-dimethyl-ethoxy)phenyl]piperazin-1-yl]ethyl]-1-methyl-[1,2,4]triazolo[5,1-f]purin-2-one | |
| A11 | 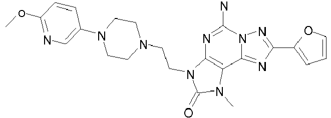 | ¹H NMR(400MHz,DMSO): δ 2.61 (bs, 4H); 2.67-2.70 (m, 2H); 2.99 (bs, 4H); 3.57 (s, 3H); 3.76 (s, 3H); 3.96 (t, J=6.4Hz, 2H); 6.69 (d, J=9.2Hz, 1H); 6.72(dd, J=2Hz, 3.6Hz, 1H); 7.19 (d, J=3.6Hz, 1H); 7.41 (dd, J=9.2Hz, 3.2Hz, 1H); 7.74 (d, J=2.8Hz, 1H); 7.80 (bs, 2H); 7.94 (s, 1H). |
| | 5-Amino-8-(2-furyl)-3-[2-[4-(6-methoxy-3-pyridyl)piperazin-1-yl]ethyl]-1-methyl-[1,2,4]triazolo[5,1-f]purin-2-one | |
| A12 | 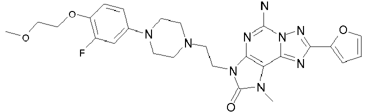 | ¹H NMR(400MHz,DMSO): δ 2.59 (bs, 4H); 2.67 (t, J=6.4Hz, 2H); 2.99(bs, 4H); 3.29 (s, 3H); 3.56 (s, 3H); 3.60-3.62 (m, 2H), 3.95 (t, J=6Hz, 2H); 4.03-4.06 (m, 2H), 6.62 (d, J=8.8Hz, 1H), 6.72(dd, J=2Hz, 3.6Hz, 1H); 6.80-6.84 (m, 1H); 6.99 (t, J=9.6Hz, 1H); 7.19(d, J=3.2Hz, 1H);7.81 (bs, 2H); 7.94(s, 1H). |
| | 5-Amino-3-[2-[4-[3-fluoro-4-(2-methoxyethoxy)phenyl]piperazin-1-yl]ethyl]-8-(2-furyl)-1-methyl-[1,2,4]triazolo[5,1-f]purin-2-one | |
| A13 | 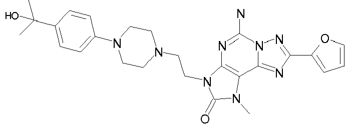 | 1H NMR (400MHz, DMSO): δ 1.37 (s, 6H); 2.58-2.62 (m, 4H); 2.66-2.68 (m, 2H); 3.03 (bs, 4H); 3.57 (s, 3H); 3.96 (t, J=6.8Hz, 2H); 4.81 (s, 1H); 6.72 (dd, J=1.6Hz, 3.2Hz, 1H); 6.83 (d, J=9.2Hz, 2H); 7.19 (d, J=3.2Hz, 1H); 7.27 (d, J=8.8Hz, 2H); 7.81 (bs, 2H); 7.94 (s, 1H). |
| | 5-Amino-8-(2-furyl)-3-[2-[4-[4-(1-hydroxy-1-methyl-ethyl)phenyl]piperazin-1-yl]ethyl]-1-methyl-[1,2,4]triazolo[5,1-f]purin-2-one | |
| A14 | 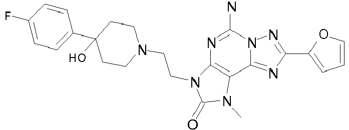 | 1H NMR (400MHz, DMSO): 1.54-1.57 (m, 2H); 1.80-1.85 (m, 2H); 2.42-2.45 (m, 2H); 2.66 (t, J=6.8Hz, 2H); 2.74-2.77 (m, 2H); 3.57 (s, 3H); 3.94 (t, J=6.8Hz, 2H); 4.85 (s, 1H); 6.72 (dd, J=1.6Hz, 3.2Hz,1H); 7.07-7.12 (m, 2H); 7.20 (d, J=3.2Hz, 1H); 7.44-7.48 (m, 2H); 7.79 (bs, 2H); 7.94 (bs, 1H). |
| | 5-Amino-3-[2-[4-(4-fluorophenyl)-4-hydroxy-1-piperidyl]ethyl]-8-(2-furyl)-1-methyl-[1,2,4]triazolo[5,1-f]purin-2-one | |
| A15 | 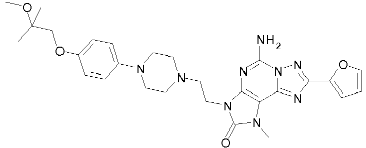 | ¹HNMR (400MHz, DMSO d6): δ 1.18 (s, 6H); 2.56(bs, 4H); 2.68 (t, J=6.8Hz, 2H); 2.95 (bs, 4H); 3.14 (s, 3H); 3.56 (s, 3H); 3.73 (s, 2H); 3.96 (t, J=6.8Hz, 2H); 6.72 (dd, J=2Hz, 3.6Hz, 1H); 6.80-6.86 (m, 4H); 7.19 (d, J=3.6Hz, 1H); 7.81 (bs, 2H); 7.94 (d, J=1.2Hz,1H). |
| | 5-Amino-8-(2-furyl)-3-[2-[4-[4-(2-methoxy-2-methyl-propoxy)phenyl]piperazin-1-yl]ethyl]-1-methyl-[1,2,4]triazolo[5,1-f]purin-2-one | |
| A16 | 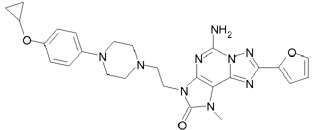 | ¹HNMR (400MHz, DMSO d6): δ 0.57-0.61 (m, 2H); 0.69-0.74 (m, 2H); 2.56-2.62 (m, 4H); 2.68 (t, J=6.4Hz, 2H); 2.96 (bs, 4H); 3.56 (s, 3H); 3.70-3.75 (m, 1H); 3.96 (t, J=6.4Hz, 2H); 6.72 (dd, J=1.6 Hz, 3.6 Hz, 1H); 6.84-6.91 (m, 4H); 7.19-7.20 (m, 1H); 7.81 (bs, 2H); 7.94 (bs, 1H). |
| | 5-Amino-3-[2-[4-[4-(cyclopropoxy) phenyl]piperazin-1-yl]ethyl]-8-(2-furyl)-1-methyl-[1,2,4]triazolo[5,1-f]purin-2-one | |
| A17 | 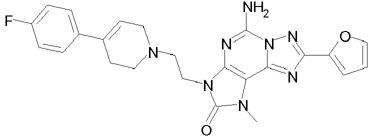 | ¹HNMR (400MHz, DMSO d6): δ 2.42 (bs, 2H); 2.71-2.78 (m, 4H); 3.17(bs, 2H); 3.56 (s, 3H); 3.99 (t, J=6.4Hz, 2H); 6.11 (bs, 1H); 6.72 (bs, 1H); 7.14(t, J=8.8Hz, 2H); 7.19 (d, J=3.2Hz, 1H); 7.43-7.46 (m, 2H); 7.82 (bs, 2H); 7.94 (s, 1H). |
| | 5-Amino-3-[2-[4-(4-fluorophenyl)-3,6-dihydro-2H-pyridin-1-yl]ethyl]-8-(2-furyl)-1-methyl-[1,2,4]triazolo[5,1-f]purin-2-one | |
| A18 | 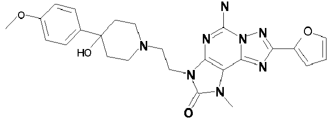 | ¹H NMR(400MHz, DMSO d6): δ1.51-1.54 (m, 2H); 1.74-1.81 (m, 2H); 2.40-2.47 (m, 2H); 2.64 (t, J=6.8Hz, 2H); 2.69-2.72 (m, 2H); 3.55 (s, 3H); 3.69 (s, 3H); 3.92 (t, J=6.8Hz, 2H); 4.66 (bs, 1H); 6.71 (dd, J=2Hz, 3.6Hz, 1H); 6.82 (d, J=8.8Hz, 2H); 7.18-7.19 (m, 1H); 7.31 (d, J=9.2Hz, 2H); 7.77 (bs, 2H); 7.92 (s, 1H). |
| | 5-Amino-8-(2-furyl)-3-[2-[4-hydroxy-4-(4-methoxyphenyl)-1-piperidyl]ethyl]-1-methyl-[1,2,4]triazolo[5,1-f]purin-2-one | |
| A19 | 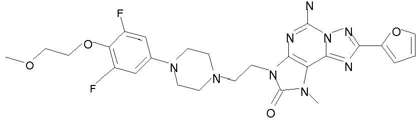 | ¹H NMR(400MHz, DMSO d6): δ 2.57 (bs, 4H); 2.65-2.69 (m, 2H); 3.06(bs, 4H); 3.27 (s, 3H); 3.56 (t, J=4Hz, 5H); 3.95 (t, J=6.4Hz, 2H); 4.03 (t, J=4.4Hz, 2H);6.64(s,1H); 6.67 (s, 1H); 6.72(dd, J=2Hz, 3.6 Hz, 1H);7.19 (d, J=3.6Hz, 1H); 7.81 (bs, 2H); 7.94 (s, 1H). |
| | 5-Amino-3-[2-[4-[3,5-difluoro-4-(2-methoxyethoxy)phenyl]piperazin-1-yl]ethyl]-8-(2-furyl)-1-methyl-[1,2,4]triazolo[5,1-f]purin-2-one | |
| A20 | 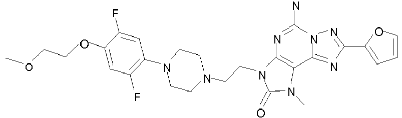 | ¹H NMR(400MHz, DMSO d6): δ 2.61 (bs, 4H); 2.67-2.70 (m, 2H); 2.87 (bs, 4H); 3.30(s, 3H); 3.57 (s, 3H); 3.61-3.63 (m, 2H); 3.95(t, J=6.4Hz, 2H); 4.10(t, J=4.4Hz, 2H); 6.72(dd, J=1.6Hz, 3.2Hz, 1H); 6.90-6.95(m, 1H); 7.08-7.14(m, 1H); 7.20(d, J=3.2Hz, 1H); 7.80(bs, 2H); 7.94(bs, 1H). |
| | 5-Amino-3-[2-[4-[2,5-difluoro-4-(2-methoxyethoxy)phenyl]piperazin-1-yl]ethyl]-8-(2-furyl)-1-methyl-[1,2,4]triazolo[5,1-f]purin-2-one | |
| A21 | 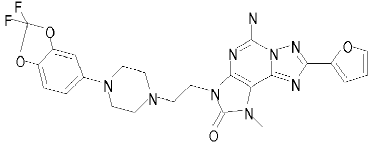 | ¹H NMR(400MHz, DMSO d6): δ 2.60 (bs, 4H); 2.69 (t, J=6.4Hz, 2H); 3.05 (bs, 4H); 3.57 (s, 3H); 3.96 (t, J=6.8Hz, 2H); 6.66 (dd, J=8.8Hz, 2.0Hz, 1H); 6.72 (bs, 1H); 7.05 (d, J=2.0Hz, 1H); 7.19-7.21 (m, 2H); 7.80 (bs, 2H); 7.94 (s, 1H). |
| | 5-Amino-3-[2-[4-(2,2-difluoro-1,3-benzodioxol-5-yl)piperazin-1-yl]ethyl]-8-(2-furyl)-1-methyl-[1,2,4]triazolo[5,1-f]purin-2-one | |
| A22 | 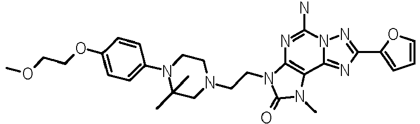 | ¹H NMR(400MHz, DMSO d6): δ 0.9 (s, 6H); 2.63 (s, 2H); 2.76 (t, J=4.8 Hz, 2H); 2.87 (t, J=4.8Hz, 2H); 2.98-3.00 (m, 2H); 3.44 (s, 3H); 3.72 (t, J=4.8Hz, 2H); 3.76 (s, 3H); 4.00 (t, J=6.8Hz, 2H); 4.06 (t, J=4.4Hz, 2H); 5.70 (bs, 2H); 6.60 (dd, J= 2.0Hz, 3.6Hz, 1H); 6.79-6.85 (m, 4H); 7.23 (d, J=3.2Hz, 1H); 7.64 (bs, 1H). |
| | 5-Amino-8-(2-furyl)-3-[2-[4-[4-(2-methoxyethoxy)phenyl]-3,3-dimethyl-piperazin-1-yl]ethyl]-1-methyl-[1,2,4]triazolo[5,1-f]purin-2-one | |
| A23 | 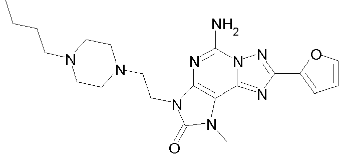 | ¹HNMR (400MHz, DMSO d6): δ 0.86 (t, J=7.2Hz, 3H); 1.23-1.30 (m, 3H);1.38 (bs, 2H); 2.21-2.45 (m, 9H); 2.61(t, J=6.4Hz, 2H); 3.55 (s, 3H); 3.90 (t, J=6.8Hz, 2H); 6.72 (dd, J=2Hz, 3.6Hz ,1H); 7.20 (dd, J=0.8Hz, 3.6Hz ,1H); 7.79 (bs, 2H); 7.93-7.94 (m, 1H). |
| | 5-Amino-3-[2-(4-butylpiperazin-1-yl)ethyl]-8-(2-furyl)-1-methyl-[1,2,4]triazolo [5,1-f]purin-2-one | |
| A24 | 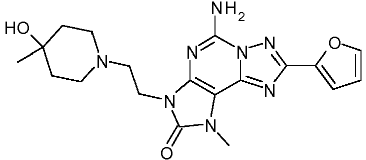 | ¹HNMR (400MHz, DMSO d6): δ 1.06 (s, 3H); 1.37-1.40 (m, 4H); 2.40-2.46 (m, 4H); 2.60 (t, J=6.8Hz, 2H); 3.55 (s, 3H); 3.89 (t, J=6.8Hz, 2H); 4.07 (s, 1H); 6.72 (dd, J=2Hz, 3.6Hz ,1H); 7.19 (d, J=3.6Hz, 1H);7.78 (s, 2H);7.93 (s, 1H) |
| | 5-Amino-8-(2-furyl)-3-[2-(4-hydroxy-4-methyl-1-piperidyl)ethyl]-1-methyl-[1,2,4]triazolo[5,1-f]purin-2-one | |
| A25 | 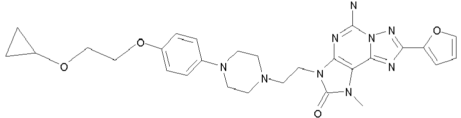 | ¹HNMR(400MHz, DMSO d6): δ 0.41-0.46 (m, 4H), 2.59 (bs, 4H); 2.66 (t, J=6.4Hz, 2H); 2.94-2.95(bs, 4H); 3.55 (s, 3H); 3.69 (t, J=4.8Hz, 3H); 3.92-3.97 (m, 4H); 6.70 (dd, J=2Hz, 3.6Hz, 1H); 6.77-6.84 (m, 4H); 7.18 (d, J=2.8Hz, 1H); 7.80 (bs, 2H); 7.93-7.94 (m, 1H). |
| | 5-Amino-3-[2-[4-[4-[2-(cyclopropoxy)ethoxy]phenyl]piperazin-1-yl]ethyl]-8-(2-furyl)-1-methyl-[1,2,4]triazolo[5,1-f]purin-2-one | |
| A26 | 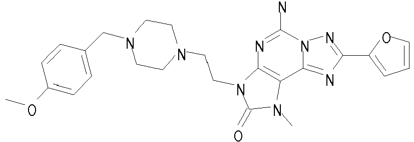 | ¹HNMR(400MHz, DMSO d6): δ 2.27-2.34 (m, 6H); 2.45-2.67 (m, 6H); 3.56(s, 3H); 3.72 (s, 3H); 3.89 (t, J=6.4Hz, 2H); 6.72 (dd, J=1.6Hz, 3.6Hz, 1H); 6.86 (d, J=8.8Hz, 2H); 7.17 (d, J=8.8Hz, 2H); 7.20 (d, J=3.6 Hz, 1H): 7.78 (bs, 2H); 7.94 (bs, 1H). |
| | 5-Amino-8-(2-furyl)-3-[2-[4-[(4-methoxyphenyl)methyl]piperazin-1-yl]ethyl]-1-methyl-[1,2,4]triazolo[5,1-f]purin-2-one | |
| A27 | 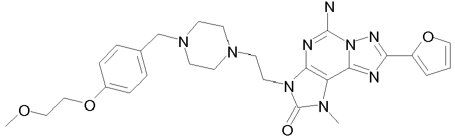 | ¹HNMR(400MHz, DMSO d6): δ 2.26-2.30(m, 4H); 2.57 -2.65 (m, 4H); 3.27 (s, 3H); 3.28-3.36 (m, 4H); 3.54 (s, 3H); 3.62 (t, J=4.8Hz, 2H); 3.68 (t, J=6.4Hz, 2H); 4.03 (t, J=4.8Hz, 2H); 6.70 (bs, 1H); 6.83(s, 1H); 6.85(s, 1H); 7.13 (bs, 1H); 7.15 (s, 1H); 7.18 (d, J=3.6Hz, 1H); 7.76 (bs, 2H); 7.92 (s, 1H). |
| | 5-Amino-8-(2-furyl)-3-[2-[4-[[4-(2-methoxyethoxy)phenyl]methyl]piperazin-1-yl]ethyl]-1-methyl-[1,2,4]triazolo[5,1-f]purin-2-one | |
| A28 | 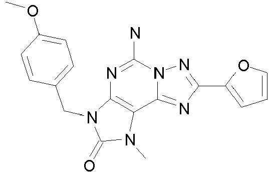 | ¹HNMR(400MHz, DMSO d6): δ 3.75 (s, 3H); 3.76 (s, 3H), 5.14 (s, 2H), 5.73(bs, 2H); 6.59 (dd, J=2Hz, 3.6Hz, 1H); 6.83-6.86 (m, 2H); 7.23 (d, J=3.2Hz, 1H); 7.43 (d, J=8.8Hz, 2H); 7.63(bs, 1H). |
| | 5-Amino-8-(2-furyl)-3-[(4-methoxyphenyl)methyl]-1-methyl-[1,2,4]triazolo[5,1-f]purin-2-one | |
| A29 | 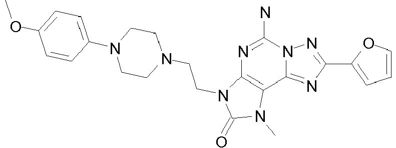 | HNMR(400MHz, DMSO d6): δ 2.61 (bs, 4H); 2.68 (bs, 2H); 2.95(bs, 4H); 3.57 (s, 3H); 3.67 (s, 3H); 3.96 (bs, 2H); 6.72 (bs, 1H); 6.78-6.86 (m, 4H); 7.19 (bs ,1H); 7.81 (bs, 2H); 7.94 (s, 1H). |
| | 5-Amino-8-(2-furyl)-3-[2-[4-(4-methoxyphenyl)piperazin-1-yl]ethyl]-1-methyl-[1,2,4]triazolo[5,1-f]purin-2-one | |
| A30 | 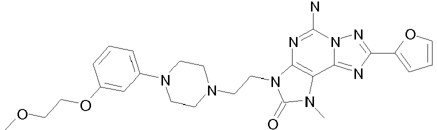 | HNMR(400MHz, DMSO d6): δ 2.59 (bs, 4H); 2.67 (t, J=6Hz, 2H); 3.06 (bs, 4H); 3.29(s, 3H); 3.56 (s, 3H); 3.62 (t, J=4.4Hz, 2H); 3.96(t, J=6.4Hz, 2H); 4.03 (t, J=4.4Hz, 2H); 6.34 (d, J=8Hz, 1H); 6.42 (s, 1H); 6.49(d, J=8Hz,1H); 6.72 (bs, 1H); 7.07 (t, J=8.4Hz, 1H); 7.20(d, J=2.8Hz, 1H); 7.81 (bs, 2H); 7.94 (s, 1H). |
| | 5-Amino-8-(2-furyl)-3-[2-[4-[3-(2-methoxyethoxy)phenyl]piperazin-1-yl]ethyl]-1-methyl-[1,2,4]triazolo[5,1-f]purin-2-one | |
| A31 | 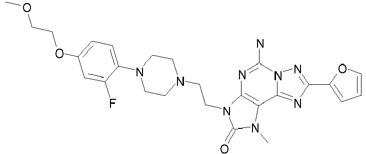 | ¹HNMR (400MHz, DMSO d6): δ 2.62 (bs, 4H); 2.68 (t, J=6.8Hz, 2H); 2.85 (bs, 4H); 3.28 (s, 3H); 3.57 (s, 3H); 3.59-3.62 (m, 2H); 3.95 (t, J=6.8Hz, 2H); 4.01-4.04 (m, 2H); 6.66-6.68 (m, 1H); 6.72 (dd, J=2 Hz,3.6Hz, 1H); 6.79 (dd, J=2.8Hz, 14Hz, 1H); 6.92 (t, J=9.6Hz, 1H); 7.19 (d, J=3.2Hz, 1H); 7.93 (bs, 2H); 7.93-7.94 (m, 1H). |
| | 5-Amino-3-[2-[4-[2-fluoro-4-(2-methoxyethoxy)phenyl]piperazin-1-yl]ethyl]-8-(2-furyl)-1-methyl-[1,2,4]triazolo[5,1-f]purin-2-one | |
| A32 | 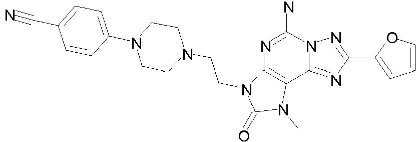 | HNMR(400MHz, DMSO d6): δ 2.59 (bs, 4H); 2.68(t, J=6.4Hz, 2H); 3.27(t, J=4.8Hz, 4H); 3.56 (s, 3H); 3.96 (t, J=6.4Hz, 2H); 6.72(dd, J=2Hz, 3.6Hz, 1H); 6.99 (d, J=8.8Hz, 2H); 7.19 (d, J=3.6Hz, 1H);7.56 (d, J=8.8Hz, 2H); 7.80 (bs, 2H); 7.93 (bs,1H). |
| | 4-[4-[2-[5-Amino-8-(2-furyl)-1-methyl-2-oxo-[1,2,4]triazolo[5,1-f]purin-3-yl]ethyl]piperazin-1-yl]benzonitrile | |
| A33 | 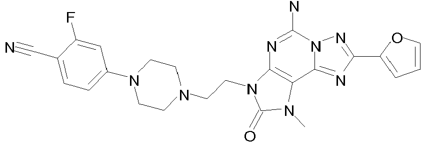 | HNMR(400MHz, DMSO d6): δ 2.57 (bs, 4H,); 2.66-2.70 (m, 2H); 3.31 (bs, 4H,); 3.56 (s, 3H); 3.96 (t, J =6.4Hz, 2H); 6.72 (dd, J=2Hz, 3.6Hz, 1H); 6.83 (dd, J=2Hz, 8.8Hz, 1H); 6.92(d, J=14Hz, 1H); 7.19 (d, J =3.2Hz, 1H); 7.58(t, J=8Hz, 1H); 7.80 (bs, 2H); 7.94(s, 1H). |
| | 4-[4-[2-[5-Amino-8-(2-furyl)-1-methyl-2-oxo-[1,2,4]triazolo[5,1-f]purin-3-yl]ethyl]piperazin-1-yl]-2-fluoro-benzonitrile. | |
| A34 | 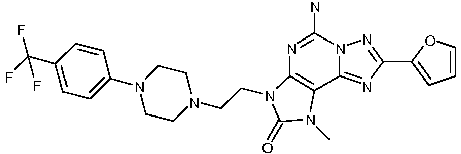 | HNMR(400MHz, DMSO d6): δ 2.61 (bs, 4H); 2.67-2.69 (m, 2H); 3.22(bs, 4H); 3.57(s, 3H); 3.97 (bs, 2H); 6.72 (bs, 1H); 7.03(d, J=8.4Hz, 2H); 7.19 (bs,1H); 7.48(d, J=8.4 Hz, 2H); 7.82 (bs, 2H); 7.94 (s, 1H). |
| | 5-Amino-8-(2-furyl)-1-methyl-3-[2-[4-[4-(trifluoromethyl)phenyl]piperazin-1-yl]ethyl]-[1,2,4]triazolo[5,1-f]purin-2-one. | |
| A35 | 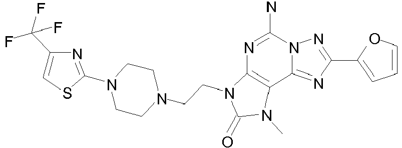 | HNMR(400MHz, DMSO d6): δ 2.60 (t, J=4.8Hz, 4H); 2.67-2.71 (m, 2H); 3.31- 3.37 (m, 4H); 3.57(s, 3H); 3.95(t, J=6.4Hz, 2H); 6.72 (dd, J=2Hz, 3.6Hz, 1H); 7.19(d, J =3.2Hz, 1H); 7.54 (d, J=0.8Hz, 1H); 7.81 (bs, 2H); 7.93 (d, J=0.8 Hz,1H). |
| | 5-Amino-8-(2-furyl)-1-methyl-3-[2-[4-[4-(trifluoromethyl)thiazol-2-yl]piperazin-1-yl]ethyl]-[1,2,4]triazolo[5,1-f]purin-2-one. | |
| A36 | 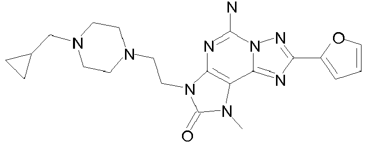 | ¹HNMR(400MHz, CDCl₃): δ 0.09 (d, J=4.4Hz, 2H); 0.50 (d, J=6.8Hz, 2H); 0.82-0.89 (m, 1H); 2.24 (d, J=6.0Hz, 2H): 2.52-2.72 (m, 8H); 2.80 (t, J=6.4Hz, 2H); 3.76 (s, 3H); 4.07 (t, J=6.8Hz, 2H); 5.89 (bs, 2H);6.61 (bs, 1H); 7.22 (d, J=2.4Hz, 1H); 7.64 (s, 1H). |
| | 5-Amino-3-[2-[4-(cyclopropylmethyl)piperazin-1-yl]ethyl]-8-(2-furyl)-1-methyl-[1,2,4]triazolo[5,1-f]purin-2-one | |
| A37 | 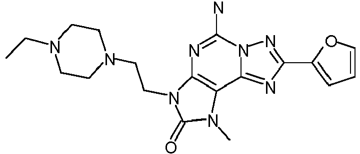 | ¹HNMR(400MHz, CDCl₃): δ 1.08 (t, J=7.6Hz, 3H); 2.39-2.41 (m, 4H); 2.45-2.52 (m, 2H); 2.62-2.66 (m, 4H); 2.78 (t, J=6.8Hz, 2H); 3.76 (s, 3H); 4.07 (t, J=6.4Hz, 2H); 5.82 (bs, 2H); 6.60 (s, 1H); 7.22 (d, J=3.2Hz, 1H); 7.64 (s, 1H). |
| | 5-Amino-3-[2-(4-ethylpiperazin-1-yl)ethyl]-8-(2-furyl)-1-methyl-[1,2,4]triazolo[5,1-f]purin-2-one | |
| A38 | 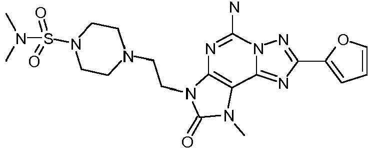 | ¹HNMR(400MHz, CDCl₃): δ 2.62 (t, J=4.4Hz, 4H); 2.79 (t, J=6.4Hz, 2H); 2.81 (s, 6H); 3.22 (t, J=4.4Hz, 4H): 3.77 (s, 3H); 4.06 (t, J=6.8Hz, 2H); 5.74 (bs, 2H); 6.60 (dd, J=2.0Hz, 3.2Hz, 1H); 7.24 (d, J=3.6Hz, 1H); 7.65 (s, 1H). |
| | 4-[2-[5-Amino-8-(2-furyl)-1-methyl-2-oxo-[1,2,4]triazolo[5,1-f]purin-3-yl]ethyl]-N,N-dimethyl-piperazine-1-sulfonamide | |
| A39 | 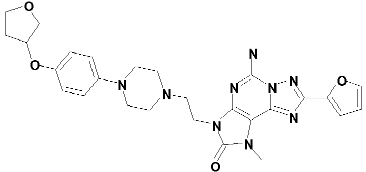 | ¹HNMR(400MHz, DMSO d6): δ 1.89-1.94 (m, 1H); 2.09-2.18 (m, 1H); 2.60 (bs, 4H); 2.67 (t, J=6.4Hz, 2H); 2.96 (bs, 4H); 3.56 (s, 3H); 3.69-3.85 (m, 4H); 3.95 (t, J=6.4Hz, 2H); 4.89 (bs, 1H); 6.72 (dd, J=2.0, 3.2Hz, 1H); 6.78 (d, J=9.2Hz, 2H); 6.85 (d, J=9.2Hz, 2H): 7.20 (d, J=3.2Hz, 1H); 7.80 (bs, 2H); 7.93 (s, 1H). |
| | 5-Amino-8-(2-furyl)-1-methyl-3-[2-[4-(4-tetrahydrofuran-3-yloxyphenyl)piperazin-1-yl]ethyl]-[1,2,4]triazolo[5,1-f]purin-2-one | |
| A40 | 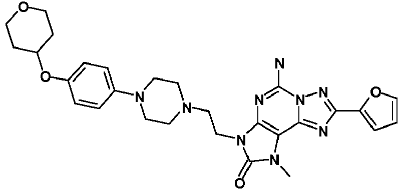 | ¹HNMR (400MHz, DMSO d6): δ 1.47-1.56 (m, 2H); 1.88-1.92 (m,2H); 2.60 (bs, 4H);2.67 (t, J=6.4Hz, 2H); 2.95 (bs, 4H); 3.57 (s, 3H); 3.57-3.61 (m, 2H); 3.79-3.84 (m, 2H); 3.95 (t, J=6.4Hz, 2H); 4.38 (sep, J=4.0Hz, 1H); 6.72 (dd, J=2.0, 3.6Hz, 1H); 6.83 (s, 4H); 7.20 (d, J=3.2Hz, 1H): 7.79 (bs, 2H); 7.94 (s, 1H). |
| | 5-Amino-8-(2-furyl)-1-methyl-3-[2-[4-(4-tetrahydropyran-4-yloxyphenyl)piperazin-1-yl]ethyl]-[1,2,4]triazolo[5,1-f]purin-2-one | |
| A41 | 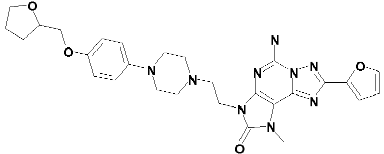 | ¹HNMR(400MHz, DMSO d6): δ 1.59-1.67 (m, 1H); 1.79-1.88 (m 2H); 1.93-2.01 (m, 1H); 2.60 (bs, 4H); 2.67 (t, J=6.4Hz, 2H); 2.95 (bs, 4H); 3.56 (s, 3H); 3.65 (dd, J=7.6Hz,14.0Hz, 1H); 3.76 (dd, J=6.8Hz, 14.4Hz, 1H); 3.80-3.86 (m, 2H); 3.95 (t, J=6.8Hz, 2H); 4.06-4.12 (m, 1H); 6.72 (dd, J=2.0, 3.2Hz, 1H); 6.78-6.85 (m, 4H); 7.29 (d, J=3.2Hz, 1H): 7.80 (bs, 2H); 7.94 (s, 1H). |
| | 5-Amino-8-(2-furyl)-l-methyl-3-[2-[4-[4-(tetrahydrofuran-2-ylmethoxy)phenyl]piperazin-1-yl]ethyl]-[1,2,4]triazolo[5,1-f]purin-2-one | |
| A42 | 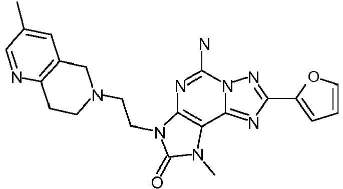 | ¹HNMR(400MHz, CDCl₃): δ 2.26 (s,3H); 2.94-2.97 (m, 6H); 3.72 (s, 2H); 3.75 (s, 3H); 4.17 (t, J=6.4Hz, 2H); 5.74 (bs, 2H); 6.59 (dd, J=1.6Hz, 3.6Hz, 1H);7.13 (s, J=3.6Hz, 1H); 7.21-7.24 (m, 1H); 7.63 (s, 1H); 8.20 (bs, 1H). |
| | 5-Amino-8-(2-furyl)-1-methyl-3-[2-(3-methyl-7,8-dihydro-5H-1,6-naphthyridin-6-yl)ethyl]-[1,2,4]triazolo[5,1-f]purin-2-one | |
| A43 | 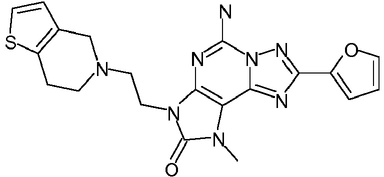 | ¹HNMR(400MHz, DMSO d6): δ 2.74 (bs, 2H); 2.79-2.86 (m, 4H); 3.55 (s, 5H); 4.00 (t, J=6.0Hz, 2H); 6.72 (bs, 1H); 6.78 (d, J=5.2Hz, 1H); 7.19 (d, J=3.2Hz, 1H); 7.24 (d, J=4.8Hz, 1H); 7.81 (bs, 2H): 7.93 (s, 1H). |
| | 5-Amino-3-[2-(6,7-dihydro-4H-thieno[3,2-c]pyridin-5-yl)ethyl]-8-(2-furyl)-1-methyl-[1,2,4]triazolo[5,1-f]purin-2-one | |
| A44 | 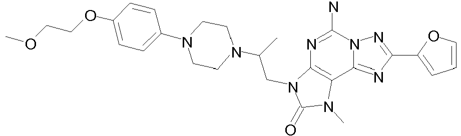 | ¹HNMR(400MHz, DMSO d6): δ 0.97 (d, J=6.4Hz, 3H); 2.84-2.86( m, 8H); 3.23 (dd, J=7.2Hz, 14.0Hz, 1H); 3.28 (s, 3H); 3.56 (s, 3H); 3.60 (t, J=4.4Hz, 2H); 3.69 (dd, J=6.8Hz, 14.0Hz, 1H); 3.93 (dd, J=8.4Hz, 13.6Hz, 1H); 3.97 (t, J=4.4Hz, 2H); 6.71-6.72 (m, 1H); 6.79 (q, J=9.6Hz, 4H); 7.19 (d, J=3.6Hz, 1H); 7.77 (bs, 2H); 7.93 (s, 1H). |
| | 5-Amino-8-(2-furyl)-3-[2-[4-[4-(2-methoxyethoxy)phenyl]piperazin-1-yl]propyl]-1-methyl-[1,2,4]triazolo[5,1-f]purin-2-one | |
| A45 | 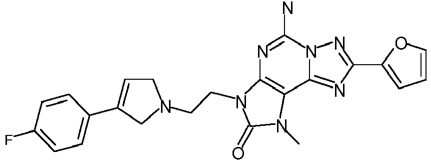 | ¹HNMR (400MHz, DMSO d6): δ 3.01 (t, J=6.4 Hz, 2H); 3.56 (s, 3H); 3.66 (bs, 2H); 3.85 (bs, 2H); 3.95 (t, J=6.4 Hz, 2H); 6.26 (s, 1H); 6.72-6.73 (m, 1H); 7.15-7.20 (m, 3H): 7.43-7.47 (m, 2H); 7.84 (bs, 2H); 7.94-7.94 (m, 1H) |
| | 5-Amino-3-[2-[3-(4-fluorophenyl)-2,5-dihydropyrrol-1-yl]ethyl]-8-(2-furyl)-1-methyl-[1,2,4]triazolo[5,1-f]purin-2-one | |
| A46 | 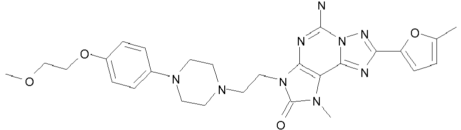 | ¹HNMR(400MHz, DMSO d6): δ 2.38 (s, 3H); 2.58 (bs, 4H); 2.66 (t, J=5.6Hz, 2H); 2.93 (bs, 4H); 3.27 (s, 3H); 3.54 (s, 3H); 3.57-3.59 (m, 2H); 3.91-3.97 (m, 4H); 6.31 (d, J=2.4Hz, 1H); 6.76-6.83 (m, 4H); 7.06 (d, J=2.8Hz, 1H); 7.75 (bs, 2H). |
| | 5-Amino-3-[2-[4-[4-(2-methoxyethoxy)phenyl]piperazin-1-yl]ethyl]-1-methyl-8-(5-methyl-2-furyl)-[1,2,4]triazolo[5,1-f]purin-2-one | |
| A47 | 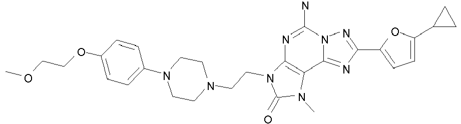 | ¹HNMR(400MHz, DMSO d6): δ 0.75-0.79 (m,2H); 0.92-0.97 (m, 2H); 2.03-2.05 (m,1H); 2.58 (bs, 4H); 2.65 (bs, 2H); 2.93 (bs, 4H); 3.27 (s, 3H); 3.53 (s, 3H); 3.59 (t, J=4.4Hz, 2H); 3.93-3.98 (m, 4H); 6.27 (d, J=3.6Hz, 1H): 6.77-6.84 (m, 4H); 7.05 (d, J=3.6Hz, 1H); 7.74 (bs, 2H). |
| | 5-Amino-8-(5-cyclopropyl-2-furyl)-3-[2-[4-[4-(2-methoxyethoxy)phenyl]piperazin-1-yl] ethyl]-1-methyl-[1,2,4]triazolo[5,1-f]purin-2-one | |
| A48 | 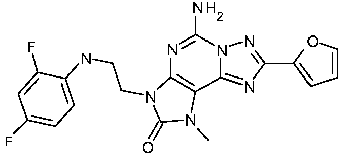 | ¹HNMR (400MHz, DMSO d6): δ 3.36-3.41 (m, 2H); 3.53 (s, 3H); 3.95 (t, J=6.8Hz, 2H); 5.6 (bs, 1H); 6.72 (dd, J=2Hz, 3.6Hz, 1H); 6.82-6.92 (m, 2H); 7.00-7.07 (m, 1H); 7.18 (d, J=3.6Hz, 1H); 7.80 (bs, 2H); 7.92 (bs, 1H) |
| | 5-Amino-3-[2-(2,4-difluoroanilino)ethyl]-8-(2-furyl)-1-methyl-[1,2,4]triazolo[5,1-f]purin-2-one | |
| A49 | 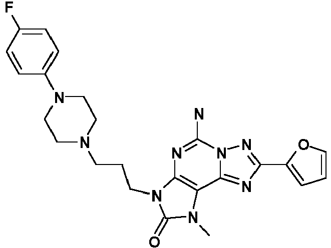 | HNMR(400MHz, CD3OD): δ 2.05 (t, J=7.6Hz , 2H), 2.51 (t, J=7.2Hz, 2H), 2.56 (t, J=5.2Hz, 4H); 2.98 (t, J=5.2Hz, 4H); 3.70(s, 3H); 4.03 (t, J=6.4Hz, 2H); 6.65 (dd, J=2Hz, 3.6Hz, 1H); 6.89-6.92 (m, 4H); 7.25 (dd, J=3.2Hz, 0.8Hz,1H); 7.75 (d, J=0.8Hz, 1H). |
| | 5-Amino-3-[3-[4-(4-fluorophenyl)piperazin-1-yl]propyl]-8-(2-furyl)-1-methyl-[1,2,4]triazolo[5,1-f]purin-2-one | |
| A50 | 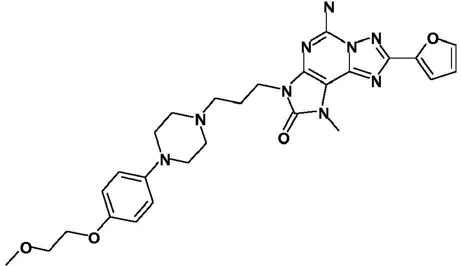 | HNMR(400MHz, DMSO d6): δ 1.89(t, J=7.2Hz, 2H), 2.38 (t, J=6.4Hz, 2H), 2.44 (bs, 4H); 2.90 (bs, 4H); 3.29 (s, 3H); 3.56 (s, 3H); 3.61 (t, J=4.8Hz, 2H); 3.87 (t, J=7.2Hz, 2H); 3.98(t, J=4.8Hz, 2H); 6.72 (dd, J=2Hz, 3.6Hz, 1H); 6.76-6.82 (m, 4H); 7.2(d, J=3.2Hz,1H); 7.80 (bs, 2H);7.94(s, 1H). |
| | 5-Amino-8-(2-furyl)-3-[3-[4-[4-(2-methoxyethoxy)phenyl]piperazin-1-yl]propyl]-1-methyl-[1,2,4]triazolo[5,1-f]purin-2-one | |
| A51 | 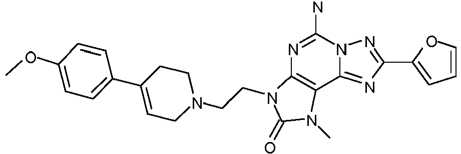 | ¹H NMR(400MHz, DMSO d6): δ 2.37 (bs, 2H); 2.69(t, J=5.2Hz, 2H); 2.74 (t, J=6.4Hz, 2H); 3.13 (bs, 2H); 3. 54 (s, 3H); 3.71 (s, 3H); 3.96 (t, J=6.4Hz, 2H); 5.99 (bs, 1H); 6.72(dd, J=2Hz, 3.6Hz, 1H); 6.85 (d, J=8.8Hz, 2H); 7.17 (d, J=3.6Hz,1H); 7.31 (d, J=8.8Hz, 2H); 7.79 (bs, 2H); 7.91(s, 1H). |
| | 5-Amino-8-(2-furyl)-3-[2-[4-(4-methoxyphenyl)-3,6-dihydro-2H-pyridin-1-yl]ethyl]-1-methyl-[1,2,4]triazolo[5,1-f]purin-2-one | |
| A52 | 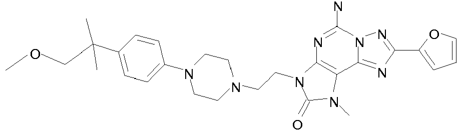 | ¹HNMR (400MHz, DMSO d6): δ 1.20 (bs, 6H); 2.60 (bs, 4H); 2.68 (t, J=6.8Hz, 2H); 3.03 (bs, 4H); 3.19 (s, 3H); 3.27 (s, 2H); 3.56 (s, 3H); 3.96 (t, J=6.4Hz, 2H); 6.72 (dd, J=2Hz, 3.6Hz, 1H); 6.82 (d, J=8.4Hz, 2H);7.17-7.20 (m, 3H); 7.80 (bs, 2H); 7.94 (s, 1H). |
| | 5-Amino-8-(2-furyl)-3-[2-[4-[4-(2-methoxy-1,1-dimethyl-ethyl)phenyl]piperazin-1-yl]ethyl]-1-methyl-[1,2,4]triazolo[5,1-f]purin-2-one | |
| A53 | 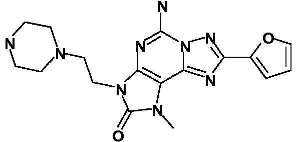 | ¹HNMR (400MHz, DMSO d6): δ 2.35 (bs, 4H); 2.57 (t, J=6.8Hz, 2H); 2.60-2.63 (m, 4H); 3.56 (s, 3H); 3.90 (t, J=6.8Hz, 2H); 6.72 (dd, J=2Hz, 3.6Hz, 1H); 7.19 (d, J=2.8Hz, 1H); 7.79 (bs, 2H); 7.94 (d, J=1.2Hz, 1H). |
| | 5-Amino-8-(2-furyl)-1-methyl-3-(2-piperazin-1-ylethyl)-[1,2,4]triazolo[5,1-f]purin-2-one | |
| A54 | 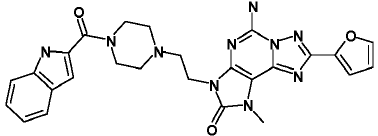 | ¹HNMR (400MHz, DMSO d6): δ 2.55 (bs, 4H); 2.68 (t, J=6Hz, 2H); 2.84 (s, 3H); 3.69 (bs, 4H); 3.96 (t, J=6Hz, 2H); 6.72 (dd, J=2Hz, 3.6Hz, 1H); 6.75 (s, 1H); 7.03 (t, J=8Hz, 1H); 7.15-7.20 (m, 2H): 7.40 (d, J=8.4Hz, 1H); 7.59 (d, J=8Hz, 1H); 7.81 (bs, 2H); 7.94 (d, J=1.6Hz, 1H), 11.59(bs,1H). |
| | 5-Amino-8-(2-furyl)-3-[2-[4-(1H-indole-2-carbonyl)piperazin-1-yl]ethyl]-1-methyl-[1,2,4]triazolo[5,1-f]purin-2-one | |
| A55 | 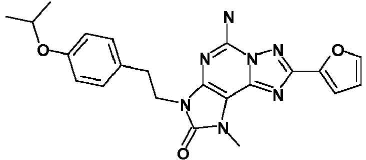 | HNMR(400MHz, DMSO d6): δ 1.21 (d, J=6Hz, 6H); 2.95 (t, J=7.6Hz, 2H); 3.55 (s, 3H); 3.98 (t, J=7.6Hz, 2H); 4.50-4.56 (m,1H), 6.73 (bs, 1H); 6.81 (d, J=8.4Hz, 2H); 7.09 (d, J=8.4Hz, 2H); 7.20 (d, J=2.4Hz, 1H); 7.81 (bs, 2H), 7.94 (s, 1H). |
| | 5-Amino-8-(2-furyl)-3-[2-(4-isopropoxyphenyl)ethyl]-1-methyl-[1,2,4]triazolo[5,1-f]purin-2-one | |
| A56 | 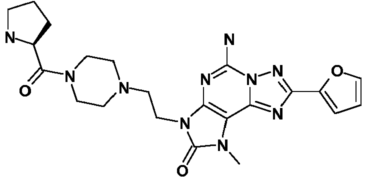 | ¹HNMR (400MHz, DMSO d6): δ 1.51-1.59 (m, 2H); 1.66-1.71 (m, 1H); 1.98-2.03 (m, 1H); 2.41-2.49 (m, 4H); 2.63-2.70 (m, 3H); 2.98-3.03 (m, 1H); 3.37-3.42 (m, 4H); 3.56 (s, 3H); 3.88 (t, J=7.6Hz, 1H); 3.93 (t, J=6.4Hz, 2H); 6.72 (dd, J=2Hz, 3.6Hz, 1H): 7.20 (d, J=3.2Hz, 1H); 7.80 (bs, 2H); 7.92-7.94 (m, 1H). |
| | 5-Amino-8-(2-furyl)-1-methyl-3-[2-[4-[(2S)-pyrrolidine-2-carbonyl]piperazin-1-yl]ethyl]-[1,2,4]triazolo[5,1-f]purin-2-one | |
| A57 | 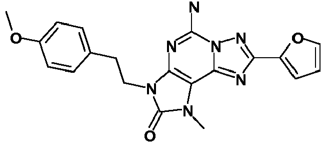 5-Amino-8-(2-furyl)-3-[2-(4-methoxyphenyl)ethyl]-1-methyl-[1,2,4]triazolo[5,1-f]purin-2-one | HNMR(400MHz, DMSO d6): δ 2.95 (t, J=8Hz, 2H); 3.52 (s, 3H); 3.69 (s, 3H), 3.97 (t, J=8Hz, 2H); 6.71 (dd, J=2Hz, 3.6Hz, 1H); 6.80 (dd, J=2Hz, 6.8Hz, 2H); 7.10 (d, J=8.8Hz, 2H); 7.18 (dd, J=0.8Hz, 3.2Hz, 1H); 7.80 (bs, 2H), 7.94 (dd, J=1Hz, 2Hz, 1H). |
| A58 | 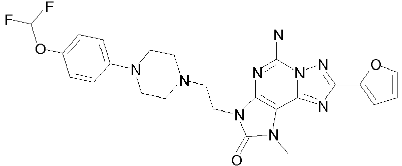 | HNMR(400MHz, DMSO d6): δ 2.61 (bs, 4H); 2.68 (bs, 2H); 3.05(bs, 4H); 3.57 (s, 3H), 3.96 (bs, 2H); 6.72 (bs, 1H); 6.92 (d, J=8Hz, 2H); 7.01 (d, J=10Hz, 2H); 7.03(d, J=148Hz, 1H); 7.19 (bs ,1H); 7.80 (bs, 2H); 7.94 (s, 1H). |
| | 5-amino-3-[2-[4-[4-(difluoromethoxy)phenyl]piperazin-1-yl]ethyl]-8-(2-furyl)-1-methyl-[1,2,4]triazolo[5,1-f]purin-2-one | |
| A59 | 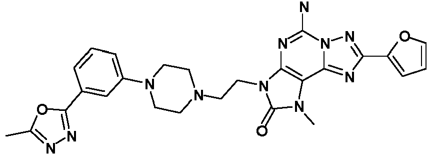 | ¹HNMR (400MHz, DMSO d6): δ 2.57 (s, 3H); 2.64 (bs, 4H); 2.70 (t, J=6.8Hz, 2H); 3.17 (bs, 4H); 3.56 (s, 3H); 3.97 (t, J=6.8Hz, 2H); 6.72 (dd, J=1.6Hz, 3.6Hz, 1H); 7.15-7.17 (m, 1H); 7.19 (d, J=3.2Hz, 1H); 7.33-7.41 (m, 3H); 7.81 (bs, 2H); 7.94(bs, 1H). |
| | 5-Amino-8-(2-furyl)-1-methyl-3-[2-[4-[3-(5-methyl-1,3,4-oxadiazol-2-yl)phenyl]piperazin-1-yl]ethyl]-[1,2,4]triazolo[5,1-f]purin-2-one | |
| A60 | 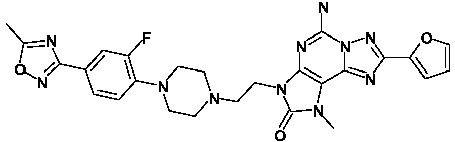 | ¹HNMR (400MHz, DMSO d6): δ 2.62 (s, 3H); 2.64 (bs, 4H); 2.69 (t, J=6.4Hz, 2H); 3.07 (bs, 4H); 3.56 (s, 3H); 3.95 (t, J=6.8Hz, 2H); 6.72 (dd, J=1.6Hz, 3.6Hz, 1H); 7.12 (t, J=8.8Hz, 1H); 7.18 (d, J=2.8Hz, 1H); 7.59-7.63 (m, 1H); 7.69-7.72 (m, 1H); 7.78 (bs, 2H); 7.92-7.93 (m, 1H). |
| | 5-Amino-3-[2-[4-[2-fluoro-4-(5-methyl-1,2,4-oxadiazol-3-yl)phenyl]piperazin-1-yl]ethyl]-8-(2-furyl)-1-methyl-[1,2,4]triazolo[5,1-f]purin-2-one | |
| A61 | 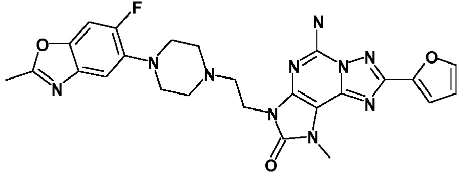 | ¹HNMR (400MHz, DMSO d6): δ 2.56 (s, 3H); 2.67 (bs, 4H); 2.70 (t, J=6.8Hz, 2H); 2.94 (bs, 4H); 3.57 (s, 3H); 3.96 (t, J=6.8Hz, 2H); 6.72 (dd, J=1.6Hz,3.6 Hz, 1H); 7.20 (dd, J=1.2Hz,3.6 Hz, 1H); 7.26 (d, J=8Hz, 1H); 7.61 (d, J=11.2Hz, 1H); 7.80 (bs, 2H); 7.93-7.94 (m, 1H) |
| | 5-Amino-3-[2-[4-(6-fluoro-2-methyl-1,3-benzoxazol-5-yl)piperazin-1-yl]ethyl]-8-(2-furyl)-1-methyl-[1,2,4]triazolo[5,1-f]purin-2-one | |
| A62 | 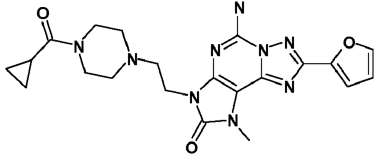 | ¹HNMR (400MHz, DMSO d6): δ 0.66-0.70 (m, 4H); 1.90-1.94 (m,1H); 2.41 (bs, 4H); 2.65 (t, J=6Hz, 2H); 3.38 (bs, 2H); 3.56 (bs, 5H); 3.93 (t, J=6.4 Hz, 2H); 6.71 (bs, 1H); 7.19 (d, J=2.4Hz, 1H); 7.79 (bs, 2H); 7.93 (bs, 1H). |
| | 5-Amino-3-[2-[4-(cyclopropanecarbonyl)piperazin-1-yl]ethyl]-8-(2-furyl)-1-methyl-[1,2,4]triazolo[5,1-f]purin-2-one | |
| A63 | 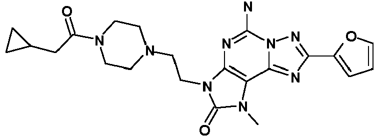 | ¹HNMR (400MHz, DMSO d6): δ 0.07-0.10 (m, 2H); 0.40-0.44 (m, 2H); 0.88-0.94 (m,1H); 2.21 (d, J=6.4Hz, 2H); 2.41-2.45 (m, 4H); 2.64 (t, J=6.4Hz, 2H); 3.38 (bs,4H); 3.56 (s, 3H); 3.93 (t, J=6.4Hz, 2H); 6.72 (dd, J=2Hz,3.6 Hz, 1H); 7.19-7.20 (m, 1H); 7.80 (bs, 2H); 7.93 (d, J=0.8 Hz, 1H). |
| | 5-Amino-3-[2-[4-(2-cyclopropylacetyl)piperazin-1-yl]ethyl]-8-(2-furyl)-1-methyl-[1,2,4]triazolo[5,1-f]purin-2-one | |

### Example B1: 5-Amino-8-(2-furyl)-3-[2-[4-[4-(2-hydroxyethoxy)phenyl]piperazin-1-yl]methyl]-1-methyl-[1,2,4]triazolo[5,1-f]purin-2-one

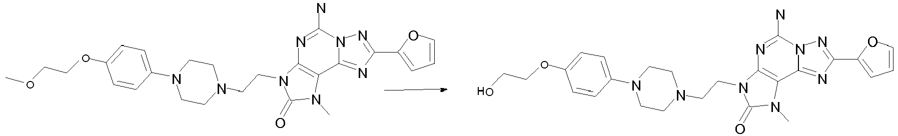

To a solution of compound 5-amino-8-(2-furyl)-3-[2-[4-[4-(2-methoxyethoxy)phenyl]piperazin-1-yl]ethyl]-1-methyl-[1,2,4]triazolo[5,1-f]purin-2-one (Example A1) (0.075g, 0.140mmol), in DCM (10ml) was added BBr₃ (0.15ml, 0. 154mmol) drop wise at 0 °C and stirred at 25°C for 20 hours. The reaction mixture was quenched with sat.NaHCO₃ (25ml) and extracted with DCM (3 x 20ml) .The crude product was purified by column chromatography to obtain 5-amino-8-(2-furyl)-3-[2-[4-[4-(2-hydroxyethoxy)phenyl]piperazin-1-yl]ethyl]-1-methyl-[1,2,4]triazolo[5,1-f]purin-2-one (70mg, 90%) as an off white solid

HNMR(400MHz, DMSO d6): δ 2.60 (bs, 4H); 2.67 (t, J=6Hz, 2H); 2.95 (bs, 4H); 3.55 (s, 3H); 3.65(q, J=5.2Hz, 2H); 3.87 (t, J=5.2Hz, 2H); 3.95 (t, J=6.4Hz, 2H); 4.8(t, J=5.2Hz, 1H); 6.71-6.72 (m, 1H); 6.78-6.85 (m, 4H); 7.19 (d, J=2.8Hz, 1H); 7.79 (bs, 2H); 7.93 (bs, 1H). Examples B2 were prepared by following similar experimental procedure of Example B1 using the appropriate intermediates

| | | |
|---|---|---|
| B2 | 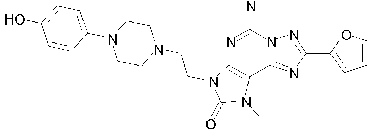 | ¹HNMR(400MHz, DMSO d6): δ 2.59 (bs, 4H); 2.67 (t, J=6.4Hz, 2H); 2.90 (bs, 4H); 3.56 (s, 3H); 3.95 (t, J=6.4Hz, 2H); 6.62 (d, J=8.8Hz, 2H); 6.72-6.74 (m, 1H); 6.74 (d, J=9.2Hz, 2H); 7.19 (d, J=3.2Hz, 1H); 7.80 (bs, 2H): 7.94 (bs, 1H); 8.80 (s, 1H). |
| | 5-Amino-8-(2-furyl)-3-[2-[4-(4-hydroxyphenyl)piperazin-1-yl]ethyl]-1-methyl-[1,2,4]triazolo[5,1-f]purin-2-one | |

### Example C1: 5-Amino-1-(cyclopropylmethyl)-3-[2-[4-(4-ethoxyphenyl)piperazin-1-yl]ethyl]-8-(2-fury)-[1,2,4]triazolo[5,1-f]purin-2-one

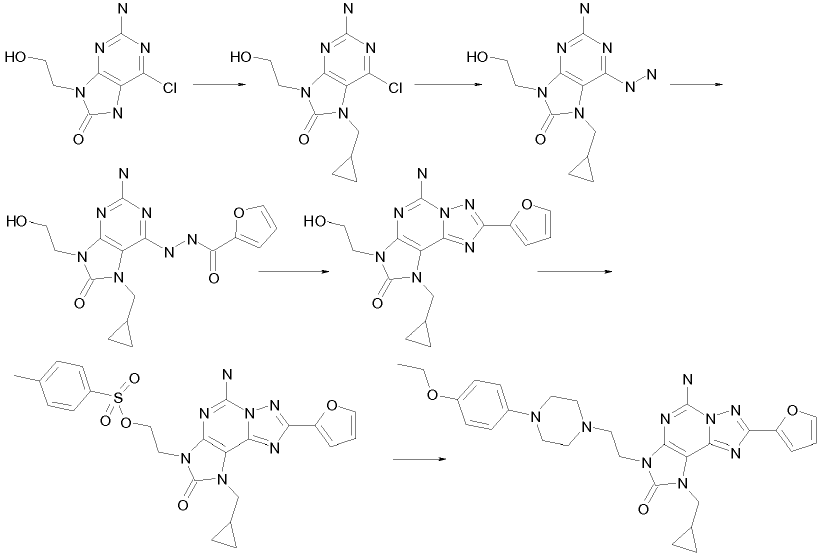

### Step-1 : 2-Amino-6-chloro-7-(cyclopropylmethyl)-9-(2-hydroxyethyl)purin-8-one (procedure is same as step-3 in example A1)

¹HNMR (400MHz, DMSO d6): δ 0.35-0.39 (m, 2H); 0.41-0.49 (m, 2H); 1.16-1.23 (m, 1H), 3.63-3.67 (m, 2H); 3.77-3.81 (m, 4H); 4.87 (t, J=5.2Hz, 1H); 6.71 (bs, 2H)

### Step-2 : 2-Amino-7-(cyclopropylmethyl)-6-hydrazino-9-(2-hydroxyethyl)purin-8-one (procedure is same as step-4 in example A1)

¹HNMR (400MHz, DMSO d6): δ 0.28-0.30 (m, 2H); 0.35-0.37 (m, 2H); 1.00-1.04 (m, 1H), 3.57-3.66 (m, 2H); 3.72-3.79 (m, 4H); 4.33 (bs, 2H); 4.88 (t, J=5.6Hz, 1H); 6.00 (bs, 2H); 7.52 (bs, 1H).

### Step-3: N'-[2-Amino-7-(cyclopropylmethyl)-9-(2-hydroxyethyl)-8-oxo-purin-6-yl]furan-2-carbohydrazide

### (procedure is same as step-5 in example A1)

Crude product was used in next step

### Step-4: 5-Amino-1-(cyclopropylmethyl)-8-(2-furyl)-3-(2-hydroxyethyl)-[1,2,4]triazolo[5,1-f]purin-2-one

### (procedure is same as step-6 in example A1)

¹HNMR (400MHz, DMSO d6): δ 0.46 (d, J=6.8Hz, 4H); 1.34-1.36 (m, 1H), 3.69-3.72 (m, 2H); 3.85-3.90 (m, 4H); 4.90 (t, J=5.6Hz, 1H); 6.72 (bs, 1H); 7.19 (d, J=3.2Hz, 1H); 7.81 (bs, 2H); 7.95 (s, 1H).

### Step-5: 2-[5-Amino-1-(cyclopropylmethyl)-8-(2-furyl)-2-oxo-[1,2,4]triazolo[5,1-f]purin-3-yl]ethyl 4-methylbenzenesulfonate

### (procedure is same as step-7 in example A1)

¹HNMR (400MHz, DMSO d6): δ 0.51 (d, J=7.6Hz, 4H); 1.34-1.36 (m, 1H); 1.99 (s, 3H); 3.79 (d, J=7.2Hz, 2H); 4.02 (t, J=4.8Hz, 2H); 4.47 (t, J=4.8Hz, 2H); 6.73-6.75 (m, 1H); 6.99 (d, J=8Hz, 2H); 7.22-7.23 (m, 1H); 7.42 (d, J=8Hz, 2H); 7.77 (bs, 2H); 7.97 (bs, 1H).

### Step-6: 5-Amino-1-(cyclopropylmethyl)-3-[2-[4-(4-ethoxyphenyl)piperazin-1-yl]ethyl]-8-(2-furyl)-[1,2,4]triazolo[5,1-f]purin-2-one (procedure is same as step-8 in example A1)

¹HNMR (400MHz, DMSO d6): δ 0.46 (bs, 4H); 1.27 (t, J=6.4Hz, 3H); 1.29 (bs, 1H); 2.59 (bs, 4H); 2.69 (s, 2H); 2.93 (bs, 4H); 3.85-3.97 (m, 6H); 6.72 (bs, 1H); 6.76-6.82 (m, 4H); 7.19 (bs, 1H); 7.82 (bs, 2H); 7.94 (s, 1H).

Examples C2-C4 was prepared by following similar experimental procedure of Example C1 using the appropriate intermediates

| **Ex. No** | **Structure / IUPAC name** | **NMR** |
|---|---|---|
| **C2** | 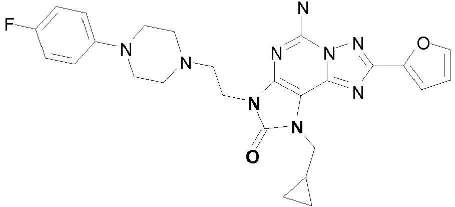 | ¹HNMR (400MHz, DMSO d6): δ 0.44 (d, J= 8 Hz, 4H); 1.32-1.35 (m, 1H); 2.60 (bs, 4H); 2.70 (t, J=7.6Hz, 2H); 3.00 (bs, 4H); 3.86 (d, J=7.2Hz, 2H); 3.98 (t, J=6.8Hz, 2H); 6.72 (bs, 1H); 6.89-6.93 (m, 2H); 7.00-7.05 (m, 2H); 7.19 (d, J=3.6Hz, 1H); 7.83 (bs, 2H); 7.94 (s, 1H). |
| | 5-Amino-1-(cyclopropylmethyl)-3-[2-[4-(4-fluorophenyl)piperazin-1-yl]ethyl]-8-(2-furyl)-[1,2,4]triazolo[5,1-f]purin-2-one | |
| **C3** | 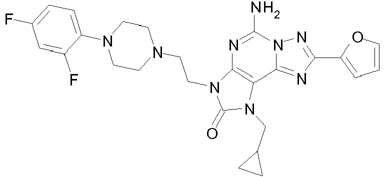 5-Amino-1-(cyclopropylmethyl)-3-[2-[4-(2,4-difluorophenyl)piperazin-1-yl]ethyl]-8-(2-furyl)-[1,2,4]triazolo[5,1-f]purin-2-one | ¹H NMR(400 MHz, DMSO d6): δ 0.44-0.47 (m, 4H); 1.32-1.37 (m, 1H); 2.62 (bs, 4H); 2.71 (t, J=6.4Hz, 2H); 2.89 (bs, 4H); 3.87 (d, J=7.2Hz, 2H); 3.97 (t,J=6.4Hz, 2H); 6.72 (dd, J=2Hz, 3.2Hz, 1H); 6.94-7.04 (m, 2H); 7.17-7.21 (m, 2H); 7.81 (bs, 2H); 7.94 (bs, 1H). |
| **C4** | 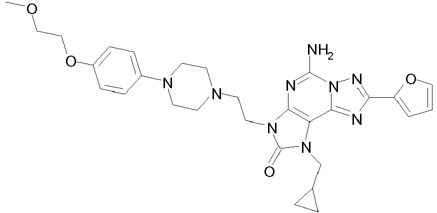 | ¹H NMR(400 MHz, DMSO d6): δ 0.43-0.46 (m, 4H); 1.32-1.37 (m, 1H); 2.57-2.62 (m, 4H); 2.68-2.72 (m, 2H); 2.92-2.96 (m, 4H); 3.29 (s, 3H); 3.58-3.62 (m, 2H); 3.86 (d, J=7.2Hz, 2H); 3.96-3.99 (m, 4H); 6.72 (dd, J=1.6Hz, 3.6Hz, 1H); 6.78-6.85 (m, 4H); 7.18 (d, J=3.2Hz, 1H); 7.82 (bs, 2H); 7.94 (bs, 1H). |
| | 5-Amino-1-(cyclopropylmethyl)-8-(2-furyl)-3-[2-[4-[4-(2-methoxyethoxy)phenyl]piperazin-1-yl]ethyl]-[1,2,4]triazolo[5,1-f]purin-2-one | |

### Example D1 5-Amino-1-(cyclopropylmethyl)-3-[2-(4-fluorophenoxy)ethyl]-8-(2-furyl)-[1,2,4]triazolo[5,1-f]purin-2-one

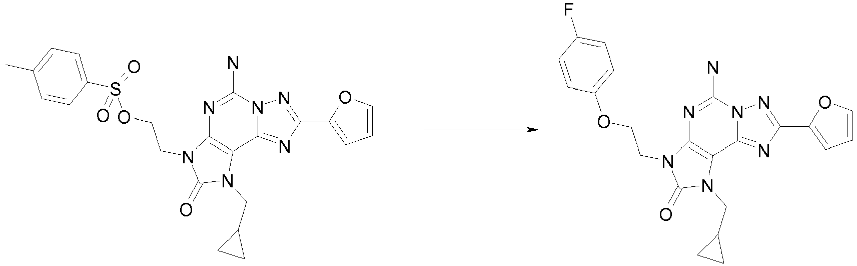

A mixture of 2-[5-amino-1-(cyclopropylmethyl)-8-(2-furyl)-2-oxo-[1,2,4]triazolo[5,1-f]purin-3-yl]ethyl 4-methylbenzenesulfonate obtained in step 7 of C1 (0.045g, 0.088mmol), 4-fluorophenol (0.018g, 0.176mmol) and K₂CO₃ (0.036g, 0.22mmol) were taken in DMF (2ml) and stirred at 80 °C for 16 hours. To the reaction mixture water (25ml) was added and extracted with ethyl acetate (3 x 20ml) .The crude product was purified by column chromatography to 5-amino-1-(cyclopropylmethyl)-3-[2-(4-fluorophenoxy)ethyl]-8-(2-furyl)-[1,2,4]triazolo[5,1-f]purin-2-one (8mg, 20%) as an off white solid
¹HNMR (400MHz, DMSO d6): δ 0.46 (d, J=8.4Hz, 4H); 1.32-1.36 (m, 1H); 3.86 (d, J=7.2Hz, 2H); 4.18 (t, J=5.6Hz, 2H); 4.30 (t, J=6Hz, 2H); 6.72-6.73 (m, 1H); 6.90-6.94 (m, 2H); 7.06-7.11 (m, 2H); 7.19 (d, J=3.2Hz, 1H); 7.87 (bs, 2H); 7.95 (d, J=0.8Hz, 1H). Examples D2-D3 was prepared by following similar experimental procedure of Example D1 using the appropriate intermediates

| | | |
|---|---|---|
| D2 | 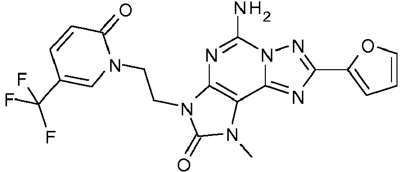 | ¹H NMR(400 MHz, DMSO d6): δ 3.50 (s, 3H); 4.12-4.17 (m, 2H); 4.23-4.28 (m, 2H); 6.49 (d, J=9.6Hz, 1H); 6.72 (dd, J=2Hz, 3.2Hz, 1H);7.20 (d, J=3.2Hz, 1H); 7.54 (dd, J=9.6Hz, 2.8Hz, 1H); 7.70 (bs, 2H); 7.93 (s, 1H); 7.94 (s, 1H). |
| | 5-Amino-8-(2-furyl)-1-methyl-3-[2-[2-oxo-5-(trifluoromethyl)-1-pyridyl]ethyl]-[1,2,4]triazolo[5,1-f]purin-2-one | |
| D3 | 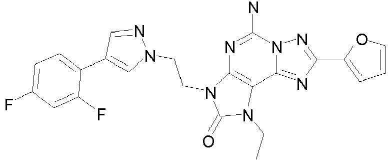 | ¹H NMR(400MHz, DMSO): δ 1.27 (t, J=6.8Hz, 3H);3.97 (q, J=6.8Hz, 2H); 4.21 (bs, 2H); 4.55 (bs, 2H); 6.72 (bs, 1H);7 .07 (t, J=8Hz, 1H); 7.19-7.28 (m, 2H); 7.66(q, J=7.6Hz, 1H); 7.83 (bs, 3H); 7.95 (s, 1H); 8.02 (s, 1H). |
| | 5-Amino-3-[2-[4-(2,4-difluorophenyl)pyrazol-1-yl]ethyl]-1-ethyl-8-(2-furyl)-[1,2,4]triazolo[5,1-f]purin-2-one | |

### Example E1: 1-[2-[5-Amino-1-(cyclopropylmethyl)-8-(2-furyl)-2-oxo-[1,2,4]triazolo[5,1-f]purin-3-yl]ethyl]pyrazole-4-carboxylic acid

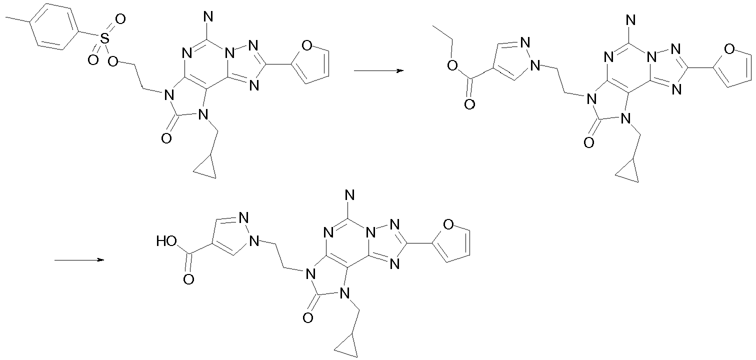

### Step-1: Ethyl 1-[2-[5-amino-1-(cyclopropylmethyl)-8-(2-furyl)-2-oxo-[1,2,4]triazolo[5,1-f]purin-3-yl] ethyl]pyrazole-4-carboxylate

### (carried out as described in Example D1)

¹HNMR (400MHz, DMSO d6): δ 0.41-0.43 (m, 4H);1.21 (t, J=6.8Hz, 3H); 1.32-1.36 (m, 1H); 3.79 (d, J=7.2Hz, 2H); 4.15 (q, J=7.2Hz, 2H); 4.21 (t, J=5.2Hz, 2H); 4.55 t, J=5.2Hz, 2H); 6.72-6.73 (m, 1H); 7.20 (d, J=2.8Hz, 1H); 7.75 (s, 1H); 7.83 (bs, 2H); 7.95 (s, 1H); 8.22 (s, 1H).

### Step-2: 1-[2-[5-Amino-1-(cyclopropylmethyl)-8-(2-furyl)-2-oxo-[1,2,4]triazolo[5,1-f]purin-3-yl]ethyl]pyrazole-4-carboxylic acid

A mixture of ethyl 1-[2-[5-amino-1-(cyclopropylmethyl)-8-(2-furyl)-2-oxo-[1,2,4]triazolo[5,1-f]purin-3-yl]ethyl]pyrazole-4-carboxylate obtained in step 1 (0.055g, 0.115mmol) was dissolved in solution of tetrahydrofuran(6ml) and methanol(4ml). To this reaction mixture a solution of lithium hydroxide (0.013g, 0.576mmol, in 2ml water) was added and stirred at 25-27 °C for 16 hours. The reaction mixture was concentrated and acidified with saturated solution of citric acid to get P^{H}~2-3 and solid obtained was filtered off and washed with water (10ml) followed by diethyl ether (15ml). It was dried to obtain 1-[2-[5-amino-1-(cyclopropylmethyl)-8-(2-furyl)-2-oxo-[1,2,4]triazolo[[5,1-f]purin-3-yl]ethyl]pyrazole-4-carboxylic acid (0.025g, 49 %) as an off white solid.
¹HNMR(400MHz, DMSO d6 HNMR(400MHz, DMSO d6): δ 0.29-0.31 (m, 4H); 1.12-1.16 (m, 1H); 3.67 (d, J=6.8Hz, 2H); 4.08 (bs, 2H); 4.42 (bs, 2H); 6.60 (bs, 1H); 7.07 (bs,1H); 7.57 (s, 1H); 7.71 (bs, 2H); 7.83 (s, 1H); 8.03 (s, 1H); 12.05 (bs, 1H).

Examples E2 was prepared by following similar experimental procedure of Example E1 using the appropriate intermediates

| | | |
|---|---|---|
| E2 | 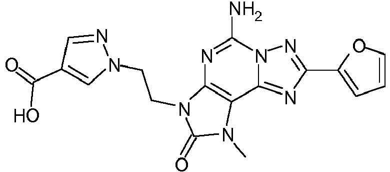 | ¹H NMR(400 MHz, DMSO d6): δ 3.53 (s, 3H); 4.18 (t, J=5.6Hz, 2H); 4.49 (t, J=6.0Hz, 2H); 6.72-6.73 (m, 1H); 7.20 (d, J=3.2Hz, 1H); 7.57 (bs, 1H); 7.82 (bs, 2H); 7.94 (bs, 2H) |
| | 1-[2-[5-Amino-8-(2-furyl)-1-methyl-2-oxo-[1,2,4]triazolo[[5,1-f]purin-3-yl]ethyl]pyrazole-4-carboxylic acid | |

### Example F1: 1-[2-[5-amino-1-(cyclopropylmethyl)-8-(2-furyl)-2-oxo-[1,2,4]triazolo[5,1-f]purin-3-yl]ethyl]-N-cyclopropyl-pyrazole-4-carboxamide

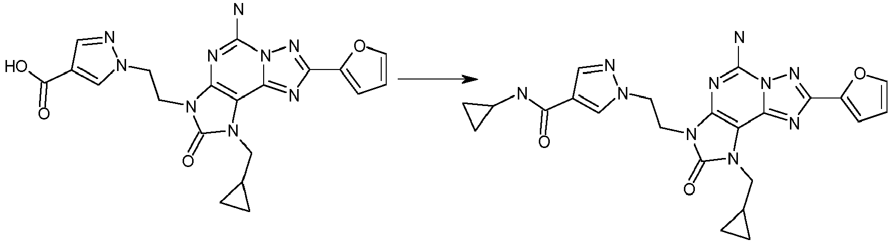

### Step-1 1-[2-[5-Amino-1-(cyclopropylmethyl)-8-(2-furyl)-2-oxo-[1,2,4]triazolo[5,1-f]purin-3-yl]ethyl]-N-cyclopropyl-pyrazole-4-carboxamide

A mixture of 1-[2-[5-amino-1-(cyclopropylmethyl)-8-(2-furyl)-2-oxo-[1,2,4]triazolo[5,1-f]purin-3-yl]ethyl]pyrazole-4-carboxylic acid (0.050g, 0.111mmol) was taken in thionyl chloride (0.04ml, 0.557mmol) and stirred at 75 °C for 2 hours. The reaction mixture was concentrated and residue was dissolved in DCM (2ml). To the above solution cyclopropyl amine (0.015ml, 0.222mmol) was added at 0 °C and stirred at same temperature for 2 hours and filtered to obtain 1-[2-[5-amino-1-(cyclopropylmethyl)-8-(2-furyl)-2-oxo-[1,2,4]triazolo[5,1-f]purin-3-yl]ethyl]-N-cyclopropyl-pyrazole-4-carboxamide (5.0mg, 9 %) as an off white solid.
¹H NMR(400 MHz, DMSO d6): δ 0.4-0.42 (m, 4H); 0.58-0.62 (m, 2H); 0.72-0.76 (m, 2H); 1.21 (bs, 2H); 2.64-2.66 (m, 1H); 3.77 (d, J=7.2Hz, 2H); 4.16 (bs, 2H); 4.50 (bs, 2H); 6.70-6.71 (m, 1H); 7.18 (d, J=3.2Hz, 1H); 7.71 (s, 1H); 7.82 (bs, 2H); 7.93 (bs, 1H); 7.99 (s, 1H).

Examples F2-F12 was prepared by following similar experimental procedure of Example F1 using the appropriate intermediates

| | | |
|---|---|---|
| F2 | 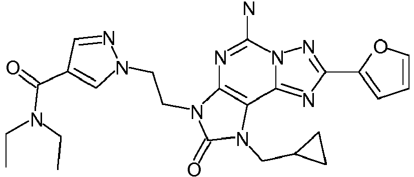 | ¹H NMR(400 MHz, DMSO d6): δ 0.41-0.43 (m, 4H); 1.01 (t, J=7.2Hz, 6H); 1.23-1.27 (m, 1H); 3.24-3.30 (m, 4H); 3.80 (d, J=6.8Hz, 2H); 4.20 (t, J=6.4Hz, 2H); 4.54 (t, J=6.4Hz, 2H); 6.72 (bs, 1H); 7.19 (d, J=3.2Hz, 1H); 7.55 (s, 1H); 7.81(bs, 1H); 7.86(bs, 2H); 7.94 (s, 1H). |
| | 1-[2-[5-amino-1-(cyclopropylmethyl)-8-(2-furyl)-2-oxo-[1,2,4]triazolo[5,1-f]purin-3-yl]ethyl]-N,N-diethyl-pyrazole-4-carboxamide | |
| F3 | 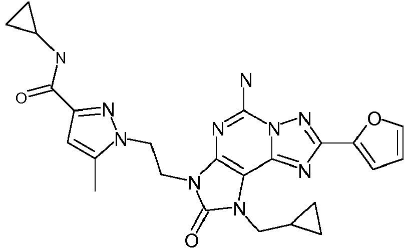 | ¹H NMR(400 MHz, DMSO d6): δ 0.43-0.48 (m, 6H); 0.55-0.58 (m, 2H); 1.28-1.32 (m, 1H); 2.22(s, 3H); 2.64-2.70(m, 1H); 3.81 (d, J=6.8Hz, 2H); 4.15(t, J=6.4Hz, 2H); 4.42 (t, J=6.4Hz, 2H); 6.33 (s, 1H); 6.72 (dd, J=1.6Hz, 3.6Hz, 1H); 7.18 (d, J=3.2Hz, 1H); 7.68 (d, J=4Hz, 1H); 7.81 (bs, 2H); 7.94 (s, 1H). |
| | 1-[2-[5-Amino-1-(cyclopropylmethyl)-8-(2-furyl)-2-oxo-[1,2,4]triazolo[5,1-f]purin-3-yl]ethyl]-N-cyclopropyl-5-methyl-pyrazole-3-carboxamide | |
| F4 | 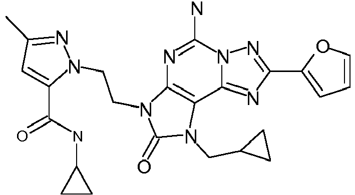 | ¹H NMR(400 MHz, DMSO d6): δ 0.43-0.4 (m, 6H); 0.58-0.62 (m, 2H); 1.26-1.30 (m, 1H); 1.97 (s, 3H); 2.64-2.69 (m, 1H); 3.79 (d, J=6.8Hz, 2H); 4.15 (t, J=4.8Hz, 2H); 4.76 (t, J=4.8Hz, 2H); 6.49 (s, 1H); 6.72 (dd, J=1.6Hz, 3.6Hz, 1H); 7.18 (d, J=3.2Hz, 1H); 7.74 (bs, 2H); 7.95 (s, 1H); 8.21 (d, J=3.6Hz, 1H). |
| | 2-[2-[5-Amino-1-(cyclopropylmethyl)-8-(2-furyl)-2-oxo-[1,2,4]triazolo[5,1-f]purin-3-yl]ethyl]-N-cyclopropyl-5-methyl-pyrazole-3-carboxamide | |
| F5 | 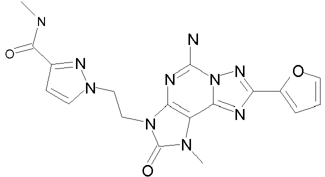 | ¹H NMR(400 MHz, DMSO d6): δ 2.66 (d, J=5.2Hz, 3H); 3.51 (s, 3H); 4.20 (t, J=6Hz, 2H); 4.52 (t, J=6.4Hz, 2H); 6.52 (d, J=2.4Hz, 1H); 6.71 (bs, 1H); 7.18 (d, J=2.8Hz, 1H); 7.70 (d, J=2.4Hz, 1H); 7.78 (bs, 2H); 7.81-7.86(m,1H); 7.92(bs, 1H). |
| | 1-[2-[5-Amino-8-(2-furyl)-1-methyl-2-oxo-[1,2,4]triazolo[5,1-f]purin-3-yl]ethyl]-N-methyl-pyrazole-3-carboxamide | |
| F6 | 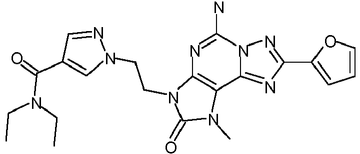 1-[2-[5-Amino-8-(2-furyl)-1-methyl-2-oxo-[1,2,4]triazolo[5,1-f]purin-3-yl]ethyl]-N,N-diethyl-pyrazole-4-carboxamide | ¹H NMR(400 MHz, DMSO d6): δ 1.02 (t, J=6.8Hz, 6H); 3.25-3.34 (m, 4H); 3.52 (s, 3H); 4.19 (t, J=5.6Hz, 2H); 4.52 (t, J=5.6Hz, 2H); 6.72 (bs, 1H); 7.20 (d, J=3.2Hz, 1H); 7.58 (s,1H); 7.79 (bs, 2H); 7.89 (s, 1H); 7.94 (s, 1H) |
| F7 | 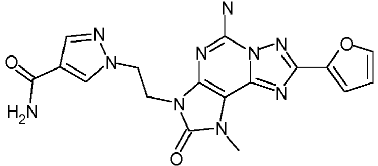 | ¹H NMR(400 MHz, DMSO d6): δ 3.52 (s, 3H); 4.18 (t, J=6Hz, 2H); 4.51 (t, J=6Hz, 2H); 6.71-6.72 (bs, 1H); 6.96 (bs, 1H); 7.20 (d, J=2.8Hz, 1H); 7.46 (bs, 1H); 7.58 (s, 1H); 7.82 (bs, 2H); 7.94 (s, 1H); 8.03 (s, 1H). |
| | 1-[2-[5-amino-8-(2-furyl)-1-methyl-2-oxo-[1,2,4]triazolo[5,1-f]purin-3-yl]ethyl]pyrazole-4-carboxamide | |
| F8 | 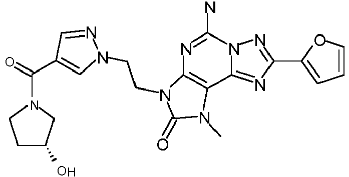 | ¹H NMR(400 MHz, DMSO d6): δ 1.77-1.87 (m, 2H); 3.42-3.48 (m, 2H); 3.53 (s, 3H); 3.59-3.66 (m, 2H); 4.20 (m, 2H); 4.30(bs, 1H); 4.53 (t, J=5.6Hz, 2H); 4.97 (d, J=24.4Hz, 1H); 6.73 (bs, 1H); 7.20 (d, J=3.2Hz, 1H); 7.70 (d, J=6.4Hz, 1H); 7.81 (bs, 2H); 7.95 (s, 1H); 8.10 (d, J=4.4Hz, 1H). |
| | 5-Amino-8-(2-furyl)-3-[2-[4-[(3R)-3-hydroxypyrrolidine-1-carbonyl]pyrazol-1-yl]ethyl]-1-methyl-[1,2,4]triazolo[5,1-f]purin-2-one | |
| F9 | 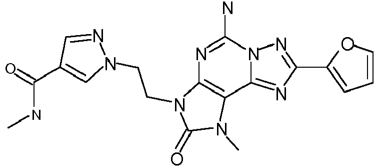 | ¹H NMR(400 MHz, DMSO d6): δ 2.64 (d, J= 4.4Hz, 3H); 3.50 (s, 3H); 4.15 (t, J=5.6Hz, 2H); 4.50 (t, J=5.6Hz, 2H); 6.70 (dd, J=2Hz, 3.6Hz, 1H); 7.18 (dd, J=0.4Hz, 3.6Hz, 1H); 7.72 (s, 1H); 7.80 (bs, 2H); 7.92-7.93 (m, 2H); 8.02 (s, 1H). |
| | 1-[2-[5-Amino-8-(2-furyl)-1-methyl-2-oxo-[1,2,4]triazolo[5,1-f]purin-3-yl]ethyl]-N-methyl-pyrazole-4-carboxamide | |
| F10 | 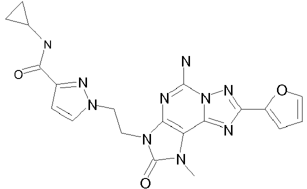 1-[2-[5-Amino-8-(2-furyl)-1-methyl-2-oxo-[1,2,4]triazolo[5,1-f]purin-3-yl]ethyl]-N-cyclopropyl-pyrazole-3-carboxamide | ¹H NMR(400 MHz, DMSO d6): δ 0.46-0.50 (m, 2H); 0.55-0.60 (m, 2H); 2.67-2.71 (m, 1H); 3.51 (s, 3H); 4.18 (t, J=6.4Hz, 2H); 4.51 (t, J=6Hz, 2H); 6.52-6.54 (m, 1H); 6.72(dd, J=2Hz, 3.6Hz, 1H); 7.18 (d, J=3.2Hz, 1H); 7.71 (d, J=2.4Hz, 1H); 7.78 (bs, 2H); 7.84 (d, J=4.4Hz, 1H); 7.92-7.93 (d, J=1.6Hz, 1H). |
| F11 | 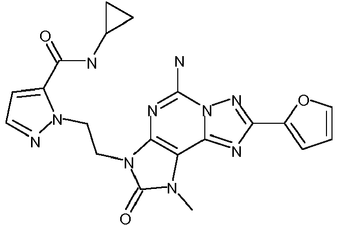 | ¹H NMR(400 MHz, DMSO d6): δ 0.41-0.45 (m, 2H); 0.56-0.60 (m, 2H); 3.06-3.09 (m, 1H); 3.47 (s, 3H); 4.14 (t, J=5.2Hz, 2H); 4.84 (t, J=4.8Hz, 2H); 6.68-6.71 (m, 1H); 7.17 (d, J=3.2Hz, 1H); 7.29 (d, J=2Hz, 1H); 7.69 (bs, 2H); 7.92 (s, 1H); 8.14 (s, 1H); 8.28 (d, J=4Hz, 1H). |
| | 1-[2-[5-Amino-8-(2-furyl)-1-methyl-2-oxo-[1,2,4]triazolo[5,1-f]purin-3-yl]ethyl]-N-cyclopropyl-pyrazole-4-carboxamide | |
| F12 | 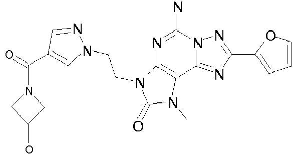 | ¹H NMR(400 MHz, DMSO d6): δ 3.50 (s,3H); 3.61-3.65 (m, 1H); 3.93-3.96 (m, 1H); 4.05-4.12 (m, 1H); 4.17 (t, J=5.6Hz, 2H); 4.35-4.46 (m, 2H); 4.50 (t, J=6.0Hz, 2H); 5.72 (d, J=6.4Hz, 1H); 6.70 (dd, J=1.6 Hz,3.6 Hz, 1H); 7.18 (d, J=3.6Hz, 1H); 7.64 (s, 1H); 7.79 (bs, 2H); 7.92 (s, 1H); 8.05 (s, 1H). |
| | 5-Amino-8-(2-furyl)-3-[2-[4-(3-hydroxyazetidine-1-carbonyl)pyrazol-1-yl]ethyl]-1-methyl-[1,2,4]triazolo[5,1-f]purin-2-one | |

### Example G1: 5-Amino-1-ethyl-8-(2-furyl)-3-[2-[4-[4-(2-methoxyethoxy)phenyl]piperazin-1-yl]ethyl]-[1,2,4]triazolo[5,1-f]purin-2-one

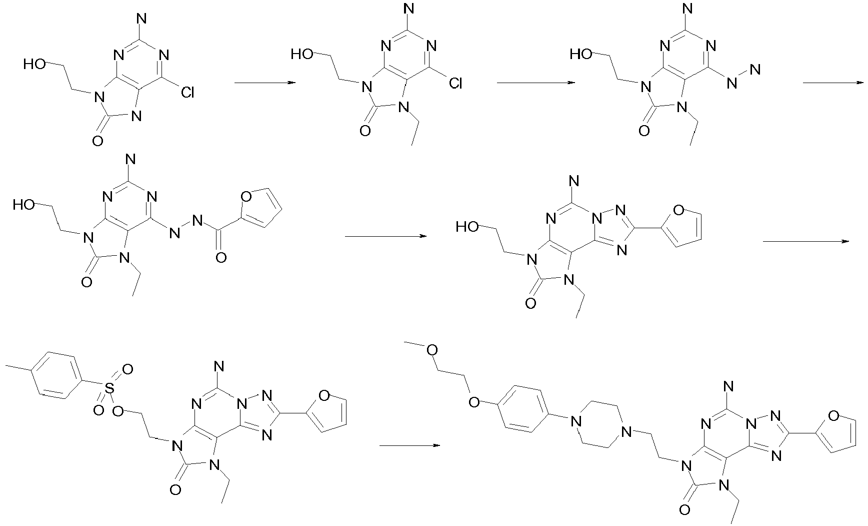

### Step-1 : 2-Amino-6-chloro-7-ethyl-9-(2-hydroxyethyl)purin-8-one

### (Procedure is same as step-3 in example A1)

¹HNMR (400MHz, DMSO d6): δ 1.21 (t, J=7.2Hz, 3H); 3.64 (s, 2H); 3.78 (t, J=6Hz, 2H); 3.92 (q, J=7.2Hz, 2H); 4.92 (bs, 1H); 6.7 (bs, 2H).

### Step-2 : 2-Amino-7-ethyl-6-hydrazino-9-(2-hydroxyethyl)purin-8-one

### (Procedure is same as step-4 in example A1)

¹HNMR (400MHz, DMSO d6): δ 1.07 (t, J=6.8Hz, 3H); 3.59 (q, J=6Hz, 2H); 3.72 (t, J=6Hz, 2H); 3.91 (q, J=6.8Hz, 2H); 4.32 (bs, 2H); 4.86 (t, J=5.6Hz, 1H); 5.99 (bs, 2H), 7.55 (bs, 1H).

### Step-3: N'-[2-Amino-7-ethyl-9-(2-hydroxyethyl)-8-oxo-purin-6-yl]furan-2carbohydrazide (Procedure is same as step-5 in example A1)

Crude product was used in next step

### Step-4: 5-Amino-1-ethyl-8-(2-furyl)-3-(2-hydroxyethyl)-[1,2,4]triazolo[5,1-f]purin-2-one (Procedure is same as step-6 in example A1)

¹HNMR (400MHz, DMSO d6): δ 1.34 (t, J=7.2Hz, 3H); 3.67 (q, J=5.6Hz, 2H); 3.84 (t, J=5.6Hz, 2H); 4.01 (q, J=7.2Hz, 2H); 4.87 (t, J=6Hz, 1H); 6.70 (bs, 1H); 7.17 (d, J=2.8Hz, 1H); 7.18 (bs, 2H); 7.92 (bs, 1H).

### Step-5: 2-[5-Amino-1-ethyl-8-(2-furyl)-2-oxo-[1,2,4]triazolo[5,1-f]purin-3-yl]ethyl 4-methylbenzenesulfonate (procedure is same as step-7 in example A1)

¹HNMR (400MHz, DMSO d6): δ 1.35 (t, J=7.2Hz, 3H); 2.00 (s, 3H); 3.95-4.00 (m, 4H); 4.47 (bs, 2H); 6.74 (s, 1H); 7.00 (d, J=7.6Hz, 2H); 7.22 (s, 1H); 7.42 (d, J=7.6Hz, 2H); 7.78 (bs, 2H); 7.97 (bs, 1H).

### Step-6: 5-Amino-1-ethyl-8-(2-furyl)-3-[2-[4-[4-(2-methoxyethoxy)phenyl]piperazin-1-yl]ethyl]-[1,2,4]triazolo[5,1-f]purin-2-one (procedure is same as step-8 in example A1)

HNMR(400MHz, DMSO d6): δ 1.35 (t, J=7.2Hz, 3H); 2.60 (bs, 4H); 2.68 (t, J=6.8Hz, 2H); 2.95 (bs, 4H); 3.28(s, 3H);3.61 (t, J=4.4Hz, 2H); 3.94-4.04 (m, 6H); 6.72 (dd, J=2Hz, 3.6Hz, 1H); 6.78-6.85 (m, 4H); 7.19 (d, J=3.2Hz, 1H); 7.81(bs, 2H); 7.94 (s, 1H).

Examples G2-G4 was prepared by following similar experimental procedure of Example G1 using the appropriate intermediates

| **Ex. No** | **Structure / IUPAC name** | **NMR** |
|---|---|---|
| **G2** | 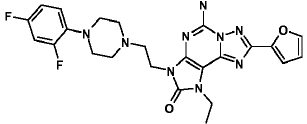 | HNMR(400MHz, DMSO d6): δ 1.35 (t, J=7.2Hz, 3H); 2.62(bs, 4H); 2.69 (t, J=6.4Hz, 2H); 2.89 (bs, 4H); 3.95 (t, J=6.4Hz, 2H); 4.00-4.04 (m, 2H); 6.72 (bs, 1H); 6.94-7.05 (m, 3H); 7.17-7.19 (m, 1H); 7.80 (bs, 2H); 7.94 (s, 1H). |
| | 5-Amino-3-[2-[4-(2,4-difluorophenyl)piperazin-1-yl]ethyl]-1-ethyl-8-(2-furyl)-[1,2,4]triazolo[5,1-f]purin-2-one | |
| **G3** | 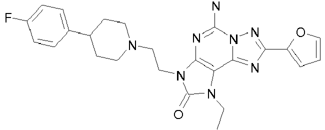 | HNMR(400MHz, DMSO): δ 1.36 (t, J=6.8Hz, 3H); 1.48-1.56(m, 3H); 1.69 (d, J=12Hz, 2H); 2.08 (t, J=10.8 Hz, 2H); 2.66 (t, J=6Hz ,2H); 3.04 (d, J=10.8Hz, 2H); 3.94 (t, J=6Hz, 2H); 4.04 (q, J=7.2Hz, 2H); 6.72 (bs, 1H); 7.08 (t, J=8.8Hz, 2H) ; 7.19-7.25 (m, 3H); 7.80 (bs, 2H); 7.95 (s, 1H). |
| | 5-Amino-1-ethyl-3-{2-[4-(4-fluoro-p henyl)-piperidin-1-yl]-ethyl}-8-fur an-2-yl-1,3-dihydro-[1,2,4]triazolo [5,1-f]purin-2-one | |
| **G4** | 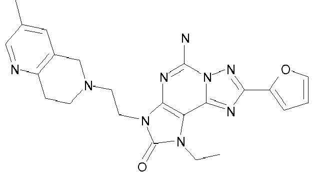 5-Amino-1-ethyl-8-(2-furyl)-3-[2-(3-methyl-7,8-dihydro-5H-1,6-naphthyridin-6-yl)ethyl]-[1,2,4]triazolo[5,1-f]purin-2-one | ¹HNMR(400MHz, DMSO d6): δ 1.30 (t, □□ J=7.2Hz, 3H); 2.19 (s, 3H); 2.74-2.76 (m□□, 2H); 2.82 (t, J=6.8Hz, 4H); 3.62 (s, 2H); 3.97-4.02 (m, 4H); 6.69 (dd, J=1.6Hz,3.2Hz, 1H);7.16 (dd, J=0.8Hz, 3.2Hz, 1H); 7.24 (bs, 1H); 7.79 (bs, 2H); 7.91 (dd, J=0.4Hz, 1.6Hz, 1H); 8.11 (bs, 1H). |

### Example H1: 5-Amino-8-(2-furyl)-3-[2-[4-[4-(2-methoxyethoxy)phenyl]piperazin-1-yl]ethyl]-1-(2,2,2-trifluoroethyl)-[1,2,4]triazolo[5,1-f]purin-2-one

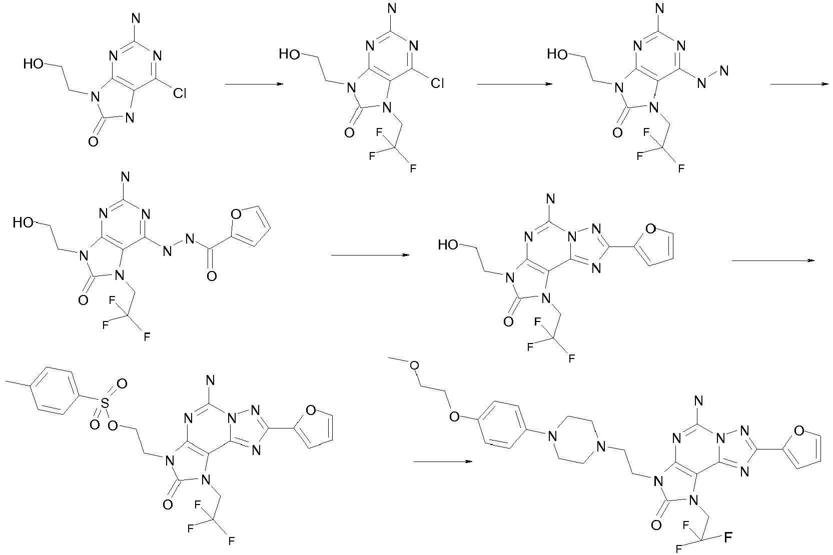

### Step-1 : 2-Amino-6-chloro-9-(2-hydroxyethyl)-7-(2,2,2-trifluoroethyl)purin-8-one (procedure is same as step-3 in example A1)

Crude product was used in next step

### Step-2 : 2-Amino-6-hydrazino-9-(2-hydroxyethyl)-7-(2,2,2-trifluoroethyl)purin-8-one (procedure is same as step-4 in example A1)

¹HNMR (400MHz, DMSO d6): δ 3.59 (t, J=6.4Hz, 2H); 3.73 (t, J=6.4Hz, 2H); 4.37 (bs, 2H); 4.88 (q, J=9.2Hz, 3H); 6.06 (bs, 2H); 7.66 (s, 1H).

### Step-3: N'-[2-Amino-9-(2-hydroxyethyl)-8-oxo-7-(2,2,2-trifluoroethyl)purin-6-yl]furan-2-carbohydrazide (procedure is same as step-5 in example A1)

¹HNMR (400MHz, DMSO d6): δ 3.60-3.64 (m, 2H); 3.78 (t, J=6.4Hz, 2H); 4.91 (q, J=8.8Hz, 3H); 6.15 (bs, 2H); 6.66 (dd, J=2Hz, 3.6Hz, 1H); 7.27 (d, J=3.2Hz, 1H); 7.90 (s, 1H); 8.44 (s, 1H); 10.28 (s, 1H).

### Step-4: 5-Amino-8-(2-furyl)-3-(2-hydroxyethyl)-1-(2,2,2-trifluoroethyl) [1,2,4]triazolo[5,1-f]purin-2-one(procedure is same as step-6 in example A1)

¹HNMR (400MHz, DMSO d6): δ 3.72 (q, J=6.4Hz, 2H); 3.89 (t, J=6.4Hz, 2H); 4.80 (q, J=8.8Hz, 2H); 4.92 (t, J=6Hz, 1H); 6.72 (dd, J=2Hz, 3.6Hz, 1H); 7.19 (d, J=3.2Hz, 1H); 7.95 (bs, 3H).

### Step-5: 2-[5-Amino-8-(2-furyl)-2-oxo-1-(2,2,2-trifluoroethyl)-[1,2,4]triazolo[5,1-f]purin-3-yl]ethyl 4-methylbenzenesulfonate (procedure is same as step-7 in example A1)

¹HNMR (400MHz, DMSO d6): δ 1.96(s,3H); 4.02 (t, J=4Hz, 2H); 4.45 (t, J=4Hz, 2H); 4.74 (q, J=8.8Hz, 2H); 6.72 (bs, 1H); 6.98 (d, J=8Hz, 1H); 7.20 (d, J=3.2Hz, 1H); 7.40 (d, J=8Hz, 3H); 7.89 (bs, 2H); 7.95 (s, 1H).

### Step-6: 5-Amino-8-(2-furyl)-3-[2-[4-[4-(2-methoxyethoxy)phenyl]piperazin-1-yl]ethyl]-1-(2,2,2-trifluoroethyl)-[1,2,4]triazolo[5,1-f]purin-2-one (procedure is same as step-8 in example A1)

HNMR(400MHz, DMSO d6): δ 2.59 (bs, 4H); 2.70 (t, J=6.8Hz, 2H); 2.93 (bs, 4H); 3.28 (s, 3H); 3.61 (t, J=4.8Hz, 2H); 3.97-4.00 (m, 4H); 4.79 (q, J=8.8Hz, 2H); 6.72 (dd, J=2Hz, 3.6Hz, 1H); 6.78-6.84 (m, 4H); 7.19 (d, J=3.2Hz, 1H); 7.95(s, 1H); 7.96 (bs, 2H).

Examples H2 was prepared by following similar experimental procedure of Example H1 using the appropriate intermediates

| **Ex. No** | **Structure / IUPAC name** | **NMR** |
|---|---|---|
| **H2** | 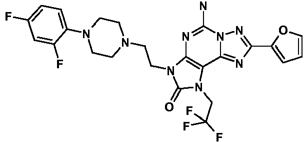 | HNMR(400MHz, DMSO d6): δ 2.63 (bs, 4H); 2.72 (t, J=6Hz, 2H) ; 2.89 (bs, 4H); 4.00 (t, J=6.0Hz, 2H); 4.82 (q, J=9.2 Hz, 2H); 6.74 (bs, 1H); 6.97-7.02 (m, 2H); 7.19-7.21 (m, 2H); 7.97 (bs, 3H). |
| | 5-Amino-3-{2-[4-(2,4-difluoro-pheny l)-piperazin-1-yl]-ethyl}-8-furan-2 -yl-1-(2,2,2-trifluoro-ethyl)-1,3-d ihydro-[1,2,4]triazolo[5,1-f]purin-2-one | |

### Example I1: 5-Amino-8-(2-furyl)-3-[2-[4-[4-(2-methoxyethoxy)phenyl]piperazin-1-yl]ethyl]-1-(2-methoxyethyl)-[1,2,4]triazolo[5,1-f]purin-2-one)

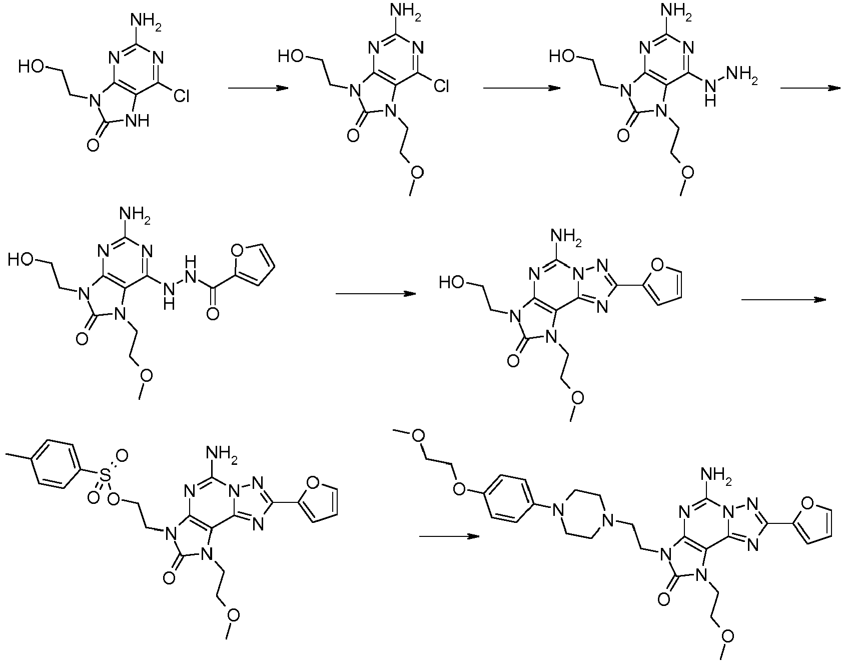

### Step-1: 2-Amino-6-chloro-9-(2-hydroxyethyl)-7-(2-methoxyethyl)purin-8-one (procedure is same as step-3 in example A1)

¹HNMR (400MHz, DMSO d6): δ 3.24(s,3H); 3.56 (t, J=6Hz, 2H); 3.62 (q, J=5.2Hz, 2H); 3.78 (t, J=6Hz, 2H); 4.05 (t, J=6Hz, 2H); 4.86 (t, J=6Hz, 1H); 6.71 (bs, 2H).

### Step-2 : 2-Amino-6-hydrazino-9-(2-hydroxyethyl)-7-(2-methoxyethyl)purin-8-one (procedure is same as step-4 in example A1)

¹HNMR (400MHz, DMSO d6): δ 3.24(s, 3H); 3.49 (t, J=5.2Hz, 2H); 3.58-3.60 (m, 2H); 3.72 (t, J=6Hz, 2H); 3.99 (t, J=4.8Hz, 2H); 4.28 (bs, 2H); 4.87 (t, J=5Hz, 1H); 6.02 (bs, 2H); 7.46 (bs, 1H).

### Step-3: N'-[2-Amino-9-(2-hydroxyethyl)-7-(2-methoxyethyl)-8-oxo-purin-6-yl]furan-2-carbohydrazide (procedure is same as step-5 in example A1)

¹HNMR (400MHz, DMSO d6): δ 3.28 (s, 3H); 3.57-3.63 (m, 4H); 3.75 (t, J=6Hz, 2H); 4.05 (t, J=4.8Hz, 2H); 4.90 (bs, 1H); 6.02 (bs, 2H); 6.66 (dd, J=2Hz, 3.6 Hz, 1H); 7.26 (d, J=3.2 Hz, 1H); 7.90 (s, 1H); 8.45 (bs, 1H); 10.33 (s, 1H).

### Step-4: 5-Amino-8-(2-furyl)-3-(2-hydroxyethyl)-1-(2-methoxyethyl)-[1,2,4]triazolo[5,1-f]purin-2-one (procedure is same as step-6 in example A1)

¹HNMR (400MHz, DMSO d6): δ 3.25 (s, 3H); 3.69 (q, J=6Hz, 2H); 3.78 (t, J=6Hz, 2H); 3.87 (t, J=6Hz, 2H); 4.15 (t, J=6Hz, 2H); 4.89 (t, J=5.2Hz, 1H); 6.72 (bs, 1H); 7.19 (d, J=3.6Hz, 1H); 7.81 (bs, 2H); 7.94(s, 1H).

### Step-5: 2-[5-Amino-8-(2-furyl)-1-(2-methoxyethyl)-2-oxo-[1,2,4]triazolo[5,1-f]purin-3-yl]ethyl 4-methylbenzenesulfonate (procedure is same as step-7 in example A1)

¹HNMR (400MHz, DMSO d6): δ 1.98 (s, 3H); 3.27 (s, 3H); 3.77 (t, J=5.6Hz, 2H); 3.99 (t, J=5.2Hz, 2H); 4.09 (t, J=5.2Hz, 2H); 4.43 (t, J= 4.8Hz, 2H); 6.72 (dd, J=2Hz, 3.6Hz, 1H); 6.95 (d, J=8Hz, 2H); 7.19 (dd, J=0.8Hz, 3.6Hz, 1H); 7.40 (d, J=8.4Hz, 2H); 7.73 (bs, 2H); 7.95(d, J=1.2Hz, 1H).

### Step-6: 5-Amino-8-(2-furyl)-3-[2-[4-[4-(2-methoxyethoxy)phenyl]piperazin-1-yl]ethyl]-1-(2-methoxyethyl)-[1,2,4]triazolo[5,1-f]purin-2-one (procedure is same as step-8 in example A1)

HNMR(400MHz, DMSO d6): δ 2.60 (bs, 4H); 2.69(t, J=6Hz, 2H); 2.94(bs, 4H); 3.22 (s, 3H); 3.28 (s, 3H);3.59-3.62(m, 2H); 3.77 (t, J=5.6Hz, 2H); 3.94- 3.99 (m, 4H); 4.15(t, J=5.2Hz, 2H); 6.71(dd, J=2Hz , 3.6Hz, 1H); 6.79-6.85 (m, 4H); 7.17 (d, J=3.2Hz, 1H); 7.81 (bs, 2H); 7.94 (s, 1H).

Examples 12 was prepared by following similar experimental procedure of Example I121 using the appropriate intermediates

| **Ex. No** | **Structure / IUPAC name** | **NMR** |
|---|---|---|
| **I2** | 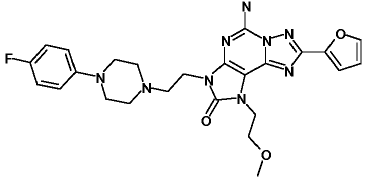 | HNMR(400MHz, DMSO d6): δ 2.60 (t, J=4.4Hz, 4H); 2.69 (t, J=6.4Hz, 2H); 3.00 (bs, 4H); 3.22 (s, 3H); 3.77 (t, J=6Hz, 2H); 3.97 (t, J=6.4Hz, 2H); 4.16 (t, J=5.6Hz, 2H); 6.71(dd, J=2Hz, 3.6Hz, 1H); 6.89-6.93 (m, 2H); 7.00 (t, J=8.8Hz, 2H); 7.17 (d, J =2.4Hz, 1H); 7.82 (bs, 2H); 7.94 (bs, 1H). |
| | 5-amino-3-[2-[4-(4-fluorophenyl)piperazin-1-yl]ethyl]-8-(2-furyl)-1-(2-methoxyethyl)-[1,2,4]triazolo[5,1-f]purin-2-one | |

### Example J1: 5-Amino-3-[2-[4-(4-fluorophenyl)piperazin-1-yl]ethyl]-8-(2-furyl)-1-(2-hydroxyethyl)-[1,2,4]triazolo[5,1-f]purin-2-one one

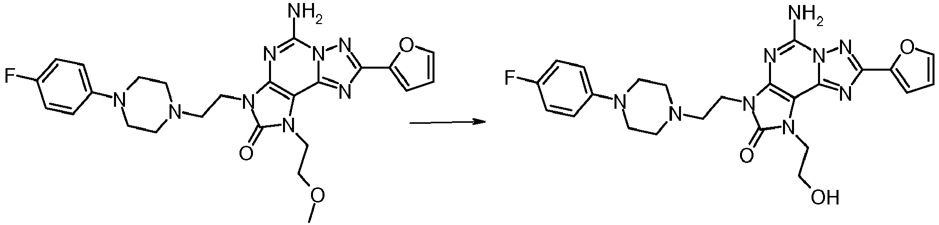

To a solution of compound 5-amino-3-[2-[4-(4-fluorophenyl)piperazin-1-yl]ethyl]-8-(2-furyl)-1-(2-methoxyethyl)-[1,2,4]triazolo[5,1-f]purin-2-one (Example I2) (0.050g, 0.095mmol), in DCM (3ml) was added BBr₃ (0.1ml, 0.105mmol) drop wise at 0 °C and stirred at 25°C for 20 hours. The reaction mixture was quenched with sat.NaHCO₃ (25ml) and extracted with DCM (3 x 20ml) .The crude product was purified by LCMS to obtain 5-Amino-3-[2-[4-(4-fluorophenyl)piperazin-1-yl]ethyl]-8-(2-furyl)-1-(2-hydroxyethyl)-[1,2,4]triazolo[5,1-f]purin-2-one (5mg, 10%) as an off white solid
HNMR(400MHz, DMSO d6): δ 2.60 (bs, 4H); 2.67 (t, J=6.8Hz, 2H); 3.0(t, J=4.8Hz, 4H); 3.80 (q, J=6Hz, 2H); 3.95 (t, J=6.4Hz, 2H); 4.03 (t, J=6.4Hz, 2H); 4.84 (t, J=5.2Hz, 1H); 6.71 (dd, J=2Hz, 3.6Hz, 1H); 6.89-6.92 (m, 2H); 7.01 (t, J=9.2Hz, 2H), 7.17 (dd, J =0.8Hz, 3.2Hz, 1H); 7.79 (bs, 2H); 7.92 (dd, J=0.8Hz, 1.6Hz, 1H).

### Example K1: 5-Amino-1-cyclopropyl-8-(2-furyl)-3-[2-[4-[4-(2-methoxyethoxy)phenyl]piperazin-1-yl]ethyl]-[1,2,4]triazolo[5,1-f]purin-2-one

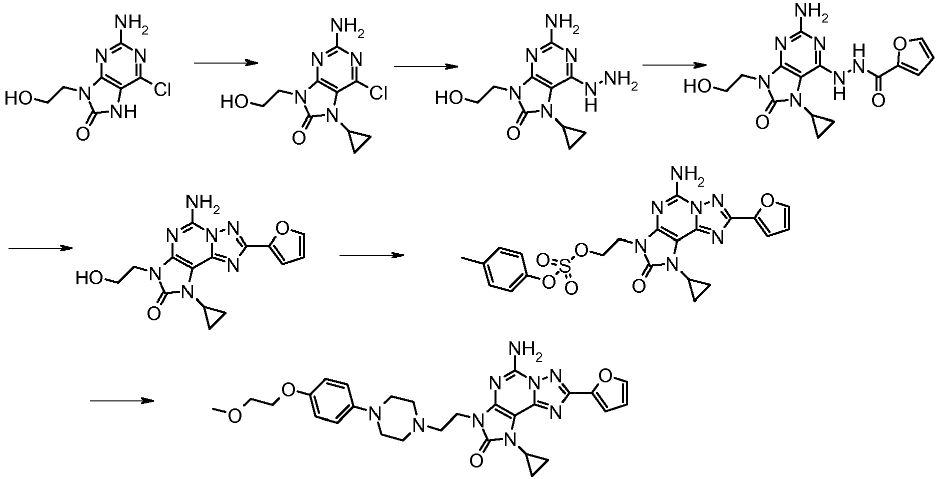

### Step-1: 2-Amino-6-chloro-7-cyclopropyl-9-(2-hydroxyethyl)purin-8-one

A mixture of cyclopropylboronic acid (0.369g, 44mmol), 2-amino-6-chloro-9-(2-hydroxyethyl)-7H-purin-8-one (5g, 22mmol) and Na₂CO₃ (0.462g, 44mmol) were taken in dichloroethane (20ml) and dimethylformamide (10ml). A suspension of Cu(OAc)₂ (0.399g, 22mmol) and bipyridine (0.343g, 22mmol) in hot dichloroethane (30ml) was added to the above mixture.The mixture was stirred at 70 °C for 18 hours under air. The resulting mixture was cooled to room temperature, and a saturated aqueous NH₄Cl solution was added, followed by water. The organic layer was separated and the aqueous layer was extracted three times with DCM. The combined organic layers were washed with brine, dried over Na₂SO₄, and concentrated under reduced pressure to get crude 2-amino-6-chloro-7-cyclopropyl-9-(2-hydroxyethyl)purin-8-one (1.6g, 27%) as an off white solid.
¹HNMR (400MHz, DMSO d6): δ 0.97-1.02 (m, 4H); 2.89-2.92 (m, 1H); 3.61-3.67 (m, 2H); 3.71-3.74 (m, 2H); 4.83 (t, J=12.8Hz, 1H); 6.67 (bs, 2H)

### Step-2: 2-Amino-7-cyclopropyl-6-hydrazino-9-(2-hydroxyethyl)purin-8-one (Procedure is same as step-4 in example A1)

¹HNMR (400MHz, DMSO d6): δ 0.84-0.87 (m, 2H); 0.96 -1.01 (m, 2H); 3.03-3.06 (m, 1H); 3.54-3.60 (m, 2H); 3.66 (t, J=6Hz, 2H); 4.32 (bs, 2H); 4.85 (t, J=5.6Hz, 1H); 5.99 (bs, 2H); 7.23 (bs, 1H)

### Step-3: N'-[2-Amino-7-cyclopropyl-9-(2-hydroxyethyl)-8-oxo-purin-6-yl]furan-2-carbohydrazide (Procedure is same as step-5 in example A1)

Crude material was taken for next reaction.

### Step-4: 5-Amino-1-cyclopropyl-8-(2-furyl)-3-(2-hydroxyethyl)-[1,2,4]triazolo[5,1-f]purin-2-one (Procedure is same as step-6 in example A1)

¹HNMR (400MHz, DMSO d6): δ 1.00-1.12 (m, 4H); 3.13-3.18 (m, 1H); 3.65-3.69 (m, 2H); 3.83 (t, J=6.4Hz, 2H); 4.88 (t, J=5.6Hz, 1H); 6.72 (dd, J=2Hz, 3.6Hz, 1H); 7.19 (d, J=3.2Hz, 1H); 7.79 (bs, 2H); 7.94-7.95 (m, 1H)

### Step-5: 2-[5-Amino-1-cyclopropyl-8-(2-furyl)-2-oxo-[1,2,4]triazolo[5,1-f]purin-3-yl]ethyl p-tolyl sulfate (Procedure is same as step-7 in example A1)

¹HNMR (400MHz, DMSO d6): δ 1.01-1.08 (m, 4H); 1.98 (s, 3H); 3.06-3.11 (m, 1H); 3.95 (t, J=4.4Hz, 2H); 4.44 (t, J=4.8Hz, 2H); 6.71-6.73 (m, 1H); 6.99 (d, J=8.4Hz, 2H); 7.19 (d, J=3.6Hz, 1H); 7.41 (d, J=8.4Hz, 2H); 7.74 (bs, 2H); 7.94 (s, 1H)

### Step-6: 5-Amino-1-cyclopropyl-8-(2-furyl)-3-[2-[4-[4-(2-methoxyethoxy)phenyl]piperazin-1-yl]ethyl]-[1,2,4]triazolo[5,1-f]purin-2-one (ADV-1005876) (Procedure is same as step-8 in example A1)

1H NMR (400MHz, DMSO): δ 1.05-1.08 (m, 4H); 2.56(bs, 4H); 2.66 (t, J=6.4Hz, 2H); 2.95 (bs, 4H); 3.15-3.19 (m, 1H); 3.29 (s, 3H); 3.60-3.62 (m, 2H); 3.92 (t, J=6.4Hz, 2H); 3.98-4.00 (m, 2H); 6.72 (dd, J=2Hz, 3.6Hz,1H); 6.79-6.86 (m, 4H); 7.18 (d, J=3.2Hz, 1H); 7.79 (bs, 2H); 7.94 (bs, 1H).

### Example L1: 5-Amino-8-(2-furyl)-3-[2-[4-[4-(2-methoxyethoxy)phenyl]piperazin-1-yl]ethyl]-1-(2,2,2-trifluoroethyl)-[1,2,4]triazolo[5,1-f]purin-2-one

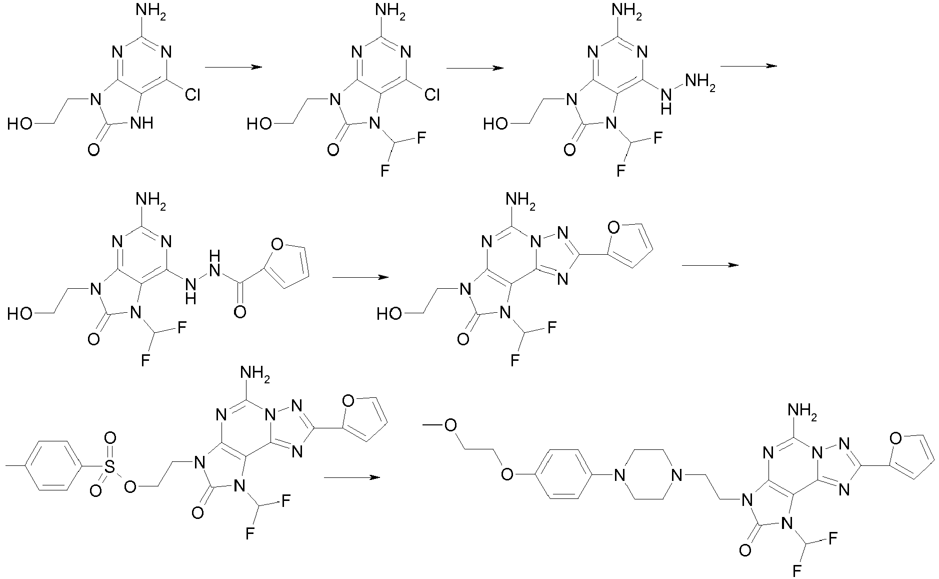

### Step-1 : 2-Amino-6-chloro-7-(difluoromethyl)-9-(2-hydroxyethyl)purin-8-one (procedure is same as step-3 in example A1)

¹HNMR (400MHz, DMSO d6): δ 3.66-3.69 (m, 2H); 3.79 (t, J=6Hz, 2H); 4.91 (t, J=6Hz, 1H); 7.04 (bs, 2H); 7.66 (t, J=58Hz, 1H).

### Step-2 : 2-Amino-7-(difluoromethyl)-6-hydrazino-9-(2-hydroxyethyl)purin-8-one (Procedure is same as step-4 in example A1)

¹HNMR (400MHz, DMSO d6): δ 3.63 (bs, 2H); 3.74 (t, J=6Hz, 2H); 4.54 (bs, 2H); 4.91 (bs, 1H); 6.36 (bs, 2H); 7.70 (t, J=58Hz, 1H).

### Step-3 N'-[2-Amino-7-(difluoromethyl)-9-(2-hydroxyethyl)-8-oxo-purin-6-yl]furan-2-carbohydrazide (procedure is same as step-5 in example A1)

Crude product was used in next step

### Step-4: 5-Amino-1-(difluoromethyl)-8-(2-furyl)-3-(2-hydroxyethyl)-[1,2,4]triazolo[5,1-f]purin-2-one (procedure is same as step-6 in example A1)

¹HNMR (400MHz, DMSO d6): δ 3.72 (q, J=6Hz, 2H); 3.87 (t, J=5.6Hz, 2H); 4.94 (t, J=6Hz, 1H); 6.72 (dd, J=2Hz, 3.6Hz, 1H); 7.19 (d, J=3.2Hz, 1H); 7.72 (t, J=58Hz, 1H); 7.95 (s, 1H); 8.12 (bs, 2H).

### Step-5: 2-[5-Amino-1-(difluoromethyl)-8-(2-furyl)-2-oxo-[1,2,4]triazolo[5,1-f]purin-3-yl]ethyl 4-methylbenzenesulfonate (procedure is same as step-7 in example A1)

¹HNMR (400MHz, DMSO d6): δ 1.98 (s, 3H); 4.02 (t, J=4.8Hz, 2H); 4.47 (t, J=5.2Hz, 2H); 6.74 (dd, J=2Hz, 3.6Hz, 1H); 7.01 (d, J=8Hz, 2H); 7.22 (d, J=2.8Hz, 1H); 7.45 (d, J=8Hz, 2H); 7.69 (t, J=58Hz, 1H); 7.97 (s, 1H); 8.11 (bs, 2H).

### Step-6: 5-Amino-1-(difluoromethyl)-8-(2-furyl)-3-[2-[4-[4-(2-methoxyethoxy)p henyl]piperazin-1-yl]ethyl]-[1,2,4]triazolo[5,1-f]purin-2-one (procedure is same as step-8 in example A1)

HNMR(400MHz, DMSO d6): δ 2.60(bs, 4H); 2.70 (t, J=6.8Hz, 2H); 2.93 (bs, 4H); 3.28 (s, 3H); 3.59-3.61 (m, 2H); 3.95-3.99 (m, 4H); 6.72 (dd, J=1.6Hz, 3.6Hz, 1H); 6.78-6.85 (m, 4H); 7.18 (d, J=2.8Hz, 1H); 7.73(t, J=58Hz, 1H); 7.94 (d, J=0.8Hz, 1H); 8.13 (bs, 2H).

### Example M1: 5-Amino-3-[2-[4-[4-(2-methoxyethoxy)phenyl]piperazin-1-yl]ethyl]-1-methyl-8-thiazol-2-yl-[1,2,4]triazolo[5,1-f]purin-2-one (ADV-1005738)

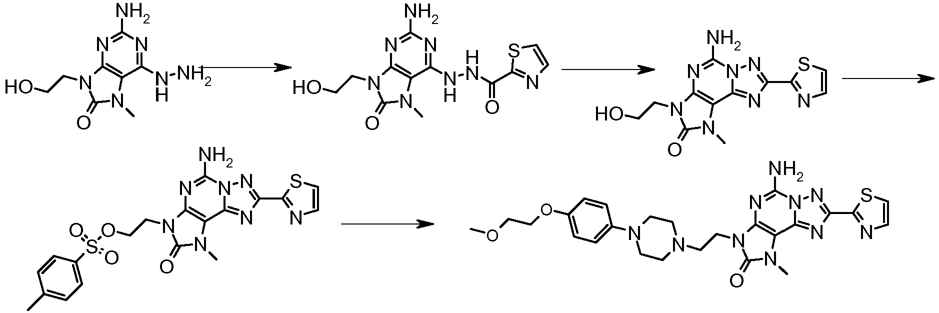

### Step-1: N'-[2-Amino-9-(2-hydroxyethyl)-7-methyl-8-oxo-purin-6-yl]thiazole-2-carbohydrazide (procedure is same as step-5 in example A1)

¹HNMR (400MHz, DMSO d6): δ 3.43 (s, 3H); 3.58-3.63 (m, 2H); 3.74 (t, J=5.6Hz, 2H); 4.88 (t, J=6.0Hz, 1H); 5.98 (s, 2H); 8.09-8.12 (m, 2H); 8.53 (s, 1H); 10.65 (s, 1H).

### Step-2: 5-Amino-3-(2-hydroxyethyl)-1-methyl-8-thiazol-2-yl-[1,2,4]triazolo[5,1-f]purin-2-one (procedure is same as step-6 in example A1)

¹HNMR (400MHz, DMSO d6): δ 3.58 (s, 3H); 3.70 (q, J=5.6Hz, 2H); 3.87 (t, J=6.4Hz, 2H); 4.89 (t, J=6.0Hz, 1H): 7.90 (bs, 2H); 8.02 (d, J=3.2Hz, 1H); 8.09 (d, J=3.2Hz, 1H).

### Step-3: 2-(5-Amino-1-methyl-2-oxo-8-thiazol-2-yl-[1,2,4]triazolo[5,1-f]purin-3-yl)ethyl 4 methylbenzenesulfonate (procedure is same as step-7 in example A1)

¹HNMR (400MHz, DMSO d6): δ 2.03 (s, 3H); 3.51 (s, 3H); 4.01 (t, J=4.4Hz, 2H); 4.49 (t, J=4.4Hz, 2H); 7.03 (d, J=8.0Hz, 2H): 7.41 (d, J=8.0Hz, 2H); 7.89 (bs, 2H); 8.04 (d, J=3.2Hz, 1H); 8.12 (d, J=3.2Hz, 1H).

### Step-4: 5-Amino-3-[2-[4-[4-(2-methoxyethoxy)phenyl]piperazin-1-yl]ethyl]-1-methyl-8-thiazol-2-yl-[1,2,4]triazolo[5,1-f]purin-2-one (procedure is same as step-8 in example A1)

¹HNMR (400MHz, DMSO d6): δ 2.59 (bs,4H); 2.67 (t, J=6.8Hz, 2H); 2.94 (bs, 4H);3.27 (s, 3H); 3.56 (s, 3H); 3.59 (t, J=4.4Hz, 2H); 3.93-3.98 (m, 4H); 6.76-6.83 (m, 4H); 7.90 (bs, 2H): 8.00 (d, J=3.2Hz, 1H); 7.41 (d, J=2.8Hz, 1H).

Examples M2-M9 was prepared by following similar experimental procedure of Example M1 using the appropriate intermediates

| **Ex. No** | **Structure / IUPAC name** | **NMR** |
|---|---|---|
| M2 | 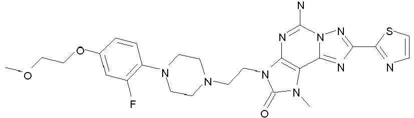 | ¹HNMR (400MHz, DMSO d6): δ 2.60(bs, 4H); 2.67(t, J=7.2 Hz, 2H); 2.84 (bs, 4H); 3.26 (s,3H); 3.57 (s, 3H); 3.59 (t, J=4.4Hz, 2H); 3.94 (t, J=6.4Hz, 2H);4.00 (t, J=4.4Hz, 2H); 6.65 (dd, J=2.4, 8.8Hz, 1H); 6.77 (dd, J=2.4, 14.4Hz, 1H); 6.91 (t, J=9.2Hz, 1H); 7.89 (bs, 2H): 8.00 (d, J=3.2Hz, 1H); 8.07 (d, J=3.2Hz, 1H). |
| | 5-Amino-3-[2-[4-[3-fluoro-4-(2-methoxyethoxy)phenyl]piperazin-1-yl]ethyl]-1-methyl-8-thiazol-2-yl-[1,2,4]triazolo[5,1-f]purin-2-one | |
| M3 | 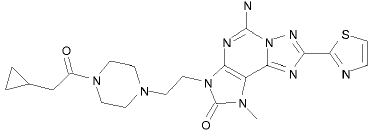 | ¹HNMR (400MHz, CDCl₃): δ 0.14-0.18 (m, 2H); 0.53-0.57 (m, 2H); 1.00-1.02 (m,1H); 2.25 (d, J=6.8Hz, 2H); 2.56 (bs, 4H); 2.78 (t, J=6.4Hz, 2H); 3.41 (t, J=5.2Hz, 2H); 3.59 (bs,2H); 3.79 (s, 3H); 4.08 (t, J=6.4Hz, 2H); 5.85 (bs, 2H); 7.57 (d, J=3.2Hz, 1H); 8.06 (d, J=3.2Hz, 1H). |
| | 5-Amino-3-[2-[4-(2-cyclopropylacetyl)piperazin-1-yl]ethyl]-1-methyl-8-thiazol-2-yl-[1,2,4]triazolo[5,1-f]purin-2-one | |
| M4 | 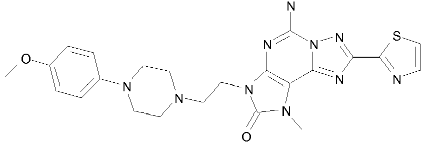 | ¹HNMR (400MHz, DMSO d6): δ 2.61 (bs, 4H); 2.68 (t, J=5.2Hz, 2H); 2.95 (bs, 4H); 3.58 (s, 3H); 3.66 (s, 3H); 3.97 (t, J=6.4Hz, 2H); 6.79 (d, J=9.2Hz, 2H); 6.86 (d, J=9.2Hz, 2H); 7.92 (bs, 2H), 8.02 (d, J=3.2Hz, 1H); 8.09 (d, J=3.6Hz, 1H). |
| | 5-Amino-3-[2-[4-(4-methoxyphenyl)piperazin-1-yl]ethyl]-1-methyl-8-thiazol-2-yl-[1,2,4]triazolo[5,1-f]purin-2-one | |
| M5 | 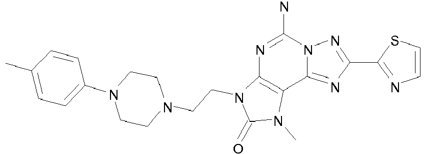 | ¹HNMR (400MHz, DMSO d6): δ 2.18 (s, 3H); 2.61 (bs, 4H); 2.68 (t, J=6.8Hz, 2H); 3.01 (bs, 4H); 3.58 (s, 3H); 3.97 (t, J=6.0Hz, 2H); 6.80 (d, J=8.4Hz, 2H); 7.00 (d, J=8.4Hz, 2H); 7.92 (bs, 2H);8.02 (d, J=2.8Hz, 1H); 8.09 (d, J=3.2Hz, 1H). |
| | 5-Amino-1-methyl-3-[2-[4-(p-tolyl)piperazin-1-yl]ethyl]-8-thiazol-2-yl-[1,2,4]triazolo[5,1-f]purin-2-one | |
| M6 | 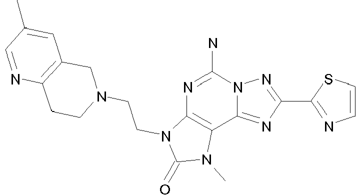 | ¹HNMR (400MHz, DMSO d6): δ 2.21 (s, 3H), 2.75-2.79 (m, 2H); 2.83-2.85 (m, 4H); 3.57 (s, 3H); 3.64 (s, 2H); 4.03 (t, J=6.4Hz, 2H); 7.28 (bs, 1H);7.93 (bs, 2H); 8.01 (d, J=3.6Hz, 1H); 8.08 (d, J=2.8Hz, 1H); 8.14 (bs, 1H). |
| | 5-Amino-1-methyl-3-[2-(3-methyl-7,8-dihydro-5H-1,6-naphthyridin-6-yl)ethyl]-8-thiazol-2-yl-[1,2,4]triazolo[5,1-f]purin-2-one | |
| M7 | 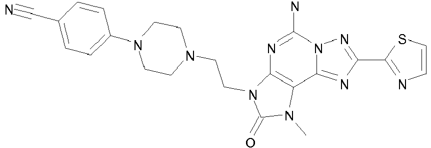 | ¹HNMR (400MHz, DMSO d6): δ 2.59(bs, 4H); 2.68 (t, J=6.0Hz, 2H); 3.27 (bs, 4H); 3.58 (s, 3H); 3.97 (t, J=6.0Hz, 2H); 6.99 (d, J=8.8Hz, 2H); 7.57 (d, J=8.4Hz, 2H); 7.93 (bs,2H); 8.02 (d, J=2.8Hz, 1H); 8.09 (d, J=3.2Hz, 1H). |
| | 4-[4-[2-(5-Amino-1-methyl-2-oxo-8-thiazol-2-yl-[1,2,4]triazolo[5,1-f]purin-3-yl)ethyl]piperazin-1-yl]benzonitrile | |
| M8 | 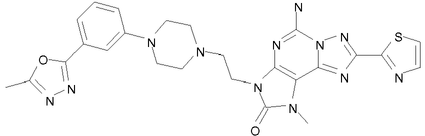 | ¹HNMR (400MHz, DMSO d6): δ 2.58 (s, 3H); 2.65 (bs, 4H); 2.71 (t, J=6.0Hz, 2H); 3.18 (bs, 4H); 3.59 (s, 3H); 3.99 (t, J=6.4Hz, 2H); 7.18 (d, J=7.2Hz, 1H); 7.35 (d, J=7.2Hz, 1H); 7.38-7.42 (m, 2H); 7.95 (bs, 2H); 8.03 (d, J=3.2Hz, 1H); 8.10 (d, J=3.2Hz, 1H). |
| | 5-Amino-1-methyl-3-[2-[4-[3-(5-methyl-1,3,4-oxadiazol-2-yl)phenyl]piperazin-1-yl]ethyl]-8-thiazol-2-yl-[1,2,4]triazolo[5,1-f]purin-2-one | |
| M9 | 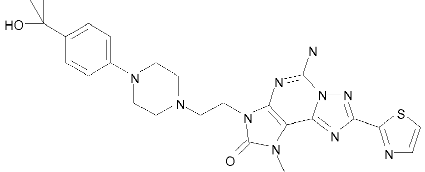 | ¹H NMR(400MHz,DMSO): δ 1.37 (m, 6H); 2.61 (m, 4H); 2.67-2.71 (m, 2H); 3.04 (bs, 4H); 3.58 (s, 3H); 3.97 (bs, 2H); 4.82 (bs, 1H); 6.83 (d, J=8.4Hz, 2H); 7.27 (d, J=8.0Hz, 2H); 7.92 (bs, 2H); 8.03 (bs, 1H); 8.09 (bs, 1H). |
| | 5-amino-3-[2-[4-[4-(1-hydroxy-1-methylethyl)phenyl]piperazin-1-yl]ethyl]-1-methyl-8-thiazol-2-yl-[1,2,4]triazolo[5,1-f]purin-2-one | |

### Example N1 : 5-Amino-3-[2-[4-[4-(2-methoxyethoxy)phenyl]piperazin-1-yl]ethyl]-1-methyl-8-(2-pyridyl)-[1,2,4]triazolo[5,1-f]purin-2-one

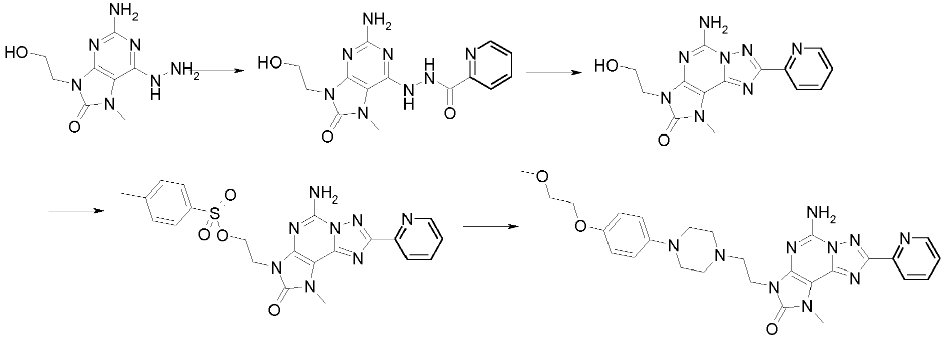

### Step-1: N'-[2-Amino-9-(2-hydroxyethyl)-7-methyl-8-oxo-purin-6-yl]pyridine-2 carbohydrazide (procedure is same step-5 as in example A1)

Crude product was used in next step

### Step-2: 5-Amino-3-(2-hydroxyethyl)-1-methyl-8-(2-pyridyl)-[1,2,4]triazolo[5,1-f]purin-2-one (procedure is same as step-6 in example A1)

¹HNMR (400MHz, DMSO d6): δ 3.60 (s, 3H); 3.69-3.71 (m, 2H); 3.88 (t, J=6Hz, 2H); 4.89 (t, J=6Hz, 1H); 7.55 (t, J=6Hz, 1H); 7.86 (bs, 2H); 8.01 (t, J=7.6Hz, 1H); 8.29 (d, J=7.6Hz, 1H); 8.75 (d, J=4.4Hz, 1H).

### Step-3: 2-[5-Amino-1-methyl-2-oxo-8-(2-pyridyl)-[1,2,4]triazolo[5,1-f]purin-3-yl]ethyl 4-methylbenzenesulfonate (procedure is same as step-7 in example A1)

¹HNMR (400MHz, DMSO d6): δ 1.99 (s, 3H); 3.53 (s, 3H); 4.01 (bs, 2H); 4.49 (bs, 2H); 7.01 (d, J=8Hz, 2H); 7.42 (d, J=8Hz, 2H); 7.58 (t, J=6Hz, 1H); 7.85 (bs, 2H); 8.04 (t, J=8Hz, 1H); 8.31 (d, J=8Hz, 1H); 8.78 (d, J=4.8Hz, 1H).

### Step-4: 5-Amino-3-[2-[4-[4-(2-methoxyethoxy)phenyl]piperazin-1-yl]ethyl]-1-methyl-8-(2-pyridyl)-[1,2,4]triazolo[5,1-f]purin-2-one (procedure is same as step-8 in example A1)

HNMR(400MHz, DMSO d6): δ 2.61 (bs, 4H); 2.69 (t, J=6.4Hz, 2H); 2.95(bs, 4H); 3.28 (s, 3H); 3.60 (t, J=4.8Hz, 5H); 3.95-3.99 (m, 4H); 6.78-6.86 (m, 4H); 7.53-7.56 (m, 1H); 7.87 (bs, 2H); 7.98-8.03 (m, 1H); 8.29 (d, J=8Hz, 1H); 8.74-8.75 (m, 1H)

Examples N2-N4 was prepared by following similar experimental procedure of EXAMPLE N1 using the appropriate intermediates

| **Ex. No** | **Structure / IUPAC name** | **NMR** |
|---|---|---|
| **N2** | 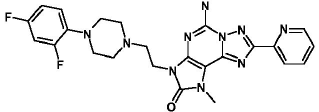 | HNMR(400MHz, DMSO d6): δ 2.64 (bs, 4H); 2.69 (bs, 2H); 2.91(bs, 4H); 3.62 (s, 3H); 3.97 (bs, 2H); 6.95-7.06 (m, 2H); 7.19 (t, J=10Hz, 1H); 7.56 (t, J=6Hz, 1H); 7.87 (bs, 2H); 8.02 (t, J=7.6 Hz, 1H); 8.29 (d, J=7.6Hz, 1H); 8.75-8.76 (m, 1H) |
| | 5-Amino-3-[2-[4-(2,4-difluorophenyl)piperazin-1-yl]ethyl]-1-methyl-8-(2-pyridyl)-[1,2,4]triazolo[5,1-f]purin-2-one | |
| **N3** | 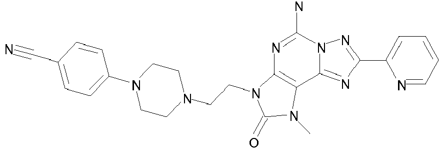 | ¹H NMR(400MHz, DMSO d6): δ 2.58 (bs, 4H); 2.64-2.68 (m, 2H); 3.25 (bs, 4H); 3.59 (s, 3H); 3.94-3.98 (m, 2H); 6.98 (d, J=8.8Hz, 2H); 7.52-7.56 (m, 3H); 7.85 (bs, 2H); 7.97-8.01 (m, 1H); 8.27 (d, J=7.6Hz, 1H); 8.73 (d, J=4.8Hz, 1H). |
| | 4-[4-[2-[5-Amino-1-methyl-2-oxo-8-(2-pyridyl)-[1,2,4]triazolo[5,1-f]purin-3-yl]ethyl]piperazin-1-yl]benzonitrile | |
| **N4** | 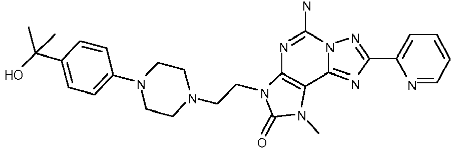 | ¹H NMR(400MHz, DMSO d6): δ 1.37 (s, 6H); 2.62 (bs, 4H); 2.67-2.70 (m, 2H); 3.04 (bs, 4H); 3.61 (s, 3H); 3.98 (bs, 2H); 4.81 (s, 1H); 6.83 (d, J=8.4Hz, 2H); 7.27 (d, J=8Hz, 2H); 7.54-7.57 (m, 1H); 7.87 (bs, 2H); 8.01 (t, J=7.2Hz, 1H); 8.29 (d, J=7.6Hz, 1H); 8.75 (d, J=4Hz, 1H). |
| | 5-Amino-3-[2-[4-[4-(1-hydroxy-1-methyl-ethyl)phenyl]piperazin-1-yl]ethyl]-1-methyl-8-(2-pyridyl)-[1,2,4]triazolo[5,1-f]purin-2-one | |

### Example O1: 5-Amino-3-[2-[4-[4-(2-methoxyethoxy)phenyl]piperazin-1-yl]ethyl]-1-methyl-8-pyrazin-2-yl-[1,2,4]triazolo[5,1-f]purin-2-one

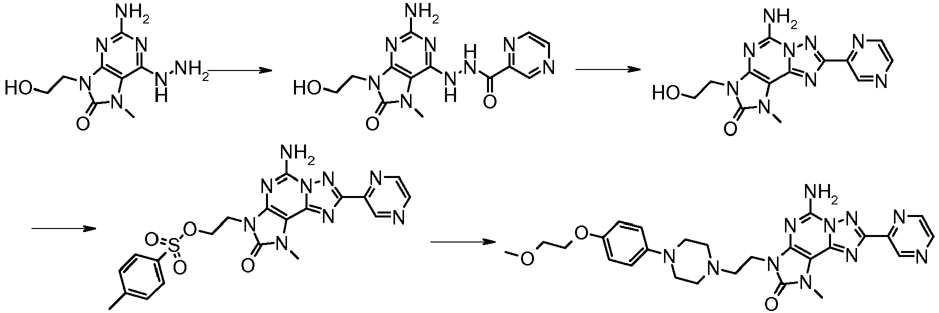

### Step1: N'-[2-Amino-9-(2-hydroxyethyl)-7-methyl-8-oxo-purin-6-yl]pyrazine-2-carbohydrazide (procedure is same as step-5 in example A1)

¹H NMR (400MHz, DMSO d6): δ 3.45 (s, 3H); 3.58-3.64 (m, 2H); 3.75 (t, J=6.4Hz, 2H); 5.97 (s, 2H); 8.54 (s, 1H); 8.80 (s, 1H); 8.92 (d, J=2Hz, 1H); 9.21 (s, 1H); 10.68 (s, 1H).

### Step 2: 5-Amino-3-(2-hydroxyethyl)-1-methyl-8-pyrazin-2-yl-[1,2,4]triazolo[5,1-f]purin-2-one (procedure is same as step-6 in example A1)

¹H NMR (400MHz, DMSO d6): δ 3.58 (s, 3H); 3.65-3.70 (m, 2H); 3.86 (t, J=6Hz, 2H); 4.88 (t, J=6Hz, 1H); 7.91 (bs, 2H); 8.79-8.82 (m, 2H); 9.44 (s, 1H).

### Step 3: 2-(5-Amino-1-methyl-2-oxo-8-pyrazin-2-yl-[1,2,4]triazolo[5,1-f]purin-3-yl)ethyl 4-methylbenzenesulfonate (procedure is same as step-7 in example A1)

¹H NMR (400MHz, DMSO d6): δ 2.01 (s, 3H); 3.53 (s, 3H); 4.02 (bs, 2H); 4.48 (bs, 2H); 7.02 (d, J=7.6Hz, 2H); 7.42 (d, J=8Hz, 2H); 7.92 (bs, 2H); 8.82-8.87 (m, 2H); 9.48 (s, 1H).

### Step 4: 5-Amino-3-[2-[4-[4-(2-methoxyethoxy)phenyl]piperazin-1-yl]ethyl]-1-methyl-8-pyrazin-2-yl-[1,2,4]triazolo[5,1-flpurin-2-(procedure is same as step-8 in example A1)

¹H NMR(400 MHz, DMSO d6): δ 2.61 (bs, 4H); 2.69 (t, J=6.4Hz, 2H); 2.96 (bs, 4H); 3.29 (s, 3H); 3.61 (bs, 5H); 3.97-3.99 (m, 4H); 6.79-6.86 (m, 4H); 7.95 (bs, 2H); 8.82-8.84 (m, 2H); 9.46 (s, 1H).

Examples 02-03 was prepared by following similar experimental procedure of EXAMPLE O1 using the appropriate intermediates

| | | |
|---|---|---|
| O2 | 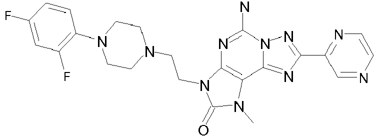 | ¹H NMR(400 MHz, DMSO d6): δ 2.64 (bs, 4H); 2.71 (t, J=6.0Hz, 2H); 2.91 (bs, 4H); 3.62 (s, 3H); 3.98 (t, J=6.0Hz, 2H); 6.94-7.06 (m, 2H); 7.15-7.21 (m, 1H); 7.94 (bs,2H); 8.82-8.85 (m, 2H); 9.46 (d, J=1.6Hz,1H). |
| | 5-Amino-3-[2-[4-(2,4-difluorophenyl)piperazin-1-yl]ethyl]-1-methyl-8-pyrazin-2-yl-[1,2,4]triazolo[5,1-f]purin-2-one | |
| O3 | 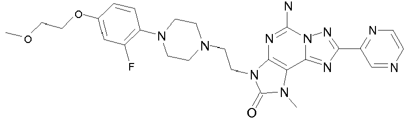 | ¹H NMR(400 MHz, DMSO d6): δ 2.60 (bs, 4H); 2.68 (t, J=6Hz, 2H); 2.84 (bs, 4H); 3.26 (s, 3H); 3.59 (bs, 5H); 3.92-3.97 (m, 2H); 3.99-4.01 (m, 2H); 6.65 (dd, J=8.8Hz, 2.4Hz, 1H); 6.78 (dd, J=14.4Hz, 2.8Hz, 1H); 6.91 (t, J=9.6Hz, 1H); 7.92 (bs, 2H); 8.79-8.83 (m, 2H); 9.44 (s, 1H). |
| | 5-Amino-3-[2-[4-[2-fluoro-4-(2-methoxyethoxy)phenyl]piperazin-1-yl]ethyl]-1-methyl-8-pyrazin-2-yl-[1,2,4]triazolo[5,1 -f]purin-2-one | |

### Example: P1: 5-Amino-8-(2-furyl)-3-[[1-(4-methoxyphenyl)pyrrolidin-3-yl]methyl]-1-methyl-[1,2,4]triazolo[5,1-f]purin-2-one

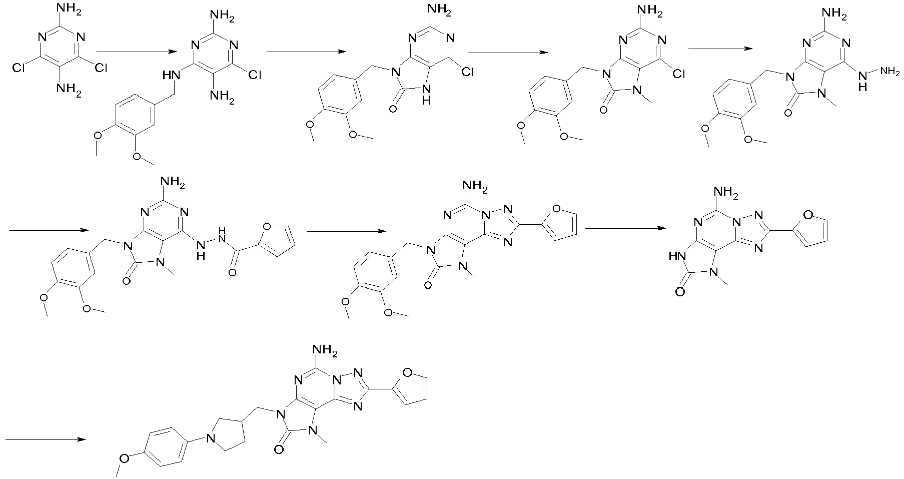

### Step-1: 6-Chloro-N4-[(2,4-dimethoxyphenyl)methyl]pyrimidine-2,4,5-triamine

A mixture of 4,6-dichloropyrimidine-2,5-diamine (5.5ml, 30.72mmol), ethanol (50ml), 2,6-dichloropyrimidin-4-amine (5.0g, 27.93mmol), and potassium carbonate (2.3g, 16.75mmol) were heated to 85-90 °C for 16 hours. Reaction mixture was cooled to room temperature., and filterd through celite bed, washed with ethanol and evaporated to dryness to obtain pure 6-Chloro-N4-[(2,4-dimethoxyphenyl)methyl]pyrimidine-2,4,5-triamine (8.4 g, 97 %). ¹HNMR(400MHz, DMSO d6): δ 3.73 (s, 3H); 3.79 (s, 3H); 3.98 (bs, 2H); 4.42 (d, J=5.6Hz, 2H); 5.64 (s, 2H); 6.46 (dd, J=2.4Hz, 8.4Hz, 1H); 6.55 (d, J=2.4Hz, 1H); 6.71 (t, J=6.0Hz, 1H); 7.10 (d, J=8.4Hz, 1H).

### Step-2: 2-Amino-6-chloro-9-[(3,4-dimethoxyphenyl)methyl]-7H-purin-8-one

A mixture of 6-Chloro-N4-[(2,4-dimethoxyphenyl)methyl]pyrimidine-2,4,5-triamine (8.4g, 27.11mmol), acetonitrile (400ml), potassium carbonate (11.2g, 81.33mmol) and p-nitrophenyl chloroformate (11.0g, 54.22mmol) were stirred at 25 °C for 48 hours. Reaction mixture was cooled to room temperature and filtered through celite bed, washed with cold acetonitrile and evaporated to dryness to obtain pure 2-Amino-6-chloro-9-[(3,4-dimethoxyphenyl)methyl]-7H-purin-8-one (9.0g, 98%)

Crude product was taken in next step

### Step-3: 2-Amino-6-chloro-9-[(3,4-dimethoxyphenyl)methyl]-7-methyl-purin-8-one

A mixture of 2-amino-6-chloro-9-[(3,4-dimethoxyphenyl)methyl]-7H-purin-8-one (8.8g, 26.21mmol), DMF (90ml), potassium carbonate (5.4g, 39.31mmol) and methyl iodide (1.96ml, 31.45mmol) stirred at 25 °C for 3 hours. Reaction mixture was cooled to 0 °C Water was added and solid obtained was filterd off to obtain 2-amino-6-chloro-9-[(3,4-dimethoxyphenyl)methyl]-7-methyl-purin-8-one (9.0g, 98 %).
¹HNMR(400MHz, DMSO d6): δ 3.72 (s, 3H); 3.82 (s, 3H); 3.90 (s, 3H); 4.73 (s, 2H); 6.35 (bs, 2H); 6.38-6.41 (m, 1H); 6.56-6.58 (m, 1H); 6.61-6.63 (m, 1H).

### Step-4: 2-Amino-9-[(3,4-dimethoxyphenyl)methyl]-6-hydrazino-7-methyl-purin-8-one

A mixture of 2-amino-6-chloro-9-[(3,4-dimethoxyphenyl)methyl]-7-methyl-purin-8-one obtained in step 3 (9.0g, 25.73mmol), Hydrazine hydrate (16ml ,257.3mmol) and ethanol (675ml) were heated at 90-95 °C for 40 hours. The reaction mixture was concentrated and solid obtained was filtered off and dried to obtain 2-amino-9-[(3,4-dimethoxyphenyl)methyl]-6-hydrazino-7-methyl-purin-8-one (6.5g, 74 %) as an off white solid.
¹HNMR(400MHz, DMSO d6): δ 3.40 (s, 3H); 3.69 (s, 3H); 3.80 (s, 3H); 4.29 (bs,2H); 4.72 (bs, 2H); 5.97(bs, 2H); 6.37 (dd, J=2.0Hz, 8.4Hz, 1H); 6.49-6.54 (m, 2H); 7.66 (s, 1H).

### Step-5:N'-[2-amino-9-[(3,4-dimethoxyphenyl)methyl]-7-methyl-8-oxo-purin-6-yl]furan-2-carbohydrazide

2-amino-9-[(3,4-dimethoxyphenyl)methyl]-6-hydrazino-7-methyl-purin-8-one (4.6g, 13.38mmol) obtained in step 4, 2-furoic acid (1.5g, 13.38mmol), HOBT (1.8g, 13.38mmol) and N-methylmorpholine (2.20ml, 20.07mmol) were taken in dimethylformamide (15ml). EDC.HCl (3.8g, 20.07mmol) was added to the reaction mixture and stirred at 25-27 °C for 3 hours. Reaction mixture was cooled to 0 °C. Water was added and solid obtained was filtered off, washed with cold water, n-hexane and dried to obtain N'-[2-amino-9-[(3,4-dimethoxyphenyl)methyl]-7-methyl-8-oxo-purin-6-yl]furan-2-carbohydrazide (5.5g, 95 %) as an off white solid.
¹HNMR(400MHz, DMSO d6): δ 3.48 (s, 3H); 3.71(s, 3H); 3.83 (s, 3H); 4.76 (bs, 2H); 5.99 (bs, 2H); 6.41 (dd, J=2.4Hz, 8.4Hz, 1H); 6.57-6.59 (m, 2H); 6.67 (dd, J=2.0Hz, 3.6Hz, 1H); 7.25 (d, J=3.6Hz, 1H); 7.90 (d, J=0.8Hz, 1H); 8.42 (s, 1H); 10.28 (s, 1H).

### Step-6:5-Amino-3-[(3,4-dimethoxyphenyl)methyl]-8-(2-furyl)-1-methyl-[1,2,4]triazolo[5,1-f]purin-2-one

A mixture of N'-[2-amino-9-[(3,4-dimethoxyphenyl)methyl]-7-methyl-8-oxo-purin-6-yl]furan-2-carbohydrazide obtained in step 5 (5.5g, 12.51mmol), N,O-bistrimethylsilylacetamide (21.4ml, 87.61mmol) and hexamethyldisilazane (49.5ml, 312.91mmol) were heated at 145-150 °C for 3 hours. The reaction mixture was quenched with methanol (100ml) and water (100ml) and solvent was concentrated. The solid obtained was filtered off and washed with water (30ml) followed by diethyl ether (100ml) to obtain 5-amino-3-[(3,4-dimethoxyphenyl)methyl]-8-(2-furyl)-1-methyl-[1,2,4]triazolo[5,1-f]purin-2-one (5.1g, 97 %) as an off white solid.
¹HNMR(400MHz, DMSO d6): δ 3.61 (s, 3H); 3.72 (s, 3H); 3.85 (s, 3H); 4.88 (bs, 2H); □6.39 (dd, J=2.4Hz, 8.4Hz, 1H); 6.58 (d, J=2.4Hz, 1H); 6.68 (d, J=8.4Hz, 1H); 6.73 (dd, J=2.0,3.6Hz, 1H); 7.21 (d, J=3.2Hz, 1H); 7.78 (bs, 2H); 7.94 (s, 1H).

### Step-7: 5-Amino-8-(2-furyl)-1-methyl-3H-[1,2,4]triazolo[5,1-f]purin-2-one

A mixture of 5-amino-3-[(3,4-dimethoxyphenyl)methyl]-8-(2-furyl)-1-methyl-[1,2,4]triazolo[5,1-f]purin-2-one (0.1g, 0.237mmol), 1,2-dichlorobenzene (5ml), and aluminium chloride (0.095g, 0.711mmol) were stirred at and heated at 160 °C for 45 minutes. Reaction mixture was cooled to room temperature and putrified by LCMS to obtain pure 5-amino-8-(2-furyl)-1-methyl-3H-[1,2,4]triazolo[5,1-f]purin-2-one (0.025g, 39 %).

Crude product was taken in next step

### Step-8: 5-Amino-8-(2-furyl)-3-[[1-(4-methoxyphenyl)pyrrolidin-3-yl]methyl]-1-methyl-[1,2,4]triazolo[5,1-f]purin-2-one

A mixture of 5-amino-8-(2-furyl)-1-methyl-3H-[1,2,4]triazolo[5,1-f]purin-2-one (0.1g, 0.368mmol), DMF (3ml), potassium carbonate (0.76g, 0.553mmol) and [1-(4-methoxyphenyl)pyrrolidin-3-yl]methyl methanesulfonate (0.115g, 0.405mmol) stirred at 60 °C for 48hours. Reaction mixture was cooled to 0 °C. Water was added and solid obtained was filtered off. The crude product was purified by HPLC/MS to obtain 5-amino-8-(2-furyl)-3-[[1-(4-methoxyphenyl)pyrrolidin-3-yl]methyl]-1-methyl-[1,2,4]triazolo[5,1-f]purin-2-one (0.012g, 7 %).
¹HNMR(400MHz, DMSO d6): δ 1.77-1.84 (m,1H); 1.98-2.04 (m,1H); 2.89(pent, J=6.8Hz, 1H); 3.08-3.18 (m, 2H); 3.22-3.29 (m, 2H); 3.52 (s, 3H); 3.64 (s, 3H); 3.84-3.88 (m, 2H); 6.48 (d, J=9.2Hz, 2H); 6.72 (dd, J=2.0Hz, 3.6Hz, 1H); 6.78 (d, J=8.8Hz, 2H); 7.21 (d, J=2.8Hz, 1H); 7.82 (bs, 2H); 7.94 (d, J=0.8Hz, 1H).

Examples P2 was prepared by following similar experimental procedure of EXAMPLE P1 using the appropriate intermediates

| **Ex. No** | **Structure / IUPAC name** | **NMR** |
|---|---|---|
| P2 | 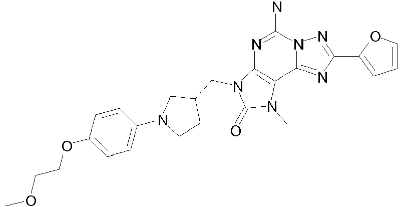 | ¹HNMR(400MHz, DMSO d6): δ 1.75-1.83 (m,1H); 1.98-2.04 (m,1H); 2.89 (pent, J=6.8Hz, 1H); 3.08-3.18 (m, 3H); 3.22-3.27 (m, 1H); 3.28 (s, 3H); 3.31-3.33 (m, 2H); 3.58 (s, 3H); 3.86-3.3.88 (m, 2H); 3.92-3.96 (m, 2H); 6.46 (d, J=8.8Hz, 2H); 6.72 (dd, J=2.0,3.6Hz, 1H); 6.79 (d, J=9.2Hz, 2H); 7.20 (d, J=3.2Hz, 1H); 7.82 (bs, 2H); 7.94 (bs, 1H). |
| | 5-Amino-8-(2-furyl)-3-[[1-[4-(2-methoxyethoxy)phenyl]pyrrolidin-3-yl]methyl]-1-methyl-[1,2,4]triazolo[5,1-f]purin-2-one hyl}-1-methyl-1,3-dihydro-[1,2,4]tr iazolo[5,1-i]purin-2-one | |

### Example Q1: 5-amino-8-(2-furyl)-3-[2-[4-[4-(2-methoxyethoxy)phenyl]piperazin-1-yl]ethyl]-1-methyl-[1,2,4]triazolo[5,1-f]purine-2-thione

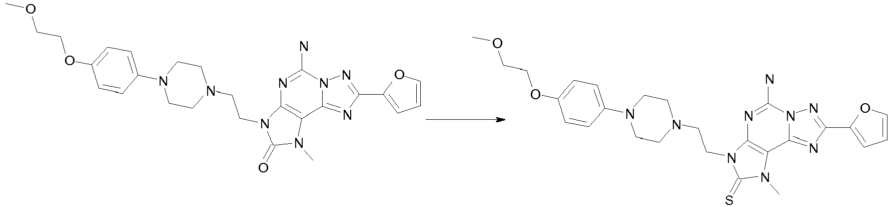

A mixture of 5-amino-8-(2-furyl)-3-[2-[4-[4-(2-methoxyethoxy)phenyl]piperazin-1-yl]ethyl]-1-methyl-[1,2,4]triazolo[5,1-f]purin-2-one (1.1g, 2mmol), Lawessoms reagent (0.83g, 2mmol) and toluene (20ml) were heated in sealed tube at 140-150 °C for 16 hours. The mixture was cooled and to the residue water (100ml) was added and extracted with dichloromethane (2x100ml). The crude product was purified by column chromatography to obtain 5-amino-8-(2-furyl)-3-[2-[4-[4-(2methoxyethoxy)phenyl] piperazin-1-yl]ethyl]-1-methyl-[1,2,4]triazolo[5,1-f]purine-2-thione. (11mg,) as an off white solid.
HNMR(400MHz, DMSO d6): δ 2.64 (bs, 4H); 2.75 (t, J=6Hz, 2H); 2.96 (bs, 4H); 3.28 (s, 3H); 3.60 (t, J=5Hz, 2H); 3.91 (s, 3H); 3.98 (t, J=5Hz, 2H); 4.34 (t, J=6Hz, 2H); 6.74-6.75 (m, 1H); 6.79-6.85 (m, 4H); 7.23 (d, J=3Hz, 1H); 7.96 (s, 1H); 8.07 (bs, 2H).

### Example R1: 8-(2-furyl)-3-[2-[4-[4-(2-methoxyethoxy)phenyl]piperazin-1-yl] ethyl] -1-methyl-5-(methylamino)-[1,2,4]triazolo[5,1-f]purin-2-one ADV-1006127

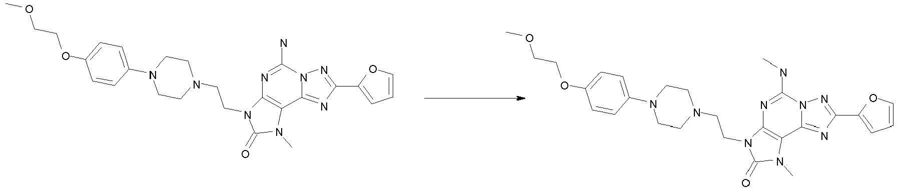

A mixture of 5-amino-8-(2-furyl)-3-[2-[4-[4-(2-methoxyethoxy)phenyl]piperazin-1-yl]ethyl]-1-methyl-[1,2,4]triazolo[5,1-f]purin-2-one (0.1g, 0.18mmol) obtained in example 1, NaH (0.043g, 0.18mmol) and methyl iodode (0.1ml, 0.18mmol)in DMF (2ml) were stirred at 21 °C for 3 hours. To the reaction mixture water (10ml) was added and solid obtained was filtered. The crude product was purified by column chromatography to obtain 8-(2-furyl)-3-[2-[4-[4-(2-methoxyethoxy)phenyl]piperazin-1-yl]ethyl]-1-methyl-5-(methylamino)-[1,2,4]triazolo[5,1-f]purin-2-one. (5mg, 5%) as an off white solid.
HNMR(400MHz, DMSO d6): δ 2.56 (bs, 4H); 2.71 (t, J=6Hz, 2H); 2.73 (bs, 4H); 3.02 (d, J=5Hz, 3H); 3.03 (s, 3H); 3.57 (s, 3H); 3.59-3.61 (m, 2H); 3.96-4.01 (m, 4H); 6.72-6.73 (m, 1H); 6.77-6.84 (m, 4H); 7.19 (d, J=3Hz, 1H); 7.93-7.94 (m, 1H); 8.05 (d, J=5Hz, 1H).

The list of examples below, but not limited to these, are synthesized following general synthesis described above:
5-Amino-3-{2-[4-(4-fluoro-phenyl)-piperazin-1-yl]-ethyl}-8-isothiazol-5-yl-1-methyl-1,3-dihydro-[1,2,4]triazolo[5,1-i]purin-2-one,
5-Amino-8-isothiazol-5-yl-3-(2-{4-[4-(2-methoxy-ethoxy)-phenyl]-piperazin-1-yl}-ethyl)-1-methyl-1,3-dihydro-[1,2,4]triazolo[5,1-i]purin-2-one,
5-Amino-3-[2-[4-[2-fluoro-4-(2-methoxyethoxy)phenyl]piperazin-1-yl]ethyl]-8-isothiazol-5-yl-1-methyl-1,2,4]triazolo[5,1-f]purin-2-one,
5-Amino-8-isoxazol-5-yl-3-[2-[4-[4-(2-methoxyethoxy)phenyl]piperazin-1-yl]ethyl]-1-methyl-[1,2,4]triazolo[5,1-f]purin-2-one,
5-Amino-3-[2-[4-[4-(2-methoxyethoxy)phenyl]piperazin-1-yl]ethyl]-1-methyl-8-oxazol-2-yl-[1,2,4]triazolo[5,1-f]purin-2-one,
5-Amino-3-{2-[4-(4-methoxy-phenyl)-piperazin-1-yl]-ethyl}-1-methyl-8-prop-1-ynyl-1,3-dihydro-[1,2,4]triazolo[5,1-i]purin-2-one,
5-Amino-3-(2-{4-[4-(2-methoxy-ethoxy)-phenyl]-piperazin-1-yl}-ethyl)-1-methyl-8-prop-1-ynyl-1,3-dihydro-[1,2,4]triazolo[5,1-i]purin-2-one,
5-Amino-3-{2-[4-(4-fluoro-benzoyl)-piperazin-1-yl]-ethyl}-8-furan-2-yl-1-methyl-1,3-dihydro-[1,2,4]triazolo[5,1-i]purin-2-one,
5-Amino-3-(2-dimethylamino-ethyl)-8-furan-2-yl-1-methyl-1,3-dihydro-[1,2,4]triazolo[5,1-i]purin-2-one,
5-Amino-8-furan-2-yl-3-[3-(4-methoxy-phenyl)-propyl]-1-methyl-1,3-dihydro-[1,2,4]triazolo[5,1-i]purin-2-one,
5-Amino-8-furan-2-yl-1-methyl-3-(2-pyrazol-1-yl-ethyl)-1,3-dihydro-[1,2,4]triazolo[5,1-i]purin-2-one,
5-Amino-8-furan-2-yl-3-(2-{4-[4-(2-methoxy-ethoxy)-phenyl]-pyrazol-1-yl}-ethyl)-1-methyl-1,3-dihydro-[1,2,4]triazolo[5,1-i]purin-2-one,
5-Amino-8-furan-2-yl-3-{2-[3-(4-methoxy-phenyl)-pyrrol-1-yl]-ethyl}-1-methyl-1,3-dihydro-[1,2,4]triazolo[5,1-i]purin-2-one,
5-Amino-8-furan-2-yl-3-{2-[4-(4-methoxy-phenyl)-imidazol-1-yl]-ethyl}-1-methyl-1,3-dihydro-[1,2,4]triazolo[5,1-i]purin-2-one,
5-Amino-8-furan-2-yl-3-{2-[4-(4-methoxy-phenyl)-[1,2,3]triazol-1-yl]-ethyl}-1-methyl-1,3-dihydro-[1,2,4]triazolo[5,1-i]purin-2-one,
5-Amino-3-[2-(1,3-dihydro-isoindol-2-yl)-ethyl]-8-furan-2-yl-1-methyl-1,3-dihydro-[1,2,4]triazolo[5,1-i]purin-2-one,
5-Amino-8-furan-2-yl-1-methyl-3-(2-piperidin-1-yl-ethyl)-1,3-dihydro-[1,2,4]triazolo[5,1-i]purin-2-one,
5-Amino-8-furan-2-yl-1-methyl-3-(2-pyrrolidin-1-yl-ethyl)-1,3-dihydro-[1,2,4]triazolo[5,1-i]purin-2-one,
5-Amino-8-furan-2-yl-1-methyl-3-[2-(3-methyl-7,8-dihydro-5H-[1,6]naphthyridin-6-yl)-ethyl]-1,3-dihydro-[1,2,4]triazolo[5,1-i]purin-2-one,
5-Amino-8-furan-2-yl-3-{2-[4-(2-methoxy-ethoxy)-phenoxy]-ethyl}-1-methyl-1,3-dihydro-[1,2,4]triazolo[5,1-i]purin-2-one,
5-Amino-8-furan-2-yl-3-{2-[4-(2-methoxy-ethoxy)-phenylamino]-ethyl}-1-methyl-1,3-dihydro-[1,2,4]triazolo[5,1-i]purin-2-one,
5-Amino-8-furan-2-yl-1-methyl-3-[2-(pyridin-2-yloxy)-ethyl]-1,3-dihydro-[1,2,4]triazolo[5,1-i]purin-2-one,
5-Amino-1-ethyl-3-{2-[4-(4-fluoro-phenyl)-piperazin-1-yl]-ethyl}-8-isothiazol-5-yl-1,3-dihydro-[1,2,4]triazolo[5,1-i]purin-2-one,
5-Amino-1-ethyl-8-isothiazol-5-yl-3-(2-{4-[4-(2-methoxy-ethoxy)-phenyl]-piperazin-1-yl}-ethyl)-1,3-dihydro-[1,2,4]triazolo[5,1-i]purin-2-one,
5-Amino-1-ethyl-8-furan-2-yl-3-(2-piperidin-1-yl-ethyl)-1,3-dihydro-[1,2,4]triazolo[5,1-i]purin-2-one,
5-Amino-1-ethyl-8-furan-2-yl-3-[2-(3-methyl-7,8-dihydro-5H-[1,6]naphthyridin-6-yl)-ethyl]-1,3-dihydro-[1,2,4]triazolo[5,1-i]purin-2-one,
5-Amino-3-[2-(2,4-difluoro-phenoxy)-ethyl]-1-ethyl-8-furan-2-yl-1,3-dihydro-[1,2,4]triazolo[5,1-i]purin-2-one,
5-Amino-3-[2-(2,4-difluoro-phenylamino)-ethyl]-1-ethyl-8-furan-2-yl-1,3-dihydro-[1,2,4]triazolo[5,1-i]purin-2-one,
5-Amino-1-cyclopropylmethyl-3-[2-(2,4-difluoro-phenylamino)-ethyl]-8-furan-2-yl-1,3-dihydro-[1,2,4]triazolo[5,1-i]purin-2-one,
5-Amino-1-cyclopropylmethyl-3-[2-(2,4-difluoro-phenoxy)-ethyl]-8-furan-2-yl-1,3-dihydro-[1,2,4]triazolo[5,1-i]purin-2-one,
5-Amino-1-cyclopropylmethyl-3-{2-[4-(4-fluoro-phenyl)-piperidin-1-yl]-ethyl}-8-furan-2-yl-1,3-dihydro-[1,2,4]triazolo[5,1-i]purin-2-one,
5-Amino-3-{2-[4-(4-fluoro-phenyl)-piperidin-1-yl]-ethyl}-8-furan-2-yl-1-(2,2,2-trifluoroethyl)-1,3-dihydro-[1,2,4]triazolo[5,1-i]purin-2-one,
5-Amino-8-furan-2-yl-3-{2-[4-(4-methoxy-phenyl)-piperazin-1-yl]-ethyl}-1-(2,2,2-trifluoro-ethyl)-1,3-dihydro-[1,2,4]triazolo[5,1-i]purin-2-one,
5-Amino-3-[2-(4-cyclopropylmethyl-piperazin-1-yl)-ethyl]-8-isothiazol-5-yl-1-(2,2,2-trifluoro-ethyl)-1,3-dihydro-[1,2,4]triazolo [5,1-i]purin-2-one,
(5-Amino-8-isothiazol-5-yl-3-{2-[4-(4-methoxy-phenyl)-piperazin-1-yl]-ethyl}-2-oxo-2,3-dihydro-[1,2,4]triazolo[5,1-i]purin-1-yl)-acetonitrile,
[5-Amino-3-{2-[4-(2,4-difluoro-phenyl)-piperazin-l-yl]-ethyl}-8-(3-fluoro-phenyl)-2-oxo-2,3-dihydro-[1,2,4]triazolo[5,1-i]purin-1-yl]-acetonitrile,
[5-Amino-8-furan-2-yl-3-(2-{4-[4-(2-methoxy-ethoxy)-phenyl]-piperazin-1-yl}-ethyl)-2-oxo-2,3-dihydro-[1,2,4]triazolo[5,1-i]purin-1-yl]-acetonitrile,
5-Amino-3-(2-{4-[4-(2-methoxy-ethoxy)-phenyl]-piperazin-1-yl}-ethyl)-1-methyl-8-phenyl-1,3-dihydro-[1,2,4]triazolo[5,1-i]purin-2-one,
3-[5-Amino-3-(2-{4-[4-(2-methoxy-ethoxy)-phenyl]-piperazin-1-yl}-ethyl)-1-methyl-2-oxo-2,3-dihydro-1H-[1,2,4]triazolo[5,1-i]purin-8-yl]-benzonitrile,
3-[5-Amino-3-(2-{4-[4-(2-methoxy-ethoxy)-phenyl]-piperazin-1-yl}-ethyl)-1-methyl-2-oxo-2,3-dihydro-1H-[1,2,4]triazolo[5,1-i]purin-8-yl]-benzonitrile,
5-Amino-8-furan-2-yl-1-methyl-3-vinyl-1,3-dihydro-[1,2,4]triazolo[5,1-i]purin-2-one
5-Amino-3-[3-(4-fluoro-phenyl)-prop-2-ynyl]-8-furan-2-yl-1-methyl-1,3-dihydro-[1,2,4]triazolo[5,1-i]purin-2-one,
5-Amino-8-furan-2-yl-1-methyl-3-[4-(4-methyl-piperazin-1-yl)-but-2-ynyl]-1,3-dihydro-[1,2,4]triazolo[5,1-i]purin-2-one,
5-Amino-8-furan-2-yl-1-isopropyl-3-(2-{4-[4-(2-methoxy-ethoxy)-phenyl]-piperazin-1-yl}-ethyl)-1,3-dihydro-[1,2,4]triazolo[5,1-i]purin-2-one,
5-Amino-2-benzyl-7-(2-{4-[4-(2-methoxy-ethoxy)-phenyl]-piperazin-1-yl}-ethyl)-9-methyl-7,9-dihydro-2H-[1,2,4]triazolo[3,4-i]purine-3,8-dione,
5-Amino-2-benzyl-9-methyl-7-(2-morpholin-4-yl-ethyl)-7,9-dihydro-2H-[1,2,4]triazolo[3,4-i]purine-3,8-dione,
5-Amino-2-(3-chloro-benzyl)-7-[2-(4-isopropyl-piperazin-1-yl)-ethyl]-9-methyl-7,9-dihydro-2H-[1,2,4]triazolo[3,4-i]purine-3, 8-dione,
5-Amino-2-cyclopropylmethyl-9-methyl-7-(2-morpholin-4-yl-ethyl)-7,9-dihydro-2H-[1,2,4]triazolo[3,4-i]purine-3,8-dione,
5-Amino-2-cyclopropylmethyl-7-(2,4-difluoro-benzyl)-9-methyl-7,9-dihydro-2H-[1,2,4]triazolo[3,4-i]purine-3,8-dione,
4-Amino-2-furan-2-yl-6-(2-{4-[4-(2-methoxy-ethoxy)-phenyl]-piperazin-1-yl}-ethyl)-6H-8-oxa-1,3,3a,5,6-pentaaza-as-indacen-7-one or
4-Amino-2-furan-2-yl-6-(2-{4-[4-(2-methoxy-ethoxy)-phenyl]-piperazin-1-yl}-ethyl)-8,8-dimethyl-6,8-dihydro-1,3,3a,5,6-pentaaza-as-indacen-7-one.

### Biological activity

### Radioligand Binding for A_{2A} Adenosine Receptor

Human A_{2A} adenosine receptor cDNA was stably transfected into HEK-293 cells.HEK-A_{2A} cells were harvested by trypsinization with 0.25% Trypsin-EDTA (Sigma), and washed in 1X PBS at 1500 rpm for 5 minutes at room temperature. The cells were washed twice in wash buffer containing 150 mM NaCl, 1 mM EDTA, 50 mM Tris (pH-7.4) at 1500 rpm for 10 minutes at room temperature and incubated for 10 min at 4°C in sonication buffer containing 1 mM EDTA, 5 mM (Tris pH 7.4). The cells were sonicated on ice for 6 min with six intermittent pulses of 9 second each and centrifuged at 1000Xg for 10 minutes at 4°C. The pellet was discarded and the supernatant was centrifuged at 49,000Xg for 45 minutes at 4°C. The protein pellet was resuspended in buffer containing 1 mM EDTA, 5 mM Tris (pH-7.4) supplemented with 1 Unit/ml adenosine deaminase (ADA) and incubated for 30 minutes at room temperature. The lysate was washed twice with buffer containing 1 mM EDTA, 5 mM Tris (pH-7.4) at 49,000Xg for 45 minutes at 4°C and the protein pellet was resuspended in 50 mM Tris, pH-7.4 supplemented with 1 Unit/ml ADA and 10% sucrose. Frozen aliquots were stored at -80°C.

**Competition assays** were started by mixing 2 nM [³H]-ZM-241385 with various concentrations of test compounds and 5 µg membrane protein in Reaction buffer (50 mM Tris pH 7.4, 1 mM EDTA) supplemented with 1 Unit/ml ADA. The assay reactions were incubated for 90 minutes at room temperature and stopped by filtration using 96 well-plate harvester (Molecular Devices) and washed four times with ice cold 50 mM Tris (pH 7.4). Non specific binding was determined in presence of 200 µM NECA. Radioligand binding was read at Liquid scintillation counter (Perkin Elmer) and the affinities of compounds (i.e. Kᵢ values) were calculated using GraphPad software.

Representative compounds of the present disclosure were tested and had micromolar to nanomolar activity.

| **Example No** | **A2A Cell based Functional Ki*** | **Example No** | **A2A Cell based Functional Ki*** |
|---|---|---|---|
| A1 | +++ | A62 | +++ |
| A7 | +++ | A63 | ++ |
| A9 | +++ | C1 | +++ |
| A13 | +++ | E1 | +++ |
| A31 | +++ | D3 | +++ |
| A32 | +++ | G1 | ++ |
| A36 | ++ | G2 | ++ |
| A38 | +++ | H2 | +++ |
| A39 | +++ | M1 | +++ |
| A42 | +++ | M2 | +++ |
| A57 | +++ | M3 | ++ |
| A58 | +++ | M6 | +++ |

| | | | |
|---|---|---|---|
| ++ = BINDING ACTIVITY IN THE RANGE OF 10 TO 100 nM +++ = BINDING ACTIVITY IN THE RANGE OF 0.1 TO 10 nM | | | |

## Claims

1. A compound represented by Formula I 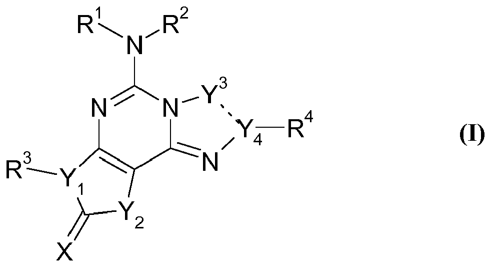 or a tautomer, polymorph, stereoisomer, solvate or a pharmaceutically acceptable salt thereof, wherein:
--- represents a single bond or a double bond;
X is selected from O, S or NR^{a};
Y₁ is selected from N or CH;
Y₂ is selected from NR⁵, O or CR⁵R⁶;
Y₃ is selected from N, CH, CH₂, C(=O) or C(=S);
Y₄ is selected from N, C or CH;
R¹ and R² are independently selected from hydrogen or alkyl;
R³ is -A-Z-B-Q;
wherein, A is absent or is a group selected from alkylene, alkenylene or alkynylene; wherein one or more methylene groups is optionally replaced by hetero atoms or groups such as -O-, -S(O)p-, -N(R^{a})-, or -C(O); alkylene, alkenylene and alkynylene is optionally substituted with -(CR^{d}R^{e})ₙOR⁷, (CR^{d}R^{e})ₙCOOR⁷, -(CR^{d}R^{e})ₙNR⁸R⁹, cyano, halogen, haloalkyl, perhaloalkyl, alkoxyalkoxy, alkyl or cycloalkyl;
Z is absent or is selected from a cycloalkyl or a heterocyclyl;
wherein cycloalkyl and heterocyclyl are unsubstituted or substituted independently with 1, 2, or 3 substituents independently selected from alkyl, alkenyl, alkynyl, acyl,-(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, halogen, haloalkyl, perhaloalkyl, azido, cyano, keto, thiocarbonyl, -SO₃H, aminocarbonylamino, nitro, -S(O)₂NR^{a}R^{a}, -NR^{b}S(O)₂R^{b} or - S(O)ₚR^{c};
B is absent or is a group selected from alkylene, alkenylene or alkynylene; wherein one or more methylene groups is optionally replaced by hetero atoms or groups such as -O-, -S(O)p-, -N(R^{a})-, or -C(O); alkylene, alkenylene and alkynylene is optionally substituted with hydroxy, amino, aminoalkyl, cyano, halogen, haloalkyl, perhaloalkyl, carboxy, carboxyalkyl, alkoxy, hydroxyalkyl, alkoxyalkyl, alkoxyalkoxy or alkyl;
Q is selected from hydrogen, alkyl, cycloalkyl, cycloalkylalkyl, aryl, arylalkyl, heterocyclyl, heterocyclylalkyl, heteroaryl or heteroarylalkyl;
wherein alkyl, cycloalkyl, cycloalkylalkyl, aryl, arylalkyl, heterocyclyl, heterocyclylalkyl, heteroaryl and heteroarylalkyl are unsubstituted or substituted independently with 1, 2, or 3 substituents independently selected from alkyl, alkenyl, alkynyl, halogen, haloalkyl, perhaloalkyl, azido, cyano, nitro, keto, thiocarbonyl, cyanoalkyl, cyanoalkylcarbonyl, -(CR^{d}R^{e})ₙOR⁷, -(CR^{d}R^{e})ₙC(O)R⁷, -(CR^{d}R^{e})ₙSR⁷, - (CR^{d}R^{e})ₙCOOR⁷, -(CR^{d}R^{e})ₙNR⁸R⁹, -(CR^{d}R^{e})ₙC(O)NR⁸R⁹, -(CR^{d}R^{e})ₙNR⁸C(O)OR⁷, - (CR^{d}R^{e})ₙNR⁸C(O)NR⁸R⁹, -NR^{b}S(O)₂R^{b}, -S(O)ₚR^{c}, -SO₃H, -S(O)₂NR^{a}R^{a}, cycloalkyl, cycloalkenyl, cycloalkylalkyl aryl, arylalkyl, heterocyclyl, heterocyclylalkyl, heteroaryl or heteroarylalkyl;
wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, alkenyl, alkynyl, haloalkyl, hydroxyalkyl, carboxyalkyl, cycloalkyl, cycloalkylalkyl, aryl, arylalkyl, heterocyclyl, heterocyclylalkyl, heteroaryl and heteroarylalkyl;
wherein alkyl, alkenyl, alkynyl, cycloalkyl, cycloalkylalkyl, arylalkyl, aryl, heteroaryl, heteroarylalkyl, heterocyclyl, and heterocyclylalkyl are unsubstituted or substituted independently with up to four substituents independently selected from alkyl, alkenyl, alkynyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, aminocarbonylamino, azido, cyano, halogen, haloalkyl, perhaloalkyl, keto, nitro, -S(O)₂NR^{b}R^{b}, -NR^{b}S(O)₂R^{b} or -S(O)ₚR^{c}, thiocarbonyl, -SO₃H, cycloalkyl, cycloalkenyl, aryl, heteroaryl or heterocyclyl ;
R⁵ and R⁶ are independently selected from the group consisting of hydrogen, hydroxy,-(CR^{d}R^{e})ₙOR⁷, (CR^{d}R^{e})ₙCOOR⁷, -(CR^{d}R^{e})ₙNR⁸R⁹, cyanoalkyl, haloalkyl, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, carbonylamino, cycloalkyl, cycloalkylalkyl, aryl, arylalkyl, heteroaryl, heteroarylalkyl, heterocyclyl or heterocyclylalkyl;
R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or S, said ring system is further optionally substituted with 1 to 4 substituents independently selected from halo, alkyl, alkenyl, alkynyl, nitro, cyano, -(CR^{d}R^{e})ₙOR⁷, -(CR^{d}R^{e})ₙSR⁷,-(CR^{d}R^{e})ₙNR⁸R⁹, oxo, alkylsulfonyl, -(CR^{d}R^{e})ₙCOOR⁷, -(CR^{d}R^{e})ₙC(O)NR⁸R⁹, cycloalkyl, cycloalkylalkyl, aryl, arylalkyl, heterocyclyl, heterocyclylalkyl, heteroaryl, or heteroarylalkyl;
R^{a} is selected from hydrogen or alkyl;
R^{b} at each occurrence is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl and heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4 and
p is 0, 1 or 2.

2. A compound of formula (I) as claimed in claim 1 or a tautomer, polymorph, stereoisomer, solvate or a pharmaceutically acceptable salt thereof, wherein
--- represents a double bond;
X is selected from O, S or NR^{a};
Y₁ is selected from N or CH;
Y₂ is selected from NR⁵ or CR⁵R⁶;
Y₃ is selected from N, CH or CH₂;
Y₄ is selected from N or C;
R¹ and R² are independently selected from hydrogen or alkyl;
R³ is -A-Z-B-Q;
wherein, A is absent or is alkylene wherein one or more methylene groups is optionally replaced by hetero atoms or groups such as -O-, -S(O)p-, -N(R^{a})-, or - C(O); alkylene is optionally substituted with -(CR^{d}R^{e})ₙOR⁷, cyano, halogen, haloalkyl, perhaloalkyl, alkyl or cycloalkyl;
Z is absent or is selected from a cycloalkyl or a heterocyclyl;
wherein cycloalkyl and heterocyclyl are unsubstituted or substituted independently with 1, 2, or 3 substituents independently selected from alkyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, halogen, haloalkyl, perhaloalkyl, azido, cyano, halogen, keto, thiocarbonyl, -SO₃H, aminocarbonylamino, nitro, -S(O)₂NR^{a}R^{a}, -NR^{b}S(O)₂R^{b} or -S(O)ₚR^{c};
B is absent or is alkylene wherein one or more methylene groups is optionally replaced by hetero atoms or groups such as -O-, -S(O)p-, -N(R^{a})-, or -C(O); alkylene is optionally substituted with hydroxy, amino, aminoalkyl, cyano, halogen, haloalkyl, perhaloalkyl, carboxy, carboxyalkyl, alkoxy, hydroxyalkyl, alkoxyalkyl, alkoxyalkoxy or alkyl;
Q is selected from hydrogen, alkyl, cycloalkyl, cycloalkylalkyl, aryl, arylalkyl, heterocyclyl, heterocyclylalkyl, heteroaryl or heteroarylalkyl;
wherein alkyl, cycloalkyl, cycloalkylalkyl, aryl, arylalkyl, heterocyclyl, heterocyclylalkyl, heteroaryl and heteroarylalkyl are unsubstituted or substituted independently with 1, 2, or 3 substituents independently selected from alkyl, alkenyl, alkynyl, alkoxy, alkoxyalkyl, haloalkyl, perhaloalkyl, azido, cyano, nitro, halogen, keto, thiocarbonyl, cyanoalkyl, cyanoalkylcarbonyl, -(CR^{d}R^{e})ₙOR⁷, -(CR^{d}R^{e})ₙC(O)R⁷, -(CR^{d}R^{e})ₙSR⁷, -(CR^{d}R^{e})ₙCOOR⁷, -(CR^{d}R^{e})ₙNR⁸R⁹, -(CR^{d}R^{e})ₙC(O)NR⁸R⁹, - (CR^{d}R^{e})ₙN^{R8}C(O)OR⁷, -(CR^{d}R^{e})ₙNR⁸C(O)NR⁸R⁹, -NR^{b}S(O)₂R^{b}, -S(O)ₚR^{c}, -SO_{3H}, - S(O)₂NR^{a}R^{a}, cycloalkyl, cycloalkenyl, cycloalkylalkyl aryl, arylalkyl, heterocyclyl, heterocyclylalkyl, heteroaryl or heteroarylalkyl;
wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, haloalkyl, hydroxyalkyl, carboxyalkyl, cycloalkyl, cycloalkylalkyl, aryl, arylalkyl, heterocyclyl, heterocyclylalkyl, heteroaryl and heteroarylalkyl;
wherein alkyl, cycloalkyl, cycloalkylalkyl, arylalkyl, aryl, heteroaryl, heteroarylalkyl, heterocyclyl, and heterocyclylalkyl are unsubstituted or independently substituted with up to four substituents independently selected from alkyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, aminocarbonylamino, azido, cyano, halogen, haloalkyl, perhaloalkyl, keto, nitro, - S(O)₂NR^{b}R^{b}, -NR^{b}S(O)₂R^{b} or -S(O)ₚR^{c}, thiocarbonyl, -SO₃H, cycloalkyl, cycloalkenyl, aryl, heteroaryl or heterocyclyl ;
R⁵ and R⁶ are independently selected from the group consisting of hydrogen, hydroxy,-(CR^{d}R^{e})ₙOR⁷, (CR^{d}R^{e})ₙCOOR⁷, -(CR^{d}R^{e})ₙNR⁸R⁹, cyanoalkyl, haloalkyl, alkoxyalkoxyalkyl, alkyl, alkenyl, alkynyl, cycloalkyl, cycloalkylalkyl, aryl, arylalkyl, heterocyclyl, heterocyclylalkyl, heteroaryl or heteroarylalkyl;
R⁷ is selected from hydrogen, alkyl, halogen, haloalkyl, carbonylamino, cycloalkyl, cycloalkylalkyl, aryl, arylalkyl, heteroaryl, heteroarylalkyl, heterocyclyl or heterocyclylalkyl;
R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or
S, said ring system is further optionally substituted with 1 to 4 substituents independently selected from halo, alkyl, alkenyl, alkynyl, nitro, cyano, -(CR^{d}R^{e})ₙOR⁷, -(CR^{d}R^{e})ₙSR⁷,-(CR^{d}R^{e})ₙNR⁸R⁹, oxo, alkylsulfonyl, -(CR^{d}R^{e})ₙCOOR⁷, -(CR^{d}R^{e})ₙC(O)NR⁸R⁹, cycloalkyl, cycloalkylalkyl, aryl, arylalkyl, heterocyclyl, heterocyclylalkyl, heteroaryl, or heteroarylalkyl;
R^{a} is selected from hydrogen or alkyl;
R^{b} each is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl and heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4 and
p is 0, 1 or 2.

3. A compound of formula (I) as claimed in claim 1 or a tautomer, polymorph, stereoisomer, solvate or a pharmaceutically acceptable salt thereof, wherein
--- represents a double bond;
X is selected from O or S;
Y₁ represents N;
Y₂ represents NR⁵;
Y₃ represents N;
Y₄ represents C;
R¹ and R² are independently selected from hydrogen or alkyl;
R³ is -A-Z-B-Q;
wherein, A is absent or is alkylene wherein one or more methylene groups is optionally replaced by hetero atoms or groups such as -O-, -S(O)p-, -N(R^{a})-, or - C(O);
Z is absent or is a heterocyclyl;
wherein the heterocyclyl is unsubstituted or substituted independently with 1, 2, or 3 substituents independently selected from alkyl, acyl, -(CR^{d}R^{e})ₙOR⁷, (CR^{d}R^{e})ₙCOOR⁷, -(CR^{d}R^{e})ₙNR⁸R⁹, haloalkyl, perhaloalkyl, cyano, halogen, keto, thiocarbonyl, -SO₃H, nitro, -S(O)₂NR^{a}R^{a}, -NR^{b}S(O)₂R^{b} or -S(O)ₚR^{c};
B is absent or is alkylene wherein one or more methylene groups is optionally replaced by hetero atoms or groups such as -O-, -S(O)p-, -N(R^{a})-, or -C(O);
Q is selected from hydrogen, alkyl, cycloalkyl, aryl, heterocyclyl, or heteroaryl;
wherein alkyl, cycloalkyl, aryl, heterocyclyl and heteroaryl are unsubstituted or independently substituted with 1, 2, or 3 substituents independently selected from alkyl, alkoxy, alkoxyalkyl, haloalkyl, perhaloalkyl, azido, cyano, nitro, halogen, keto, thiocarbonyl, cyanoalkyl, cyanoalkylcarbonyl, -(CR^{d}R^{e})ₙOR⁷, -(CR^{d}R^{e})ₙC(O)R⁷, - (CR^{d}R^{e})ₙSR⁷, -(CR^{d}R^{e})ₙCOOR⁷, -(CR^{d}R^{e})ₙNR⁸R⁹, -(CR^{d}R^{e})ₙC(O)NR⁸R⁹, - (CR^{d}R^{e})ₙNR⁸C(O)OR⁷, -(CR^{d}R^{e})ₙNR⁸C(O)NR⁸R⁹, -NR^{b}S(O)₂R^{b}, -S(O)ₚR^{c}, -SO₃H, - S(O)₂NR^{a}R^{a}, cycloalkyl, cycloalkenyl, cycloalkylalkyl aryl, arylalkyl, heterocyclyl, heterocyclylalkyl, heteroaryl or heteroarylalkyl;
wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, haloalkyl, hydroxyalkyl, carboxyalkyl, cycloalkyl, cycloalkylalkyl, aryl, arylalkyl, heterocyclyl, heterocyclylalkyl, heteroaryl and heteroarylalkyl;
wherein alkyl, cycloalkyl, cycloalkylalkyl, arylalkyl, aryl, heteroaryl, heteroarylalkyl, heterocyclyl, and heterocyclylalkyl are unsubstituted or independently substituted with up to four substituents independently selected from alkyl, acyl, -(CR^{d}R^{e})ₙOR⁷, (CR^{d}R^{e})ₙCOOR⁷, -(CR^{d}R^{e})ₙNR⁸R⁹, aminocarbonyl, alkoxycarbonylamino, aminocarbonylamino, azido, cyano, halogen, haloalkyl, perhaloalkyl, keto, nitro, - S(O)₂NR^{b}R^{b}, -NR^{b}S(O)₂R^{b} or -S(O)ₚR^{c}, thiocarbonyl, -SO₃H, cycloalkyl, cycloalkenyl, aryl, heteroaryl or heterocyclyl;
R⁵ and R⁶ are independently selected from the group consisting of hydrogen, hydroxy,-(CR^{d}R^{e})ₙOR⁷, (CR^{d}R^{e})ₙCOOR⁷, -(CR^{d}R^{e})ₙNR⁸R⁹, cyanoalkyl, haloalkyl, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, carbonylamino, cycloalkyl, cycloalkylalkyl, aryl, arylalkyl, heteroaryl, heteroarylalkyl, heterocyclyl or heterocyclylalkyl;
R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or
S, said ring system is further optionally substituted with 1 to 4 substituents independently selected from halo, alkyl, alkenyl, alkynyl, nitro, cyano, -(CR^{d}R^{e})ₙOR⁷, -(CR^{d}R^{e})ₙSR⁷,-(CR^{d}R^{e})ₙNR⁸R⁹, oxo, alkylsulfonyl, -(CR^{d}R^{e})ₙCOOR⁷, -(CR^{d}R^{e})ₙC(O)NR⁸R⁹, cycloalkyl, cycloalkylalkyl, aryl, arylalkyl, heterocyclyl, heterocyclylalkyl, heteroaryl, or heteroarylalkyl;
R^{a} is selected from hydrogen or alkyl;
R^{b} each is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl and heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl or alkyl;
n is 0, 1, 2, 3 or 4 and
p is 0, 1 or 2.

4. A compound of formula (I) as claimed in claim 1 or a tautomer, polymorph, stereoisomer, solvate or a pharmaceutically acceptable salt thereof, wherein
--- represents a double bond;
X selected from O or S;
Y₁ represents N;
Y₂ represents NR⁵;
Y₃ represents N;
Y₄ represents C;
R¹ and R² are independently selected from hydrogen or alkyl;
R³ is -A-Z-B-Q;
wherein, A is absent or is alkylene wherein one or more methylene groups is optionally replaced by hetero atoms or groups such as -O- or -N(R^{a})-;
Z is absent or is a heterocyclyl;
wherein the heterocyclyl is unsubstituted or substituted independently with 1, 2, or 3 substituents independently selected from alkyl, -(CR^{d}R^{e})ₙOR⁷, (CR^{d}R^{e})ₙCOOR⁷, haloalkyl, perhaloalkyl, cyano, halogen, keto or thiocarbonyl;
B is absent or is alkylene wherein one or more methylene groups is optionally replaced by hetero atoms or groups such as -O-, -N(R^{a})-, or -C(O);
Q is selected from hydrogen, alkyl, cycloalkyl, aryl, heterocyclyl, or heteroaryl;
wherein alkyl, cycloalkyl, aryl, heterocyclyl and heteroaryl are unsubstituted or independently substituted with 1, 2, or 3 substituents independently selected from alkyl, alkoxy, alkoxyalkyl, haloalkyl, perhaloalkyl, cyano, halogen, keto, thiocarbonyl, cyanoalkyl, -(CR^{d}R^{e})ₙOR⁷, -(CR^{d}R^{e})ₙC(O)R⁷, -(CR^{d}R^{e})ₙCOOR⁷,-(CR^{d}R^{e})ₙNR⁸R⁹, -(CR^{d}R^{e})ₙC(O)NR⁸R⁹, -(CR^{d}R^{e})ₙNR⁸C(O)OR⁷, -S(O)ₚR^{c}, -SO₃H,-S(O)₂NR^{a}R^{a}, cycloalkyl, cycloalkenyl, aryl, heterocyclyl or heteroaryl;
wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, haloalkyl, hydroxyalkyl, cycloalkyl, aryl, heterocyclyl and heteroaryl;
wherein alkyl, cycloalkyl, aryl, heteroaryl and heterocyclyl are unsubstituted or
independently substituted with up to four substituents independently selected from alkyl,-(CR^{d}R^{e})ₙOR⁷, (CR^{d}R^{e})ₙCOOR⁷, -(CR^{d}R^{e})ₙNR⁸R⁹, cyano, halogen, haloalkyl, perhaloalkyl, nitro, -S(O)₂NR^{b}R^{b}, -NR^{b}S(O)₂R^{b}, -S(O)ₚR^{c}, thiocarbonyl, -SO₃H, cycloalkyl, aryl, heteroaryl or heterocyclyl;
R⁵ is selected from the group consisting of hydrogen, hydroxy, haloalkyl, -(CR^{d}R^{e})ₙOR⁷, -(CR^{d}R^{e})ₙCOOR⁷, alkoxyalkoxyalkyl, alkyl, alkenyl, alkynyl, cycloalkyl, cycloalkylalkyl, aryl, arylalkyl, heterocyclyl, heterocyclylalkyl, heteroaryl or heteroarylalkyl;
R⁷ is selected from hydrogen, alkyl, halogen, haloalkyl, cycloalkyl, aryl, heteroaryl or heterocyclyl;
R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or
S, said ring system is further optionally substituted with 1 to 4 substituents independently selected from halo, alkyl, nitro, cyano, -(CR^{d}R^{e})ₙOR⁷, -(CR^{d}R^{e})ₙNR⁸R⁹, oxo, alkylsulfonyl, -(CR^{d}R^{e})ₙCOOR⁷ or -(CR^{d}R^{e})ₙC(O)NR⁸R⁹;
R^{a} is selected from hydrogen or alkyl;
R^{b} each is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl or heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4 and
p is 0, 1 or 2.

5. A compound of formula (I) as claimed in claim 1 or a tautomer, polymorph, stereoisomer, solvate or a pharmaceutically acceptable salt thereof, wherein
--- represents a double bond;
X is selected from O or S;
Y₁ represents N;
Y₂ represents NR⁵;
Y₃ represents N;
Y₄ represents C;
R¹ and R² are independently selected from hydrogen or alkyl;
R³ is -A-Z-B-Q;
wherein, A is absent or is alkylene wherein one or more methylene groups is optionally replaced by hetero atoms or groups such as -O- or -N(R^{a})-;
Z is absent or is a heterocyclyl selected from dihydrofuranyl, tetrahydrofuranyl, morpholinyl, pyrrolidinyl, dihydropyrrole, dihydropyranyl, tetrahydropyranyl, pyrazolidinyl, imidazolidinyl, dihydropyridinyl, tetrahydropyridinyl, piperidinyl, dihydropyrazinyl, tetrahydropyrazinyl, piperazinyl or dihydropyridinyl;
wherein the heterocyclyl is unsubstituted or substituted independently with 1, 2, or 3 substituents independently selected from alkyl, -(CR^{d}R^{e})ₙOR⁷, (CR^{d}R^{e})ₙCOOR⁷, haloalkyl, perhaloalkyl, cyano or halogen;
B is absent or is alkylene wherein one or more methylene groups is optionally replaced by hetero atoms or groups such as -O-, -N(R^{a})-, or -C(O);
Q is selected from hydrogen, alkyl, cyclopropyl, cyclopentyl, cyclohexyl, phenyl, tetrahydrofuranyl, pyrrolidinyl, tetrahydropyridinyl, tetrahydropyranyl, piperazinyl, benzodiaxolyl, tetrahydroquinolinyl, morpholinyl, tetrahydronaphthyridinyl, tetrahydrothienopyridinyl, furanyl, pyridinyl, pyrimidinyl, oxazolyl, thiazolyl, oxadiazolyl, thiadiazolyl, indolyl, quinolinyl, isoquinolinyl or benzooxazolyl;
wherein Q is unsubstituted or substituted with 1, 2, or 3 substituents independently selected from alkyl, alkoxy, alkoxyalkyl, haloalkyl, perhaloalkyl, cyano, halogen, keto, thiocarbonyl, cyanoalkyl, -(CR^{d}R^{e})ₙOR⁷, -(CR^{d}R^{e})ₙC(O)R⁷, -(CR^{d}R^{e})ₙCOOR⁷,-(CR^{d}R^{e})ₙNR⁸R⁹, -(CR^{d}R^{e})ₙC(O)NR⁸R⁹, -(CR^{d}R^{e})ₙNR⁸C(O)OR⁷, -S(O)ₚR^{c}, -SO₃H,-S(O)₂NR^{a}R^{a}, cycloalkyl, cycloalkenyl, aryl, heterocyclyl or heteroaryl;
wherein each substituent is unsubstituted or substituted with 1, 2, or 3 substituents independently selected from alkyl, carboxy, carboxyalkyl, aminocarbonyl, hydroxy, alkoxy, halogen, haloalkyl, perhaloalkyl, haloalkoxy, perhaloalkoxy, amino, substituted amino, cyano or -S(O)ₚR^{c};
R⁴ is selected from the group consisting of hydrogen, alkyl, phenyl, naphthyl, furanyl, thiazolyl, oxazolyl, thiadiazolyl, oxadiazolyl, pyrazinyl, pyridinyl and pyrimidinyl;
wherein R⁴ is unsubstituted or substituted with up to four substituents independently selected from alkyl, -(CR^{d}R^{e})ₙOR⁷, (CR^{d}R^{e})ₙCOOR⁷, -(CR^{d}R^{e})ₙNR⁸R⁹, cyano, halogen, haloalkyl, perhaloalkyl or cycloalkyl;
R⁵ is selected from the group consisting of hydrogen, hydroxy, haloalkyl, -(CR^{d}R^{e})ₙOR⁷, -(CR^{d}R^{e})ₙCOOR⁷, alkoxyalkoxyalkyl, alkyl, alkenyl, alkynyl, cycloalkyl, cycloalkylalkyl, aryl, arylalkyl, heterocyclyl, heterocyclylalkyl, heteroaryl and heteroarylalkyl;
R⁷ is selected from hydrogen, alkyl, halogen, haloalkyl, cycloalkyl, aryl, heteroaryl or heterocyclyl;
R⁸ and R⁹ are independently selected from the group consisting of hydrogen, alkyl, haloalkyl, -(CR^{d}R^{e})ₙOR⁷, -(CR^{d}R^{e})ₙC(O)R⁷, aryl, arylalkyl, heteroaryl, heteroarylalkyl, cycloalkyl, cycloalkylalkyl, heterocyclyl and heterocyclylalkyl, or
R⁸ and R⁹ taken together form a monocyclic or a bicyclic ring system which is saturated or partially unsaturated and optionally have additional heteroatoms selected from O, N or
S, said ring system is further optionally substituted with 1 to 4 substituents independently selected from halo, alkyl, nitro, cyano, -(CR^{d}R^{e})ₙOR⁷, -(CR^{d}R^{e})ₙNR⁸R⁹, oxo, alkylsulfonyl, -(CR^{d}R^{e})ₙCOOR⁷ or -(CR^{d}R^{e})ₙC(O)NR⁸R⁹;
R^{a} is selected from hydrogen or alkyl;
R^{b} at each occurrence is independently selected from the group consisting of hydrogen, alkyl, acyl, carboxyalkyl, carbonylamino, cycloalkyl, cycloalkylalkyl, aryl, arylalkyl, heteroaryl, heteroarylalkyl, heterocyclyl or heterocyclylalkyl;
R^{c} is selected from alkyl, cycloalkyl, aryl, heterocyclyl or heteroaryl;
R^{d} and R^{e} are independently selected from the group consisting of hydrogen, -OR⁷, halogen, haloalkyl, perhaloalkyl and alkyl;
n is 0, 1, 2, 3 or 4 and
p is 0, 1 or 2.

6. A compound of formula (I) as claimed in claim 1 or a tautomer, polymorph, stereoisomer, solvate or a pharmaceutically acceptable salt thereof, which is
5-Amino-8-(2-furyl)-3-[2-[4-[4-(2-methoxyethoxy)phenyl]piperazin-1-yl]ethyl]-1-methyl-[1,2,4]triazolo[5,1-f]purin-2-one,
5-Amino-8-(2-furyl)-3-(2-hydroxyethyl)-1-methyl-[1,2,4]triazolo[5,1-f]purin-2-one,
5-Amino-3-[2-[4-(2,4-difluorophenyl)piperazin-1-yl]ethyl]-8-(2-furyl)-1-methyl-[1,2,4]triazolo[5,1-f]purin-2-one,
5-Amino-8-(2-furyl)-3-[2-[4-(4-methoxyphenyl)piperazin-1-yl]ethyl]-1-methyl-[1,2,4]triazolo[5,1-f]purin-2-one,
5-Amino-8-(2-furyl)-1-methyl-3-(2-morpholinoethyl)-[1,2,4]triazolo[5,1-f]purin-2-one,
5-Amino-3-[2-[4-(2,4-difluorophenyl)-1-piperidyl]ethyl]-8-(2-furyl)-1-methyl-[1,2,4]triazolo[5,1-f]purin-2-one,
5-Amino-8-(2-furyl)-1-methyl-3-[2-[4-(5-methyl-2-pyridyl)piperazin-1-yl]ethyl]-[1,2,4]triazolo[5,1-f]purin-2-one,
5-Amino-8-(2-furyl)-1-methyl-3-[2-[4-(p-tolyl)piperazin-1-yl]ethyl]-[1,2,4]triazolo[5,1-f]purin-2-one,
5-Amino-8-(2-furyl)-1-methyl-3-[2-[4-(3-methyl-2-oxo-butyl)piperazin-1-yl]ethyl]-[1,2,4]triazolo[5,1-f]purin-2-one.
5-Amino-3-[2-[4-(2-fluoro-4-methoxy-phenyl)piperazin-1-yl]ethyl]-8-(2-furyl)-1-methyl-[1,2,4]triazolo[5,1-f]purin-2-one,
5-Amino-8-(2-furyl)-3-[2-[4-[4-(2-methoxy-1,1-dimethyl-ethoxy)phenyl]piperazin-1-yl]ethyl]-1-methyl-[1,2,4]triazolo[5,1-f]purin-2-one,
5-Amino-8-(2-furyl)-3-[2-[4-(6-methoxy-3-pyridyl)piperazin-1-yl]ethyl]-1-methyl-[1,2,4]triazolo[5,1-f]purin-2-one,
5-Amino-3-[2-[4-[3-fluoro-4-(2-methoxyethoxy)phenyl]piperazin-1-yl]ethyl]-8-(2-furyl)-1-methyl-[1,2,4]triazolo[5,1-f]purin-2-one,
5-Amino-8-(2-furyl)-3-[2-[4-[4-(1-hydroxy-1-methyl-ethyl)phenyl]piperazin-1-yl]ethyl]-1-methyl-[1,2,4]triazolo [5,1-f]purin-2-one,
5-Amino-3-[2-[4-(4-fluorophenyl)-4-hydroxy-1-piperidyl]ethyl]-8-(2- furyl)-1-methyl-[1,2,4]triazolo[5,1-f]purin-2-one,
5-Amino-8-(2-furyl)-3-[2-[4-[4-(2-methoxy-2-methyl-propoxy)phenyl]piperazin-1-yl]ethyl]-1-methyl-[1,2,4]triazolo[5,1-f]purin-2-one,
5-Amino-3-[2-[4-[4-(cyclopropoxy)phenyl]piperazin-1-yl]ethyl]-8-(2-furyl)-1-methyl-[1,2,4]triazolo[5,1-f]purin-2-one,
5-Amino-3-[2-[4-(4-fluorophenyl)-3,6-dihydro-2H-pyridin-1-yl]ethyl]-8-(2-furyl)-1-methyl-[1,2,4]triazolo[5,1-f]purin-2-one,
5-Amino-8-(2-furyl)-3-[2-[4-hydroxy-4-(4-methoxyphenyl)-1-piperidyl]ethyl]-1-methyl-[1,2,4]triazolo[5,1-f]purin-2-one,
5-Amino-3-[2-[4-[3,5-difluoro-4-(2-methoxyethoxy)phenyl]piperazin-1-yl]ethyl]-8-(2-furyl)-1-methyl-[1,2,4]triazolo[5,1-f]purin-2-one,
5-Amino-3-[2-[4-[2,5-difluoro-4-(2-methoxyethoxy)phenyl]piperazin-1-yl]ethyl]-8-(2-furyl)-1-methyl-[1,2,4]triazolo[5,1-f]purin-2-one,
5-Amino-3-[2-[4-(2,2-difluoro-1,3-benzodioxol-5-yl)piperazin-1-yl]ethyl]-8-(2-furyl)-1-methyl-[1,2,4]triazolo[5,1-f]purin-2-one,
5-Amino-8-(2-furyl)-3-[2-[4-[4-(2-methoxyethoxy)phenyl]-3,3-dimethyl-piperazin-1-yl]ethyl]-1-methyl-[1,2,4]triazolo[5,1-f]purin-2-one,
5-Amino-3-[2-(4-butylpiperazin-1-yl)ethyl]-8-(2-furyl)-1-methyl-[1,2,4]triazolo[5,1-f]purin-2-one,
5-Amino-8-(2-furyl)-3-[2-(4-hydroxy-4-methyl-1-piperidyl)ethyl]-1-methyl-[1,2,4]triazolo[5,1-f]purin-2-one,
5-Amino-3-[2-[4-[4-[2-(cyclopropoxy)ethoxy]phenyl]piperazin-1-yl]ethyl]-8-(2-furyl)-1-methyl-[1,2,4]triazolo[5,1-f]purin-2-one,
5-Amino-8-(2-furyl)-3-[2-[4-[(4-methoxyphenyl)methyl]piperazin-1-yl]ethyl]-1-methyl-[1,2,4]triazolo[5,1-f]purin-2-one,
5-Amino-8-(2-furyl)-3-[2-[4-[[4-(2-methoxyethoxy)phenyl]methyl]piperazin-1-yl]ethyl]-1-methyl-[1,2,4]triazolo [5,1-f]purin-2-one,
5-Amino-8-(2-furyl)-3-[(4-methoxyphenyl)methyl]-1-methyl-[1,2,4]triazolo[5,1-f]purin-2-one,
5-Amino-8-(2-furyl)-3-[2-[4-(4-methoxyphenyl)piperazin-1-yl]ethyl]-1-methyl-[1,2,4]triazolo[5,1-f]purin-2-one,
5-Amino-8-(2-furyl)-3-[2-[4-[3-(2-methoxyethoxy)phenyl]piperazin-1-yl]ethyl]-1-methyl-[1,2,4]triazolo[5,1-f]purin-2-one,
5-Amino-3-[2-[4-[2-fluoro-4-(2-methoxyethoxy)phenyl]piperazin-1-yl]ethyl]-8-(2-furyl)-1-methyl-[1,2,4]triazolo [5,1-f]purin-2-one,
4-[4-[2-[5-Amino-8-(2-furyl)-1-methyl-2-oxo-[1,2,4]triazolo[5,1-f]purin-3yl]ethyl]piperazin-1-yl]benzonitrile,
4-[4-[2-[5-Amino-8-(2-furyl)-1-methyl-2-oxo-[1,2,4]triazolo[5,1-f]purin-3-yl]ethyl]piperazin-1-yl]-2-fluoro-benzonitrile,
5-Amino-8-(2-furyl)-1-methyl-3-[2-[4-[4-(trifluoromethyl)phenyl]piperazin-1-yl]ethyl]-[1,2,4]triazolo[5,1-f]purin-2-one,
5-Amino-8-(2-furyl)-1-methyl-3-[2-[4-[4-(trifluoromethyl)thiazol-2-yl]piperazin-1-yl]ethyl]-[1,2,4]triazolo[5,1-f]purin-2-one,
5-Amino-3-[2-[4-(cyclopropylmethyl)piperazin-1-yl]ethyl]-8-(2-furyl)-1-methyl-[1,2,4]triazolo[5,1-f]purin-2-one,
5-Amino-3-[2-(4-ethylpiperazin-1-yl)ethyl]-8-(2-furyl)-1-methyl-[1,2,4]triazolo[5,1-f]purin-2-one,
4-[2-[5-Amino-8-(2-furyl)-1-methyl-2-oxo-[1,2,4]triazolo[5,1-f]purin-3-yl]ethyl]-N,N-dimethyl-piperazine-1-sulfonamide,
5-Amino-8-(2-furyl)-1-methyl-3-[2-[4-(4-tetrahydrofuran-3-yloxyphenyl)piperazin-1-yl]ethyl]-[1,2,4]triazolo[5,1-f]purin-2-one,
5-Amino-8-(2-furyl)-1-methyl-3-[2-[4-(4-tetrahydropyran-4-yloxyphenyl)piperazin-1-yl]ethyl]-[1,2,4]triazolo[5,1-f]purin-2-one,
5-Amino-8-(2-furyl)-1-methyl-3-[2-[4-[4-(tetrahydrofuran-2-ylmethoxy)phenyl]piperazin-1-yl]ethyl]-[1,2,4]triazolo[5,1-f]purin-2-one,
5-Amino-8-(2-furyl)-1-methyl-3-[2-(3-methyl-7,8-dihydro-5H-1,6-naphthyridin-6-yl)ethyl]-[1,2,4]triazolo[5,1-f]purin-2-one,
5-Amino-3-[2-(6,7-dihydro-4H-thieno[3,2-c]pyridin-5-yl)ethyl]-8-(2-furyl)-1-methyl-[1,2,4]triazolo[5,1-f]purin-2-one,
5-Amino-8-(2-furyl)-3-[2-[4-[4-(2-methoxyethoxy)phenyl]piperazin-1-yl]propyl]-1-methyl-[1,2,4]triazolo[5,1-f]purin-2-one,
5-Amino-3-[2-[3-(4-fluorophenyl)-2,5-dihydropyrrol-1-yl]ethyl]-8-(2-furyl)-1-methyl-[1,2,4]triazolo[5,1-f]purin-2-one,
5-Amino-3-[2-[4-[4-(2-methoxyethoxy)phenyl]piperazin-1-yl]ethyl]-1-methyl-8-(5-methyl-2-furyl)-[1,2,4]triazolo[5,1-f]purin-2-one,
5-Amino-8-(5-cyclopropyl-2-furyl)-3-[2-[4-[4-(2-methoxyethoxy)phenyl]piperazin-1-yl]ethyl]-1-methyl-[1,2,4]triazolo[5,1-f]purin-2-one,
5-Amino-3-[2-(2,4-difluoroanilino)ethyl]-8-(2-furyl)-1-methyl-[1,2,4]triazolo[5,1-f]purin-2-one,
5-Amino-3-[3-[4-(4-fluorophenyl)piperazin-1-yl]propyl]-8-(2-furyl)-1-methyl-[1,2,4]triazolo[5,1-f]purin-2-one,
5-Amino-8-(2-furyl)-3-[3-[4-[4-(2-methoxyethoxy)phenyl]piperazin-1-yl]propyl]-1-methyl-[1,2,4]triazolo[5,1-f]purin-2-one,
5-Amino-8-(2-furyl)-3-[2-[4-(4-methoxyphenyl)-3,6-dihydro-2H-pyridin-1-yl]ethyl]-1-methyl-[1,2,4]triazolo[5,1-f]purin-2-one,
5-Amino-8-(2-furyl)-3-[2-[4-[4-(2-methoxy-1,1-dimethyl-ethyl)phenyl]piperazin-1-yl]ethyl]-1-methyl-[1,2,4]triazolo[5,1-f]purin-2-one,
5-Amino-8-(2-furyl)-1-methyl-3-(2-piperazin-1-ylethyl)-[1,2,4]triazolo[5,1-f]purin-2-one,
5-Amino-8-(2-furyl)-3-[2-[4-(1H-indole-2-carbonyl)piperazin-1-yl]ethyl]-1-methyl-[1,2,4]triazolo[5,1-f]purin-2-one,
5-Amino-8-(2-furyl)-3-[2-(4-isopropoxyphenyl)ethyl]-1-methyl-[1,2,4]triazolo[5,1-f]purin-2-one,
5-Amino-8-(2-furyl)-1-methyl-3-[2-[4-[(2S)-pyrrolidine-2-carbonyl]piperazin-1-yl]ethyl]-[1,2,4]triazolo[5,1-f]purin-2-one,
5-Amino-8-(2-furyl)-3-[2-(4-methoxyphenyl)ethyl]-1-methyl-[1,2,4]triazolo[5,1-f]purin-2-one,
5-amino-3-[2-[4-[4-(difluoromethoxy)phenyl]piperazin-1-yl]ethyl]-8-(2-furyl)-1-methyl-[1,2,4]triazolo[5,1-f]purin-2-one,
5-Amino-8-(2-furyl)-1-methyl-3-[2-[4-[3-(5-methyl-1,3,4-oxadiazol-2-yl)phenyl]piperazin-1-yl]ethyl]-[1,2,4]triazolo[5,1-f]purin-2-one,
5-Amino-3-[2-[4-[2-fluoro-4-(5-methyl-1,2,4-oxadiazol-3-yl)phenyl]piperazin-1-yl]ethyl]-8-(2-furyl)-1-methyl-[1,2,4]triazolo[5,1-f]purin-2-one,
5-Amino-3-[2-[4-(6-fluoro-2-methyl-1,3-benzoxazol-5-yl)piperazin-1-yl]ethyl]-8-(2-furyl)-1-methyl-[1,2,4]triazolo[5,1-f]purin-2-one,
5-Amino-3-[2-[4-(cyclopropanecarbonyl)piperazin-1-yl]ethyl]-8-(2-furyl)-1-methyl-[1,2,4]triazolo[5,1-f]purin-2-one,
5-Amino-3-[2-[4-(2-cyclopropylacetyl)piperazin-1-yl]ethyl]-8-(2-furyl)-1-methyl-[1,2,4]triazolo[5,1-f]purin-2-one,
5-Amino-8-(2-furyl)-3-[2-[4-[4-(2-hydroxyethoxy)phenyl]piperazin-1-yl]ethyl]-1-methyl-[1,2,4]triazolo[5,1-f]purin-2-one,
5-Amino-8-(2-furyl)-3-[2-[4-(4-hydroxyphenyl)piperazin-1-yl]ethyl]-1-methyl-[1,2,4]triazolo[5,1-f]purin-2-one,
5-Amino-1-(cyclopropylmethyl)-3-[2-[4-(4-ethoxyphenyl)piperazin-1-yl]ethyl]-8-(2-furyl)-[1,2,4]triazolo[5,1-f]purin-2-one,
5-Amino-1-(cyclopropylmethyl)-3-[2-[4-(4-fluorophenyl)piperazin-1-yl]ethyl]-8-(2-furyl)-[1,2,4]triazolo[5,1-f]purin-2-one,
5-Amino-1-(cyclopropylmethyl)-3-[2-[4-(2,4-difluorophenyl)piperazin-1-yl]ethyl]-8-(2-furyl)-[1,2,4]triazolo[5,1-f]purin-2-one,
5-Amino-1-(cyclopropylmethyl)-8-(2-furyl)-3-[2-[4-[4-(2-methoxyethoxy)phenyl]piperazin-1-yl]ethyl]-[1,2,4]triazolo[5,1-f]purin-2-one,
5-Amino-1-(cyclopropylmethyl)-3-[2-(4-fluorophenoxy)ethyl]-8-(2-furyl)-[1,2,4]triazolo[5,1-f]purin-2-one,
5-Amino-8-(2-furyl)-1-methyl-3-[2-[2-oxo-5-(trifluoromethyl)-1-pyridyl]ethyl]-[1,2,4]triazolo[5,1-f]purin-2-one,
5-Amino-3-[2-[4-(2,4-difluorophenyl)pyrazol-1-yl]ethyl]-1-ethyl-8-(2-furyl)-[1,2,4]triazolo[5,1-f]purin-2-one,
1-[2-[5-Amino-1-(cyclopropylmethyl)-8-(2-furyl)-2-oxo-[1,2,4]triazolo[5,1-f]purin-3-yl]ethyl]pyrazole-4-carboxylic acid,
1-[2-[5-Amino-8-(2-furyl)-1-methyl-2-oxo-[1,2,4]triazolo[5,1-f]purin-3-yl]ethyl]pyrazole-4-carboxylic acid,
1-[2-[5-amino-1-(cyclopropylmethyl)-8-(2-furyl)-2-oxo-[1,2,4]triazolo[5,1-f]purin-3-yl]ethyl]-N-cyclopropyl-pyrazole-4-carboxamide,
1-[2-[5-amino-1-(cyclopropylmethyl)-8-(2-furyl)-2-oxo-[1,2,4]triazolo[5,1-f]purin-3-yl]ethyl]-N,N-diethyl-pyrazole-4-carboxamide,
1-[2-[5-Amino-1-(cyclopropylmethyl)-8-(2-furyl)-2-oxo-[1,2,4]triazolo[5,1-f]purin-3-yl]ethyl]-N-cyclopropyl-5-methyl-pyrazole-3-carboxamide,
2-[2-[5-Amino-1-(cyclopropylmethyl)-8-(2-furyl)-2-oxo-[1,2,4]triazolo[5,1-f]purin-3-yl]ethyl]-N-cyclopropyl-5-methyl-pyrazole-3-carboxamide,
1-[2-[5-Amino-8-(2-furyl)-1-methyl-2-oxo-[1,2,4]triazolo[5,1-f]purin-3-yl]ethyl]-N-methyl-pyrazole-3-carboxamide,
1-[2-[5-Amino-8-(2-furyl)-1-methyl-2-oxo-[1,2,4]triazolo[5,1-f]purin-3-yl]ethyl]-N,N-diethyl-pyrazole-4-carboxamide,
1-[2-[5-amino-8-(2-furyl)-1-methyl-2-oxo-[1,2,4]triazolo[5,1-f]purin-3-yl]ethyl]pyrazole-4-carboxamide,
5-Amino-8-(2-furyl)-3-[2-[4-[(3R)-3-hydroxypyrrolidine-1-carbonyl]pyrazol-1-yl]ethyl]-1-methyl-[1,2,4]triazolo[5,1-f]purin-2-one,
1-[2-[5-Amino-8-(2-furyl)-1-methyl-2-oxo-[1,2,4]triazolo [5,1-f]purin-3-yl]ethyl]-N-methyl-pyrazole-4-carboxamide,
1-[2-[5-Amino-8-(2-furyl)-1-methyl-2-oxo-[1,2,4]triazolo[5,1-f]purin-3-yl]ethyl]-N-cyclopropyl-pyrazole-3-carboxamide,
1-[2-[5-Amino-8-(2-furyl)-1-methyl-2-oxo-[1,2,4]triazolo[5,1-f]purin-3-yl]ethyl]-N-cyclopropyl-pyrazole-4-carboxamide,
5-Amino-8-(2-furyl)-3-[2-[4-(3-hydroxyazetidine-1-carbonyl)pyrazol-1-yl]ethyl]-1-methyl-[1,2,4]triazolo[5,1-f]purin-2-one,
5-Amino-1-ethyl-8-(2-furyl)-3-[2-[4-[4-(2-methoxyethoxy)phenyl]piperazin-1-yl]ethyl]-[1,2,4]triazolo[5,1-f]purin-2-one,
5-Amino-3-[2-[4-(2,4-difluorophenyl)piperazin-1-yl]ethyl]-1-ethyl-8-(2-furyl)-[1,2,4]triazolo[5,1-f]purin-2-one,
5-Amino-1-ethyl-3-{2-[4-(4-fluoro-phenyl)-piperidin-1-yl]-ethyl}-8-furan-2-yl-1,3-dihydro-[1,2,4]triazolo[5,1-f]purin-2-one,
5-Amino-1-ethyl-8-(2-furyl)-3-[2-(3-methyl-7,8-dihydro-5H-1,6-naphthyridin-6-yl)ethyl]-[1,2,4]triazolo[5,1-f]purin-2-one,
5-Amino-8-(2-furyl)-3-[2-[4-[4-(2-methoxyethoxy)phenyl]piperazin-1-yl]ethyl]-1-(2,2,2-trifluoroethyl)-[1,2,4]triazolo[5,1-f]purin-2-one,
5-Amino-3-{2-[4-(2,4-difluoro-phenyl)-piperazin-1-yl]-ethyl}-8-furan-2-yl-1-(2,2,2-trifluoro-ethyl)-1,3-dihydro-[1,2,4]triazolo[5,1-f]purin-2-one,
5-Amino-8-(2-furyl)-3-[2-[4-[4-(2-methoxyethoxy)phenyl]piperazin-1-yl]ethyl]-1-(2-methoxyethyl)-[1,2,4]triazolo[5,1-f]purin-2-one,
5-amino-3-[2-[4-(4-fluorophenyl)piperazin-1-yl] ethyl]-8-(2-furyl)-1-(2-methoxyethyl)-[1,2,4]triazolo[5,1-f]purin-2-one,
5-Amino-3-[2-[4-(4-fluorophenyl)piperazin-1-yl] ethyl]-8-(2-furyl)-1-(2-hydroxyethyl)-[1,2,4]triazolo[5,1-f]purin-2-one one,
5-Amino-1-cyclopropyl-8-(2-furyl)-3-[2-[4-[4-(2-methoxyethoxy)phenyl]piperazin-1-yl]ethyl]-[1,2,4]triazolo[5,1-f]purin-2-one,
5-Amino-8-(2-furyl)-3-[2-[4-[4-(2-methoxyethoxy)phenyl]piperazin-1-yl]ethyl]-1-(2,2,2-trifluoroethyl)-[1,2,4]triazolo[5,1-f]purin-2-one,
5-Amino-3-[2-[4-[4-(2-methoxyethoxy)phenyl]piperazin-1-yl]ethyl]-1-methyl-8-thiazol-2-yl-[1,2,4]triazolo[5,1-f]purin-2-one,
5-Amino-3-[2-[4-[3-fluoro-4-(2-methoxyethoxy)phenyl]piperazin-1-yl]ethyl]-1-methyl-8-thiazol-2-yl-[1,2,4]triazolo[5,1-f]purin-2-one,
5-Amino-3-[2-[4-(2-cyclopropylacetyl)piperazin-1-yl]ethyl]-1-methyl-8-thiazol-2-yl-[1,2,4]triazolo[5,1-f]purin-2-one,
5-Amino-3-[2-[4-(4-methoxyphenyl)piperazin-1-yl]ethyl]-1-methyl-8-thiazol-2-yl-[1,2,4]triazolo[5,1-f]purin-2-one,
5-Amino-1-methyl-3-[2-[4-(p-tolyl)piperazin-1-yl]ethyl]-8-thiazol-2-yl-[1,2,4]triazolo[5,1-f]purin-2-one,
5-Amino-1-methyl-3-[2-(3-methyl-7,8-dihydro-5H-1,6-naphthyridin-6-yl)ethyl]-8-thiazol-2-yl-[1,2,4]triazolo[5,1-f]purin-2-one,
4-[4-[2-(5-Amino-1-methyl-2-oxo-8-thiazol-2-yl-[1,2,4]triazolo[5,1-f]purin-3-yl)ethyl]piperazin-1-yl]benzonitrile,
5-Amino-1-methyl-3-[2-[4-[3-(5-methyl-1,3,4-oxadiazol-2-yl)phenyl]piperazin-1-yl]ethyl]-8-thiazol-2-yl-[1,2,4]triazolo[5,1-f]purin-2-one,
5-amino-3-[2-[4-[4-(1-hydroxy-1-methyl-ethyl)phenyl]piperazin-1-yl]ethyl]-1-methyl-8-thiazol-2-yl-[1,2,4]triazolo[5,1-f]purin-2-one,
5-Amino-3-[2-[4-[4-(2-methoxyethoxy)phenyl]piperazin-1-yl]ethyl]-1-methyl-8-(2-pyridyl)-[1,2,4]triazolo[5,1-f]purin-2-one,
5-Amino-3-[2-[4-(2,4-difluorophenyl)piperazin-1-yl]ethyl]-1-methyl-8-(2-pyridyl)-[1,2,4]triazolo[5,1-f]purin-2-one,
4-[4-[2-[5-Amino-1-methyl-2-oxo-8-(2-pyridyl)-[1,2,4]triazolo[5,1-f]purin-3-yl]ethyl]piperazin-1-yl]benzonitrile,
5-Amino-3-[2-[4-[4-(1-hydroxy-1-methyl-ethyl)phenyl]piperazin-1-yl]ethyl]-1-methyl-8-(2-pyridyl)-[1,2,4]triazolo[5,1-f]purin-2-one,
5-Amino-3-[2-[4-[4-(2-methoxyethoxy)phenyl]piperazin-1-yl]ethyl]-1-methyl-8-pyrazin-2-yl-[1,2,4]triazolo[5,1-f]purin-2-one,
5-Amino-3-[2-[4-(2,4-difluorophenyl)piperazin-1-yl]ethyl]-1-methyl-8-pyrazin-2-yl-[1,2,4]triazolo[5,1-f]purin-2-one,
5-Amino-3-[2-[4-[2-fluoro-4-(2-methoxyethoxy)phenyl]piperazin-1-yl]ethyl]-1-methyl-8-pyrazin-2-yl-[1,2,4]triazolo[5,1-f]purin-2-one,
5-Amino-8-(2-furyl)-3-[[1-(4-methoxyphenyl)pyrrolidin-3-yl]methyl]-1-methyl-[1,2,4]triazolo[5,1-f]purin-2-one,
5-Amino-8-(2-furyl)-3-[[1-[4-(2-methoxyethoxy)phenyl]pyrrolidin-3-yl]methyl]-1-methyl-[1,2,4]triazolo[5,1-f]purin-2-onehyl}-1-methyl-1,3-dihydro-[1,2,4]triazolo[5,1-i]purin-2-one,
5-amino-8-(2-furyl)-3-[2-[4-[4-(2-methoxyethoxy)phenyl]piperazin-1-yl]ethyl]-1-methyl-[1,2,4]triazolo[5,1-f]purine-2-thione,
8-(2-furyl)-3-[2-[4-[4-(2-methoxyethoxy)phenyl]piperazin-1-yl]ethyl]-1-methyl-5-(methylamino)-[1,2,4]triazolo[5,1-f]purin-2-one,
5-Amino-3-{2-[4-(4-fluoro-phenyl)-piperazin-1-yl]-ethyl}-8-isothiazol-5-yl-1-methyl-1,3-dihydro-[1,2,4]triazolo[5,1-i]purin-2-one,
5-Amino-8-isothiazol-5-yl-3-(2-{4-[4-(2-methoxy-ethoxy)-phenyl]-piperazin-1-yl}-ethyl)-1-methyl-1,3-dihydro-[1,2,4]triazolo[5,1-i]purin-2-one,
5-Amino-3-[2-[4-[2-fluoro-4-(2-methoxyethoxy)phenyl]piperazin-1-yl]ethyl]-8-isothiazol-5-yl-1-methyl-[1,2,4]triazolo[5,1-f]purin-2-one,
5-Amino-8-isoxazol-5-yl-3-[2-[4-[4-(2-methoxyethoxy)phenyl]piperazin-1-yl]ethyl]-1-methyl-[1,2,4]triazolo[5,1-f]purin-2-one,
5-Amino-3-[2-[4-[4-(2-methoxyethoxy)phenyl]piperazin-1-yl]ethyl]-1-methyl-8-oxazol-2-yl-[1,2,4]triazolo[5,1-f]purin-2-one,
5-Amino-3-{2-[4-(4-methoxy-phenyl)-piperazin-1-yl]-ethyl}-1-methyl-8-prop-1-ynyl-1,3-dihydro-[1,2,4]triazolo[5,1-i]purin-2-one,
5-Amino-3-(2-{4-[4-(2-methoxy-ethoxy)-phenyl]-piperazin-1-yl}-ethyl)-1-methyl-8-prop-1-ynyl-1,3-dihydro-[1,2,4]triazolo[5,1-i]purin-2-one,
5-Amino-3-{2-[4-(4-fluoro-benzoyl)-piperazin-1-yl]-ethyl}-8-furan-2-yl-1-methyl-1,3-dihydro-[1,2,4]triazolo[5,1-i]purin-2-one,
5-Amino-3-(2-dimethylamino-ethyl)-8-furan-2-yl-1-methyl-1,3-dihydro-[1,2,4]triazolo[5,1-i]purin-2-one,
5-Amino-8-furan-2-yl-3-[3-(4-methoxy-phenyl)-propyl]-1-methyl-1,3-dihydro-[1,2,4]triazolo[5,1-i]purin-2-one,
5-Amino-8-furan-2-yl-1-methyl-3-(2-pyrazol-1-yl-ethyl)-1,3-dihydro-[1,2,4]triazolo[5,1-i]purin-2-one,
5-Amino-8-furan-2-yl-3-(2-{4-[4-(2-methoxy-ethoxy)-phenyl]-pyrazol-1-yl}-ethyl)-1-methyl-1,3-dihydro-[1,2,4]triazolo[5,1-i]purin-2-one,
5-Amino-8-furan-2-yl-3-{2-[3-(4-methoxy-phenyl)-pyrrol-1-yl]-ethyl}-1-methyl-1,3-dihydro-[1,2,4]triazolo[5,1-i]purin-2-one,
5-Amino-8-furan-2-yl-3-{2-[4-(4-methoxy-phenyl)-imidazol-1-yl]-ethyl}-1-methyl-1,3-dihydro-[1,2,4]triazolo[5,1-i]purin-2-one,
5-Amino-8-furan-2-yl-3-{2-[4-(4-methoxy-phenyl)-[1,2,3]triazol-1-yl]-ethyl}-1-methyl-1,3-dihydro-[1,2,4]triazolo[5,1-i]purin-2-one,
5-Amino-3-[2-(1,3-dihydro-isoindol-2-yl)-ethyl]-8-furan-2-yl-1-methyl-1,3-dihydro-[1,2,4]triazolo[5,1-i]purin-2-one,
5-Amino-8-furan-2-yl-1-methyl-3-(2-piperidin-1-yl-ethyl)-1,3-dihydro-[1,2,4]triazolo[5,1-i]purin-2-one,
5-Amino-8-furan-2-yl-1-methyl-3-(2-pyrrolidin-1-yl-ethyl)-1,3-dihydro-[1,2,4]triazolo[5,1-i]purin-2-one,
5-Amino-8-furan-2-yl-1-methyl-3-[2-(3-methyl-7,8-dihydro-5H-[1,6]naphthyridin-6-yl)-ethyl]-1,3-dihydro-[1,2,4]triazolo[5,1-i]purin-2-one,
5-Amino-8-furan-2-yl-3-{2-[4-(2-methoxy-ethoxy)-phenoxy]-ethyl}-1-methyl-1,3-dihydro-[1,2,4]triazolo[5,1-i]purin-2-one,
5-Amino-8-furan-2-yl-3-{2-[4-(2-methoxy-ethoxy)-phenylamino]-ethyl}-1-methyl-1,3-dihydro-[1,2,4]triazolo[5,1-i]purin-2-one,
5-Amino-8-furan-2-yl-1-methyl-3-[2-(pyridin-2-yloxy)-ethyl]-1,3-dihydro-[1,2,4]triazolo[5,1-i]purin-2-one,
5-Amino-1-ethyl-3-{2-[4-(4-fluoro-phenyl)-piperazin-1-yl]-ethyl}-8-isothiazol-5-yl-1,3-dihydro-[1,2,4]triazolo[5,1-i]purin-2-one,
5-Amino-1-ethyl-8-isothiazol-5-yl-3-(2-{4-[4-(2-methoxy-ethoxy)-phenyl]-piperazin-1 - yl}-ethyl)-1,3-dihydro-[1,2,4]triazolo[5,1-i]purin-2-one,
5-Amino-1-ethyl-8-furan-2-yl-3-(2-piperidin-1-yl-ethyl)-1,3-dihydro-[1,2,4]triazolo[5,1-i]purin-2-one,
5-Amino-1-ethyl-8-furan-2-yl-3-[2-(3-methyl-7,8-dihydro-5H-[1,6]naphthyridin-6-yl)-ethyl]-1,3-dihydro-[1,2,4]triazolo[5,1-i]purin-2-one,
5-Amino-3-[2-(2,4-difluoro-phenoxy)-ethyl]-1-ethyl-8-furan-2-yl-1,3-dihydro-[1,2,4]triazolo[5,1-i]purin-2-one,
5-Amino-3-[2-(2,4-difluoro-phenylamino)-ethyl]-1-ethyl-8-furan-2-yl-1,3-dihydro-[1,2,4]triazolo[5,1-i]purin-2-one,
5-Amino-1-cyclopropylmethyl-3-[2-(2,4-difluoro-phenylamino)-ethyl]-8-furan-2-yl-1,3-dihydro-[1,2,4]triazolo[5,1-i]purin-2-one,
5-Amino-1-cyclopropylmethyl-3-[2-(2,4-difluoro-phenoxy)-ethyl]-8-furan-2-yl-1,3-dihydro-[1,2,4]triazolo[5,1-i]purin-2-one,
5-Amino-1-cyclopropylmethyl-3-{2-[4-(4-fluoro-phenyl)-piperidin-1-yl]-ethyl}-8-furan-2-yl-1,3-dihydro-[1,2,4]triazolo[5,1-i]purin-2-one,
5-Amino-3-{2-[4-(4-fluoro-phenyl)-piperidin-1-yl]-ethyl}-8-furan-2-yl-1-(2,2,2-trifluoroethyl)-1,3-dihydro-[1,2,4]triazolo[5,1-i]purin-2-one,
5-Amino-8-furan-2-yl-3-{2-[4-(4-methoxy-phenyl)-piperazin-1-yl]-ethyl}-1-(2,2,2-trifluoro-ethyl)-1,3-dihydro-[1,2,4]triazolo[5,1-i]purin-2-one,
5-Amino-3-[2-(4-cyclopropylmethyl-piperazin-1-yl)-ethyl]-8-isothiazol-5-yl-1-(2,2,2-trifluoro-ethyl)-1,3-dihydro-[1,2,4]triazolo[5,1-i]purin-2-one,
(5-Amino-8-isothiazol-5-yl-3-{2-[4-(4-methoxy-phenyl)-piperazin-1-yl]-ethyl}-2-oxo-2,3-dihydro-[1,2,4]triazolo[5,1-i]purin-1-yl)-acetonitrile,
[5-Amino-3-{2-[4-(2,4-difluoro-phenyl)-piperazin-l-yl]-ethyl}-8-(3-fluoro-phenyl)-2-oxo-2,3-dihydro-[1,2,4]triazolo[5,1-i]purin-1-yl]-acetonitrile,
[5-Amino-8-furan-2-yl-3-(2-{4-[4-(2-methoxy-ethoxy)-phenyl]-piperazin-1-yl}-ethyl)-2-oxo-2,3-dihydro-[1,2,4]triazolo[5,1-i]purin-1-yl]-acetonitrile,
5-Amino-3-(2-{4-[4-(2-methoxy-ethoxy)-phenyl]-piperazin-1-yl}-ethyl)-1-methyl-8-phenyl-1,3-dihydro-[1,2,4]triazolo[5,1-i]purin-2-one,
3-[5-Amino-3-(2-{4-[4-(2-methoxy-ethoxy)-phenyl]-piperazin-1-yl}-ethyl)-1-methyl-2-oxo-2,3-dihydro-1H-[1,2,4]triazolo[5,1-i]purin-8-yl]-benzonitrile,
3-[5-Amino-3-(2-{4-[4-(2-methoxy-ethoxy)-phenyl]-piperazin-1-yl}-ethyl)-1-methyl-2-oxo-2,3-dihydro-1H-[1,2,4]triazolo[5,1-i]purin-8-yl]-benzonitrile,
5-Amino-8-furan-2-yl-1-methyl-3-vinyl-1,3-dihydro-[1,2,4]triazolo[5,1-i]purin-2-one
5-Amino-3-[3-(4-fluoro-phenyl)-prop-2-ynyl]-8-furan-2-yl-1-methyl-1,3-dihydro-[1,2,4]triazolo[5,1-i]purin-2-one,
5-Amino-8-furan-2-yl-1-methyl-3-[4-(4-methyl-piperazin-1-yl)-but-2-ynyl]-1,3-dihydro-[1,2,4]triazolo[5,1-i]purin-2-one,
5-Amino-8-furan-2-yl-1-isopropyl-3-(2-{4-[4-(2-methoxy-ethoxy)-phenyl]-piperazin-1-yl}-ethyl)-1,3-dihydro-[1,2,4]triazolo[5,1-i]purin-2-one,
5-Amino-2-benzyl-7-(2-{4-[4-(2-methoxy-ethoxy)-phenyl]-piperazin-1-yl}-ethyl)-9-methyl-7,9-dihydro-2H-[1,2,4]triazolo[3,4-i]purine-3,8-dione,
5-Amino-2-benzyl-9-methyl-7-(2-morpholin-4-yl-ethyl)-7,9-dihydro-2H-[1,2,4]triazolo[3,4-i]purine-3,8-dione,
5-Amino-2-(3-chloro-benzyl)-7-[2-(4-isopropyl-piperazin-1-yl)-ethyl]-9-methyl-7,9-dihydro-2H-[1,2,4]triazolo[3,4-i]purine-3,8-dione,
5-Amino-2-cyclopropylmethyl-9-methyl-7-(2-morpholin-4-yl-ethyl)-7,9-dihydro-2H-[1,2,4]triazo lo [3,4-i]purine-3, 8-dione,
5-Amino-2-cyclopropylmethyl-7-(2,4-difluoro-benzyl)-9-methyl-7,9-dihydro-2H-[1,2,4]triazolo[3,4-i]purine-3,8-dione,
4-Amino-2-furan-2-yl-6-(2-{4-[4-(2-methoxy-ethoxy)-phenyl]-piperazin-1-yl}-ethyl)-6H-8-oxa-1,3,3a,5,6-pentaaza-as-indacen-7-one, or
4-Amino-2-furan-2-yl-6-(2-{4-[4-(2-methoxy-ethoxy)-phenyl]-piperazin-1-yl}-ethyl)-8,8-dimethyl-6,8-dihydro-1,3,3a,5,6-pentaaza-as-indacen-7-one.

7. A pharmaceutical composition comprising, as an active ingredient, at least one compound of formula (I), as claimed in any of the claims 1 to 6, or a tautomer, polymorph, stereoisomer, solvate or a pharmaceutically acceptable salt thereof, together with one or more pharmaceutically acceptable carriers or excipients.

8. A compound of formula (I) as claimed in any of the claims 1 to 6, or a tautomer, polymorph, stereoisomer, solvate or a pharmaceutically acceptable salt thereof, for use in treating a disease or disorder susceptible to improvement by antagonism of A_{2A} receptor.

9. A compound of formula (I), as claimed in any of the claims 1 to 6, or a tautomer, polymorph, stereoisomer, solvate or a pharmaceutically acceptable salt thereof, for use in treating Parkinsons disease, restless leg syndrome, Alzheimers disease, neurodegenerative disorder, inflammation, wound healing, dermal fibrosis, nocturnal myoclonus, cerebral ischaemia, myocardial ischemia, Huntington's disease, multiple system atrophy, corticobasal degeneration, Wilson's disease or other disorders of basal ganglia which results in dyskinesias, post traumatic stress disorder, hepatic cirrhosis, sepsis, spinal cord injury, retinopathy, hypertension, social memory impairment, depression, neuroprotection, narcolepsy or other sleep related disorders, attention deficit hyperactivity disorder, drug addiction, post traumatic stress disorder and vascular injury.

10. A pharmaceutical composition comprising, a compound of formula (I) as claimed in any of the claims 1 to 6, or a tautomer, polymorph, stereoisomer, solvate or a pharmaceutically acceptable salt thereof, in combination with one or more therapeutically active agents.

11. The pharmaceutical composition as claimed in claim 10 wherein, the therapeutically active agent is selected from anti-inflammatory agent, anti-diabetic agent, antihypertensive agent or anti-dyslipidemic agent.

12. The pharmaceutical composition as claimed in claim 10, wherein the therapeutically active agent is selected from anticholinergic agent, antimuscarinic agent, steroid, LTB4 (leukotriene B4) antagonist, dopamine receptor agonists, phosphodiesterase 4 inhibitor, beta-2 adrenergic receptor agonist, insulin, insulin derivatives and mimetics, insulin secretagogues, insulinotropic sulfonylurea receptor ligands, thiazolidone derivatives, glycogen synthase kinase-3 inhibitor, sodium-dependent glucose co-transporter inhibitor, glycogen phosphorylase A inhibitor, biguanide, alpha-glucosidase inhibitor, glucagon like peptide-1 (GLP-1), GLP-1 analogs and GLP-1 mimetics, modulators of peroxisome proliferator-activated receptors, dipeptidyl peptidase IV inhibitor, stearoyl-CoA desaturase-1 inhibitor, diacylglycerol acyltransferase 1 and 2 inhibitor, acetyl CoA carboxylase 2 inhibitor, and breakers of advanced glycation end products, loop diuretics, angiotensin converting enzyme inhibitor, inhibitor of the Na-K-ATPase membrane pump such as digoxin, neutralendopeptidase (NEP) inhibitor, ACE/NEP inhibitors, angiotensin II antagonists, renin inhibitors, β-adrenergic receptor blockers, inotropic agents, calcium channel blockers, aldosterone receptor antagonists, and aldosterone synthase inhibitors, 3-hydroxy-3-methyl-glutaryl coenzyme A reductase inhibitor, HDL increasing compounds such as cholesterol ester transfer protein inhibitor, squalene synthase inhibitor, farnesoid X receptor and liver X receptor ligand, cholestyramine, fibrates, nicotinic acid, or aspirin.

13. A pharmaceutical composition comprising, a compound of formula (I) as claimed in any of the claims 1 to 6, or a tautomer, polymorph, stereoisomer, solvate or a pharmaceutically acceptable salt thereof, in combination with one to three other agents useful in treating Parkinson's disease, in a pharmaceutically acceptable carrier.

14. The pharmaceutical composition as claimed in claim 13, wherein the other agent is selected from L-DOPA, dopaminergic agonists, MAO-B inhibitors, DOPA decarboxylase inhibitors, COMT inhibitors and NMDA receptor antagonists.

## Patentansprüche

1. Verbindung der Formel (I) 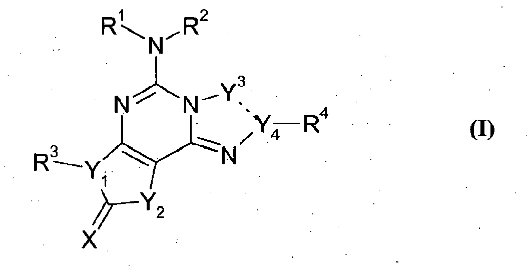 oder ein Tautomer, Polymorph, Stereoisomer, Solvat oder pharmazeutisch verträgliches Salz davon, wobei:
--- eine Einfachbindung oder eine Doppelbindung darstellt;
X ausgewählt ist aus O, S oder NR^{a};
Y₁ ausgewählt ist aus N oder CH;
Y₂ ausgewählt ist aus NR⁵, O oder CR⁵R⁶;
Y₃ ausgewählt ist aus N, CH, CH₂, C(=O) oder C(=S);
Y₄ ausgewählt ist aus N, C oder CH;
R¹ und R² unabhängig ausgewählt sind aus Wasserstoff oder Alkyl;
R³ -A-Z-B-Q ist;
wobei A fehlt oder eine Gruppe ist, die ausgewählt ist aus Alkylen, Alkenylen oder Alkinylen; wobei ein oder mehr Methylengruppen wahlweise durch Heteroatome oder Gruppen wie -O-, -S(O)ₚ-, -N(R^{a})- oder -C(O)- ersetzt sind; Alkylen, Alkenylen und Alkinylen wahlweise mit -(CR^{d}R^{e})ₙOR⁷, -(CR^{d}R^{e})ₙCOOR⁷, -(CR^{d}R^{e})ₙNR⁸R⁹, Cyan, Halogen, Haloalkyl, Perhaloalkyl, Alkoxyalkoxy, Alkyl oder Cycloalkyl substituiert sind;
Z fehlt oder ausgewählt ist aus einem Cycloalkyl oder einem Heterocyclyl;
wobei Cyclalkyl und Heterocyclyl unsubstituiert sind oder unabhängig voneinander substituiert sind mit 1, 2 oder 3 Substituenten, die unabhängig ausgewählt sind aus Alkyl, Alkenyl, Alkinyl, Acyl, -(CR^{d}R^{e})ₙOR⁷, -(CR^{d}R^{e})ₙCOOR⁷, -(CR^{d}R^{e})ₙNR⁸R⁹, Aminocarbonyl, Alkoxycarbonylamino, Halogen, Haloalkyl, Perhaloalkyl, Azido, Cyan, Keto, Thiocarbonyl, -SO₃H, Aminocarbonylamino, Nitro, -S(O)₂NR^{a}R^{a}, -NR^{b}S(O)₂R^{b} oder -S(O)ₚR^{c},
B fehlt oder eine Gruppe ist, die ausgewählt ist aus Alkylen, Alkenylen oder Alkinylen;
wobei ein oder mehr Methylengruppen wahlweise durch Heteroatome oder Gruppen wie -O-, -S(O)p-, -N(R^{a})- oder -C(O)- ersetzt sind; Alkylen, Alkenylen und Alkinylen wahlweise mit Hydroxy, Amino, Aminoalkyl, Cyan, Halogen, Haloalkyl, Perhaloalkyl, Carboxy, Carboxyalkyl, Alkoxy, Hydroxyalkyl, Alkoxyalkyl, Alkoxyalkoxy oder Alkyl substituiert sind;
Q ausgewählt ist aus Wasserstoff, Alkyl, Cycloalkyl, Cycloalkylalkyl, Aryl, Arylalkyl, Heterocyclyl, Heterocyclylalkyl, Heteroaryl oder Heteroarylalkyl,
wobei Alkyl, Cycloalkyl, Cycloalkylalkyl, Aryl, Arylalkyl, Heterocyclyl, Heterocyclylalkyl, Heteroaryl und Heteroarylalkyl unsubstituiert sind oder unabhängig voneinander substituiert sind mit 1, 2 oder 3 Substituenten, die unabhängig ausgewählt sind aus Alkyl, Alkenyl, Alkinyl, Halogen, Haloalkyl, Perhaloalkyl, Azido, Cyan, Nitro, Keto, Thiocarbonyl, Cyanoalkyl, Cyanoalkylcarbonyl, -(CR^{d}R^{e})ₙOR⁷, -(CR^{d}R^{e})ₙC(O)R⁷, -(CR^{d}R^{e})ₙSR⁷, -(CR^{d}R^{e})ₙCOOR⁷, -(CR^{d}R^{e})ₙNR⁸R⁹, -(CR^{d}R^{e})ₙC(O)NR⁸R⁹, -(CR^{d}R^{e})ₙNR⁸C(O)OR⁷, -(CR^{d}R^{e})ₙNR⁸C(O)NR⁸R⁹, -NR^{b}S(O)₂R^{b}, -S(O)ₚR^{c}, -SO₃H, -S(O)₂NR^{a}R^{a}, Cycloalkyl, Cycloalkenyl, Cycloalkylalkyl, Aryl, Arylalkyl, Heterocyclyl, Heterocyclylalkyl, Heteroaryl oder Heteroarylalkyl;
wobei jeder Substituent unsubstituiert ist oder substituiert ist mit 1, 2 oder 3 Substituenten, die unabhängig ausgewählt sind aus Alkyl, Carboxy, Carboxyalkyl, Aminocarbonyl, Hydroxy, Alkoxy, Halogen, Haloalkyl, Perhaloalkyl, Haloalkoxy, Perhaloalkoxy, Amino, substituiertem Amino, Cyan oder -S(O)ₚR^{c};
R⁴ ausgewählt ist aus der Gruppe bestehend aus Wasserstoff, Alkyl, Alkenyl, Alkinyl, Haloalkyl, Hydroxyalkyl, Carboxyalkyl, Cycloalkyl, Cyclaoalkylalkyl, Aryl, Arylalkyl, Heterocyclyl, Heterocyclylalkyl, Heteroaryl und Heteroarylalkyl;
wobei Alkyl, Alkenyl, Alkinyl, Cycloalkyl, Cycloalkylalkyl, Arylalkyl, Aryl, Heteroaryl, Heteroarylalkyl, Heterocyclyl und Heterocyclylalkyl unsubstituiert sind oder unabhängig voneinander substituiert sind mit bis zu vier Substituenten, die unabhängig ausgewählt sind aus Alkyl, Alkenyl, Alkinyl, Acyl, -(CR^{d}R^{e})ₙOR⁷, -(CR^{d}R^{e})ₙCOOR⁷, -(CR^{d}R^{e})ₙNR⁸R⁹, Aminocarbonyl, Alkoxycarbonylamino, Aminocarbonylamino, Azido, Cyan, Halogen, Haloalkyl, Perhaloalkyl, Keto, Nitro, -S(O)₂NR^{b}R^{b}, -NR^{b}S(O)₂R^{b} oder -S(O)ₚR^{c}, Thiocarbonyl, -SO₃H, Cycloalkyl, Cycloalkenyl, Aryl, Heteroaryl oder Heterocyclyl;
R⁵ und R⁶ unabhängig ausgewählt sind aus der Gruppe bestehend aus Wasserstoff, Hydroxy, -(CR^{d}R^{e})ₙOR⁷, -(CR^{d}R^{e})ₙCOOR⁷, -(CR^{d}R^{e})ₙNR⁸R⁹, Cyanoalkyl, Haloalkyl, Alkoxyalkoxyalkyl, Alkyl, Alkenyl, Alkinyl, Cycloalkyl, Cyclaoalkylalkyl, Aryl, Arylalkyl, Heterocyclyl, Heterocyclylalkyl, Heteroaryl und Heteroarylalkyl;
R⁷ ausgewählt ist aus Wasserstoff, Alkyl, Halogen, Haloalkyl, Carbonylamino, Cycloalkyl, Cyclaoalkylalkyl, Aryl, Arylalkyl, Heteroaryl, Heteroarylalkyl, Heterocyclyl oder Heterocyclylalkyl;
R⁸ und R⁹ unabhängig ausgewählt sind aus der Gruppe bestehend aus Wasserstoff, Alkyl, Haloalkyl, -(CR^{d}R^{e})ₙOR⁷, -(CR^{d}R^{e})ₙC(O)R⁷, Aryl, Arylalkyl, Heteroaryl, Heteroarylalkyl, Cycloalkyl, Cycloalkylalkyl, Heterocyclyl und Heterocyclylalkyl, oder
R⁸ und R⁹ zusammen ein monocyclisches oder ein bicyclisches Ringsystem bilden, das gesättigt oder teilweise ungesättigt ist und wahlweise weitere Heteroatome aufweist, die ausgewählt sind aus O, N oder S, wobei das Ringsystem weiterhin wahlweise mit 1 bis 4 Substituenten substituiert ist, die unabhängig ausgewählt sind aus Halo, Alkyl, Alkenyl, Alkinyl, Nitro, Cyan, -(CR^{d}R^{e})ₙOR⁷, -(CR^{d}R^{e})ₙSR⁷, -(CR^{d}R^{e})ₙNR⁸R⁹, Oxo, Alkylsulfonyl, -(CR^{d}R^{e})ₙCOOR⁷, -(CR^{d}R^{e})ₙC(O)NR⁸R⁹, Cycloalkyl, Cycloalkylalkyl, Aryl, Arylalkyl, Heterocyclyl, Heterocyclylalkyl, Heteroaryl oder Heteroarylalkyl;
R^{a} ausgewählt ist aus Wasserstoff oder Alkyl;
R^{b} bei jedem Auftreten unabhängig ausgewählt ist aus der Gruppe bestehend aus Wasserstoff, Alkyl, Acyl, Carboxyalkyl, Carbonylamino, Cycloalkyl, Cycloalkylalkyl, Aryl, Arylalkyl, Heteroaryl, Heteroarylalkyl, Heterocyclyl und Heterocyclylalkyl;
R^{c} ausgewählt ist aus Alkyl, Cycloalkyl, Aryl, Heterocyclyl oder Heteroaryl;
R^{d} und R^{e} unabhängig ausgewählt sind aus der Gruppe bestehend aus Wasserstoff, -OR⁷, Halogen, Haloalkyl, Perhaloalkyl und Alkyl;
n 0, 1, 2, 3 oder 4 ist und
p 0, 1 oder 2 ist.

2. Verbindung der Formel (I) nach Anspruch 1 oder ein Tautomer, Polymorph, Stereoisomer, Solvat oder pharmazeutisch verträgliches Salz davon, wobei
--- eine Doppelbindung darstellt;
X ausgewählt ist aus O, S oder NR^{a};
Y₁ ausgewählt ist aus N oder CH;
Y₂ ausgewählt ist aus NR⁵ oder CR⁵R⁶;
Y₃ ausgewählt ist aus N, CH oder CH₂;
Pa ausgewählt ist aus N oder C;
R¹ und R² unabhängig ausgewählt sind aus Wasserstoff oder Alkyl;
R³ -A-Z-B-Q ist;
wobei A fehlt oder Alkylen ist, wobei ein oder mehr Methylengruppen wahlweise durch Heteroatome oder Gruppen wie -O-, -S(O)p-, -N(R^{a})- oder -C(O)- ersetzt sind; Alkylen wahlweise mit -(CR^{d}R^{e})ₙOR⁷, Cyan, Halogen, Haloalkyl, Perhaloalkyl, Alkyl oder Cycloalkyl substituiert ist;
Z fehlt oder ausgewählt ist aus einem Cycloalkyl oder einem Heterocyclyl;
wobei Cyclalkyl und Heterocyclyl unsubstituiert sind oder unabhängig voneinander substituiert sind mit 1, 2 oder 3 Substituenten, die unabhängig ausgewählt sind aus Alkyl, Acyl, -(CR^{d}R^{e})ₙOR⁷, -(CR^{d}R^{e})ₙCOOR⁷, -(CR^{d}R^{e})ₙNR⁸R⁹, Aminocarbonyl, Alkoxycarbonylamino, Halogen, Haloalkyl, Perhaloalkyl, Azido, Cyan, Halogen, Keto, Thiocarbonyl, -SO₃H, Aminocarbonylamino, Nitro, -S(O)₂NR^{a}R^{a}, -NR^{b}S(O)₂R^{b} oder -S(O)ₚR^{c},
B fehlt oder Alkylen ist, wobei ein oder mehr Methylengruppen wahlweise durch Heteroatome oder Gruppen wie -O-, -S(O)p-, -N(R^{a})- oder -C(O)- ersetzt sind; Alkylen wahlweise mit Hydroxy, Amino, Aminoalkyl, Cyan, Halogen, Haloalkyl, Perhaloalkyl, Carboxy, Carboxyalkyl, Alkoxy, Hydroxyalkyl, Alkoxyalkyl, Alkoxyalkoxy oder Alkyl substituiert sind;
Q ausgewählt ist aus Wasserstoff, Alkyl, Cycloalkyl, Cycloalkylalkyl, Aryl, Arylalkyl, Heterocyclyl, Heterocyclylalkyl, Heteroaryl oder Heteroarylalkyl,
wobei Alkyl, Cycloalkyl, Cycloalkylalkyl, Aryl, Arylalkyl, Heterocyclyl, Heterocyclylalkyl, Heteroaryl und Heteroarylalkyl unsubstituiert sind oder unabhängig voneinander substituiert sind mit 1, 2 oder 3 Substituenten, die unabhängig ausgewählt sind aus Alkyl, Alkenyl, Alkinyl, Alkoxy, Alkoxyalkyl, Haloalkyl, Perhaloalkyl, Azido, Cyan, Nitro, Halogen, Keto, Thiocarbonyl, Cyanoalkyl, Cyanoalkylcarbonyl, -(CR^{d}R^{e})ₙOR⁷, -(CR^{d}R^{e})ₙC(O)R⁷, -(CR^{d}R^{e})ₙSR⁷, -(CR^{d}R^{e})ₙCOOR⁷, -(CR^{d}R^{e})ₙNR⁸R⁹, -(CR^{d}R^{e})ₙC(O)NR⁸R⁹, -(CR^{d}R^{e})ₙNR⁸C(O)OR⁷, -(CR^{d}R^{e})ₙNR⁸C(O)NR⁸R⁹, -NR^{b}S(O)₂R^{b}, -S(O)ₚR^{c}, -SO₃H, -S(O)₂NR^{a}R^{a}, Cycloalkyl, Cycloalkenyl, Cycloalkylalkyl, Aryl, Arylalkyl, Heterocyclyl, Heterocyclylalkyl, Heteroaryl oder Heteroarylalkyl;
wobei jeder Substituent unsubstituiert ist oder substituiert ist mit 1, 2 oder 3 Substituenten, die unabhängig ausgewählt sind aus Alkyl, Carboxy, Carboxyalkyl, Aminocarbonyl, Hydroxy, Alkoxy, Halogen, Haloalkyl, Perhaloalkyl, Haloalkoxy, Perhaloalkoxy, Amino, substituiertem Amino, Cyan oder -S(O)ₚR^{c},
R⁴ ausgewählt ist aus der Gruppe bestehend aus Wasserstoff, Alkyl, Haloalkyl, Hydroxyalkyl, Carboxyalkyl, Cycloalkyl, Cyclaoalkylalkyl, Aryl, Arylalkyl, Heterocyclyl, Heterocyclylalkyl, Heteroaryl und Heteroarylalkyl;
wobei Alkyl, Cycloalkyl, Cycloalkylalkyl, Arylalkyl, Aryl, Heteroaryl, Heteroarylalkyl, Heterocyclyl und Heterocyclylalkyl unsubstituiert sind oder unabhängig voneinander substituiert sind mit bis zu vier Substituenten, die unabhängig ausgewählt sind aus Alkyl, Acyl, -(CR^{d}R^{e})ₙOR⁷, -(CR^{d}R^{e})ₙCOOR⁷, -(CR^{d}R^{e})ₙNR⁸R⁹, Aminocarbonyl, Alkoxycarbonylamino, Aminocarbonylamino, Azido, Cyan, Halogen, Haloalkyl, Perhaloalkyl, Keto, Nitro, -S(O)₂NR^{b}R^{b}, -NR^{b}S(O)₂R^{b} oder -S(O)ₚR^{c}, Thiocarbonyl, -SO₃H, Cycloalkyl, Cycloalkenyl, Aryl, Heteroaryl oder Heterocyclyl;
R⁵ und R⁶ unabhängig ausgewählt sind aus der Gruppe bestehend aus Wasserstoff, Hydroxy, -(CR^{d}R^{e})ₙOR⁷, -(CR^{d}R^{e})ₙCOOR⁷, -(CR^{d}R^{e})ₙNR⁸R⁹, Cyanoalkyl, Haloalkyl, Alkoxyalkoxyalkyl, Alkyl, Alkenyl, Alkinyl, Cycloalkyl, Cyclaoalkylalkyl, Aryl, Arylalkyl, Heterocyclyl, Heterocyclylalkyl, Heteroaryl oder Heteroarylalkyl;
R⁷ ausgewählt ist aus Wasserstoff, Alkyl, Halogen, Haloalkyl, Carbonylamino, Cycloalkyl, Cyclaoalkylalkyl, Aryl, Arylalkyl, Heteroaryl, Heteroarylalkyl, Heterocyclyl oder Heterocyclylalkyl;
R⁸ und R⁹ unabhängig ausgewählt sind aus der Gruppe bestehend aus Wasserstoff, Alkyl, Haloalkyl, -(CR^{d}R^{e})ₙOR⁷, -(CR^{d}R^{e})ₙC(O)R⁷, Aryl, Arylalkyl, Heteroaryl, Heteroarylalkyl, Cycloalkyl, Cycloalkylalkyl, Heterocyclyl und Heterocyclylalkyl, oder
R⁸ und R⁹ zusammen ein monocyclisches oder ein bicyclisches Ringsystem bilden, das gesättigt oder teilweise ungesättigt ist und wahlweise weitere Heteroatome aufweist, die ausgewählt sind aus O, N oder S, wobei das Ringsystem weiterhin wahlweise mit 1 bis 4 Substituenten substituiert ist, die unabhängig ausgewählt sind aus Halo, Alkyl, Alkenyl, Alkinyl, Nitro, Cyan, -(CR^{d}R^{e})ₙOR⁷, -(CR^{d}R^{e})ₙSR⁷, -(CR^{d}R^{e})ₙNR⁸R⁹, Oxo, Alkylsulfonyl, -(CR^{d}R^{e})ₙCOOR⁷, -(CR^{d}R^{e})ₙC(O)NR⁸R⁹, Cycloalkyl, Cycloalkylalkyl, Aryl, Arylalkyl, Heterocyclyl, Heterocyclylalkyl, Heteroaryl oder Heteroarylalkyl;
R^{a} ausgewählt ist aus Wasserstoff oder Alkyl;
R^{b} bei jedem Auftreten unabhängig ausgewählt ist aus der Gruppe bestehend aus Wasserstoff, Alkyl, Acyl, Carboxyalkyl, Carbonylamino, Cycloalkyl, Cycloalkylalkyl, Aryl, Arylalkyl, Heteroaryl, Heteroarylalkyl, Heterocyclyl und Heterocyclylalkyl;
R^{c} ausgewählt ist aus Alkyl, Cycloalkyl, Aryl, Heterocyclyl oder Heteroaryl;
R^{d} und R^{e} unabhängig ausgewählt sind aus der Gruppe bestehend aus Wasserstoff, -OR⁷, Halogen, Haloalkyl, Perhaloalkyl und Alkyl;
n 0, 1, 2, 3 oder 4 ist und
p 0, 1 oder 2 ist.

3. Verbindung der Formel (I) nach Anspruch 1 oder ein Tautomer, Polymorph, Stereoisomer, Solvat oder pharmazeutisch verträgliches Salz davon, wobei
--- eine Doppelbindung darstellt;
X ausgewählt ist aus O oder S;
Y₁ N bedeutet;
Y₂ NR⁵ bedeutet;
Y₃ N bedeutet;
Y₄ C bedeutet;
R¹ und R² unabhängig ausgewählt sind aus Wasserstoff oder Alkyl;
R³ -A-Z-B-Q ist;
wobei A fehlt oder Alkylen ist, wobei ein oder mehr Methylengruppen wahlweise durch Heteroatome oder Gruppen wie -O-, -S(O)p-, -N(R^{a})- oder -C(O)- ersetzt sind;
Z fehlt oder ein Heterocyclyl ist;
wobei das Heterocyclyl unsubstituiert ist oder unabhängig substituiert ist mit 1, 2 oder 3 Substituenten, die unabhängig ausgewählt sind aus Alkyl, Acyl, -(CR^{d}R^{e})ₙOR⁷, -(CR^{d}R^{e})ₙCOOR⁷, -(CR^{d}R^{e})ₙNR⁸R⁹, Haloalkyl, Perhaloalkyl, Cyan, Halogen, Keto, Thiocarbonyl, -SO₃H, Nitro, -S(O)₂NR^{a}R^{a}, -NR^{b}S(O)₂R^{b} oder -S(O)ₚR^{c},
B fehlt oder Alkylen ist, wobei ein oder mehr Methylengruppen wahlweise durch Heteroatome oder Gruppen wie -O-, -S(O)ₚ-, -N(R^{a})- oder -C(O)- ersetzt sind;
Q ausgewählt ist aus Wasserstoff, Alkyl, Cycloalkyl, Aryl, Heterocyclyl oder Heteroaryl,
wobei Alkyl, Cycloalkyl, Aryl, Heterocyclyl und Heteroaryl unsubstituiert sind oder unabhängig voneinander substituiert sind mit 1, 2 oder 3 Substituenten, die unabhängig ausgewählt sind aus Alkyl, Alkoxy, Alkoxyalkyl, Haloalkyl, Perhaloalkyl, Azido, Cyan, Nitro, Halogen, Keto, Thiocarbonyl, Cyanoalkyl, Cyanoalkylcarbonyl, -(CR^{d}R^{e})ₙOR⁷, -(CR^{d}R^{e})ₙC(O)R⁷, -(CR^{d}R^{e})ₙSR⁷, -(CR^{d}R^{e})ₙCOOR⁷, -(CR^{d}R^{e})ₙNR⁸R⁹, -(CR^{d}R^{e})ₙC(O)NR⁸R⁹, -(CR^{d}R^{e})ₙNR⁸C(O)OR⁷, -(CR^{d}R^{e})ₙNR⁸C(O)NR⁸R⁹, -NR^{b}S(O)₂R^{b}, -S(O)ₚR^{c}, -SO₃H, -S(O)₂NR^{a}R^{a}, Cycloalkyl, Cycloalkenyl, Cycloalkylalkyl, Aryl, Arylalkyl, Heterocyclyl, Heterocyclylalkyl, Heteroaryl oder Heteroarylalkyl;
wobei jeder Substituent unsubstituiert ist oder substituiert ist mit 1, 2 oder 3 Substituenten, die unabhängig ausgewählt sind aus Alkyl, Carboxy, Carboxyalkyl, Aminocarbonyl, Hydroxy, Alkoxy, Halogen, Haloalkyl, Perhaloalkyl, Haloalkoxy, Perhaloalkoxy, Amino, substituiertem Amino, Cyan oder -S(O)ₚR^{c},
R⁴ ausgewählt ist aus der Gruppe bestehend aus Wasserstoff, Alkyl, Haloalkyl, Hydroxyalkyl, Carboxyalkyl, Cycloalkyl, Cyclaoalkylalkyl, Aryl, Arylalkyl, Heterocyclyl, Heterocyclylalkyl, Heteroaryl und Heteroarylalkyl;
wobei Alkyl, Cycloalkyl, Cycloalkylalkyl, Arylalkyl, Aryl, Heteroaryl, Heteroarylalkyl, Heterocyclyl und Heterocyclylalkyl unsubstituiert sind oder unabhängig voneinander substituiert sind mit bis zu vier Substituenten, die unabhängig ausgewählt sind aus Alkyl, Acyl, -(CR^{d}R^{e})ₙOR⁷, -(CR^{d}R^{e})ₙCOOR⁷, -(CR^{d}R^{e})ₙNR⁸R⁹, Aminocarbonyl, Alkoxycarbonylamino, Aminocarbonylamino, Azido, Cyan, Halogen, Haloalkyl, Perhaloalkyl, Keto, Nitro, -S(O)₂NR^{b}R^{b}, -NR^{b}S(O)₂R^{b} oder -S(O)ₚR^{c}, Thiocarbonyl, -SO₃H, Cycloalkyl, Cycloalkenyl, Aryl, Heteroaryl oder Heterocyclyl;
R⁵ und R⁶ unabhängig ausgewählt sind aus der Gruppe bestehend aus Wasserstoff, Hydroxy, -(CR^{d}R^{e})ₙOR⁷, -(CR^{d}R^{e})ₙCOOR⁷, -(CR^{d}R^{e})ₙNR⁸R⁹, Cyanoalkyl, Haloalkyl, Alkoxyalkoxyalkyl, Alkyl, Alkenyl, Alkinyl, Cycloalkyl, Cyclaoalkylalkyl, Aryl, Arylalkyl, Heterocyclyl, Heterocyclylalkyl, Heteroaryl und Heteroarylalkyl;
R⁷ ausgewählt ist aus Wasserstoff, Alkyl, Halogen, Haloalkyl, Carbonylamino, Cycloalkyl, Cyclaoalkylalkyl, Aryl, Arylalkyl, Heteroaryl, Heteroarylalkyl, Heterocyclyl oder Heterocyclylalkyl;
R⁸ und R⁹ unabhängig ausgewählt sind aus der Gruppe bestehend aus Wasserstoff, Alkyl, Haloalkyl, -(CR^{d}R^{e})ₙOR⁷, -(CR^{d}R^{e})ₙC(O)R⁷, Aryl, Arylalkyl, Heteroaryl, Heteroarylalkyl, Cycloalkyl, Cycloalkylalkyl, Heterocyclyl und Heterocyclylalkyl, oder
R⁸ und R⁹ zusammen ein monocyclisches oder ein bicyclisches Ringsystem bilden, das gesättigt oder teilweise ungesättigt ist und wahlweise weitere Heteroatome aufweist, die ausgewählt sind aus O, N oder S, wobei das Ringsystem weiterhin wahlweise mit 1 bis 4 Substituenten substituiert ist, die unabhängig ausgewählt sind aus Halo, Alkyl, Alkenyl, Alkinyl, Nitro, Cyan, -(CR^{d}R^{e})ₙOR⁷, -(CR^{d}R^{e})ₙSR⁷, -(CR^{d}R^{e})ₙNR⁸R⁹, Oxo, Alkylsulfonyl, -(CR^{d}R^{e})ₙCOOR⁷, -(CR^{d}R^{e})ₙC(O)NR⁸R⁹, Cycloalkyl, Cycloalkylalkyl, Aryl, Arylalkyl, Heterocyclyl, Heterocyclylalkyl, Heteroaryl oder Heteroarylalkyl;
R^{a} ausgewählt ist aus Wasserstoff oder Alkyl;
R^{b} jeweils unabhängig ausgewählt ist aus der Gruppe bestehend aus Wasserstoff, Alkyl, Acyl, Carboxyalkyl, Carbonylamino, Cycloalkyl, Cycloalkylalkyl, Aryl, Arylalkyl, Heteroaryl, Heteroarylalkyl, Heterocyclyl und Heterocyclylalkyl;
R^{c} ausgewählt ist aus Alkyl, Cycloalkyl, Aryl, Heterocyclyl oder Heteroaryl;
R^{d} und R^{e} unabhängig ausgewählt sind aus der Gruppe bestehend aus Wasserstoff, -OR⁷, Halogen, Haloalkyl, Perhaloalkyl und Alkyl;
n 0, 1, 2, 3 oder 4 ist und
p 0, 1 oder 2 ist.

4. Verbindung der Formel (I) nach Anspruch 1 oder ein Tautomer, Polymorph, Stereoisomer, Solvat oder pharmazeutisch verträgliches Salz davon, wobei
--- eine Doppelbindung darstellt;
X ausgewählt ist aus O oder S;
Y₁ N bedeutet;
Y₂ NR⁵ bedeutet;
Y₃ N bedeutet;
Y₄ C bedeutet;
R¹ und R² unabhängig ausgewählt sind aus Wasserstoff oder Alkyl;
R³ -A-Z-B-Q ist;
wobei A fehlt oder Alkylen ist, wobei ein oder mehr Methylengruppen wahlweise durch Heteroatome oder Gruppen wie -O-, oder -N(R^{a})- ersetzt sind;
Z fehlt oder ein Heterocyclyl ist;
wobei das Heterocyclyl unsubstituiert ist oder unabhängig substituiert ist mit 1, 2 oder 3 Substituenten, die unabhängig ausgewählt sind aus Alkyl, -(CR^{d}R^{e})ₙOR⁷, -(CR^{d}R^{e})ₙCOOR⁷, Haloalkyl, Perhaloalkyl, Cyan, Halogen, Keto oder Thiocarbonyl:
B fehlt oder Alkylen ist, wobei ein oder mehr Methylengruppen wahlweise durch Heteroatome oder Gruppen wie -O-, -N(R^{a})- oder -C(O)- ersetzt sind;
Q ausgewählt ist aus Wasserstoff, Alkyl, Cycloalkyl, Aryl, Heterocyclyl oder Heteroaryl,
wobei Alkyl, Cycloalkyl, Aryl, Heterocyclyl und Heteroaryl unsubstituiert sind oder unabhängig voneinander substituiert sind mit 1, 2 oder 3 Substituenten, die unabhängig ausgewählt sind aus Alkyl, Alkoxy, Alkoxyalkyl, Haloalkyl, Perhaloalkyl, Cyan, Halogen, Keto, Thiocarbonyl, Cyanoalkyl, -(CR^{d}R^{e})ₙOR⁷, -(CR^{d}R^{e})ₙC(O)R⁷, -(CR^{d}R^{e})ₙCOOR⁷, -(CR^{d}R^{e})ₙNR⁸R⁹, -(CR^{d}R^{e})ₙC(O)NR⁸R⁹, -(CR^{d}R^{e})ₙNR⁸C(O)OR⁷, -S(O)ₚR^{c}, -SO₃H, -S(O)₂NR^{a}R^{a}, Cycloalkyl, Cycloalkenyl, Aryl, Heterocyclyl oder Heteroaryl;
wobei jeder Substituent unsubstituiert ist oder substituiert ist mit 1, 2 oder 3 Substituenten, die unabhängig ausgewählt sind aus Alkyl, Carboxy, Carboxyalkyl, Aminocarbonyl, Hydroxy, Alkoxy, Halogen, Haloalkyl, Perhaloalkyl, Haloalkoxy, Perhaloalkoxy, Amino, substituiertem Amino, Cyan oder -S(O)ₚR^{c},
R⁴ ausgewählt ist aus der Gruppe bestehend aus Wasserstoff, Alkyl, Haloalkyl, Hydroxyalkyl, Cycloalkyl, Aryl, Heterocyclyl und Heteroaryl;
wobei Alkyl, Cycloalkyl, Aryl, Heteroaryl und Heterocyclyl unsubstituiert sind oder unabhängig voneinander substituiert sind mit bis zu vier Substituenten, die unabhängig ausgewählt sind aus Alkyl, -(CR^{d}R^{e})ₙOR⁷, -(CR^{d}R^{e})ₙCOOR⁷, -(CR^{d}R^{e})ₙNR⁸R⁹, Cyan, Halogen, Haloalkyl, Perhaloalkyl, Nitro, -S(O)₂NR^{b}R^{b}, -NR^{b}S(O)₂R^{b}, -S(O)ₚR^{c}, Thiocarbonyl, -SO₃H, Cycloalkyl, Aryl, Heteroaryl oder Heterocyclyl;
R⁵ ausgewählt ist aus der Gruppe bestehend aus Wasserstoff, Hydroxy, Haloalkyl, -(CR^{d}R^{e})ₙOR⁷, -(CR^{d}R^{e})ₙCOOR⁷, Alkoxyalkoxyalkyl, Alkyl, Alkenyl, Alkinyl, Cycloalkyl, Cyclaoalkylalkyl, Aryl, Arylalkyl, Heterocyclyl, Heterocyclylalkyl, Heteroaryl oder Heteroarylalkyl;
R⁷ ausgewählt ist aus Wasserstoff, Alkyl, Halogen, Haloalkyl, Cycloalkyl, Aryl, Heteroaryl oder Heterocyclyl;
R⁸ und R⁹ unabhängig ausgewählt sind aus der Gruppe bestehend aus Wasserstoff, Alkyl, Haloalkyl, -(CR^{d}R^{e})ₙOR⁷, -(CR^{d}R^{e})ₙC(O)R⁷, Aryl, Arylalkyl, Heteroaryl, Heteroarylalkyl, Cycloalkyl, Cycloalkylalkyl, Heterocyclyl und Heterocyclylalkyl, oder
R⁸ und R⁹ zusammen ein monocyclisches oder ein bicyclisches Ringsystem bilden, das gesättigt oder teilweise ungesättigt ist und wahlweise weitere Heteroatome aufweist, die ausgewählt sind aus O, N oder S, wobei das Ringsystem weiterhin wahlweise mit 1 bis 4 Substituenten substituiert ist, die unabhängig ausgewählt sind aus Halo, Alkyl, Nitro, Cyan, -(CR^{d}R^{e})ₙOR⁷, -(CR^{d}R^{e})ₙNR⁸R⁹, Oxo, Alkylsulfonyl, -(CR^{d}R^{e})ₙCOOR⁷ oder -(CR^{d}R^{e})ₙC(O)NR⁸R⁹;
R^{a} ausgewählt ist aus Wasserstoff oder Alkyl;
R^{b} jeweils unabhängig ausgewählt ist aus der Gruppe bestehend aus Wasserstoff, Alkyl, Acyl, Carboxyalkyl, Carbonylamino, Cycloalkyl, Cycloalkylalkyl, Aryl, Arylalkyl, Heteroaryl, Heteroarylalkyl, Heterocyclyl oder Heterocyclylalkyl;
R^{c} ausgewählt ist aus Alkyl, Cycloalkyl, Aryl, Heterocyclyl oder Heteroaryl;
R^{d} und R^{e} unabhängig ausgewählt sind aus der Gruppe bestehend aus Wasserstoff, -OR⁷, Halogen, Haloalkyl, Perhaloalkyl und Alkyl;
n 0, 1, 2, 3 oder 4 ist und
p 0, 1 oder 2 ist.

5. Verbindung der Formel (I) nach Anspruch 1 oder ein Tautomer, Polymorph, Stereoisomer, Solvat oder pharmazeutisch verträgliches Salz davon, wobei
--- eine Doppelbindung darstellt;
X ausgewählt ist aus O oder S;
Y₁ N bedeutet;
Y₂ NR⁵ bedeutet;
Y₃ N bedeutet;
Y₄ C bedeutet;
R¹ und R² unabhängig ausgewählt sind aus Wasserstoff oder Alkyl;
R³ -A-Z-B-Q ist;
wobei A fehlt oder Alkylen ist, wobei ein oder mehr Methylengruppen wahlweise durch Heteroatome oder Gruppen wie -O-, oder -N(R^{a})- ersetzt sind;
Z fehlt oder ein Heterocyclyl ist, das ausgewählt ist aus Dihydrofuranyl, Tetrahydrofuranyl, Morpholinyl, Pyrrolidinyl, Dihydropyrrol, Dihydropyranyl, Tetrahydropyranyl, Pyrazolidinyl, Imidazolidinyl, Dihydropyridinyl, Tetrahydropyridinyl, Piperidinyl, Dihydropyrazinyl, Tetrahydropyrazinyl, Piperazinyl oder Dihydropyridinyl;
wobei das Heterocyclyl unsubstituiert ist oder unabhängig substituiert ist mit 1, 2 oder 3 Substituenten, die unabhängig ausgewählt sind aus Alkyl, -(CR^{d}R^{e})ₙOR⁷, -(CR^{d}R^{e})ₙCOOR⁷, Haloalkyl, Perhaloalkyl, Cyan oder Halogen:
B fehlt oder Alkylen ist, wobei ein oder mehr Methylengruppen wahlweise durch Heteroatome oder Gruppen wie -O-, -N(R^{a})- oder -C(O)- ersetzt sind;
Q ausgewählt ist aus Wasserstoff, Alkyl, Cyclopropyl, Cyclopentyl, Cyclohexyl, Phenyl, Tetrahydrofuranyl, Pyrrolidinyl, Tetrahydropyridinyl, Tetrahydropyranyl, Piperazinyl, Benzodiaxolyl, Tetrahydrochinolinyl, Morpholinyl, Tetrahydronaphthyridinyl, Tetrahydrothienopyridinyl, Furanyl, Pyridinyl, Pyrimidinyl, Oxazolyl, Thiazolyl, Oxadiazolyl, Thiadiazolyl, Indolyl, Chinolinyl, Isochinolinyl oder Benzooxazolyl;
wobei Q unsubstituiert ist oder substituiert ist mit 1, 2 oder 3 Substituenten, die unabhängig ausgewählt sind aus Alkyl, Alkoxy, Alkoxyalkyl, Haloalkyl, Perhaloalkyl, Cyan, Halogen, Keto, Thiocarbonyl, Cyanoalkyl, -(CR^{d}R^{e})ₙOR⁷, -(CR^{d}R^{e})ₙC(O)R⁷, -(CR^{d}R^{e})ₙCOOR⁷, -(CR^{d}R^{e})ₙNR⁸R⁹, -(CR^{d}R^{e})ₙC(O)NR⁸R⁹, -(CR^{d}R^{e})ₙNR⁸C(O)OR⁷, -S(O)ₚR^{c}, -SO₃H, -S(O)₂NR^{a}R^{a}, Cycloalkyl, Cycloalkenyl, Aryl, Heterocyclyl oder Heteroaryl;
wobei jeder Substituent unsubstituiert ist oder substituiert ist mit 1, 2 oder 3 Substituenten, die unabhängig ausgewählt sind aus Alkyl, Carboxy, Carboxyalkyl, Aminocarbonyl, Hydroxy, Alkoxy, Halogen, Haloalkyl, Perhaloalkyl, Haloalkoxy, Perhaloalkoxy, Amino, substituiertem Amino, Cyan oder -S(O)ₚR^{c},
R⁴ ausgewählt ist aus der Gruppe bestehend aus Wasserstoff, Alkyl, Phenyl, Naphthyl, Furanyl, Thiazolyl, Oxazolyl, Thiadiazolyl, Oxadiazolyl, Pyrazinyl, Pyridinyl, und Pyrimidinyl;
wobei R⁴ unsubstituiert ist oder substituiert ist mit bis zu vier Substituenten, die unabhängig ausgewählt sind aus Alkyl, -(CR^{d}R^{e})ₙOR⁷, -(CR^{d}R^{e})ₙCOOR⁷, -(CR^{d}R^{e})ₙNR⁸R⁹, Cyan, Halogen, Haloalkyl, Perhaloalkyl, Nitro oder Cycloalkyl;
R⁵ ausgewählt ist aus der Gruppe bestehend aus Wasserstoff, Hydroxy, Haloalkyl, -(CR^{d}R^{e})ₙOR⁷, -(CR^{d}R^{e})ₙCOOR⁷, Alkoxyalkoxyalkyl, Alkyl, Alkenyl, Alkinyl, Cycloalkyl, Cyclaoalkylalkyl, Aryl, Arylalkyl, Heterocyclyl, Heterocyclylalkyl, Heteroaryl und Heteroarylalkyl;
R⁷ ausgewählt ist aus Wasserstoff, Alkyl, Halogen, Haloalkyl, Cycloalkyl, Aryl, Heteroaryl oder Heterocyclyl;
R⁸ und R⁹ unabhängig ausgewählt sind aus der Gruppe bestehend aus Wasserstoff, Alkyl, Haloalkyl, -(CR^{d}R^{e})ₙOR⁷, -(CR^{d}R^{e})ₙC(O)R⁷, Aryl, Arylalkyl, Heteroaryl, Heteroarylalkyl, Cycloalkyl, Cycloalkylalkyl, Heterocyclyl und Heterocyclylalkyl, oder
R⁸ und R⁹ zusammen ein monocyclisches oder ein bicyclisches Ringsystem bilden, das gesättigt oder teilweise ungesättigt ist und wahlweise weitere Heteroatome aufweist, die ausgewählt sind aus O, N oder S, wobei das Ringsystem weiterhin wahlweise mit 1 bis 4 Substituenten substituiert ist, die unabhängig ausgewählt sind aus Halo, Alkyl, Nitro, Cyan, -(CR^{d}R^{e})nOR⁷, -(CR^{d}R^{e})ₙNR⁸R⁹, Oxo, Alkylsulfonyl, -(CR^{d}R^{e})ₙCOOR⁷ oder -(CR^{d}R^{e})ₙC(O)NR⁸R⁹;
R^{a} ausgewählt ist aus Wasserstoff oder Alkyl;
R^{b} bei jedem Auftreten unabhängig ausgewählt ist aus der Gruppe bestehend aus Wasserstoff, Alkyl, Acyl, Carboxyalkyl, Carbonylamino, Cycloalkyl, Cycloalkylalkyl, Aryl, Arylalkyl, Heteroaryl, Heteroarylalkyl, Heterocyclyl oder Heterocyclylalkyl;
R^{c} ausgewählt ist aus Alkyl, Cycloalkyl, Aryl, Heterocyclyl oder Heteroaryl;
R^{d} und R^{e} unabhängig ausgewählt sind aus der Gruppe bestehend aus Wasserstoff, -OR⁷, Halogen, Haloalkyl, Perhaloalkyl und Alkyl;
n 0, 1, 2, 3 oder 4 ist und
p 0, 1 oder 2 ist.

6. Verbindung der Formel (I) nach Anspruch 1 oder ein Tautomer, Polymorph, Stereoisomer, Solvat oder pharmazeutisch verträgliches Salz davon, nämlich:
5-Amino-8-(2-furyl)-3-[2-[4-[4-(2-methoxyethoxy)phenyl]piperazin-1-yl]ethyl]-1-methyl-[1,2,4]triazolo[5,1-f]purin-2-on,
5-Amino-8-(2-furyl)-3-(2-hydroxyethyl)-1-methyl-[1,2,4]triazolo[5,1-f]purin-2-on,
5-Amino-3-[2-[4-(2,4-difluorphenyl)piperazin-1-yl]ethyl]-8-(2-furyl)-1-methyl-[1,2,4]triazolo[5,1-f]purin-2-on,
5-Amino-8-(2-furyl)-3-[2-[4-(4-methoxyphenyl)piperazin-1-yl]ethyl]-1-methyl-[1,2,4]triazolo[5,1-f]purin-2-on,
5-Amino-8-(2-furyl)-1-methyl-3-(2-morpholinoethyl)-[1,2,4]triazolo[5,1-f]purin-2-on,
5-Amino-3-[2-[4-(2,4-difluorphenyl)-1-piperidyl]ethyl]-8-(2-furyl)-1-methyl-[1,2,4]triazolo[5,1-f]purin-2-on,
5-Amino-8-(2-furyl)-1-methyl-3-[2-[4-(5-methyl-2-pyridyl)piperazin-1-yl]ethyl]-[1,2,4]triazolo[5,1-f]purin-2-on,
5-Amino-8-(2-furyl)-1-methyl-3-[2-[4-(p-tolyl)piperazin-1-yl]ethyl]-[1,2,4]triazolo-[5,1-f]purin-2-on,
5-Amino-8-(2-furyl)-1-methyl-3-[2-[4-(3-methyl-2-oxo-butyl)piperazin-1-yl]ethyl]-[1,2,4]triazolo[5,1-f]purin-2-on,
5-Amino-3-[2-[4-(2-fluor-4-methoxyphenyl)piperazin-1-yl]ethyl]-8-(2-furyl)-1-methyl-[1,2,4]triazolo[5,1-f]purin-2-on,
5-Amino-8-(2-furyl)-3-[2-[4-[4-(2-methoxy-1,1-dimethylethoxy)phenyl]piperazin-1-yl]ethyl]-1-methyl-[1,2,4]triazolo[5,1-f]purin-2-on,
5-Amino-8-(2-furyl)-3-[2-[4-(6-methoxy-3-pyridyl)piperazin-1-yl]ethyl]-1-methyl-[1,2,4]triazolo[5,1-f]purin-2-on,
5-Amino-3-[2-[4-[3-fluor-4-(2-methoxyethoxy)phenyl]piperazin-1-yl]ethyl]-8-(2-furyl)-1-methyl-[1,2,4]triazolo[5,1-f]purin-2-on,
5-Amino-8-(2-furyl)-3-[2-[4-[4-(1-hydroxy-1-methylethyl)phenyl]piperazin-1-yl]ethyl]-1-methyl-[1,2,4]triazolo[5,1-f]purin-2-on,
5-Amino-3-[2-[4-(4-fluorphenyl)-4-hydroxy-1-piperidyl]ethyl]-8-(2-furyl)-1-methyl-[1,2,4]triazolo[5,1-f]purin-2-on,
5-Amino-8-(2-furyl)-3-[2-[4-[4-(2-methoxy-2-methylpropoxy)phenyl]piperazin-1-yl]ethyl]-1-methyl-[1,2,4]triazolo[5,1-f]purin-2-on,
5-Amino-3-[2-[4-[4-(cyclopropoxy)phenyl]piperazin-1-yl]ethyl]-8-(2-furyl)-1-methyl-[1,2,4]triazolo[5,1-f]purin-2-on,
5-Amino-3-[2-[4-(4-fluorphenyl)-3,6-dihydro-2H-pyridin-1-yl]ethyl]-8-(2-furyl)-1-methyl-[1,2,4]triazolo[5,1-f]purin-2-on,
5-Amino-8-(2-furyl)-3-[2-[4-hydroxy-4-(4-methoxyphenyl)-1-piperidyl]ethyl]-1-methyl-[1,2,4]triazolo[5,1-f]purin-2-on,
5-Amino-3-[2-[4-[3,5-difluor-4-(2-methoxyethoxy)phenyl]piperazin-1-yl]ethyl]-8-(2-furyl)-1-methyl-[1,2,4]triazolo[5,1-f]purin-2-on,
5-Amino-3-[2-[4-[2,5-difluor-4-(2-methoxyethoxy)phenyl]piperazin-1-yl]ethyl]-8-(2-furyl)-1-methyl-[1,2,4]triazolo[5,1-f]purin-2-on,
5-Amino-3-[2-[4-(2,2-difluor-1,3-benzodioxol-5-yl)piperazin-1-yl]ethyl]-8-(2-furyl)-1-methyl-[1,2,4]triazolo[5,1-f]purin-2-on,
5-Amino-8-(2-furyl)-3-[2-[4-[4-(2-methoxyethoxy)phenyl]-3,3-dimethylpiperazin-1-yl]ethyl]-1-methyl-[1,2,4]triazolo[5,1-f]purin-2-on,
5-Amino-3-[2-(4-butylpiperazin-1-yl)ethyl]-8-(2-furyl)-1-methyl-[1,2,4]triazolo-[5,1-f]purin-2-on,
5-Amino-8-(2-furyl)-3-[2-(4-hydroxy-4-methyl-1-piperidyl)ethyl]-1-methyl-[1,2,4]triazolo[5,1-f]purin-2-on,
5-Amino-3-[2-[4-[4-[2-(cyclopropoxy)ethoxy]phenyl]piperazin-1-yl]ethyl]-8-(2-furyl)-1-methyl-[1,2,4]triazolo[5,1-f]purin-2-on,
5-Amino-8-(2-furyl)-3-[2-[4-[(4-methoxyphenyl)methyl]piperazin-1-yl]ethyl]-1-methyl-[1,2,4]triazolo[5,1-f]purin-2-on,
5-Amino-8-(2-furyl)-3-[2-[4-[[4-(2-methoxyethoxy)phenyl]methyl]piperazin-1-yl]ethyl]-1-methyl-[1,2,4]triazolo[5,1-f]purin-2-on,
5-Amino-8-(2-furyl)-3-[(4-methoxyphenyl)methyl]-1-methyl-[1,2,4]triazol-[5,1-f]purin-2-on,
5-Amino-8-(2-furyl)-3-[2-[4-(4-methoxyphenyl)piperazin-1-yl]ethyl]-1-methyl-[1,2,4]triazolo[5,1-f]purin-2-on,
5-Amino-8-(2-furyl)-3-[2-[4-[3-(2-methoxyethoxy)phenyl]piperazin-1-yl]ethyl]-1-methyl-[1,2,4]triazolo[5,1-f]purin-2-on,
5-Amino-3-[2-[4-[2-fluor-4-(2-methoxyethoxy)phenyl]piperazin-1-yl]ethyl]-8-(2-furyl)-1-methyl-[1,2,4]triazolo[5,1-f]purin-2-on,
4-[4-[2-[5-Amino-8-(2-furyl)-1-methyl-2-oxo-[1,2,4]triazolo[5,1-f]purin-3yl]ethyl]piperazin-1-yl]benzonitril,
4-[4-[2-[5-Amino-8-(2-furyl)-1-methyl-2-oxo-[1,2,4]triazolo[5,1-f]purin-3-yl]ethyl]piperazin-1-yl]-2-fluorbenzonitril,
5-Amino-8-(2-furyl)-1-methyl-3-[2-[4-[4-(trifluormethyl)phenyl]piperazin-1-yl]ethyl]-[1,2,4]triazolo[5,1-f]purin-2-on,
5-Amino-8-(2-furyl)-1-methyl-3-[2-[4-[4-(trifluormethyl)thiazol-2-yl]piperazin-1-yl]ethyl]-[1,2,4]triazolo[5,1-f]purin-2-on,
5-Amino-3-[2-[4-(cyclopropylmethyl)piperazin-1 -yl]ethyl]-8-(2-furyl)-1 -methyl-[1,2,4]triazolo[5,1-f]purin-2-on,
5-Amino-3-[2-(4-ethylpiperazin-1-yl)ethyl]-8-(2-furyl)-1-methyl-[1,2,4]triazol-[5,1-f]purin-2-on,
4-[2-[5-Amino-8-(2-furyl)-1-methyl-2-oxo-[1,2,4]triazolo[5,1-f]purin-3-yl]ethyl]-N,N-dimethylpiperazin-1-sulfonamid,
5-Amino-8-(2-furyl)-1-methyl-3-[2-[4-(4-tetrahydrofuran-3-yloxyphenyl)piperazin-1-yl]ethyl]-[1,2,4]triazolo[5,1-f]purin-2-on,
5-Amino-8-(2-furyl)-1-methyl-3-[2-[4-(4-tetrahydropyran-4-yloxyphenyl)piperazin-1-yl]ethyl]-[1,2,4]triazolo[5,1-f]purin-2-on,
5-Amino-8-(2-furyl)-1-methyl-3-[2-[4-[4-(tetrahydrofuran-2-ylmethoxy)phenyl]piperazin-1-yl]ethyl]-[1,2,4]triazolo[5,1-f]purin-2-on,
5-Amino-8-(2-furyl)-1-methyl-3-[2-(3-methyl-7,8-dihydro-5H-1,6-naphthyridin-6-yl)ethyl]-[1,2,4]triazolo[5,1-f]purin-2-on,
5-Amino-3-[2-(6,7-dihydro-4H-thieno[3,2-c]pyridin-5-yl)ethyl]-8-(2-furyl)-1-methyl-[1,2,4]triazolo[5,1-f]purin-2-on,
5-Amino-8-(2-furyl)-3-[2-[4-[4-(2-methoxyethoxy)phenyl]piperazin-1-yl]propyl]-1-methyl-[1,2,4]triazolo[5,1-f]purin-2-on,
5-Amino-3-[2-[3-(4-fluorphenyl)-2,5-dihydropyrrol-1-yl]ethyl]-8-(2-furyl)-1-methyl-[1,2,4]triazolo[5,1-f]purin-2-on,
5-Amino-3-[2-[4-[4-(2-methoxyethoxy)phenyl]piperazin-1-yl]ethyl]-1-methyl-8-(5-methyl-2-furyl)-[1,2,4]triazolo[5,1-f]purin-2-on,
5-Amino-8-(5-cyclopropyl-2-furyl)-3-[2-[4-[4-(2-methoxyethoxy)phenyl]piperazin-1-yl]ethyl]-1-methyl-[1,2,4]triazolo[5,1-f]purin-2-on,
5-Amino-3-[2-(2,4-difluoranilino)ethyl]-8-(2-furyl)-1-methyl-[1,2,4]triazolo-[5,1-f]purin-2-on,
5-Amino-3-[3-[4-(4-fluorphenyl)piperazin-1 -yl]propyl]-8-(2-furyl)-1 -methyl-[1,2,4]triazolo[5,1-f]purin-2-on,
5-Amino-8-(2-furyl)-3-[3-[4-[4-(2-methoxyethoxy)phenyl]piperazin-1-yl]propyl]-1-methyl-[1,2,4]triazolo[5,1-f]purin-2-on,
5-Amino-8-(2-furyl)-3-[2-[4-(4-methoxyphenyl)-3,6-dihydro-2H-pyridin-1-yl]ethyl]-1-methyl-[1,2,4]triazolo[5,1-f]purin-2-on,
5-Amino-8-(2-furyl)-3-[2-[4-[4-(2-methoxy-1,1-dimethylethyl)phenyl]piperazin-1-yl]ethyl]-1-methyl-[1,2,4]triazolo[5,1-f]purin-2-on,
5-Amino-8-(2-furyl)-1-methyl-3-(2-piperazin-1-ylethyl)-[1,2,4]triazolo[5,1-f]purin-2-on,
5-Amino-8-(2-furyl)-3-[2-[4-(1H-indol-2-carbonyl)piperazin-1-yl]ethyl]-1-methyl-[1,2,4]triazolo[5,1-f]purin-2-on,
5-Amino-8-(2-furyl)-3-[2-(4-isopropoxyphenyl)ethyl]-1-methyl-[1,2,4]triazolo-[5,1-f]purin-2-on,
5-Amino-8-(2-furyl)-1-methyl-3-[2-[4-[(2S)-pyrrolidin-2-carbonyl]piperazin-1-yl]ethyl]-[1,2,4]triazolo[5,1-f]purin-2-on,
5-Amino-8-(2-furyl)-3-[2-(4-methoxyphenyl)ethyl]-1-methyl-[1,2,4]triazolo-[5,1-f]purin-2-on,
5-Amino-3-[2-[4-[4-(difluormethoxy)phenyl]piperazin-1-yl]ethyl]-8-(2-furyl)-1-methyl-[1,2,4]triazolo[5,1-f]purin-2-on,
5-Amino-8-(2-furyl)-1-methyl-3-[2-[4-[3-(5-methyl-1,3,4-oxadiazol-2-yl)phenyl]piperazin-1-yl]ethyl]-[1,2,4]triazolo[5,1-f]purin-2-on,
5-Amino-3-[2-[4-[2-fluor-4-(5-methyl-1,2,4-oxadiazol-3-yl)phenyl]piperazin-1-yl]ethyl]-8-(2-furyl)-1-methyl-1,2,4]triazolo[5,1-f]purin-2-on,
5-Amino-3-[2-[4-(6-fluor-2-methyl-1,3-benzoxazol-5-yl)piperazin-1-yl]ethyl]-8-(2-furyl)-1-methyl-[1,2,4]triazolo[5,1-f]purin-2-on,
5-Amino-3-[2-[4-(cyclopropancarbonyl)piperazin-1-yl]ethyl]-8-(2-furyl)-1-methyl-[1,2,4]triazolo[5,1-f]purin-2-on,
5-Amino-3-[2-[4-(2-cyclopropylacetyl)piperazin-1-yl]ethyl]-8-(2-furyl)-1-methyl-[1,2,4]triazolo[5,1-f]purin-2-on,
5-Amino-8-(2-furyl)-3-[2-[4-[4-(2-hydroxyethoxy)phenyl]piperazin-1-yl]ethyl]-1-methyl-[1,2,4]triazolo[5,1-f]purin-2-on,
5-Amino-8-(2-furyl)-3-[2-[4-(4-hydroxyphenyl)piperazin-1-yl]ethyl]-1-methyl-[1,2,4]triazolo[5,1-f]purin-2-on,
5-Amino-1-(cyclopropylmethyl)-3-[2-[4-(4-ethoxyphenyl)piperazin-1-yl]ethyl]-8-(2-furyl)-[1,2,4]triazolo[5,1-f]purin-2-on,
5-Amino-1-(cyclopropylmethyl)-3-[2-[4-(4-fluorphenyl)piperazin-1-yl]ethyl]-8-(2-furyl)-[1,2,4]triazolo[5,1-f]purin-2-on,
5-Amino-1-(cyclopropylmethyl)-3-[2-[4-(2,4-difluorphenyl)piperazin-1-yl]ethyl]-8-(2-furyl)-[1,2,4]triazolo[5,1-f]purin-2-on,
5-Amino-1-(cyclopropylmethyl)-8-(2-furyl)-3-[2-[4-[4-(2-methoxyethoxy)phenyl]-piperazin-1-yl]ethyl]-[1,2,4]triazolo[5,1-f]purin-2-on,
5-Amino-1-(cyclopropylmethyl)-3-[2-(4-fluorphenoxy)ethyl]-8-(2-furyl)-[1,2,4]triazolo[5,1-f]purin-2-on,
5-Amino-8-(2-furyl)-1-methyl-3-[2-[2-oxo-5-(trifluormethyl)-1-pyridyl]ethyl]-[1,2,4]triazolo[5,1-f]purin-2-on,
5-Amino-3-[2-[4-(2,4-difluorphenyl)pyrazol-1-yl]ethyl]-1-ethyl-8-(2-furyl)-[1,2,4]triazolo[5,1-f]purin-2-on,
1-[2-[5-Amino-1-(cyclopropylmethyl)-8-(2-furyl)-2-oxo-[1,2,4]triazolo[5,1-f]purin-3-yl]ethyl]pyrazol-4-carbonsäure,
1-[2-[ 5-Amino-8-(2-furyl)-1-methyl-2-oxo-[1,2,4]triazolo[5,1-f]purin-3-yl]ethyl]pyrazol-4-carbonsäure,
1-[2-[5-Amino-1-(cyclopropylmethyl)-8-(2-furyl)-2-oxo-[1,2,4]triazolo[5,1-f]purin-3-yl]ethyl]-N-cyclopropyl-pyrazol-4-carboxamid,
1-[2-[5-Amino-1-(cyclopropylmethyl)-8-(2-furyl)-2-oxo-[1,2,4]triazolo[5,1-f]purin-3-yl]ethyl]-N,N-diethylpyrazol-4-carboxamid,
1-[2-[5-Amino-1-(cyclopropylmethyl)-8-(2-furyl)-2-oxo-[1,2,4]triazolo[5,1-f]purin-3-yl]ethyl]-N-cyclopropyl-5-methylpyrazol-3-carboxamid,
2-[2-[5-Amino-1-(cyclopropylmethyl)-8-(2-furyl)-2-oxo-[1,2,4]triazolo[5,1-f]purin-3-yl]ethyl]-N-cyclopropyl-5-methylpyrazol-3-carboxamid,
1-[2-[5-Amino-8-(2-furyl)-1-methyl-2-oxo-[1,2,4]triazolo[5,1-f]purin-3-yl]ethyl]-N-methylpyrazol-3-carboxamid,
1-[2-[5-Amino-8-(2-furyl)-1-methyl-2-oxo-[1,2,4]triazolo[5,1-f]purin-3-yl]ethyl]-N,N-diethylpyrazol-4-carboxamid,
1-[2-[5-Amino-8-(2-furyl)-1-methyl-2-oxo-[1,2,4]triazolo[5,1-f]purin-3-yl]ethyl]pyrazol-4-carboxamid,
5-Amino-8-(2-furyl)-3-[2-[4-[(3R)-3-hydroxypyrrolidin-1-carbonyl]pyrazol-1-yl]ethyl]-1-methyl-[1,2,4]triazolo[5,1-f]purin-2-on,
1-[2-[5-Amino-8-(2-furyl)-1-methyl-2-oxo-[1,2,4]triazolo[5,1-f]purin-3-yl]ethyl]-N-methylpyrazol-4-carboxamid,
1-[2-[5-Amino-8-(2-furyl)-1-methyl-2-oxo-[1,2,4]triazolo[5,1-f]purin-3-yl]ethyl]-N-cyclopropylpyrazol-3-carboxamid,
1-[2-[5-Amino-8-(2-furyl)-1-methyl-2-oxo-[1,2,4]triazolo[5,1-f]purin-3-yl]ethyl]-N-cyclopropylpyrazol-4-carboxamid,
5-Amino-8-(2-furyl)-3-[2-[4-(3-hydroxyazetidin-1-carbonyl)pyrazol-1-yl]ethyl]-1-methyl-[1,2,4]triazolo[5,1-f]purin-2-on,
5-Amino-1-ethyl-8-(2-furyl)-3-[2-[4-[4-(2-methoxyethoxy)phenyl]piperazin-1-yl]ethyl]-[1,2,4]triazolo[5,1-f]purin-2-on,
5-Amino-3-[2-[4-(2,4-difluorphenyl)piperazin-1-yl]ethyl]-1-ethyl-8-(2-furyl)-[1,2,4]triazolo[5,1-f]purin-2-on,
5-Amino-1-ethyl-3-{2-[4-(4-fluorphenyl)-piperidin-1-yl]-ethyl}-8-furan-2-yl-1,3-dihydro-[1,2,4]triazolo[5,1-f]purin-2-on,
5-Amino-1-ethyl-8-(2-furyl)-3-[2-(3-methyl-7,8-dihydro-5H-1,6-naphthyridin-6-yl)ethyl]-[1,2,4]triazolo[5,1-f]purin-2-on,
5-Amino-8-(2-furyl)-3-[2-[4-[4-(2-methoxyethoxy)phenyl]piperazin-1-yl]ethyl]-1-(2,2,2-trifluorethyl)-[1,2,4]triazolo[5,1-f]purin-2-on,
5-Amino-3-{2-[4-(2,4-difluorphenyl)-piperazin-1-yl]-ethyl}-8-furan-2-yl-1-(2,2,2-trifluorethyl)-1,3-dihydro-[1,2,4]triazolo[5,1-f]purin-2-on,
5-Amino-8-(2-furyl)-3-[2-[4-[4-(2-methoxyethoxy)phenyl]piperazin-1-yl]ethyl]-1-(2-methoxyethyl)-[1,2,4]triazolo[5,1-f]purin-2-on,
5-Amino-3-[2-[4-(4-fluorphenyl)piperazin-1-yl]ethyl]-8-(2-furyl)-1-(2-methoxyethyl)-[1,2,4]triazolo[5,1-f]purin-2-on,
5-Amino-3-[2-[4-(4-fluorphenyl)piperazin-1-yl]ethyl]-8-(2-furyl)-1-(2-hydroxyethyl)-[1,2,4]triazolo[5,1-f]purin-2-on,
5-Amino-1-cyclopropyl-8-(2-furyl)-3-[2-[4-[4-(2-methoxyethoxy)phenyl]piperazin-1-yl]ethyl]-[1,2,4]triazolo[5,1-f]purin-2-on,
5-Amino-8-(2-furyl)-3-[2-[4-[4-(2-methoxyethoxy)phenyl]piperazin-1-yl]ethyl]-1-(2,2,2-trifluorethyl)-[1,2,4]triazolo[5,1-f]purin-2-on,
5-Amino-3-[2-[4-[4-(2-methoxyethoxy)phenyl]piperazin-1-yl]ethyl]-1-methyl-8-thiazol-2-yl-[1,2,4]triazolo[5,1-f]purin-2-on,
5-Amino-3-[2-[4-[3-fluor-4-(2-methoxyethoxy)phenyl]piperazin-1-yl]ethyl]-1-methyl-8-thiazol-2-yl-[1,2,4]triazolo[5,1-f]purin-2-on,
5-Amino-3-[2-[4-(2-cyclopropylacetyl)piperazin-1-yl]ethyl]-1-methyl-8-thiazol-2-yl-[1,2,4]triazolo[5,1-f]purin-2-on,
5-Amino-3-[2-[4-(4-methoxyphenyl)piperazin-1-yl]ethyl]-1-methyl-8-thiazol-2-yl-[1,2,4]triazolo[5,1-f]purin-2-on,
5-Amino-1-methyl-3-[2-[4-(p-tolyl)piperazin-1-yl]ethyl]-8-thiazol-2-yl-[1,2,4]triazolo[5,1-f]purin-2-on,
5-Amino-1-methyl-3-[2-(3-methyl-7,8-dihydro-5H-1,6-naphthyridin-6-yl)ethyl]-8-thiazol-2-yl-[1,2,4]triazolo[5,1-f]purin-2-on,
4-[4-[2-(5-Amino-1-methyl-2-oxo-8-thiazol-2-yl-[1,2,4]triazolo[5,1-f]purin-3-yl)ethyl]piperazin-1-yl]benzonitril,
5-Amino-1-methyl-3-[2-[4-[3-(5-methyl-1,3,4-oxadiazol-2-yl)phenyl]piperazin-1-yl]ethyl]-8-thiazol-2-yl-[1,2,4]triazolo[5,1-f]purin-2-on,
5-Amino-3-[2-[4-[4-(1-hydroxy-1-methylethyl)phenyl]piperazin-1-yl]ethyl]-1-methyl-8-thiazol-2-yl-[1,2,4]triazolo[5,1-f]purin-2-on,
5-Amino-3-[2-[4-[4-(2-methoxyethoxy)phenyl]piperazin-1-yl]ethyl]-1-methyl-8-(2-pyridyl)-[1,2,4]triazolo[5,1-f]purin-2-on,
5-Amino-3-[2-[4-(2,4-difluorphenyl)piperazin-1-yl]ethyl]-1-methyl-8-(2-pyridyl)-[1,2,4]triazolo[5,1-f]purin-2-on,
4-[4-[2-[5-Amino-1-methyl-2-oxo-8-(2-pyridyl)-[1,2,4]triazolo[5,1-f]purin-3-yl]ethyl]piperazin-1-yl]benzonitril,
5-Amino-3-[2-[4-[4-(1-hydroxy-1-methylethyl)phenyl]piperazin-1-yl]ethyl]-1-methyl-8-(2-pyridyl)-[1,2,4]triazolo[5,1-f]purin-2-on,
5-Amino-3-[2-[4-[4-(2-methoxyethoxy)phenyl]piperazin-1-yl]ethyl]-1-methyl-8-pyrazin-2-yl-[1,2,4]triazolo[5,1-f]purin-2-on,
5-Amino-3-[2-[4-(2,4-difluorphenyl)piperazin-1-yl]ethyl]-1-methyl-8-pyrazin-2-yl-[1,2,4]triazolo[5,1-f]purin-2-on,
5-Amino-3-[2-[4-[2-fluor-4-(2-methoxyethoxy)phenyl]piperazin-1-yl]ethyl]-1-methyl-8-pyrazin-2-yl-[1,2,4]triazolo[5,1-f]purin-2-on,
5-Amino-8-(2-furyl)-3-[[1-(4-methoxyphenyl)pyrrolidin-3-yl]methyl]-1-methyl-[1,2,4]triazolo[5,1-f]purin-2-on,
5-Amino-8-(2-furyl)-3-[[1-[4-(2-methoxyethoxy)phenyl]pyrrolidin-3-yl]methyl]-1-ethyl-[1,2,4]triazolo[5,1-f]purin-2-onyl]-1-methyl-1,3-dihydro-[1,2,4]triazolo[5,1-i]purin-2-on,
5-Amino-8-(2-furyl)-3-[2-[4-[4-(2-methoxyethoxy)phenyl]piperazin-1-yl]ethyl]-1-methyl-[1,2,4]triazolo[5,1-f]purin-2-thion,
8-(2-Furyl)-3-[2-[4-[4-(2-methoxyethoxy)phenyl]piperazin-1-yl]ethyl]-1-methyl-5-(methylamino)-[1,2,4]triazolo[5,1-f]purin-2-on,
5-Amino-3-{2-[4-(4-fluorphenyl)-piperazin-1-yl]-ethyl}-8-isothiazol-5-yl-1-methyl-1,3-dihydro-[1,2,4]triazolo[5,1-i]purin-2-on,
5-Amino-8-isothiazol-5-yl-3-(2-{4-[4-(2-methoxyethoxy)-phenyl]-piperazin-1-yl}-ethyl)-1-methyl-1,3-dihydro-[1,2,4]triazolo[5,1-i]purin-2-on,
5-Amino-3-[2-[4-[2-fluor-4-(2-methoxyethoxy)phenyl]piperazin-1-yl]ethyl]-8-isothiazol-5-yl-1-methyl-[1,2,4]triazolo[5,1-f]purin-2-on,
5-Amino-8-isoxazol-5-yl-3-[2-[4-[4-(2-methoxyethoxy)phenyl]piperazin-1-yl]ethyl]-1-methyl-[1,2,4]triazolo[5,1-f]purin-2-on,
5-Amino-3-[2-[4-[4-(2-methoxyethoxy)phenyl]piperazin-1-yl]ethyl]-1-methyl-8-oxazol-2-yl-[1,2,4]triazolo[5,1-f]purin-2-on,
5-Amino-3-{2-[4-(4-methoxyphenyl)-piperazin-1-yl]-ethyl}-1-methyl-8-prop-1-inyl-1,3-dihydro-[1,2,4]triazolo[5,1-i]purin-2-on,
5-Amino-3-(2-{4-[4-(2-methoxyethoxy)-phenyl]-piperazin-1-yl}-ethyl)-1-methyl-8-prop-1-inyl-1,3-dihydro-[1,2,4]triazolo[5,1-i]purin-2-on,
5-Amino-3-{2-[4-(4-fluorbenzoyl)-piperazin-1-yl]-ethyl}-8-furan-2-yl-1-methyl-1,3-dihydro-[1,2,4]triazolo[5,1-i]purin-2-on,
5-Amino-3-(2-dimethylaminoethyl)-8-furan-2-yl-1-methyl-1,3-dihydro-[1,2,4]triazolo[5,1-i]purin-2-on,
5-Amino-8-furan-2-yl-3-[3-(4-methoxyphenyl)-propyl]-1-methyl-1,3-dihydro-[1,2,4]triazolo[5,1-i]purin-2-on,
5-Amino-8-furan-2-yl-1-methyl-3-(2-pyrazol-1-yl-ethyl)-1,3-dihydro-[1,2,4]triazolo[5,1-i]purin-2-on,
5-Amino-8-furan-2-yl-3-(2-{4-[4-(2-methoxyethoxy)-phenyl]-pyrazol-1-yl}-ethyl)-1-methyl-1,3-dihydro-[1,2,4]triazolo[5,1-i]purin-2-on,
5-Amino-8-furan-2-yl-3-{2-[3-(4-methoxyphenyl)-pyrrol-1-yl]-ethyl}-1-methyl-1,3-dihydro-[1,2,4]triazolo[5,1-i]purin-2-on,
5-Amino-8-furan-2-yl-3-{2-[4-(4-methoxyphenyl)-imidazol-1-yl]-ethyl}-1-methyl-1,3-dihydro-[1,2,4]triazolo[5,1-i]purin-2-on,
5-Amino-8-furan-2-yl-3-{2-[4-(4-methoxyphenyl)-[1,2,3]triazol-1-yl]-ethyl}-1-methyl-1,3-dihydro-[1,2,4]triazolo[5,1-i]purin-2-on,
5-Amino-3-[2-(1,3-dihydroisoindol-2-yl)-ethyl]-8-furan-2-yl-1-methyl-1,3-dihydro-[1,2,4]triazolo[5,1-i]purin-2-on,
5-Amino-8-furan-2-yl-1-methyl-3-(2-piperidin-1-yl-ethyl)-1,3-dihydro-[1,2,4]triazolo[5,1-i]purin-2-on,
5-Amino-8-furan-2-yl-1-methyl-3-(2-pyrrolidin-1-yl-ethyl)-1,3-dihydro-[1,2,4]triazolo[5,1-i]purin-2-on,
5-Amino-8-furan-2-yl-1-methyl-3-[2-(3-methyl-7,8-dihydro-5H-[1,6]naphthyridin-6-yl)-ethyl]-1,3-dihydro-[1,2,4]triazolo[5,1-i]purin-2-on,
5-Amino-8-furan-2-yl-3-{2-[4-(2-methoxyethoxy)-phenoxy]-ethyl}-1-methyl-1,3-dihydro-[1,2,4]triazolo[5,1-i]purin-2-on,
5-Amino-8-furan-2-yl-3-{2-[4-(2-methoxyethoxy)-phenylamino]-ethyl}-1-methyl-1,3-dihydro-[1,2,4]triazolo[5,1-i]purin-2-on,
5-Amino-8-furan-2-yl-1-methyl-3-[2-(pyridin-2-yloxy)-ethyl]-1,3-dihydro-[1,2,4]triazolo[5,1-i]purin-2-on,
5-Amino-1-ethyl-3-{2-[4-(4-fluorphenyl)-piperazin-1-yl]-ethyl}-8-isothiazol-5-yl-1,3-dihydro-[1,2,4]triazolo[5,1-i]purin-2-on,
5-Amino-1-ethyl-8-isothiazol-5-yl-3-(2-{4-[4-(2-methoxyethoxy)-phenyl]-piperazin-1-yl}-ethyl)-1,3-dihydro-[1,2,4]triazolo[5,1-i]purin-2-on,
5-Amino-1-ethyl-8-furan-2-yl-3-(2-piperidin-1-yl-ethyl)-1,3-dihydro-[1,2,4]triazolo[5,1-i]purin-2-on,
5-Amino-1-ethyl-8-furan-2-yl-3-[2-(3-methyl-7,8-dihydro-5H-[1,6]naphthyridin-6-yl)-ethyl]-1,3-dihydro-[1,2,4]triazolo[5,1-i]purin-2-on,
5-Amino-3-[2-(2,4-difluorphenoxy)-ethyl]-1-ethyl-8-furan-2-yl-1,3-dihydro-[1,2,4]triazolo[5,1-i]purin-2-on,
5-Amino-3-[2-(2,4-difluorphenylamino)-ethyl]-1-ethyl-8-furan-2-yl-1,3-dihydro-[1,2,4]triazolo[5,1-i]purin-2-on,
5-Amino-1-cyclopropylmethyl-3-[2-(2,4-difluorphenylamino)-ethyl]-8-furan-2-yl-1,3-dihydro-[1,2,4]triazolo[5,1-i]purin-2-on,
5-Amino-1 -cyclopropylmethyl-3-[2-(2,4-difluorphenoxy)-ethyl]-8-furan-2-yl-1,3-dihydro-[1,2,4]triazolo[5,1-i]purin-2-on,
5-Amino-1-cyclopropylmethyl-3-{2-[4-(4-fluorphenyl)-piperidin-1-yl]-ethyl}-8-furan-2-yl-1,3-dihydro-[1,2,4]triazolo[5,1-i]purin-2-on,
5-Amino-3-{2-[4-(4-fluorphenyl)-piperidin-1-yl]-ethyl}-8-furan-2-yl-1-(2,2,2-trifluorethyl)-1,3-dihydro-[1,2,4]triazolo[5,1-i]purin-2-on,
5-Amino-8-furan-2-yl-3-{2-[4-(4-methoxyphenyl)-piperazin-1-yl]-ethyl}-1-(2,2,2-trifluorethyl)-1,3-dihydro-[1,2,4]triazolo[5,1-i]purin-2-on,
5-Amino-3-[2-(4-cyclopropylmethylpiperazin-1-yl)-ethyl]-8-isothiazol-5-yl-1-(2,2,2-trifluorethyl)-1,3-dihydro-[1,2,4]triazolo[5,1-i]purin-2-on,
(5-Amino-8-isothiazol-5-yl-3-{2-[4-(4-methoxyphenyl)-piperazin-1-yl]-ethyl}-2-oxo-2,3-dihydro-[1,2,4]triazolo[5,1-i]purin-1-yl)-acetonitril,
[5-Amino-3-{2-[4-(2,4-difluorphenyl)-piperazin-1-yl]-ethyl}-8-(3-fluorphenyl)-2-oxo-2,3-dihydro-[1,2,4]triazolo[5,1-i]purin-1-yl]-acetonitril,
[5-Amino-8-furan-2-yl-3-(2-{4-[4-(2-methoxyethoxy)-phenyl]-piperazin-1-yl}-ethyl)-2-oxo-2,3-dihydro-[1,2,4]triazolo[5,1-i]purin-1-yl]-acetonitril,
5-Amino-3-(2-{4-[4-(2-methoxyethoxy)-phenyl]-piperazin-1-yl}-ethyl)-1-methyl-8-phenyl-1,3-dihydro-[1,2,4]triazolo[5,1-i]purin-2-on,
3-[5-Amino-3-(2-{4-[4-(2-methoxyethoxy)-phenyl]-piperazin-1-yl}-ethyl)-1-methyl-2-oxo-2,3-dihydro-1H-[1,2,4]triazolo[5,1-i]purin-8-yl]-benzonitril,
3-[5-Amino-3-(2-{4-[4-(2-methoxyethoxy)-phenyl]-piperazin-1-yl}-ethyl)-1-methyl-2-oxo-2,3-dihydro-1H-[1,2,4]triazolo[5,1-i]purin-8-yl]-benzonitril,
5-Amino-8-furan-2-yl-1-methyl-3-vinyl-1,3-dihydro-[1,2,4]triazolo[5,1-i]purin-2-on,
5-Amino-3-[3-(4-fluorphenyl)-prop-2-inyl]-8-furan-2-yl-1-methyl-1,3-dihydro-[1,2,4]triazolo[5,1-i]purin-2-on,
5-Amino-8-furan-2-yl-1-methyl-3-[4-(4-methylpiperazin-1-yl)-but-2-inyl]-1,3-dihydro-[1,2,4]triazolo[5,1-i]purin-2-on,
5-Amino-8-furan-2-yl-1-isopropyl-3-(2-{4-[4-(2-methoxyethoxy)-phenyl]-piperazin-1-yl}-ethyl)-1,3-dihydro-[1,2,4]triazolo [5,1-i]purin-2-on,
5-Amino-2-benzyl-7-(2- {4-[4-(2-methoxyethoxy)-phenyl]-piperazin-1-yl}-ethyl)-9-methyl-7,9-dihydro-2H-[1,2,4]triazolo[3,4-i]purin-3,8-dion,
5-Amino-2-benzyl-9-methyl-7-(2-morpholin-4-yl-ethyl)-7,9-dihydro-2H-[1,2,4]triazolo[3,4-i]purin-3,8-dion,
5-Amino-2-(3-chlorbenzyl)-7-[2-(4-isopropylpiperazin-1-yl)-ethyl]-9-methyl-7,9-dihydro-2H-[1,2,4]triazolo[3,4-i]purin-3,8-dion,
5-Amino-2-cyclopropylmethyl-9-methyl-7-(2-morpholin-4-yl-ethyl)-7,9-dihydro-2H-[1,2,4]triazolo[3,4-i]purin-3,8-dion,
5-Amino-2-cyclopropylmethyl-7-(2,4-difluorbenzyl)-9-methyl-7,9-dihydro-2H-[1,2,4]triazolo[3,4-i]purin-3,8-dion,
4-Amino-2-furan-2-yl-6-(2-{4-[4-(2-methoxyethoxy)-phenyl]-piperazin-1-yl}-ethyl)-6H-8-oxa-1,3,3a,5,6-pentaaza-as-indacen-7-on, oder
4-Amino-2-furan-2-yl-6-(2-{4-[4-(2-methoxyethoxy)-phenyl]-piperazin-1-yl}-ethyl)-8,8-dimethyl-6,8-dihydro-1,3,3a,5,6-pentaaza-as-indacen-7-on.

7. Pharmazeutische Zusammensetzung, umfassend als Wirkstoff wenigstens eine Verbindung der Formel (I) nach einem der Ansprüche 1 bis 6, oder ein Tautomer, Polymorph, Stereoisomer, Solvat oder pharmazeutisch verträgliches Salz davon, zusammen mit ein oder mehr pharmazeutisch verträglichen Trägern oder Hilfsstoffen.

8. Verbindung der Formel (I) nach einem der Ansprüche 1 bis 6, oder ein Tautomer, Polymorph, Stereoisomer, Solvat oder pharmazeutisch verträgliches Salz davon, zur Verwendung bei der Behandlung einer Krankheit oder eines Leidens, die bzw. das sich durch A_{2A}-Rezeptorantagonismus bessern lässt.

9. Verbindung der Formel (I) nach einem der Ansprüche 1 bis 6, oder ein Tautomer, Polymorph, Stereoisomer, Solvat oder pharmazeutisch verträgliches Salz davon, zur Verwendung bei der Behandlung von Parkinson-Krankheit, Restless-Legs-Syndrom, Alzheimer-Krankheit, neurodegenerativen Erkrankungen, Entzündungen, Wundheilung, dermaler Fibrose, nächtlichem Myoklonus, zerebraler Ischämie, Huntington-Krankheit, Multisystematrophie, corticobasaler Degeneration, Morbus Wilson oder anderen Erkrankungen von Basalganglien, die zu Dyskinesien führen, posttraumatischer Belastungsstörung, Leberzirrhose, Sepsis, Rückenmarksverletzungen, Retinopathie, Hypertonie, sozialen Gedächtnisstörungen, Depression, Neuroprotektion, Narkolepsie oder anderen Schlafstörungen, Aufmerksamkeitsdefizit-Hyperaktivitätsstörung, Medikamentenabhängigkeit, posttraumatischer Belastungsstörung und Gefäßverletzungen.

10. Pharmazeutische Zusammensetzung, umfassend eine Verbindung der Formel (I) nach einem der Ansprüche 1 bis 6, oder ein Tautomer, Polymorph, Stereoisomer, Solvat oder pharmazeutisch verträgliches Salz davon, in Kombination mit ein oder mehr therapeutischen Wirkstoffen.

11. Pharmazeutische Zusammensetzung nach Anspruch 10, wobei der therapeutische Wirkstoff ausgewählt ist aus entzündungshemmenden Mitteln, Antidiabetika, Antihypertonika oder Mitteln gegen Dyslipidämie.

12. Pharmazeutische Zusammensetzung nach Anspruch 10, wobei der therapeutische Wirkstoff ausgewählt ist aus Anticholinergika, Antimuskarinika, Steroiden, LTB4 (Leukotrien-B4)-Antagonisten, Dopaminrezeptorantagonisten, Phosphodiesterase 4-Inhibitoren, beta-2-Adrenozeptoragonisten, Insulin, Insulinderivaten und -mimetika, Insulinsekretagoga, insulinotropen Sulfonylharnstoffrezeptorliganden, Thiazolidonderivaten, Glykogensynthase-Kinase-3-Inhibitoren, Inhibitoren von natriumabhängigem Glukose-Cotransporter, Glykogenphosphorylase A-Inhibitor, Biguanid, alpha-Glukosidase-Inhibitor, Glucagon-like-Peptid 1 (GLP-1), GLP-1-Analogen und GLP-1-Mimetika, Modulatoren für Peroxisom-Proliferator-aktivierte Rezeptoren, Dipeptidylpeptidase IV-Inhibitoren, Stearoyl-CoA-Desaturase-1-Inhibitoren, Diacylglycerin-Acyltransferase 1- und 2-Inhibitoren, Acetyl-CoA-Carboxylase 2-Inhibitoren und Advanced-Glycation-End-Products-Breakers, Schleifendiuretika, Inhibitoren von Angiotensin-konvertierendem Enzym, Inhibitoren der Na-K-ATPase-Membranpumpe wie Digoxin, Neutralendopeptidase (NEP)-Inhibitoren, ACE/NEP-Inhibitoren, Angiotensin II-Antagonisten, Renininhibitoren, β-Adrenozeptor-Blockern, inotropen Mitteln, Calciumkanalblockern, Aldosteronrezeptor-Antagonisten und Aldosteronsynthase-Inhibitoren, 3-Hydroxy-3-methylglutaryl-Coenzym A-Reduktase-Inhibitoren, HDL-erhöhenden Verbindungen wie Cholesterinestertransferprotein-Inhibitoren, Squalensynthase-Inhibitoren, Farnesoid X-Rezeptor- und Leber X-Rezeptorliganden, Cholestyramin, Fribaten, Nicotinsäure oder Aspirin.

13. Pharmazeutische Zusammensetzung, umfassend eine Verbindung der Formel (I) nach einem der Ansprüche 1 bis 6, oder ein Tautomer, Polymorph, Stereoisomer, Solvat oder pharmazeutisch verträgliches Salz davon, in Kombination mit ein bis drei weiteren zur Behandlung von Parkinson-Krankheit geeigneten Mitteln in einem pharmazeutsch verträglichen Träger.

14. Pharmazeutische Zusammensetzung nach Anspruch 13, wobei das weitere Mittel ausgewählt ist aus L-DOPA, dopaminergen Agonisten, MAO-B-Inhibitoren, DOPA-Decarboxylase-Inhibitoren, COMT-Inhibitoren und NMDA-Rezeptorantagonisten.

## Revendications

1. Composé représenté par la formule I 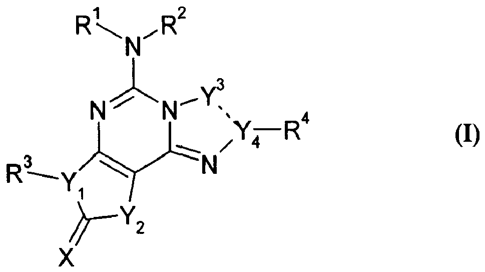 ou un tautomère, polymorphe, stéréoisomère, solvate ou sel pharmaceutiquement acceptable d'un tel composé,
formule dans laquelle :
--- représente une liaison simple ou une double liaison ;
X est choisi parmi O, S et NR^{a} ;
Y₁ est choisi parmi N et CH ;
Y₂ est choisi parmi NR⁵, O et CR⁵R⁶ ;
Y₃ est choisi parmi N, CH, CH₂, C(=O) et C(=S) ;
Y₄ est choisi parmi N, C ou CH ;
R¹ et R² sont indépendamment choisis parmi l'hydrogène et alkyle ;
R³ est -A-Z-B-Q ; où
A est absent ou est un groupe choisi parmi alkylène, alcénylène ou alcynylène ; où un ou plusieurs groupes méthylène sont éventuellement remplacés par des hétéroatomes ou des groupes tels que -O-, -S(O)ₚ-, -N(R^{a})- ou -C(O) ; les alkylène, alcénylène et alcynylène sont éventuellement substitués par un halogène, -(CR^{d}R^{e})ₙOR⁷, (CR^{d}R^{e})ₙCOOR⁷, -(CR^{d}R^{e})ₙNR⁸R⁹, cyano, halogénoalkyle, perhalogénoalkyle, alcoxyalcoxy, alkyle ou cycloalkyle ;
Z est absent ou est choisi parmi cycloalkyle et hétérocyclyle ;
où les cycloalkyle et hétérocyclyle sont non substitués ou substitués indépendamment par 1, 2 ou 3 substituants indépendamment choisis parmi un halogène, alkyle, alcényle, alcynyle, acyle, -(CR^{d}R^{e})ₙOR⁷, (CR^{d}R^{e})ₙCOOR⁷, -(CR^{d}R^{e})ₙNR⁸R⁹, aminocarbonyle, alcoxycarbonylamino, halogénoalkyle, perhalogénoalkyle, azido, cyano, céto, thiocarbonyle, -SO₃H, aminocarbonylamino, nitro, -S(O)₂NR^{a}R^{a}, -NR^{b}S(O)₂R^{b} ou-S(O)ₚR^{c} ,
B est absent ou est un groupe choisi parmi alkylène, alcénylène ou alcynylène ; où un ou plusieurs groupes méthylène sont éventuellement remplacés par des hétéroatomes ou des groupes tels que -O-, -S(O)ₚ-, -N(R^{a})- ou -C(O) ; les alkylène, alcénylène et alcynylène sont éventuellement substitués par un halogène, hydroxy, amino, aminoalkyle, cyano, halogénoalkyle, perhalogénoalkyle, carboxy, carboxyalkyle, alcoxy, hydroxyalkyle, alcoxyalkyle, alcoxyalcoxy ou alkyle ;
Q est choisi parmi l'hydrogène, alkyle, cycloalkyle, cycloalkylalkyle, aryle, arylalkyle, hétérocyclyle, hétérocyclylalkyle, hétéroaryle et hétéroarylalkyle ;
où les alkyle, cycloalkyle, cycloalkylalkyle, aryle, arylalkyle, hétérocyclyle, hétérocyclylalkyle, hétéroaryle et hétéroarylalkyle sont non substitués ou substitués indépendamment par 1, 2 ou 3 substituants indépendamment choisis parmi un halogène, alkyle, alcényle, alcynyle, halogénoalkyle, perhalogénoalkyle, azido, cyano, nitro, céto, thiocarbonyle, cyanoalkyle, cyanoalkylcarbonyle, -(CR^{d}R^{e})ₙOR⁷, - (CR^{d}R^{e})ₙC(O)R⁷, -(CR^{d}R^{e})ₙSR⁷, -(CR^{d}R^{e})ₙCOOR⁷, -(CR^{d}R^{e})ₙNR⁸R⁹,-(CR^{d}R^{e})ₙC(O)NR⁸R⁹, -(CR^{d}R^{e})ₙNR⁸C(O)OR⁷, -(CR^{d}R^{e})ₙNR⁸C(O)NR⁸R⁹,-NR^{b}S(O)₂R^{b}, -S(O)ₚR^{c}, -SO₃H, -S(O)₂NR^{a}R^{a}, cycloalkyle, cycloalcényle, cycloalkylalkyle, aryle, arylalkyle, hétérocyclyle, hétérocyclylalkyle, hétéroaryle ou hétéroarylalkyle ;
où chaque substituant est non substitué ou substitué par 1, 2 ou 3 substituants indépendamment choisis parmi un halogène, alkyle, carboxy, carboxyalkyle, aminocarbonyle, hydroxy, alcoxy, halogénoalkyle, perhalogénoalkyle, halogénoalcoxy, perhalogénoalcoxy, amino, amino substitué, cyano et -S(O)ₚR^{c} ;
R⁴ est choisi dans l'ensemble constitué par l'hydrogène, alkyle, alcényle, alcynyle, halogénoalkyle, hydroxyalkyle, carboxyalkyle, cycloalkyle, cycloalkylalkyle, aryle, arylalkyle, hétérocyclyle, hétérocyclylalkyle, hétéroaryle et hétéroarylalkyle ;
où les alkyle, alcényle, alcynyle, cycloalkyle, cycloalkylalkyle, arylalkyle, aryle, hétéroaryle, hétéroarylalkyle, hétérocyclyle et hétérocyclylalkyle sont non substitués ou substitués indépendamment par jusqu'à quatre substituants indépendamment choisis parmi un halogène, alkyle, alcényle, alcynyle, acyle, -(CR^{d}R^{e})ₙOR⁷,-(CR^{d}R^{e})ₙCOOR⁷, -(CR^{d}R^{e})ₙNR⁸R⁹, aminocarbonyle, alcoxycarbonylamino, aminocarbonylamino, azido, cyano, halogénoalkyle, perhalogénoalkyle, céto, nitro, -S(O)₂NR^{b}R^{b}, -NR^{b}S(O)₂R^{b} et -S(O)ₚR^{c}, thiocarbonyle, -SO₃H, cycloalkyle, cycloalcényle, aryle, hétéroaryle et hétérocyclyle ;
R⁵ et R⁶ sont indépendamment choisis dans l'ensemble constitué par l'hydrogène, hydroxy, -(CR^{d}R^{e})ₙOR⁷, (CR^{d}R^{e})ₙCOOR⁷, -(CR^{d}R^{e})ₙNR⁸R⁹, cyanoalkyle, halogénoalkyle, alcoxyalcoxyalkyle, alkyle, alcényle, alcynyle, cycloalkyle, cycloalkylalkyle, aryle, arylalkyle, hétérocyclyle, hétérocyclylalkyle, hétéroaryle et hétéroarylalkyle ;
R⁷ est choisi parmi l'hydrogène, un halogène, alkyle, halogénoalkyle, carbonylamino, cycloalkyle, cycloalkylalkyle, aryle, arylalkyle, hétéroaryle, hétéroarylalkyle, hétérocyclyle et hétérocyclylalkyle ;
R⁸ et R⁹ sont indépendamment choisis dans l'ensemble constitué par l'hydrogène, alkyle, halogénoalkyle, -(CR^{d}R^{e})ₙOR⁷, -(CR^{d}R^{e})ₙC(O)R⁷, aryle, arylalkyle, hétéroaryle, hétéroarylalkyle, cycloalkyle, cycloalkylalkyle, hétérocyclyle et hétérocyclylalkyle,
ou bien R⁸ et R⁹, pris ensemble, forment un système cyclique monocyclique ou bicyclique qui est saturé ou partiellement insaturé et a éventuellement des hétéroatomes additionnels choisis parmi O, N et S, ledit système cyclique étant en outre éventuellement substitué par 1 à 4 substituants indépendamment choisis parmi halogéno, alkyle, alcényle, alcynyle, nitro, cyano, -(CR^{d}R^{e})ₙOR⁷, -(CR^{d}R^{e})ₙSR⁷,-(CR^{d}R^{e})ₙNR⁸R⁹, oxo, alkylsulfonyle, -(CR^{d}R^{e})ₙCOOR⁷, -(CR^{d}R^{e})ₙC(O)NR⁸R⁹, cycloalkyle, cycloalkylalkyle, aryle, arylalkyle, hétérocyclyle, hétérocyclylalkyle, hétéroaryle, et hétéroarylalkyle ;
R^{a} est choisi parmi l'hydrogène et alkyle ;
R^{b}, à chaque occurrence, est indépendamment choisi dans l'ensemble constitué par l'hydrogène, alkyle, acyle, carboxyalkyle, carbonylamino, cycloalkyle, cycloalkylalkyle, aryle, arylalkyle, hétéroaryle, hétéroarylalkyle, hétérocyclyle et hétérocyclylalkyle ;
R^{c} est choisi parmi alkyle, cycloalkyle, aryle, hétérocyclyle et hétéroaryle ;
R^{d} et R^{e} sont indépendamment choisis dans l'ensemble constitué par l'hydrogène, un halogène, -OR⁷, halogénoalkyle, perhalogénoalkyle et alkyle ;
n vaut 0, 1, 2, 3 ou 4 ; et
p vaut 0, 1 ou 2.

2. Composé de formule (I) selon la revendication 1 ou un tautomère, polymorphe, stéréoisomère, solvate ou sel pharmaceutiquement acceptable d'un tel composé, dans lequel
--- représente une double liaison ;
X est choisi parmi O, S et NR^{a} ;
Y₁ est choisi parmi N et CH ;
Y₂ est choisi parmi NR⁵ et CR⁵R⁶ ;
Y₃ est choisi parmi N, CH et CH₂ ;
Y₄ est choisi parmi N et C ;
R¹ et R² sont indépendamment choisis parmi l'hydrogène et alkyle ;
R³ est -A-Z-B-Q ; où
A est absent ou est un alkylène, où un ou plusieurs groupes méthylène sont éventuellement remplacés par des hétéroatomes ou des groupes tels que -O-, -S(O)ₚ-, -N(R^{a})- ou -C(O) ; l'alkylène est éventuellement substitué par un halogène, - (CR^{d}R^{e})ₙOR⁷, cyano, halogénoalkyle, perhalogénoalkyle, alkyle ou cycloalkyle ;
Z est absent ou est choisi parmi cycloalkyle et hétérocyclyle ;
où les cycloalkyle et hétérocyclyle sont non substitués ou substitués indépendamment par 1, 2 ou 3 substituants indépendamment choisis parmi un halogène, alkyle, acyle, -(CR^{d}R^{e})ₙOR⁷, -(CR^{d}R^{e})ₙCOOR⁷, -(CR^{d}R^{e})ₙNR⁸R⁹, aminocarbonyle, alcoxycarbonylamino, halogénoalkyle, perhalogénoalkyle, azido, cyano, céto, thiocarbonyle, -SO₃H, aminocarbonylamino, nitro, -S(O)₂NR^{a}R^{a}, -NR^{b}S(O)₂R^{b} et -S(O)ₚR^{c} ;
B est absent ou est un alkylène, où un ou plusieurs groupes méthylène sont éventuellement remplacés par des hétéroatomes ou des groupes tels que -O-, -S(O)ₚ-, -N(R^{a})- ou -C(O) ; l'alkylène est éventuellement substitué par un halogène, hydroxy, amino, aminoalkyle, cyano, halogénoalkyle, perhalogénoalkyle, carboxy, carboxyalkyle, alcoxy, hydroxyalkyle, alcoxyalkyle, alcoxyalcoxy ou alkyle ;
Q est choisi parmi l'hydrogène, alkyle, cycloalkyle, cycloalkylalkyle, aryle, arylalkyle, hétérocyclyle, hétérocyclylalkyle, hétéroaryle et hétéroarylalkyle ;
où les alkyle, cycloalkyle, cycloalkylalkyle, aryle, arylalkyle, hétérocyclyle, hétérocyclylalkyle, hétéroaryle et hétéroarylalkyle sont non substitués ou substitués indépendamment par 1, 2 ou 3 substituants indépendamment choisis parmi un halogène, alkyle, alcényle, alcynyle, alcoxy, alcoxyalkyle, halogénoalkyle, perhalogénoalkyle, azido, cyano, nitro, céto, thiocarbonyle, cyanoalkyle, cyanoalkylcarbonyle, -(CR^{d}R^{e})ₙOR⁷, -(CR^{d}R^{e})ₙC(O)R⁷, -(CR^{d}R^{e})ₙSR⁷,-(CR^{d}R^{e})ₙCOOR⁷, -(CR^{d}R^{e})ₙNR⁸R⁹, -(CR^{d}R^{e})ₙC(O)NR⁸R⁹, -(CR^{d}R^{e})ₙNR⁸C(O)OR⁷,-(CR^{d}R^{e})ₙNR⁸C(O)NR⁸R⁹, -NR^{b}S(O)₂R^{b}, -S(O)ₚR^{c}, -SO₃H, -S(O)₂NR^{a}R^{a}, cycloalkyle, cycloalcényle, cycloalkylalkyle, aryle, arylalkyle, hétérocyclyle, hétérocyclylalkyle, hétéroaryle et hétéroarylalkyle ;
où chaque substituant est non substitué ou substitué par 1, 2 ou 3 substituants indépendamment choisis parmi un halogène, alkyle, carboxy, carboxyalkyle, aminocarbonyle, hydroxy, alcoxy, halogénoalkyle, perhalogénoalkyle, halogénoalcoxy, perhalogénoalcoxy, amino, amino substitué, cyano et -S(O)ₚR^{c} ;
R⁴ est choisi dans l'ensemble constitué par l'hydrogène, alkyle, halogénoalkyle, hydroxyalkyle, carboxyalkyle, cycloalkyle, cycloalkylalkyle, aryle, arylalkyle, hétérocyclyle, hétérocyclylalkyle, hétéroaryle et hétéroarylalkyle ;
où les alkyle, cycloalkyle, cycloalkylalkyle, arylalkyle, aryle, hétéroaryle, hétéroarylalkyle, hétérocyclyle et hétérocyclylalkyle sont non substitués ou substitués indépendamment par jusqu'à quatre substituants indépendamment choisis parmi un halogène, alkyle, acyle, -(CR^{d}R^{e})ₙOR⁷, -(CR^{d}R^{e})ₙCOOR⁷, -(CR^{d}R^{e})ₙNR⁸R⁹, aminocarbonyle, alcoxycarbonylamino, aminocarbonylamino, azido, cyano, halogénoalkyle, perhalogénoalkyle, céto, nitro, -S(O)₂NR^{b}R^{b}, -NR^{b}S(O)₂R^{b} et-S(O)ₚR^{c}, thiocarbonyle, -SO₃H, cycloalkyle, cycloalcényle, aryle, hétéroaryle et hétérocyclyle ;
R⁵ et R⁶ sont indépendamment choisis dans l'ensemble constitué par l'hydrogène, hydroxy, -(CR^{d}R^{e})ₙOR⁷, -(CR^{d}R^{e})ₙCOOR⁷, -(CR^{d}R^{e})ₙNR⁸R⁹, cyanoalkyle, halogénoalkyle, alcoxyalcoxyalkyle, alkyle, alcényle, alcynyle, cycloalkyle, cycloalkylalkyle, aryle, arylalkyle, hétérocyclyle, hétérocyclylalkyle, hétéroaryle et hétéroarylalkyle ;
R⁷ est choisi parmi l'hydrogène, un halogène, alkyle, halogénoalkyle, carbonylamino, cycloalkyle, cycloalkylalkyle, aryle, arylalkyle, hétéroaryle, hétéroarylalkyle, hétérocyclyle et hétérocyclylalkyle ;
R⁸ et R⁹ sont indépendamment choisis dans l'ensemble constitué par l'hydrogène, alkyle, halogénoalkyle, -(CR^{d}R^{e})ₙOR⁷, -(CR^{d}R^{e})ₙC(O)R⁷, aryle, arylalkyle, hétéroaryle, hétéroarylalkyle, cycloalkyle, cycloalkylalkyle, hétérocyclyle et hétérocyclylalkyle,
ou bien R⁸ et R⁹, pris ensemble, forment un système cyclique monocyclique ou bicyclique qui est saturé ou partiellement insaturé et a éventuellement des hétéroatomes additionnels choisis parmi O, N et S, ledit système cyclique étant en outre éventuellement substitué par 1 à 4 substituants indépendamment choisis parmi halogéno, alkyle, alcényle, alcynyle, nitro, cyano, -(CR^{d}R^{e})ₙOR⁷, -(CR^{d}R^{e})ₙSR⁷, - (CR^{d}R^{e})ₙNR⁸R⁹, oxo, alkylsulfonyle, -(CR^{d}R^{e})ₙCOOR⁷, -(CR^{d}R^{e})ₙC(O)NR⁸R⁹, cycloalkyle, cycloalkylalkyle, aryle, arylalkyle, hétérocyclyle, hétérocyclylalkyle, hétéroaryle, et hétéroarylalkyle ;
R^{a} est choisi parmi l'hydrogène et alkyle ;
chaque R^{b} est indépendamment choisi dans l'ensemble constitué par l'hydrogène, alkyle, acyle, carboxyalkyle, carbonylamino, cycloalkyle, cycloalkylalkyle, aryle, arylalkyle, hétéroaryle, hétéroarylalkyle, hétérocyclyle et hétérocyclylalkyle ;
R^{c} est choisi parmi alkyle, cycloalkyle, aryle, hétérocyclyle et hétéroaryle ;
R^{d} et R^{e} sont indépendamment choisis dans l'ensemble constitué par l'hydrogène, un halogène, -OR⁷, halogénoalkyle, perhalogénoalkyle et alkyle ;
n vaut 0, 1, 2, 3 ou 4 ; et
p vaut 0, 1 ou 2.

3. Composé de formule (I) selon la revendication 1 ou un tautomère, polymorphe, stéréoisomère, solvate ou sel pharmaceutiquement acceptable d'un tel composé, dans lequel
--- représente une double liaison ;
X est choisi parmi O et S ;
Y₁ représente N ;
Y₂ représente NR⁵;
Y₃ représente N ;
Y₄ représente C ;
R¹ et R² sont indépendamment choisis parmi l'hydrogène et alkyle ;
R³ est -A-Z-B-Q ; où
A est absent ou est un alkylène, où un ou plusieurs groupes méthylène sont éventuellement remplacés par des hétéroatomes ou des groupes tels que -O-, -S(O)ₚ-, -N(R^{a})- ou -C(O) ;
Z est absent ou est un hétérocyclyle ;
où l'hétérocyclyle est non substitué ou substitué indépendamment par 1, 2 ou 3 substituants indépendamment choisis parmi un halogène, alkyle, acyle, -(CR^{d}R^{e})ₙOR⁷, -(CR^{d}R^{e})ₙCOOR⁷, -(CR^{d}R^{e})ₙNR⁸R⁹, halogénoalkyle, perhalogénoalkyle, cyano, céto, thiocarbonyle, -SO₃H, nitro, -S(O)₂NR^{a}R^{a},-NR^{b}S(O)₂R^{b} et -S(O)ₚR^{c} ;
B est absent ou est un alkylène, où un ou plusieurs groupes méthylène sont éventuellement remplacés par des hétéroatomes ou des groupes tels que -O-, -S(O)ₚ-, -N(R^{a})- ou -C(O) ;
Q est choisi parmi l'hydrogène, alkyle, cycloalkyle, aryle, hétérocyclyle et hétéroaryle ;
où les alkyle, cycloalkyle, aryle, hétérocyclyle et hétéroaryle sont non substitués ou substitués indépendamment par 1, 2 ou 3 substituants indépendamment choisis parmi un halogène, alkyle, alcoxy, alcoxyalkyle, halogénoalkyle, perhalogénoalkyle, azido, cyano, nitro, céto, thiocarbonyle, cyanoalkyle, cyanoalkylcarbonyle, -(CR^{d}R^{e})ₙOR⁷,-(CR^{d}R^{e})ₙC(O)R⁷, -(CR^{d}R^{e})ₙSR⁷, -(CR^{d}R^{e})ₙCOOR⁷, -(CR^{d}R^{e})ₙNR⁸R⁹,-(CR^{d}R^{e})ₙC(O)NR⁸R⁹, -(CR^{d}R^{e})ₙNR⁸C(O)OR⁷, -(CR^{d}R^{e})ₙNR⁸C(O)NR⁸R⁹,-NR^{b}S(O)₂R^{b}, -S(O)ₚR^{c}, -SO₃H, -S(O)₂NR^{a}R^{a}, cycloalkyle, cycloalcényle, cycloalkylalkyle, aryle, arylalkyle, hétérocyclyle, hétérocyclylalkyle, hétéroaryle et hétéroarylalkyle ;
où chaque substituant est non substitué ou substitué par 1, 2 ou 3 substituants indépendamment choisis parmi un halogène, alkyle, carboxy, carboxyalkyle, aminocarbonyle, hydroxy, alcoxy, halogénoalkyle, perhalogénoalkyle, halogénoalcoxy, perhalogénoalcoxy, amino, amino substitué, cyano et -S(O)ₚR^{c} ;
R⁴ est choisi dans l'ensemble constitué par l'hydrogène, alkyle, halogénoalkyle, hydroxyalkyle, carboxyalkyle, cycloalkyle, cycloalkylalkyle, aryle, arylalkyle, hétérocyclyle, hétérocyclylalkyle, hétéroaryle et hétéroarylalkyle ;
où les alkyle, cycloalkyle, cycloalkylalkyle, arylalkyle, aryle, hétéroaryle, hétéroarylalkyle, hétérocyclyle et hétérocyclylalkyle sont non substitués ou substitués indépendamment par jusqu'à quatre substituants indépendamment choisis parmi un halogène, alkyle, acyle, -(CR^{d}R^{e})ₙOR⁷, -(CR^{d}R^{e})ₙCOOR⁷, -(CR^{d}R^{e})ₙNR⁸R⁹, aminocarbonyle, alcoxycarbonylamino, aminocarbonylamino, azido, cyano, halogénoalkyle, perhalogénoalkyle, céto, nitro, -S(O)₂NR^{b}R^{b}, -NR^{b}S(O)₂R^{b} et-S(O)ₚR^{c}, thiocarbonyle, -SO₃H, cycloalkyle, cycloalcényle, aryle, hétéroaryle et hétérocyclyle ;
R⁵ et R⁶ sont indépendamment choisis dans l'ensemble constitué par l'hydrogène, hydroxy, -(CR^{d}R^{e})ₙOR⁷, -(CR^{d}R^{e})ₙCOOR⁷, -(CR^{d}R^{e})ₙNR⁸R⁹, cyanoalkyle, halogénoalkyle, alcoxyalcoxyalkyle, alkyle, alcényle, alcynyle, cycloalkyle, cycloalkylalkyle, aryle, arylalkyle, hétérocyclyle, hétérocyclylalkyle, hétéroaryle et hétéroarylalkyle ;
R⁷ est choisi parmi l'hydrogène, un halogène, alkyle, halogénoalkyle, carbonylamino, cycloalkyle, cycloalkylalkyle, aryle, arylalkyle, hétéroaryle, hétéroarylalkyle, hétérocyclyle et hétérocyclylalkyle ;
R⁸ et R⁹ sont indépendamment choisis dans l'ensemble constitué par l'hydrogène, alkyle, halogénoalkyle, -(CR^{d}R^{e})ₙOR⁷, -(CR^{d}R^{e})ₙC(O)R⁷, aryle, arylalkyle, hétéroaryle, hétéroarylalkyle, cycloalkyle, cycloalkylalkyle, hétérocyclyle et hétérocyclylalkyle,
ou bien R⁸ et R⁹, pris ensemble, forment un système cyclique monocyclique ou bicyclique qui est saturé ou partiellement insaturé et a éventuellement des hétéroatomes additionnels choisis parmi O, N et S, ledit système cyclique étant en outre éventuellement substitué par 1 à 4 substituants indépendamment choisis parmi halogéno, alkyle, alcényle, alcynyle, nitro, cyano, -(CR^{d}R^{e})ₙOR⁷, -(CR^{d}R^{e})ₙSR⁷, - (CR^{d}R^{e})nNR⁸R⁹, oxo, alkylsulfonyle, -(CR^{d}R^{e})ₙCOOR⁷, -(CR^{d}R^{e})ₙC(O)NR⁸R⁹, cycloalkyle, cycloalkylalkyle, aryle, arylalkyle, hétérocyclyle, hétérocyclylalkyle, hétéroaryle, et hétéroarylalkyle ;
R^{a} est choisi parmi l'hydrogène et alkyle ;
chaque R^{b} est indépendamment choisi dans l'ensemble constitué par l'hydrogène, alkyle, acyle, carboxyalkyle, carbonylamino, cycloalkyle, cycloalkylalkyle, aryle, arylalkyle, hétéroaryle, hétéroarylalkyle, hétérocyclyle et hétérocyclylalkyle ;
R^{c} est choisi parmi alkyle, cycloalkyle, aryle, hétérocyclyle et hétéroaryle ;
R^{d} et R^{e} sont indépendamment choisis dans l'ensemble constitué par l'hydrogène, un halogène, -OR⁷, halogénoalkyle, perhalogénoalkyle et alkyle ;
n vaut 0, 1, 2, 3 ou 4 ; et
p vaut 0, 1 ou 2.

4. Composé de formule (I) selon la revendication 1 ou un tautomère, polymorphe, stéréoisomère, solvate ou sel pharmaceutiquement acceptable d'un tel composé, dans lequel
--- représente une double liaison ;
X est choisi parmi O et S ;
Y₁ représente N ;
Y₂ représente NR⁵;
Y₃ représente N ;
Y₄ représente C ;
R¹ et R² sont indépendamment choisis parmi l'hydrogène et alkyle ;
R³ est -A-Z-B-Q ; où
A est absent ou est un alkylène, où un ou plusieurs groupes méthylène sont éventuellement remplacés par des hétéroatomes ou des groupes tels que -O- ou - N(R^{a})- ;
Z est absent ou est un hétérocyclyle ;
où l'hétérocyclyle est non substitué ou substitué indépendamment par 1, 2 ou 3 substituants indépendamment choisis parmi un halogène, alkyle, - (CR^{d}R^{e})ₙOR⁷, -(CR^{d}R^{e})ₙCOOR⁷, halogénoalkyle, perhalogénoalkyle, cyano, céto ou thiocarbonyle ;
B est absent ou est un alkylène, où un ou plusieurs groupes méthylène sont éventuellement remplacés par des hétéroatomes ou des groupes tels que -O-, -N(R^{a})-ou -C(O) ;
Q est choisi parmi l'hydrogène, alkyle, cycloalkyle, aryle, hétérocyclyle et hétéroaryle ;
où les alkyle, cycloalkyle, aryle, hétérocyclyle et hétéroaryle sont non substitués ou substitués indépendamment par 1, 2 ou 3 substituants indépendamment choisis parmi un halogène, alkyle, alcoxy, alcoxyalkyle, halogénoalkyle, perhalogénoalkyle, cyano, céto, thiocarbonyle, cyanoalkyle, (CR^{d}R^{e})ₙOR⁷, -(CR^{d}R^{e})ₙC(O)R⁷, -(CR^{d}R^{e})ₙCOOR⁷, -(CR^{d}R^{e})ₙNR⁸R⁹, -(CR^{d}R^{e})ₙC(O)NR⁸R⁹, -(CR^{d}R^{e})ₙNR⁸C(O)OR⁷, -S(O)ₚR^{c}, -SO₃H,-S(O)₂NR^{a}R^{a}, cycloalkyle, cycloalcényle, aryle, hétérocyclyle et hétéroaryle ;
où chaque substituant est non substitué ou substitué par 1, 2 ou 3 substituants indépendamment choisis parmi un halogène, alkyle, carboxy, carboxyalkyle, aminocarbonyle, hydroxy, alcoxy, halogénoalkyle, perhalogénoalkyle, halogénoalcoxy, perhalogénoalcoxy, amino, amino substitué, cyano et -S(O)ₚR^{c} ;
R⁴ est choisi dans l'ensemble constitué par l'hydrogène, alkyle, halogénoalkyle, hydroxyalkyle, cycloalkyle, aryle, hétérocyclyle et hétéroaryle ;
où les alkyle, cycloalkyle, aryle, hétéroaryle et hétérocyclyle sont non substitués ou substitués indépendamment par jusqu'à quatre substituants indépendamment choisis parmi un halogène, alkyle, -(CR^{d}R^{e})ₙOR⁷, -(CR^{d}R^{e})ₙCOOR⁷, -(CR^{d}R^{e})ₙNR⁸R⁹, cyano, halogénoalkyle, perhalogénoalkyle, nitro, -S(O)₂NR^{b}R^{b}, -NR^{b}S(O)₂R^{b} et -S(O)ₚR^{c}, thiocarbonyle, -SO₃H, cycloalkyle, aryle, hétéroaryle et hétérocyclyle ;
R⁵ est choisi dans l'ensemble constitué par l'hydrogène, hydroxy, halogénoalkyle, - (CR^{d}R^{e})ₙOR⁷, -(CR^{d}R^{e})ₙCOOR⁷, alcoxyalcoxyalkyle, alkyle, alcényle, alcynyle, cycloalkyle, cycloalkylalkyle, aryle, arylalkyle, hétérocyclyle, hétérocyclylalkyle, hétéroaryle et hétéroarylalkyle ;
R⁷ est choisi parmi l'hydrogène, un halogène, halogénoalkyle, cycloalkyle, aryle, hétéroaryle et hétérocyclyle ;
R⁸ et R⁹ sont indépendamment choisis dans l'ensemble constitué par l'hydrogène, alkyle, halogénoalkyle, -(CR^{d}R^{e})ₙOR⁷, -(CR^{d}R^{e})ₙC(O)R⁷, aryle, arylalkyle, hétéroaryle, hétéroarylalkyle, cycloalkyle, cycloalkylalkyle, hétérocyclyle et hétérocyclylalkyle,
ou bien R⁸ et R⁹, pris ensemble, forment un système cyclique monocyclique ou bicyclique qui est saturé ou partiellement insaturé et a éventuellement des hétéroatomes additionnels choisis parmi O, N et S, ledit système cyclique étant en outre éventuellement substitué par 1 à 4 substituants indépendamment choisis parmi halogéno, alkyle, nitro, cyano, -(CR^{d}R^{e})ₙOR⁷, -(CR^{d}R^{e})ₙNR⁸R⁹, oxo, alkylsulfonyle,-(CR^{d}R^{e})ₙCOOR⁷ et -(CR^{d}R^{e})ₙC(O)NR⁸R⁹ ;
R^{a} est choisi parmi l'hydrogène et alkyle ;
chaque R^{b} est indépendamment choisi dans l'ensemble constitué par l'hydrogène, alkyle, acyle, carboxyalkyle, carbonylamino, cycloalkyle, cycloalkylalkyle, aryle, arylalkyle, hétéroaryle, hétéroarylalkyle, hétérocyclyle et hétérocyclylalkyle ;
R^{c} est choisi parmi alkyle, cycloalkyle, aryle, hétérocyclyle et hétéroaryle ;
R^{d} et R^{e} sont indépendamment choisis dans l'ensemble constitué par l'hydrogène, un halogène, -OR⁷, halogénoalkyle, perhalogénoalkyle et alkyle ;
n vaut 0, 1, 2, 3 ou 4 ; et
p vaut 0, 1 ou 2.

5. Composé de formule (I) selon la revendication 1 ou un tautomère, polymorphe, stéréoisomère, solvate ou sel pharmaceutiquement acceptable d'un tel composé, dans lequel
--- représente une double liaison ;
X est choisi parmi O et S ;
Y₁ représente N ;
Y₂ représente NR⁵;
Y₃ représente N ;
Y₄ représente C ;
R¹ et R² sont indépendamment choisis parmi l'hydrogène et alkyle ;
R³ est -A-Z-B-Q ; où
A est absent ou est un alkylène, où un ou plusieurs groupes méthylène sont éventuellement remplacés par des hétéroatomes ou des groupes tels que -O- ou-N(R^{a})- ;
Z est absent ou est un hétérocyclyle choisi parmi dihydrofuranyle, tétrahydrofuranyle, morpholinyle, pyrrolidinyle, dihydropyrrole, dihydropyranyle, tétrahydropyranyle, pyrazolidinyle, imidazolidinyle, dihydropyridinyle, tétrahydropyridinyle, pipéridinyle, dihydropyrazinyle, tétrahydropyrazinyle, pipérazinyle et dihydropyridinyle ;
où l'hétérocyclyle est non substitué ou substitué indépendamment par 1, 2 ou 3 substituants indépendamment choisis parmi un halogène, alkyle,-(CR^{d}R^{e})ₙOR⁷, -(CR^{d}R^{e})ₙCOOR⁷, halogénoalkyle, perhalogénoalkyle ou cyano ;
B est absent ou est un alkylène, où un ou plusieurs groupes méthylène sont éventuellement remplacés par des hétéroatomes ou des groupes tels que -O-, -N(R^{a})-ou -C(O) ;
Q est choisi parmi l'hydrogène, alkyle, cyclopropyle, cyclopentyle, cyclohexyle, phényle, tétrahydrofuranyle, pyrrolidinyle, tétrahydropyridinyle, tétrahydropyranyle, pipérazinyle, benzodiazolyle, tétrahydroquinolinyle, morpholinyle, tétrahydronaphtyridinyle, tétrahydrothiénopyridinyle, furanyle, pyridinyle, pyrimidinyle, oxazolyle, thiazolyle, oxadiazolyle, thiadiazolyle, indolyle, quinolinyle, isoquinolinyle et benzoxazolyle ;
où Q est non substitué ou substitué par 1, 2 ou 3 substituants indépendamment choisis parmi un halogène, alkyle, alcoxy, alcoxyalkyle, halogénoalkyle, perhalogénoalkyle, cyano, céto, thiocarbonyle, cyanoalkyle, -(CR^{d}R^{e})ₙOR⁷, -(CR^{d}R^{e})ₙC(O)R⁷,-(CR^{d}R^{e})ₙCOOR⁷, -(CR^{d}R^{e})ₙNR⁸R⁹, -(CR^{d}R^{e})ₙC(O)NR⁸R⁹,-(CR^{d}R^{e})ₙNR⁸C(O)OR⁷, -S(O)ₚR^{c}, -SO₃H, -S(O)₂NR^{a}R^{a}, cycloalkyle, cycloalcényle, aryle, hétérocyclyle et hétéroaryle ;
où chaque substituant est non substitué ou substitué par 1, 2 ou 3 substituants indépendamment choisis parmi un halogène, alkyle, carboxy, carboxyalkyle, aminocarbonyle, hydroxy, alcoxy, halogénoalkyle, perhalogénoalkyle, halogénoalcoxy, perhalogénoalcoxy, amino, amino substitué, cyano et -S(O)ₚR^{c} ;
R⁴ est choisi dans l'ensemble constitué par l'hydrogène, alkyle, phényle, naphtyle, furanyle, thiazolyle, oxazolyle, thiadiazolyle, oxadiazolyle, pyrazinyle, pyridinyle et pyrimidinyle ;
où R⁴ est non substitué ou substitué par jusqu'à quatre substituants indépendamment choisis parmi un halogène, alkyle, -(CR^{d}R^{e})ₙOR⁷, (CR^{d}R^{e})ₙCOOR⁷, -(CR^{d}R^{e})ₙNR⁸R⁹, cyano, halogénoalkyle, perhalogénoalkyle et cycloalkyle ;
R⁵ est choisi dans l'ensemble constitué par l'hydrogène, hydroxy, halogénoalkyle, - (CR^{d}R^{e})ₙOR⁷, -(CR^{d}R^{e})ₙCOOR⁷, alcoxyalcoxyalkyle, alkyle, alcényle, alcynyle, cycloalkyle, cycloalkylalkyle, aryle, arylalkyle, hétérocyclyle, hétérocyclylalkyle, hétéroaryle et hétéroarylalkyle ;
R⁷ est choisi parmi l'hydrogène, un halogène, alkyle, halogénoalkyle, cycloalkyle, aryle, hétéroaryle et hétérocyclyle;
R⁸ et R⁹ sont indépendamment choisis dans l'ensemble constitué par l'hydrogène, alkyle, halogénoalkyle, -(CR^{d}R^{e})ₙOR⁷, -(CR^{d}R^{e})ₙC(O)R⁷, aryle, arylalkyle, hétéroaryle, hétéroarylalkyle, cycloalkyle, cycloalkylalkyle, hétérocyclyle et hétérocyclylalkyle,
ou bien R⁸ et R⁹, pris ensemble, forment un système cyclique monocyclique ou bicyclique qui est saturé ou partiellement insaturé et a éventuellement des hétéroatomes additionnels choisis parmi O, N et S, ledit système cyclique étant en outre éventuellement substitué par 1 à 4 substituants indépendamment choisis parmi halogéno, alkyle, nitro, cyano, -(CR^{d}R^{e})ₙOR⁷, -(CR^{d}R^{e})ₙNR⁸R⁹, oxo, alkylsulfonyle,-(CR^{d}R^{e})ₙCOOR⁷ et -(CR^{d}R^{e})ₙC(O)NR⁸R⁹ ;
R^{a} est choisi parmi l'hydrogène et alkyle ;
R^{b}, à chaque occurrence, est indépendamment choisi dans l'ensemble constitué par l'hydrogène, alkyle, acyle, carboxyalkyle, carbonylamino, cycloalkyle, cycloalkylalkyle, aryle, arylalkyle, hétéroaryle, hétéroarylalkyle, hétérocyclyle et hétérocyclylalkyle ;
R^{c} est choisi parmi alkyle, cycloalkyle, aryle, hétérocyclyle et hétéroaryle ;
R^{d} et R^{e} sont indépendamment choisis dans l'ensemble constitué par l'hydrogène, un halogène, -OR⁷, halogénoalkyle, perhalogénoalkyle et alkyle ;
n vaut 0, 1, 2, 3 ou 4 ; et
p vaut 0, 1 ou 2.

6. Composé de formule (I) selon la revendication 1 ou un tautomère, polymorphe, stéréoisomère, solvate ou sel pharmaceutiquement acceptable d'un tel composé, qui est l'un des suivants :
5-amino-8-(2-furyl)-3-[2-[4-[4-(2-méthoxyéthoxy)phényl]pipérazin-1-yl]éthyl]-1-méthyl-[1,2,4]triazolo[5,1-f]purin-2-one,
5-amino-8-(2-furyl)-3-(2-hydroxyéthyl)-1-méthyl-[1,2,4]triazolo[5,1-f]purin-2-one,
5-amino-3-[2-[4-(2,4-difluorophényl)pipérazin-1-yl]éthyl]-8-(2-furyl)-1-méthyl-[1,2,4]triazolo[5,1-f]purin-2-one,
5-amino-8-(2-furyl)-3-[2-[4-(4-méthoxyphényl)pipérazin-1-yl]éthyl]-1-méthyl-[1,2,4]triazolo[5,1-f]purin-2-one,
5-amino-8-(2-furyl)-1-méthyl-3-(2-morpholinoéthyl)-[1,2,4]triazolo[5,1-f]purin-2-one,
5-amino-3-[2-[4-(2,4-difluorophényl)-1-pipéridyl]éthyl]-8-(2-furyl)-1-méthyl-[1,2,4]triazolo[5,1-f]purin-2-one,
5-amino-8-(2-furyl)-1-méthyl-3-[2-[4-(5-méthyl-2-pyridyl)pipérazin-1-yl]éthyl]-[1,2,4]triazolo[5,1-f]purin-2-one,
5-amino-8-(2-furyl)-1-méthyl-3-[2-[4-(p-tolyl)pipérazin-1-yl]éthyl]-[1,2,4]triazolo[5,1-f]purin-2-one,
5-amino-8-(2-furyl)-1-méthyl-3-[2-[4-(3-méthyl-2-oxobutyl)pipérazin-1-yl]éthyl]-[1,2,4]triazolo[5,1-f]purin-2-one,
5-amino-3-[2-[4-(2-fluoro-4-méthoxyphényl)pipérazin-1-yl]éthyl]-8-(2-furyl)-1-méthyl-[1,2,4]triazolo[5,1-f]purin-2-one,
5-amino-8-(2-furyl)-3-[2-[4-[4-(2-méthoxy-1,1-diméthyléthoxy)phényl]pipérazin-1-yl]éthyl]-1-méthyl-[1,2,4]triazolo[5,1-f]purin-2-one,
5-amino-8-(2-furyl)-3-[2-[4-(6-méthoxy-3-pyridyl)pipérazin-1-yl]éthyl]-1-méthyl-[1,2,4]triazolo[5,1-f]purin-2-one,
5-amino-3-[2-[4-[3-fluoro-4-(2-méthoxyéthoxy)phényl]pipérazin-1-yl]éthyl]-8-(2-furyl)-1-méthyl-[1,2,4]triazolo[5,1-f]purin-2-one,
5-amino-8-(2-furyl)-3-[2-[4-[4-(1-hydroxy-1-méthyléthyl)phényl]pipérazin-1-yl]éthyl]-1-méthyl-[1,2,4]triazolo[5,1-f]purin-2-one,
5-amino-3-[2-[4-(4-fluorophényl)-4-hydroxy-1-pipéridyl]éthyl]-8-(2-furyl)-1-méthyl-[1,2,4]triazolo[5,1-f]purin-2-one,
5-amino-8-(2-furyl)-3-[2-[4-[4-(2-méthoxy-2-méthylpropoxy)phényl]pipérazin-1-yl] éthyl]-1-méthyl-[1,2,4]triazolo[5,1-f]purin-2-one,
5-amino-3-[2-[4-[4-(cyclopropoxy)phényl]pipérazin-1-yl]éthyl]-8-(2-furyl)-1-méthyl-[1,2,4]triazolo[5,1-f]purin-2-one,
5-amino-3-[2-[4-(4-fluorophényl)-3,6-dihydro-2H-pyridin-1-yl]éthyl]-8-(2-furyl)-1-méthyl-[1,2,4]triazolo[5,1-f]purin-2-one,
5-amino-8-(2-furyl)-3-[2-[4-hydroxy-4-(4-méthoxyphényl)-1-pipéridyl]éthyl]-1-méthyl-[1,2,4]triazolo[5,1-f]purin-2-one,
5-amino-3-[2-[4-[3,5-difluoro-4-(2-méthoxyéthoxy)phényl]pipérazin-1-yl]éthyl]-8-(2-furyl)-1-méthyl-[1,2,4]triazolo[5,1-f]purin-2-one,
5-amino-3-[2-[4-[2,5-difluoro-4-(2-méthoxyéthoxy)phényl]pipérazin-1-yl]éthyl]-8-(2-furyl)-1-méthyl-[1,2,4]triazolo[5,1-f]purin-2-one,
5-amino-3-[2-[4-(2,2-difluoro-1,3-benzodioxol-5-yl)pipérazin-1-yl]éthyl]-8-(2-furyl)-1-méthyl-[1,2,4]triazolo[5,1-f]purin-2-one,
5-amino-8-(2-furyl)-3-[2-[4-[4-(2-méthoxyéthoxy)phényl]-3,3-diméthylpipérazin-1-yl]éthyl]-1-méthyl-[1,2,4]triazolo[5,1-f]purin-2-one,
5-amino-3-[2-(4-butylpipérazin-1-yl)éthyl]-8-(2-furyl)-1-méthyl-[1,2,4]triazolo[5,1-f]purin-2-one,
5-amino-8-(2-furyl)-3-[2-(4-hydroxy-4-méthyl-1-pipéridyl)éthyl]-1-méthyl-[1,2,4]triazolo[5,1-f]purin-2-one,
5-amino-3-[2-[4-[4-[2-(cyclopropoxy)éthoxy]phényl]pipérazin-1-yl]éthyl]-8-(2-furyl)-1-méthyl-[1,2,4]triazolo[5,1-f]purin-2-one,
5-amino-8-(2-furyl)-3-[2-[4-[(4-méthoxyphényl)méthyl]pipérazin-1-yl]éthyl]-1-méthyl-[1,2,4]triazolo[5,1-f]purin-2-one,
5-amino-8-(2-furyl)-3-[2-[4-[[4-(2-méthoxyéthoxy)phényl]méthyl]pipérazin-1-yl]éthyl]-1-méthyl-[1,2,4]triazolo[5,1-f]purin-2-one,
5-amino-8-(2-furyl)-3-[(4-méthoxyphényl)méthyl]-1-méthyl-[1,2,4]triazolo[5,1-f]purin-2-one,
5-amino-8-(2-furyl)-3-[2-[4-(4-méthoxyphényl)pipérazin-1-yl]éthyl]-1-méthyl-[1,2,4]triazolo[5,1-f]purin-2-one,
5-amino-8-(2-furyl)-3-[2-[4-[3-(2-méthoxyéthoxy)phényl]pipérazin-1-yl]éthyl]-1-méthyl-[1,2,4]triazolo[5,1-f]purin-2-one,
5-amino-3-[2-[4-[2-fluoro-4-(2-méthoxyéthoxy)phényl]pipérazin-1-yl]éthyl]-8-(2-furyl)-1-méthyl-[1,2,4]triazolo[5,1-f]purin-2-one,
4-[4-[2-[5-amino-8-(2-furyl)-1-méthyl-2-oxo-[1,2,4]triazolo[5,1-f]purin-3-yl]éthyl]pipérazin-1-yl]benzonitrile,
4-[4-[2-[5-amino-8-(2-furyl)-1-méthyl-2-oxo-[1,2,4]triazolo[5,1-f]purin-3-yl]éthyl]pipérazin-1-yl]-2-fluorobenzonitrile,
5-amino-8-(2-furyl)-1-méthyl-3-[2-[4-[4-(trifluorométhyl)phényl]pipérazin-1-yl]éthyl]-[1,2,4]triazolo[5,1-f]purin-2-one,
5-amino-8-(2-furyl)-1-méthyl-3-[2-[4-[4-(trifluorométhyl)thiazol-2-yl]pipérazin-1-yl]éthyl]-[1,2,4]triazolo[5,1-f]purin-2-one,
5-amino-3-[2-[4-(cyclopropylméthyl)pipérazin-1-yl]éthyl]-8-(2-furyl)-1-méthyl-[1,2,4]triazolo[5,1-f]purin-2-one,
5-amino-3-[2-(4-éthylpipérazin-1-yl)éthyl]-8-(2-furyl)-1-méthyl-[1,2,4]triazolo[5,1-f]purin-2-one,
4-[2-[5-amino-8-(2-furyl)-1-méthyl-2-oxo-[1,2,4]triazolo[5,1-f]purin-3-yl]éthyl]-N,N-diméthylpipérazine-1-sulfonamide,
5-amino-8-(2-furyl)-1-méthyl-3-[2-[4-(4-tétrahydrofuran-3-yloxyphényl)pipérazin-1-yl]éthyl]-[1,2,4]triazolo[5,1-f]purin-2-one,
5-amino-8-(2-furyl)-1-méthyl-3-[2-[4-(4-tétrahydropyran-4-yloxyphényl)pipérazin-1-yl]éthyl]-[1,2,4]triazolo[5,1-f]purin-2-one,
5-amino-8-(2-furyl)-1-méthyl-3-[2-[4-[4-(tétrahydrofuran-2-ylméthoxy)phényl]pipérazin-1-yl]éthyl]-[1,2,4]triazolo[5,1-f]purin-2-one,
5-amino-8-(2-furyl)-1-méthyl-3-[2-(3-méthyl-7,8-dihydro-5H-1,6-naphtyridin-6-yl)éthyl]-[1,2,4]triazolo[5,1-f]purin-2-one,
5-amino-3-[2-(6,7-dihydro-4H-thiéno[3,2-c]pyridin-5-yl)éthyl]-8-(2-furyl)-1-méthyl-[1,2,4]triazolo[5,1-f]purin-2-one,
5-amino-8-(2-furyl)-3-[2-[4-[4-(2-méthoxyéthoxy)phényl]pipérazin-1-yl]propyl]-1-méthyl-[1,2,4]triazolo[5,1-f]purin-2-one,
5-amino-3-[2-[3-(4-fluorophényl)-2,5-dihydropyrrol-1-yl]éthyl]-8-(2-furyl)-1-méthyl-[1,2,4]triazolo[5,1-f]purin-2-one,
5-amino-3-[2-[4-[4-(2-méthoxyéthoxy)phényl]pipérazin-1-yl]éthyl]-1-méthyl-8-(5-méthyl-2-furyl)-[1,2,4]triazolo[5,1-f]purin-2-one,
5-amino-8-(5-cyclopropyl-2-furyl)-3-[2-[4-[4-(2-méthoxyéthoxy)phényl]pipérazin-1-yl]éthyl]-1-méthyl-[1,2,4]triazolo[5,1-f]purin-2-one,
5-amino-3-[2-(2,4-difluoroanilino)éthyl]-8-(2-furyl)-1-méthyl-[1,2,4]triazolo[5,1-f]purin-2-one,
5-amino-3-[3-[4-(4-fluorophényl)pipérazin-1-yl]propyl]-8-(2-furyl)-1-méthyl-[1,2,4]triazolo[5,1-f]purin-2-one,
5-amino-8-(2-furyl)-3-[3-[4-[4-(2-méthoxyéthoxy)phényl]pipérazin-1-yl]propyl]-1-méthyl-[1,2,4]triazolo[5,1-f]purin-2-one,
5-amino-8-(2-furyl)-3-[2-[4-(4-méthoxyphényl)-3,6-dihydro-2H-pyridin-1-yl]éthyl]-1-méthyl-[1,2,4]triazolo[5,1-f]purin-2-one,
5-amino-8-(2-furyl)-3-[2-[4-[4-(2-méthoxy-1,1-diméthyléthyl)phényl]pipérazin-1-yl]éthyl]-1-méthyl-[1,2,4]triazolo[5,1-f]purin-2-one,
5-amino-8-(2-furyl)-1-méthyl-3-(2-pipérazin-1-yléthyl)-[1,2,4]triazolo[5,1-f]purin-2-one,
5-amino-8-(2-furyl)-3-[2-[4-(1H-indole-2-carbonyl)pipérazin-1-yl]éthyl]-1-méthyl-[1,2,4]triazolo[5,1-f]purin-2-one,
5-amino-8-(2-furyl)-3-[2-(4-isopropoxyphényl)éthyl]-1-méthyl-[1,2,4]triazolo[5,1-f]purin-2-one,
5-amino-8-(2-furyl)-1-méthyl-3-[2-[4-[(2S)-pyrrolidine-2-carbonyl]pipérazin-1-yl]éthyl]-[1,2,4]triazolo[5,1-f]purin-2-one,
5-amino-8-(2-furyl)-3-[2-(4-méthoxyphényl)éthyl]-1-méthyl-[1,2,4]triazolo[5,1-f]purin-2-one,
5-amino-3-[2-[4-[4-(difluorométhoxy)phényl]pipérazin-1-yl]éthyl]-8-(2-furyl)-1-méthyl-[1,2,4]triazolo[5,1-f]purin-2-one,
5-amino-8-(2-furyl)-1-méthyl-3-[2-[4-[3-(5-méthyl-1,3,4-oxadiazol-2-yl)phényl]pipérazin-1-yl]éthyl]-[1,2,4]triazolo[5,1-f]purin-2-one,
5-amino-3-[2-[4-[2-fluoro-4-(5-méthyl-1,2,4-oxadiazol-3-yl)phényl]pipérazin-1-yl]éthyl]-8-(2-furyl)-1-méthyl-[1,2,4]triazolo[5,1-f]purin-2-one,
5-amino-3-[2-[4-(6-fluoro-2-méthyl-1,3-benzoxazol-5-yl)pipérazin-1-yl]éthyl]-8-(2-furyl)-1-méthyl-[1,2,4]triazolo[5,1-f]purin-2-one,
5-amino-3-[2-[4-(cyclopropanecarbonyl)pipérazin-1-yl]éthyl]-8-(2-furyl)-1-méthyl-[1,2,4]triazolo[5,1-f]purin-2-one,
5-amino-3-[2-[4-(2-cyclopropylacétyl)pipérazin-1-yl]éthyl]-8-(2-furyl)-1-méthyl-[1,2,4]triazolo[5,1-f]purin-2-one,
5-amino-8-(2-furyl)-3-[2-[4-[4-(2-hydroxyéthoxy)phényl]pipérazin-1-yl]éthyl]-1-méthyl-[1,2,4]triazolo[5,1-f]purin-2-one,
5-amino-8-(2-furyl)-3-[2-[4-(4-hydroxyphényl)pipérazin-1-yl]éthyl]-1-méthyl-[1,2,4]triazolo[5,1-f]purin-2-one,
5-amino-1-(cyclopropylméthyl)-3-[2-[4-(4-éthoxyphényl)pipérazin-1-yl]éthyl]-8-(2-furyl)-[1,2,4]triazolo[5,1-f]purin-2-one,
5-amino-1-(cyclopropylméthyl)-3-[2-[4-(4-fluorophényl)pipérazin-1-yl]éthyl]-8-(2-furyl)-[1,2,4]triazolo[5,1-f]purin-2-one,
5-amino-1-(cyclopropylméthyl)-3-[2-[4-(2,4-difluorophényl)pipérazin-1-yl]éthyl]-8-(2-furyl)-[1,2,4]triazolo[5,1-f]purin-2-one,
5-amino-1-(cyclopropylméthyl)-8-(2-furyl)-3-[2-[4-[4-(2-méthoxyéthoxy)phényl]pipérazin-1-yl]éthyl]-[1,2,4]triazolo[5,1-f]purin-2-one,
5-amino-1-(cyclopropylméthyl)-3-[2-(4-fluorophénoxy)éthyl]-8-(2-furyl)-[1,2,4]triazolo[5,1-f]purin-2-one,
5-amino-8-(2-furyl)-1-méthyl-3-[2-[2-oxo-5-(trifluorométhyl)-1-pyridyl]éthyl]-[1,2,4]triazolo[5,1-f]purin-2-one,
5-amino-3-[2-[4-(2,4-difluorophényl)pyrazol-1-yl]éthyl]-1-éthyl-8-(2-furyl)-[1,2,4]triazolo[5,1-f]purin-2-one,
acide 1-[2-[5-amino-1-(cyclopropylméthyl)-8-(2-furyl)-2-oxo-[1,2,4]triazolo[5,1-f]purin-3-yl]éthyl]pyrazole-4-carboxylique,
acide 1-[2-[5-amino-8-(2-furyl)-1-méthyl-2-oxo-[1,2,4]triazolo[5,1-f]purin-3-yl]éthyl]pyrazole-4-carboxylique,
1-[2-[5-amino-1-(cyclopropylméthyl)-8-(2-furyl)-2-oxo-[1,2,4]triazolo[5,1-f]purin-3-yl]éthyl]-N-cyclopropyl-pyrazole-4-carboxamide,
1-[2-[5-amino-1-(cyclopropylméthyl)-8-(2-furyl)-2-oxo-[1,2,4]triazolo[5,1-f]purin-3-yl]éthyl]-N,N-diéthylpyrazole-4-carboxamide,
1-[2-[5-amino-1-(cyclopropylméthyl)-8-(2-furyl)-2-oxo-[1,2,4]triazolo[5,1-f]purin-3-yl]éthyl]-N-cyclopropyl-5-méthylpyrazole-3-carboxamide,
2-[2-[5-amino-1-(cyclopropylméthyl)-8-(2-furyl)-2-oxo-[1,2,4]triazolo[5,1-f]purin-3-yl]éthyl]-N-cyclopropyl-5-méthylpyrazole-3-carboxamide,
1-[2-[5-amino-8-(2-furyl)-1-méthyl-2-oxo-[1,2,4]triazolo[5,1-f]purin-3-yl]éthyl]-N-méthylpyrazole-3-carboxamide,
1-[2-[5-amino-8-(2-furyl)-1-méthyl-2-oxo-[1,2,4]triazolo[5,1-f]purin-3-yl]éthyl]-N,N-diéthylpyrazole-4-carboxamide,
1-[2-[5-amino-8-(2-furyl)-1-méthyl-2-oxo-[1,2,4]triazolo[5,1-f]purin-3-yl]éthyl]pyrazole-4-carboxamide,
5-amino-8-(2-furyl)-3-[2-[4-[(3R)-3-hydroxypyrrolidine-1-carbonyl]pyrazol-1-yl]éthyl]-1-méthyl-[1,2,4]triazolo[5,1-f]purin-2-one,
1-[2-[5-amino-8-(2-furyl)-1-méthyl-2-oxo-[1,2,4]triazolo[5,1-f]purin-3-yl]éthyl]-N-méthylpyrazole-4-carboxamide,
1-[2-[5-amino-8-(2-furyl)-1-méthyl-2-oxo-[1,2,4]triazolo[5,1-f]purin-3-yl]éthyl]-N-cyclopropylpyrazole-3-carboxamide,
1-[2-[5-amino-8-(2-furyl)-1-méthyl-2-oxo-[1,2,4]triazolo[5,1-f]purin-3-yl]éthyl]-N-cyclopropylpyrazole-4-carboxamide,
5-amino-8-(2-furyl)-3-[2-[4-(3-hydroxyazétidine-1-carbonyl)pyrazol-1-yl]éthyl]-1-méthyl-[1,2,4]triazolo[5,1-f]purin-2-one,
5-amino-1-éthyl-8-(2-furyl)-3-[2-[4-[4-(2-méthoxyéthoxy)phényl]pipérazin-1-yl]éthyl]-[1,2,4]triazolo[5,1-f]purin-2-one,
5-amino-3-[2-[4-(2,4-difluorophényl)pipérazin-1-yl]éthyl]-1-éthyl-8-(2-furyl)-[1,2,4]triazolo[5,1-f]purin-2-one,
5-amino-1-éthyl-3-{2-[4-(4-fluorophényl)pipéridin-1-yl]éthyl}-8-furan-2-yl-1,3-dihydro-[1,2,4]triazolo[5,1-f]purin-2-one,
5-amino-1-éthyl-8-(2-furyl)-3-[2-(3-méthyl-7,8-dihydro-5H-1,6-naphtyridin-6-yl)éthyl]-[1,2,4]triazolo[5,1-f]purin-2-one,
5-amino-8-(2-furyl)-3-[2-[4-[4-(2-méthoxyéthoxy)phényl]pipérazin-1-yl]éthyl]-1-(2,2,2-trifluoroéthyl)-[1,2,4]triazolo[5,1-f]purin-2-one,
5-amino-3-{2-[4-(2,4-difluorophényl)pipérazin-1-yl]éthyl}-8-furan-2-yl-1-(2,2,2-trifluoroéthyl)-1,3-dihydro-[1,2,4]triazolo[5,1-f]purin-2-one,
5-amino-8-(2-furyl)-3-[2-[4-[4-(2-méthoxyéthoxy)phényl]pipérazin-1-yl]éthyl]-1-(2-méthoxyéthyl)-[1,2,4]triazolo[5,1-f]purin-2-one,
5-amino-3-[2-[4-(4-fluorophényl)pipérazin-1-yl]éthyl]-8-(2-furyl)-1-(2-méthoxyéthyl)-[1,2,4]triazolo[5,1-f]purin-2-one,
5-amino-3-[2-[4-(4-fluorophényl)pipérazin-1-yl]éthyl]-8-(2-furyl)-1-(2-hydroxyéthyl)-[1,2,4]triazolo[5,1-f]purin-2-one,
5-amino-1-cyclopropyl-8-(2-furyl)-3-[2-[4-[4-(2-méthoxyéthoxy)phényl]pipérazin-1-yl]éthyl]-[1,2,4]triazolo[5,1-f]purin-2-one,
5-amino-8-(2-furyl)-3-[2-[4-[4-(2-méthoxyéthoxy)phényl]pipérazin-1-yl]éthyl]-1-(2,2,2-trifluoroéthyl)-[1,2,4]triazolo[5,1-f]purin-2-one,
5-amino-3-[2-[4-[4-(2-méthoxyéthoxy)phényl]pipérazin-1-yl]éthyl]-1-méthyl-8-thiazol-2-yl-[1,2,4]triazolo[5,1-f]purin-2-one,
5-amino-3-[2-[4-[3-fluoro-4-(2-méthoxyéthoxy)phényl]pipérazin-1-yl]éthyl]-1-méthyl-8-thiazol-2-yl-[1,2,4]triazolo[5,1-f]purin-2-one,
5-amino-3-[2-[4-(2-cyclopropylacétyl)pipérazin-1-yl]éthyl]-1-méthyl-8-thiazol-2-yl-[1,2,4]triazolo[5,1-f]purin-2-one,
5-amino-3-[2-[4-(4-méthoxyphényl)pipérazin-1-yl]éthyl]-1-méthyl-8-thiazol-2-yl-[1,2,4]triazolo[5,1-f]purin-2-one,
5-amino-1-méthyl-3-[2-[4-(p-tolyl)pipérazin-1-yl]éthyl]-8-thiazol-2-yl-[1,2,4]triazolo[5,1-f]purin-2-one,
5-amino-1-méthyl-3-[2-(3-méthyl-7,8-dihydro-5H-1,6-naphtyridin-6-yl)éthyl]-8-thiazol-2-yl-[1,2,4]triazolo[5,1-f]purin-2-one,
4-[4-[2-(5-amino-1-méthyl-2-oxo-8-thiazol-2-yl-[1,2,4]triazolo[5,1-f]purin-3-yl)éthyl]pipérazin-1-yl]benzonitrile,
5-amino-1-méthyl-3-[2-[4-[3-(5-méthyl-1,3,4-oxadiazol-2-yl)phényl]pipérazin-1-yl]éthyl]-8-thiazol-2-yl-[1,2,4]triazolo[5,1-f]purin-2-one,
5-amino-3-[2-[4-[4-(1-hydroxy-1-méthyléthyl)phényl]pipérazin-1-yl]éthyl]-1-méthyl-8-thiazol-2-yl-[1,2,4]triazolo[5,1-f]purin-2-one,
5-amino-3-[2-[4-[4-(2-méthoxyéthoxy)phényl]pipérazin-1-yl]éthyl]-1-méthyl-8-(2-pyridyl)-[1,2,4]triazolo[5,1-f]purin-2-one,
5-amino-3-[2-[4-(2,4-difluorophényl)pipérazin-1-yl]éthyl]-1-méthyl-8-(2-pyridyl)-[1,2,4]triazolo[5,1-f]purin-2-one,
4-[4-[2-[5-amino-1-méthyl-2-oxo-8-(2-pyridyl)-[1,2,4]triazolo[5,1-f]purin-3-yl]éthyl]pipérazin-1-yl]benzonitrile,
5-amino-3-[2-[4-[4-(1-hydroxy-1-méthyléthyl)phényl]pipérazin-1-yl]éthyl]-1-méthyl-8-(2-pyridyl)-[1,2,4]triazolo[5,1-f]purin-2-one,
5-amino-3-[2-[4-[4-(2-méthoxyéthoxy)phényl]pipérazin-1-yl]éthyl]-1-méthyl-8-pyrazin-2-yl-[1,2,4]triazolo[5,1-f]purin-2-one,
5-amino-3-[2-[4-(2,4-difluorophényl)pipérazin-1-yl]éthyl]-1-méthyl-8-pyrazin-2-yl-[1,2,4]triazolo[5,1-f]purin-2-one,
5-amino-3-[2-[4-[2-fluoro-4-(2-méthoxyéthoxy)phényl]pipérazin-1-yl]éthyl]-1-méthyl-8-pyrazin-2-yl-[1,2,4]triazolo[5,1-f]purin-2-one,
5-amino-8-(2-furyl)-3-[[1-(4-méthoxyphényl)pyrrolidin-3-yl]méthyl]-1-méthyl-[1,2,4]triazolo[5,1-f]purin-2-one,
5-amino-8-(2-furyl)-3-[[1-[4-(2-méthoxyéthoxy)phényl]pyrrolidin-3-yl]méthyl]-1-méthyl-[1,2,4]triazolo[[5,1-f]purin-2-one,
hyl}-1-méthyl-1,3-dihydro-[1,2,4]triazolo[5,1-i]purin-2-one,
5-amino-8-(2-furyl)-3-[2-[4-[4-(2-méthoxyéthoxy)phényl]pipérazin-1-yl]éthyl]-1-méthyl-[1,2,4]triazolo[5,1-f]purine-2-thione,
8-(2-furyl)-3-[2-[4-[4-(2-méthoxyéthoxy)phényl]pipérazin-1-yl]éthyl]-1-méthyl-5-(méthylamino)-[1,2,4]triazolo[5,1-f]purin-2-one,
5-amino-3-{2-[4-(4-fluorophényl)pipérazin-1-yl]éthyl}-8-isothiazol-5-yl-1-méthyl-1,3-dihydro-[1,2,4]triazolo[5,1-i]purin-2-one,
5-amino-8-isothiazol-5-yl-3-(2-{4-[4-(2-méthoxyéthoxy)phényl]pipérazin-1-yl}éthyl)-1-méthyl-1,3-dihydro-[1,2,4]triazolo[5,1-i]purin-2-one,
5-amino-3-[2-[4-[2-fluoro-4-(2-méthoxyéthoxy)phényl]pipérazin-1-yl]éthyl]-8-isothiazol-5-yl-1-méthyl-[1,2,4]triazolo[5,1-f]purin-2-one,
5-amino-8-isoxazol-5-yl-3-[2-[4-[4-(2-méthoxyéthoxy)phényl]pipérazin-1-yl]éthyl]-1-méthyl-[1,2,4]triazolo[5,1-f]purin-2-one,
5-amino-3-[2-[4-[4-(2-méthoxyéthoxy)phényl]pipérazin-1-yl]éthyl]-1-méthyl-8-oxazol-2-yl-[1,2,4]triazolo[5,1-f]purin-2-one,
5-amino-3-{2-[4-(4-méthoxyphényl)pipérazin-1-yl]éthyl}-1-méthyl-8-prop-1-ynyl-1,3-dihydro-[1,2,4]triazolo[5,1-i]purin-2-one,
5-amino-3-(2-{4-[4-(2-méthoxyéthoxy)phényl]pipérazin-1-yl}éthyl)-1-méthyl-8-prop-1-ynyl-1,3-dihydro-[1,2,4]triazolo[5,1-i]purin-2-one,
5-amino-3-{2-[4-(4-fluorobenzoyl)pipérazin-1-yl]éthyl}-8-furan-2-yl-1-méthyl-1,3-dihydro-[1,2,4]triazolo[5,1-i]purin-2-one,
5-amino-3-(2-diméthylaminoéthyl)-8-furan-2-yl-1-méthyl-1,3-dihydro-[1,2,4]triazolo[5,1-i]purin-2-one,
5-amino-8-furan-2-yl-3-[3-(4-méthoxyphényl)propyl]-1-méthyl-1,3-dihydro-[1,2,4]triazolo[5,1-i]purin-2-one,
5-amino-8-furan-2-yl-1-méthyl-3-(2-pyrazol-1-yléthyl)-1,3-dihydro-[1,2,4]triazolo[5,1-i]purin-2-one,
5-amino-8-furan-2-yl-3-(2- {4-[4-(2-méthoxyéthoxy)phényl]pyrazol-1-yl}éthyl)-1-méthyl-1,3-dihydro-[1,2,4]triazolo[5,1-i]purin-2-one,
5-amino-8-furan-2-yl-3-{2-[3-(4-méthoxyphényl)pyrrol-1-yl]-éthyl}-1-méthyl-1,3-dihydro-[1,2,4]triazolo[5,1-i]purin-2-one,
5-amino-8-furan-2-yl-3-{2-[4-(4-méthoxyphényl)imidazol-1-yl]éthyl}-1-méthyl-1,3-dihydro-[1,2,4]triazolo[5,1-i]purin-2-one,
5-amino-8-furan-2-yl-3-{2-[4-(4-méthoxyphényl)-[1,2,3]triazol-1-yl]éthyl}-1-méthyl-1,3-dihydro-[1,2,4]triazolo[5,1-i]purin-2-one,
5-amino-3-[2-(1,3-dihydroisoindol-2-yl)-éthyl]-8-furan-2-yl-1-méthyl-1,3-dihydro-[1,2,4]triazolo[5,1-i]purin-2-one,
5-amino-8-furan-2-yl-1-méthyl-3-(2-pipéridin-1-yléthyl)-1,3-dihydro-[1,2,4]triazolo[5,1-i]purin-2-one,
5-amino-8-furan-2-yl-1-méthyl-3-(2-pyrrolidin-1-yléthyl)-1,3-dihydro-[1,2,4]triazolo[5,1-i]purin-2-one,
5-amino-8-furan-2-yl-1-méthyl-3-[2-(3-méthyl-7,8-dihydro-5H-[1,6]naphtyridin-6-yl)éthyl]-1,3-dihydro-[1,2,4]triazolo[5,1-i]purin-2-one,
5-amino-8-furan-2-yl-3-{2-[4-(2-méthoxyéthoxy)phénoxy]éthyl}-1-méthyl-1,3-dihydro-[1,2,4]triazolo[5,1-i]purin-2-one,
5-amino-8-furan-2-yl-3-{2-[4-(2-méthoxyéthoxy)phénylamino]éthyl}-1-méthyl-1,3-dihydro-[1,2,4]triazolo[5,1-i]purin-2-one,
5-amino-8-furan-2-yl-1-méthyl-3-[2-(pyridin-2-yloxy)éthyl]-1,3-dihydro-[1,2,4]triazolo[5,1-i]purin-2-one,
5-amino-1-éthyl-3-{2-[4-(4-fluorophényl)pipérazin-1-yl]éthyl}-8-isothiazol-5-yl-1,3-dihydro-[1,2,4]triazolo[5,1-i]purin-2-one,
5-amino-1-éthyl-8-isothiazol-5-yl-3-(2-{4-[4-(2-méthoxyéthoxy)phényl]-pipérazin-1-yl}éthyl)-1,3-dihydro-[1,2,4]triazolo[5,1-i]purin-2-one,
5-amino-1-éthyl-8-furan-2-yl-3-(2-pipéridin-1-yléthyl)-1,3-dihydro-[1,2,4]triazolo[5,1-i]purin-2-one,
5-amino-1-éthyl-8-furan-2-yl-3-[2-(3-méthyl-7,8-dihydro-5H-[1,6]naphtyridin-6-yl)éthyl]-1,3-dihydro-[1,2,4]triazolo[5,1-i]purin-2-one,
5-amino-3-[2-(2,4-difluorophénoxy)éthyl]-1-éthyl-8-furan-2-yl-1,3-dihydro-[1,2,4]triazolo[5,1-i]purin-2-one,
5-amino-3-[2-(2,4-difluorophénylamino)éthyl]-1-éthyl-8-furan-2-yl-1,3-dihydro-[1,2,4]triazolo[5,1-i]purin-2-one,
5-amino-1-cyclopropylméthyl-3-[2-(2,4-difluorophénylamino)éthyl]-8-furan-2-yl-1,3-dihydro-[1,2,4]triazolo[5,1-i]purin-2-one,
5-amino-1-cyclopropylméthyl-3-[2-(2,4-difluorophénoxy)éthyl]-8-furan-2-yl-1,3-dihydro-[1,2,4]triazolo[5,1-i]purin-2-one,
5-amino-1-cyclopropylméthyl-3-{2-[4-(4-fluorophényl)pipéridin-1-yl]éthyl}-8-furan-2-yl-1,3-dihydro-[1,2,4]triazolo[5,1-i]purin-2-one,
5-amino-3-{2-[4-(4-fluorophényl)pipéridin-1-yl]éthyl}-8-furan-2-yl-1-(2,2,2-trifluoroéthyl)-1,3-dihydro-[1,2,4]triazolo[5,1-i]purin-2-one,
5-amino-8-furan-2-yl-3-{2-[4-(4-méthoxyphényl)pipérazin-1-yl]éthyl}-1-(2,2,2-trifluoroéthyl)-1,3-dihydro-[1,2,4]triazolo[5,1-i]purin-2-one,
5-amino-3-[2-(4-cyclopropylméthylpipérazin-1-yl)éthyl]-8-isothiazol-5-yl-1-(2,2,2-trifluoroéthyl)-1,3-dihydro-[1,2,4]triazolo[5,1-i]purin-2-one,
(5-amino-8-isothiazol-5-yl-3-{2-[4-(4-méthoxyphényl)pipérazin-1-yl]éthyl}-2-oxo-2,3-dihydro-[1,2,4]triazolo[5,1-i]purin-1-yl)acétonitrile,
[5-amino-3-{2-[4-(2,4-difluorophényl)pipérazin-1-yl]éthyl}-8-(3-fluorophényl)-2-oxo-2,3-dihydro-[1,2,4]triazolo[5,1-i]purin-1-yl]acétonitrile,
[5-amino-8-furan-2-yl-3-(2-{4-[4-(2-méthoxyéthoxy)phényl]pipérazin-1-yl}éthyl)-2-oxo-2,3-dihydro-[1,2,4]triazolo[5,1-i]purin-1-yl]-acétonitrile,
5-amino-3-(2-{4-[4-(2-méthoxyéthoxy)phényl]pipérazin-1-yl}éthyl)-1-méthyl-8-phényl-1,3-dihydro-[1,2,4]triazolo[5,1-i]purin-2-one,
3-[5-amino-3-(2-{4-[4-(2-méthoxyéthoxy)phényl]pipérazin-1-yl}éthyl)-1-méthyl-2-oxo-2,3-dihydro-1H-[1,2,4]triazolo[5,1-i]purin-8-yl]benzonitrile,
3-[5-amino-3-(2-{4-[4-(2-méthoxyéthoxy)phényl]pipérazin-1-yl}éthyl)-1-méthyl-2-oxo-2,3-dihydro-1H-[1,2,4]triazolo[5,1-i]purin-8-yl]benzonitrile,
5-amino-8-furan-2-yl-1-méthyl-3-vinyl-1,3-dihydro-[1,2,4]triazolo[5,1-i]purin-2-one,
5-amino-3-[3-(4-fluorophényl)prop-2-ynyl]-8-furan-2-yl-1-méthyl-1,3-dihydro-[1,2,4]triazolo[5,1-i]purin-2-one,
5-amino-8-furan-2-yl-1-méthyl-3-[4-(4-méthylpipérazin-1-yl)but-2-ynyl]-1,3-dihydro-[1,2,4]triazolo[5,1-i]purin-2-one,
5-amino-8-furan-2-yl-1-isopropyl-3-(2-{4-[4-(2-méthoxyéthoxy)phényl]pipérazin-1-yl}-éthyl)1,3-dihydro-[1,2,4]triazolo[5,1-i]purin-2-one,
5-amino-2-benzyl-7-(2- {4-[4-(2-méthoxyéthoxy)phényl]pipérazin-1-yl}éthyl)-9-méthyl-7,9-dihydro-2H-[1,2,4]triazolo[3,4-i]purine-3,8-dione,
5-amino-2-benzyl-9-méthyl-7-(2-morpholin-4-yléthyl)-7,9-dihydro-2H-[1,2,4]triazolo[3,4-i]purine-3,8-dione,
5-amino-2-(3-chlorobenzyl)-7-[2-(4-isopropylpipérazin-1-yl)éthyl]-9-méthyl-7,9-dihydro-2H-[1,2,4]triazolo[3,4-i]purine-3,8-dione,
5-amino-2-cyclopropylméthyl-9-méthyl-7-(2-morpholin-4-yléthyl)-7,9-dihydro-2H-[1,2,4]triazolo[3,4-i]purine-3,8-dione,
5-amino-2-cyclopropylméthyl-7-(2,4-difluorobenzyl)-9-méthyl-7,9-dihydro-2H-[1,2,4]triazolo[3,4-i]purine-3,8-dione,
4-amino-2-furan-2-yl-6-(2-{4-[4-(2-méthoxyéthoxy)phényl]pipérazin-1-yl}éthyl)-6H-8-oxa-1,3,3a,5,6-pentaaza-as-indacén-7-one, et
4-amino-2-furan-2-yl-6-(2-{4-[4-(2-méthoxyéthoxy)phényl]pipérazin-1-yl}éthyl)-8,8-diméthyl-6,8-dihydro-1,3,3a,5,6-pentaaza-as-indacén-7-one.

7. Composition pharmaceutique comprenant, à titre d'agent actif, au moins un composé de formule (I) selon l'une quelconque des revendications 1 à 6, ou un tautomère, polymorphe, stéréoisomère, solvate ou sel pharmaceutiquement acceptable d'un tel composé, conjointement avec un ou plusieurs véhicules ou excipients pharmaceutiquement acceptables.

8. Composé de formule (I) selon l'une quelconque des revendications 1 à 6, ou un tautomère, polymorphe, stéréoisomère, solvate ou sel pharmaceutiquement acceptable d'un tel composé, pour utilisation dans le traitement d'une maladie ou d'un trouble susceptible d'être amélioré par un antagonisme d'un récepteur A_{2A}.

9. Composé de formule (I) selon l'une quelconque des revendications 1 à 6, ou un tautomère, polymorphe, stéréoisomère, solvate ou sel pharmaceutiquement acceptable d'un tel composé, pour utilisation dans le traitement de la maladie de Parkinson, du syndrome des jambes sans repos, de la maladie d'Alzheimer, d'un trouble neurodégénératif, d'une inflammation, d'une cicatrisation, d'une fibrose dermique, d'un myoclonus nocturne, d'une ischémie cérébrale, d'une ischémie du myocarde, de la chorée de Huntington, d'une atrophie multisystémique, d'une dégénérescence cortico-basale, de la maladie de Wilson ou d'autres troubles des noyaux gris centraux ayant pour résultat des dyskinésies, d'un trouble de stress post-traumatique, d'une cirrhose hépatique, d'une sepsie, d'une lésion de la moelle épinière, d'une rétinopathie, de l'hypertension, d'une altération de la mémoire sociale, d'une dépression, d'une neuroprotection, d'une narcolepsie ou d'autres troubles du sommeil, du trouble déficitaire de l'attention avec hyperactivité, d'une pharmacodépendance, d'un trouble de stress post-traumatique, et d'une lésion vasculaire.

10. Composition pharmaceutique comprenant un composé de formule (I) selon l'une quelconque des revendications 1 à 6, ou un tautomère, polymorphe, stéréoisomère, solvate ou sel pharmaceutiquement acceptable d'un tel composé, en combinaison avec un ou plusieurs agents ayant une activité thérapeutique.

11. Composition pharmaceutique selon la revendication 10, dans laquelle l'agent ayant une activité thérapeutique est choisi parmi un agent anti-inflammatoire, un agent antidiabétique, un agent antihypertenseur et un agent antidyslipidémiant.

12. Composition pharmaceutique selon la revendication 10, dans laquelle l'agent ayant une activité thérapeutique est choisi parmi un agent anticholinergique, un agent antimuscarinique, un stéroïde, un antagoniste de LTB4 (leucotriène 4), les agonistes de récepteurs de la dopamine, un inhibiteur de phosphodiestérase 4, un agoniste de récepteur beta-2 adrénergique, l'insuline, les dérivés et imitateurs de l'insuline, les sécrétagogues d'insuline, les ligands de récepteurs de sulfonylurée insulinotropes, les dérivés de thiazolidone, un inhibiteur de glycogène synthase kinase-3, un inhibiteur de co-transporteur du glucose dépendant du sodium, un inhibiteur de glycogène phosphorylase A, un biguanide, un inhibiteur d'alpha-glucosidase, le peptide 1 analogue au glucagon (GLP-1), les analogues de GLP-1 et les imitateurs de GLP-1, les modulateurs des récepteurs activés par les proliférateurs de peroxisomes, un inhibiteur de dipeptidyle peptidase IV, un inhibiteur de stéaroyl-CoA désaturase-1, un inhibiteur de diacylglycérol acyltransférase 1 ou 2, un inhibiteur d'acétyl-CoA carboxylase 2, et les casseurs de produits finals de glycation avancés, les diurétiques de l'anse, un inhibiteur d'enzyme de conversion de l'angiotensine, un inhibiteur de pompe à Na-K-ATPase membranaire tel que la digoxine, un inhibiteur d'endopeptidase neutre (NEP), les inhibiteurs d'ACE/NEP, les antagonistes d'angiotensine II, les inhibiteurs de rénine, les bêtabloquants, les agents inotropes, les inhibiteurs calciques, les antagonistes de récepteurs d'aldostérone, et les inhibiteurs d'aldostérone synthase, un inhibiteur de 3-hydroxy-3-méthylglytaryle coenzyme A réductase, les composés augmentant les HDL tels qu'un inhibiteur de protéine de transfert d'ester de cholestérol, un inhibiteur de squalène synthase, un récepteur de farnésoïde X et un ligand de récepteur X du foie, la cholestyramine, les fibrates, l'acide nicotinique, et l'aspirine.

13. Composition pharmaceutique comprenant un composé de formule (I) selon l'une quelconque des revendications 1 à 6, ou un tautomère, polymorphe, stéréoisomère, solvate ou sel pharmaceutiquement acceptable d'un tel composé, en combinaison avec un à trois autres agents utiles dans le traitement de la maladie de Parkinson, dans un véhicule pharmaceutiquement acceptable.

14. Composition pharmaceutique selon la revendication 13, dans laquelle l'autre agent est choisi parmi le L-DOPA, les agonistes dopaminergiques, les inhibiteurs de MAO-B, les inhibiteurs de DOPA décarboxylase, les inhibiteurs de COMT et les antagonistes de récepteurs de NMDA.
